# EUROPEAN PATENT SPECIFICATION

(11) **EP 4 382 527 B1**
(45) Date of publication and mention of the grant of the patent: **30.09.2026**
(21) Application number: 22852286.8
(22) Date of filing: 04.08.2022
(51) Int. Cl.: C07D 413/06, C07D 413/14, A61K 31/42, A61K 31/4439, A61K 31/506, A61P 31/04, C07D 417/14

(54) **AROMATIC ACETYLENE DERIVATIVE, AND PREPARATION METHOD THEREFOR AND USE THEREOF**
AROMATISCHES ACETYLENDERIVAT UND HERSTELLUNGSVERFAHREN DAFÜR UND VERWENDUNG DAVON
DÉRIVÉ D'ACÉTYLÈNE AROMATIQUE, SON PROCÉDÉ DE PRÉPARATION ET SON UTILISATION

(30) Priority: 05.08.2021 CN 202110893728; 22.03.2022 CN 202210285860
(43) Date of publication of application: 12.06.2024
(73) Proprietor: Zhejiang Hisun Pharmaceutical Co., Ltd., Taizhou, Zhejiang 318000 (CN); Shanghai Aryl Pharmtech Co., Ltd., Songjiang, Shanghai 201612 (CN)
(72) Inventor: GUO, Yanghui, Shanghai 201612 (CN); MENG, Lichen, Shanghai 201612 (CN); LIAO, Weiwei, Shanghai 201612 (CN); ZHANG, Heng, Shanghai 201612 (CN); WU, Chengfei, Shanghai 201612 (CN); CHEN, Youxi, Shanghai 201612 (CN); YE, Cheng, Shanghai 201612 (CN); QIAN, Wenjian, Zhejiang 318000 (CN); CHEN, Lei, Zhejiang 318000 (CN); WU, Enguo, Zhejiang 318000 (CN)
(74) Representative: Boult Wade Tennant LLP
(86) International application number: PCT/CN2022/110173
(87) International publication number: WO 2023/011574

(56) References cited:
- WO-A1-2010/081145
- WO-A1-2010/100475
- WO-A1-2012/162635
- WO-A1-2015/085238
- WO-A1-2018/216822
- WO-A1-2020/061375
- WO-A1-2020/102572
- WO-A2-2018/208987
- CN-A- 106 061 977
- CN-A- 112 996 505
- ZICKERMANN VOLKER ET AL: "Mechanistic insight from the crystal structure of mitochondrial complex I", SCIENCE - AUTHOR MANUSCRIPT, vol. 347, no. 6217, 2 January 2015 (2015-01-02), US, pages 44 - 49, XP093290636, ISSN: 0036-8075, DOI: 10.1126/science.1259859
- BUITRAGO SANTANILLA ALEXANDER ET AL: "Nanomole-scale high-throughput chemistry for the synthesis of complex molecules SUPPORTING ONLINE MATERIAL", SCIENCE - AUTHOR MANUSCRIPT, vol. 347, no. 6217, 2 January 2015 (2015-01-02), US, pages 49 - 53, XP093290642, ISSN: 0036-8075, DOI: 10.1126/science.1259203

## Description

This application claims the priority of Chinese Patent Application No. 202110893728.1, filed with the China National Intellectual Property Administration on August 05, 2021, and titled with "AROMATIC ACETYLENE DERIVATIVE, AND PREPARATION METHOD THEREFOR AND USE THEREOF", and Chinese Patent Application No. 202210285860.9, filed with the China National Intellectual Property Administration on March 22, 2022, and titled with "AROMATIC ACETYLENE DERIVATIVE, AND PREPARATION METHOD THEREFOR AND USE THEREOF".

### FIELD

The present disclosure relates to an aromatic acetylene derivative, a preparation method thereof, a pharmaceutical composition comprising the derivative, and use of the aromatic acetylene derivative or the pharmaceutical composition as a therapeutic agent, especially as an LPXC inhibitor.

### BACKGROUND

The period from the 1930s to the 1960s is considered the golden age for the development of antibiotics. Since then, antibiotics have been widely used around the world. However, bacterial resistance problems have also emerged one after another. Drug-resistant bacteria have become a major threat to human health. Multi-drug-resistant Gram-negative bacteria are among the main pathogens of infection. Currently, there is a serious shortage of drugs used to treat multi-drug-resistant Gram-negative bacterial infections in clinical practice, and toxic drugs are still in use. Although bacterial resistance has been a hot topic in the international pharmaceutical community in recent years, research and development have progressed slowly, and very few compounds have entered clinical research at home and abroad. Therefore, it is an important issue that needs to be addressed urgently to find a new antibacterial drug for Gram-negative bacteria.

UDP-3-O-(R-3-hydroxymyristoyl)-N-acetylglucosamine deacetylase (LPXC) is a Zn²⁺-dependent metalloenzyme, and is the rate-limiting enzyme for the first step in the synthesis of lipid A. Lipid A is an important component of the outer cell membrane of Gram-negative bacteria, anchoring lipopolysaccharide to the outer cell membrane and maintaining the integrity of the cell. Lipid A also serves as a hydrophobic external barrier, preventing external factors such as antibiotics from entering cells and protecting bacteria from invasion. Additionnaly, lipid A is an active component of bacterial endotoxins and can activate the body's immune response through the intestinal mucosa into the blood, even causing severe septic shock. This is also the cause of pathogenic infections caused by Gram-negative bacteria. Therefore, inhibiting LPXC can prevent the biosynthesis of lipid A in Gram-negative bacteria, thereby effectively controlling Gram-negative bacterial infections.

At present, further understanding of the structure and characteristics of LPXC is primarily achieved through the isolation, purification, analysis and identification of LPXC crystals from *Escherichia coli, Pseudomonas aeruginosa* and hyperthermophiles. The three LPXC from different sources exhibit highly similar structures, each containing two domains with the active region situated at the junction of the two domains. Each domain contains an α-helix and a β-sheet that surrounds the α-helix, forming a "β-α-α-β" sandwich structure. Despite slight differences in the amino acid sequences of these two domains, they share the same spatial structure. In addition, each domain has a corresponding insertion region composed of β-sheets that form distinct functional regions. Research shows that LPXC has high homology in Gram-negative bacteria and has no common sequences with various mammalian enzyme systems. From a biological perspective, inhibiting LPXC will be an ideal direction for studying antibacterial drugs due to its unique advantages of broad spectrum and low toxicity.

There are currently no drugs of LPXC inhibitors available on the market. While some progress has been made in the research and application of LPXC inhibitors, they still fall short of meeting treatment needs. Inhibitors of LPXC have been disclosed, for example, in WO 2020/102572, WO 2020/061375 and WO 2018/208987. There is still huge room for improvement, and it is still necessary to continue to research and develop new LPXC inhibitors.

### SUMMARY

The scope of the invention is defined in the appended claims.

In view of the above technical problems, the present disclosure provides a compound represented by general formula (A-I) or a stereoisomer, tautomer or pharmaceutically acceptable salt thereof: 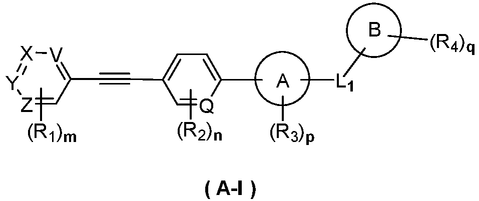 wherein:
ring A is 5- to 6-membered heteroaryl or 5- to 6-membered heterocyclyl, preferably 5-membered heteroaryl or 5-membered heterocyclyl;
ring B is 5- to 10-membered heteroaryl;
Q is C or N;
X, Y, Z, and V are each independently C or N, wherein X and Y are not N simultaneously, and Z and Y are not N simultaneously;
R₁ is the same or different, each independently being -G₁-R₅;
G₁ is selected from the group consisting of a single bond, -O-, -CH₂- and -C(=O)-;
L₁ is -(CH₂)ₛ-, preferably -CH₂-;
R₂ is the same or different, each independently selected from the group consisting of hydroxyl, cyano, halogen, alkyl, alkoxy, cycloalkyl, heterocyclyl, aryl and heteroaryl, wherein the alkyl, cycloalkyl, heterocyclyl, aryl or heteroaryl is optionally further substituted with one or more substituents selected from the group consisting of halogen, hydroxyl, cyano and alkoxy;
R₃ is the same or different, each independently selected from the group consisting of hydroxyl, cyano, halogen, alkyl and alkoxy;
alternatively, two R₃ form -C(=O)- together with the C atom to which they are connected;
R₄ is the same or different, each independently selected from the group consisting of hydroxyl, cyano, halogen, alkyl, alkoxy, cycloalkyl, heterocyclyl, heteroaryl, -C(O)R₆, -C(O)OR₆, -OC(O)R₆, -NR₇R₈, -C(O)NR₇R₈, -SO₂NR₇R₈ and -NR₇C(O)R₈, wherein the alkyl, cycloalkyl, heterocyclyl or heteroaryl is optionally further substituted with one or more substituents selected from the group consisting of halogen, hydroxyl, cyano, alkoxy, and amino;
R₅ is selected from the group consisting of cyano, halogen, alkyl, hydroxyl, alkoxy, cycloalkyl, heterocyclyl, aryl, heteroaryl, -C(O)R₆, -C(O)OR₆, -OC(O)R₆, -NR₁₂R₁₃, -C(O)NR₁₂R₁₃, -SO₂R₆, -SO₂NR₁₂R₁₃ and -NR₁₂C(O)R₁₃, wherein the alkoxy, alkyl, cycloalkyl, heterocyclyl, aryl or heteroaryl is optionally further substituted with one or more R_{A};
R_{A} is selected from the group consisting of halogen, hydroxyl, cyano, hydroxyalkyl, alkoxy, alkyl, alkenyl, cycloalkyl, heterocyclyl, aryl, heteroaryl, -C(O)R₆, -C(O)OR₆, -OC(O)R₆, -NR₇R₈, -C(O)NR₇R₈, -SO₂NR₇R₈ and -NR₇C(O)R₈, wherein the alkyl, cycloalkyl, heterocyclyl, aryl, or heteroaryl is optionally further substituted with one or more substituents selected from the group consisting of halogen, hydroxyl, cyano, amino, haloalkyl, hydroxyalkyl, alkoxy, alkyl, cycloalkyl, heterocyclyl, aryl, heteroaryl, -C(O)R₆, -C(O)OR₆, -OC(O)R₆, -NR₇R₈, -C(O)NR₇R₈, -SO₂NR₇R₈ and -NR₇C(O)R₈;
alternatively, two R_{A} form -C(O)- together with the same carbon atom to which they are connected;
R₆ is selected from the group consisting of a hydrogen atom, alkyl, cycloalkyl, heterocyclyl, aryl and heteroaryl, wherein the alkyl, cycloalkyl, heterocyclyl, aryl or heteroaryl is optionally further substituted with one or more substituents selected from the group consisting of hydroxyl, halogen, nitro, cyano, alkyl, alkoxy, haloalkyl, haloalkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
R₇, R₈, R₁₂ and R₁₃ are each independently selected from the group consisting of a hydrogen atom, hydroxyl, halogen, alkyl, alkoxy, cycloalkyl, heterocyclyl, aryl and heteroaryl, wherein the alkyl, alkoxy, cycloalkyl, heterocyclyl, aryl or heteroaryl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂R₉, -SO₂NR₁₀R₁₁ and -NR₁₀C(O)R₁₁;
alternatively, R₇ and R₈ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
alternatively, R₁₂ and R₁₃ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
R₉, R₁₀ and R₁₁ are each independently selected from the group consisting of a hydrogen atom, alkyl, amino, cycloalkyl, heterocyclyl, aryl and heteroaryl, wherein the alkyl, cycloalkyl, heterocyclyl, aryl or heteroaryl is optionally further substituted with one or more substituents selected from the group consisting of hydroxyl, halogen, nitro, amino, cyano, alkyl, alkoxy, cycloalkyl, heterocyclyl, aryl, heteroaryl, carboxyl, and a carboxylate group;
m is 0, 1, 2 or 3;
n is 0, 1 or 2, preferably 0;
p is 0, 1 or 2;
s is 1 or 2; and
q is 1, 2 or 3.

According to a preferred embodiment of the present disclosure, the present disclosure provides a compound represented by general formula (A-I) or a stereoisomer, tautomer or pharmaceutically acceptable salt thereof, which is a compound represented by general formula (I) or a stereoisomer, tautomer or pharmaceutically acceptable salt thereof: 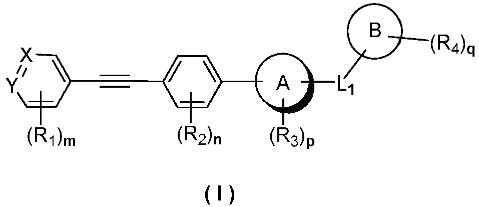 wherein:
ring A is 5- to 6-membered heteroaryl or 5- to 6-membered heterocyclyl, preferably 5-membered heteroaryl or 5-membered heterocyclyl;
ring B is 5- to 10-membered heteroaryl;
X and Y are each independently C or N, wherein X and Y are not N simultaneously;
R₁ is the same or different, each independently selected from -G₁-R₅;
G₁ is selected from the group consisting of a single bond, -CH₂- and -C(=O)-;
L₁ is -(CH₂)s-, preferably -CH₂-;
R₂ is the same or different, each independently selected from the group consisting of hydroxyl, cyano, halogen, alkyl, alkoxy, cycloalkyl, heterocyclyl, aryl and heteroaryl, wherein the alkyl, cycloalkyl, heterocyclyl, aryl or heteroaryl is optionally further substituted with one or more substituents selected from the group consisting of halogen, hydroxyl, cyano and alkoxy;
R₃ is the same or different, each independently selected from the group consisting of hydroxyl, cyano, halogen, alkyl and alkoxy;
alternatively, two R₃ form -C(=O)- together with the C atom to which they are connected;
R₄ is the same or different, each independently selected from the group consisting of hydroxyl, cyano, halogen, alkyl, alkoxy, cycloalkyl, heterocyclyl, heteroaryl, -C(O)R₆, -C(O)OR₆, -OC(O)R₆, -NR₇R₈, -C(O)NR₇R₈, -SO₂NR₇R₈ and -NR₇C(O)R₈, wherein the alkyl, cycloalkyl, heterocyclyl or heteroaryl is optionally further substituted with one or more substituents selected from the group consisting of halogen, hydroxyl, cyano, alkoxy and amino;
R₅ is selected from the group consisting of cyano, halogen, alkyl, hydroxyl, alkoxy, cycloalkyl, heterocyclyl, aryl, heteroaryl, -C(O)R₆, -C(O)OR₆, -OC(O)R₆, -NR₁₂R₁₃, -C(O)NR₁₂R₁₃, -SO₂NR₁₂R₁₃ and -NR₁₂C(O)R₁₃, wherein the alkoxy, alkyl, cycloalkyl, heterocyclyl, aryl or heteroaryl is optionally further substituted with one or more R_{A};
R_{A} is selected from the group consisting of halogen, hydroxyl, cyano, hydroxyalkyl, alkoxy, alkyl, cycloalkyl, heterocyclyl, aryl, heteroaryl, -C(O)R₆, -C(O)OR₆, -OC(O)R₆, -NR₇R₈, -C(O)NR₇R₈, -SO₂NR₇R₈ and -NR₇C(O)R₈, wherein the alkyl, cycloalkyl, heterocyclyl, aryl or heteroaryl is optionally further substituted with one or more substituents selected from the group consisting of halogen, hydroxyl, amino, haloalkyl, hydroxyalkyl, alkoxy, alkyl, cycloalkyl, heterocyclyl, aryl, heteroaryl, -C(O)R₆, -C(O)OR₆, -OC(O)R₆, -NR₇R₈, -C(O)NR₇R₈, -SO₂NR₇R₈ and -NR₇C(O)R₈;
R₆ is selected from the group consisting of a hydrogen atom, alkyl, cycloalkyl, heterocyclyl, aryl and heteroaryl, wherein the alkyl, cycloalkyl, heterocyclyl, aryl or heteroaryl is optionally further substituted with one or more substituents selected from the group consisting of hydroxyl, halogen, nitro, cyano, alkyl, alkoxy, haloalkyl, haloalkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
R₇, R₈, R₁₂ and R₁₃ are each independently selected from the group consisting of a hydrogen atom, hydroxyl, halogen, alkyl, alkoxy, cycloalkyl, heterocyclyl, aryl and heteroaryl, wherein the alkyl, alkoxy, cycloalkyl, heterocyclyl, aryl or heteroaryl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
alternatively, R₇ and R₈ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
alternatively, R₁₂ and R₁₃ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
R₉, R₁₀ and R₁₁ are each independently selected from the group consisting of a hydrogen atom, alkyl, amino, cycloalkyl, heterocyclyl, aryl and heteroaryl, wherein the alkyl, cycloalkyl, heterocyclyl, aryl or heteroaryl is optionally further substituted with one or more substituents selected from the group consisting of hydroxyl, halogen, nitro, amino, cyano, alkyl, alkoxy, cycloalkyl, heterocyclyl, aryl, heteroaryl, carboxyl and a carboxylate group;
m is 0, 1, 2 or 3;
n is 0, 1 or 2, preferably 0;
p is 0, 1 or 2;
s is 1 or 2; and
q is 1, 2 or 3.

According to a preferred embodiment of the present disclosure, the present disclosure provides a compound represented by general formula (A-I) or a stereoisomer, tautomer or pharmaceutically acceptable salt thereof, which is a compound represented by general formula (II-1), (II-2), (II-3), (II-4) or (II-5) or a stereoisomer, tautomer or pharmaceutically acceptable salt thereof: 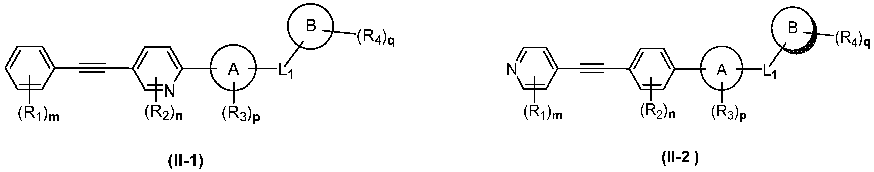 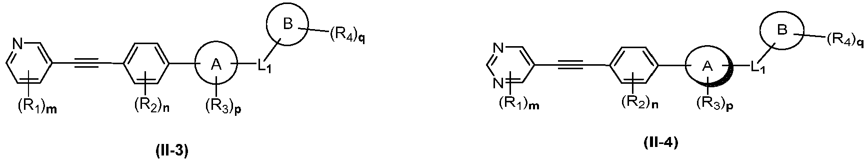 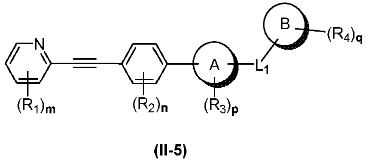 wherein, ring A, ring B, R₁-R₄, L₁, m, n, p and q are as defined in general formula (A-I).

According to a preferred embodiment of the present disclosure, the present disclosure provides a compound represented by general formula (A-I), (I) or (II-3) or a stereoisomer, tautomer or pharmaceutically acceptable salt thereof, which is a compound represented by general formula (III-1) or (III-2) or a stereoisomer, tautomer or pharmaceutically acceptable salt thereof: 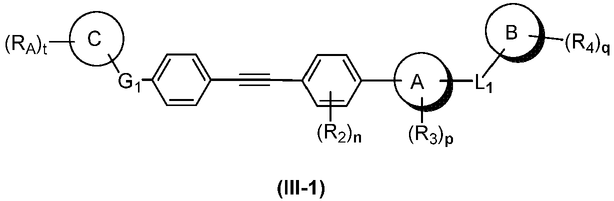 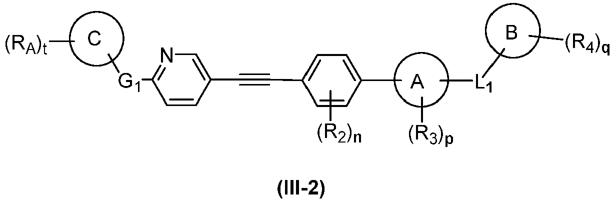 wherein:
ring C is selected from the group consisting of C₄-C₈ cycloalkyl, 5- to 6-membered heteroaryl and 4- to 8-membered heterocyclyl;
R_{A} is the same or different, each independently selected from the group consisting of haloalkyl, hydroxyalkyl, alkoxy, alkyl, alkenyl, hydroxyl, halogen, cyano, -C(O)NH₂, cycloalkyl, heterocyclyl, aryl, heteroaryl and carboxyl, wherein the alkyl, cycloalkyl, heterocyclyl, aryl or heteroaryl is optionally further substituted with a substituent selected from the group consisting of hydroxyl, cyano, carboxyl and an ester group;
alternatively, two R_{A} form -C(O)- together with the same carbon atom to which they are connected; t is 0, 1, 2 or 3;
ring A, ring B, R₂-R₄, G₁, L₁, n, p and q are as defined in general formula (A-I).

According to a preferred embodiment of the present disclosure, the present disclosure provides a compound represented by general formula (A-I), (I), (II-1), (II-2), (II-3), (II-4), (II-5), (III-1) or (III-2) or a stereoisomer, tautomer or pharmaceutically acceptable salt thereof, wherein ring A is selected from the group consisting of: 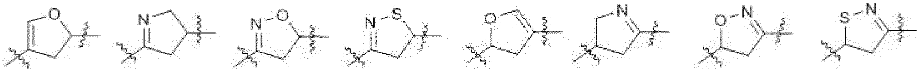 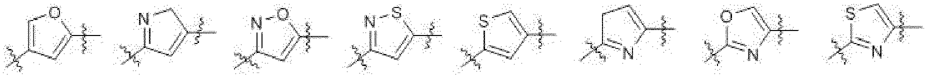 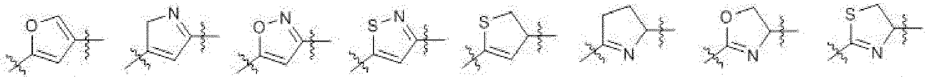 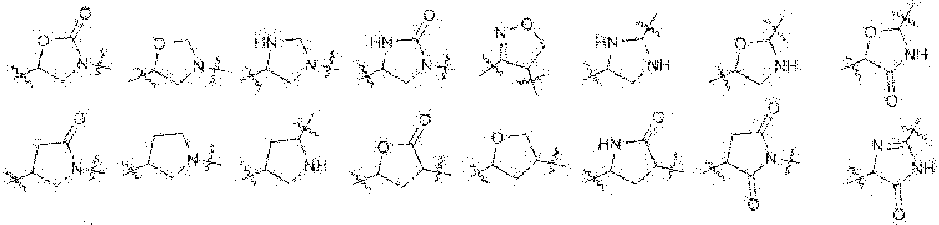 and

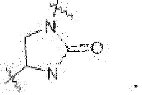

According to a preferred embodiment of the present disclosure, the present disclosure provides a compound represented by general formula (A-I), (I), (II-1), (II-2), (II-3), (II-4), (II-5), (III-1) or (III-2) or a stereoisomer, tautomer or pharmaceutically acceptable salt thereof, wherein ring B is selected from the group consisting of:

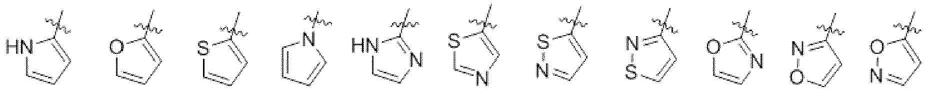

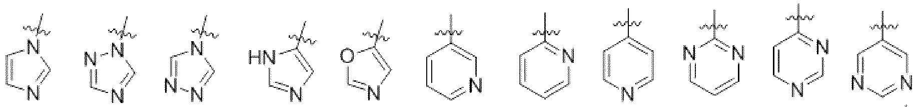

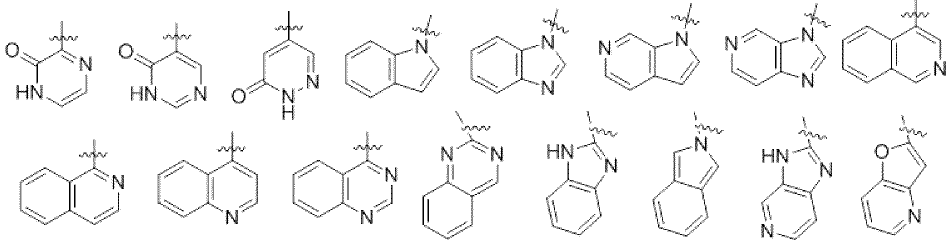

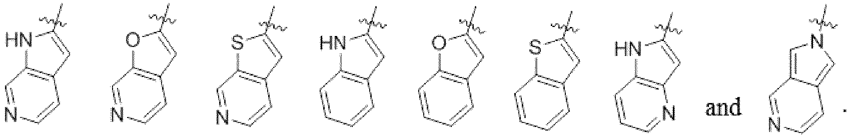

According to a preferred embodiment of the present disclosure, the present disclosure provides a compound represented by general formula (A-I), (I), (II-1), (II-2), (II-3), (II-4), (II-5), (III-1) or (III-2) or a stereoisomer, tautomer or pharmaceutically acceptable salt thereof, wherein ring C is selected from the group consisting of:

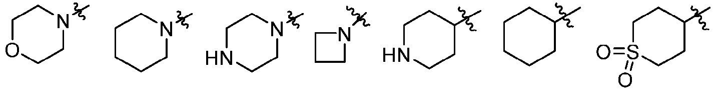

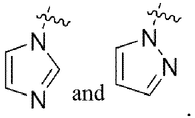

According to a preferred embodiment of the present disclosure, the present disclosure provides a compound represented by general formula (A-I), (I), (II-1), (II-2), (II-3), (II-4), (II-5), (III-1) or (III-2) or a stereoisomer, tautomer or pharmaceutically acceptable salt thereof, wherein:
R₄ is selected from the group consisting of alkyl, -C(O)R₆, -C(O)OR₆ and -C(O)NR₇R₈, wherein the alkyl is optionally further substituted with one or more substituents of hydroxyl or halogen;
R₆ is selected from the group consisting of a hydrogen atom, hydroxyl, alkyl and alkoxy;
R₇ and R₈ are each independently a hydrogen atom or alkyl.

According to a preferred embodiment of the present disclosure, the present disclosure provides a compound represented by general formula (A-I), (I), (II-1), (II-2), (II-3), (II-4), (II-5), (III-1) or (III-2) or a stereoisomer, tautomer or pharmaceutically acceptable salt thereof, wherein R₄ is selected from the group consisting of:

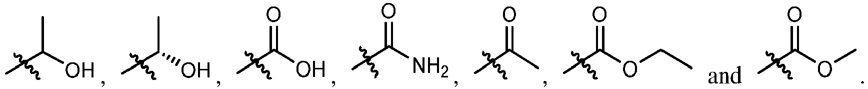

In a preferred embodiment of the present disclosure, the compound represented by general formula (A-I) is selected from the group consisting of:

| **Compound No.** | **Structure** | **Name** |
|---|---|---|
| Example 1 | 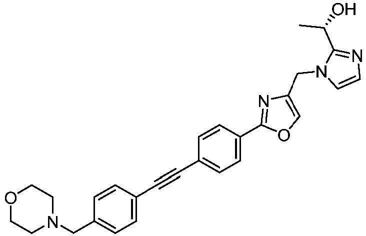 | (S)-1-(1-((2-(4-((4-(morph olinomethyl)phenyl)ethyn yl)phenyl)oxazol-4-yl)met hyl)-1H-imidazol-2-yl) ethan-1-ol |
| Example 2 | 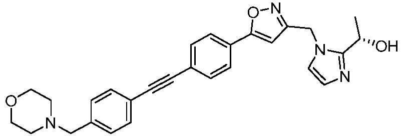 | (S)-1-(1-((5-(4-((4-(morph olinomethyl)phenyl)ethyn yl)phenyl)isoxazol-3-yl) methyl)-1H-imidazol-2-yl) ethan-1-ol |
| Example 3 | 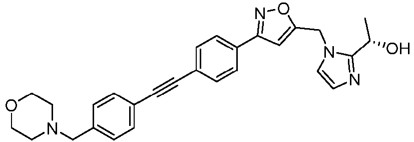 | (S)-1-(1-((3-(4-((4-(morph olinomethyl)phenyl)ethyn yl)phenyl)isoxazol-5-yl) methyl)-1H-imidazol-2-yl) ethan-1-ol |
| Example 4 | 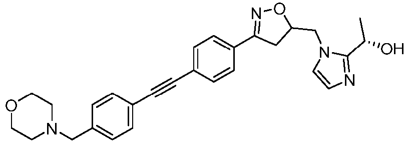 | (S)-1-(1-((3-(4-((4-(morph olinomethyl)phenyl)ethyn yl)phenyl)-4,5-dihydroiso xazol-5-yl)methyl)-1H-imidazol-2-yl)ethan-1-ol |
| Example 5 | 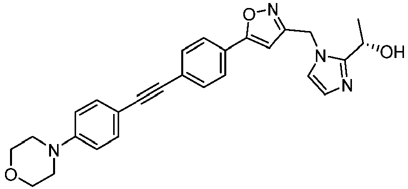 | (S)-1-(1-((5-(4-((4-morph olinophenyl))ethynyl)phen yl)isoxazol-3-yl)methyl)-1H -imidazol-2-yl)ethan-1-ol |
| Example 6 | 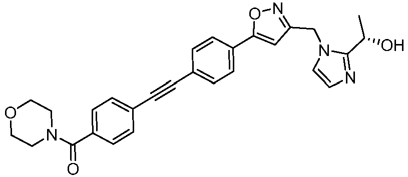 | (S)-(4-((4-(3-((2-(1-hydro xyethyl)-1H-imidazol-1-yl )methyl)isoxazol-5-yl)phe nyl)ethynyl)phenyl)(morp holino)methanone |
| Example 7 | 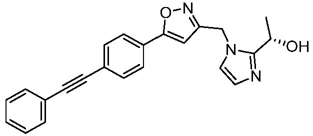 | (S)-1-(1-((5-(4-(phenyleth ynyl)phenyl)isoxazol-3-yl )methyl)-1H-imidazol-2-yl )ethan-1-ol |
| Example 8 | 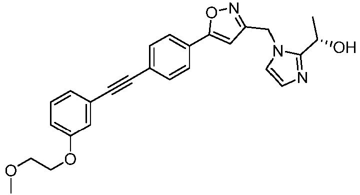 | (S)-1-(1-((5-(4-((3-(2-met hoxyethoxy)phenyl)ethyn yl)phenyl)isoxazol-3-yl) methyl)-1H-imidazol-2-yl) ethan-1-ol |
| Example 9 | 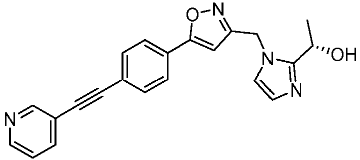 | (S)-1-(1-((5-(4-(pyridin-3-ylethynyl)phenyl)isoxazol -3-yl)methyl)-1H-imidazol -2-yl)ethan-1-ol |
| Example 10 | 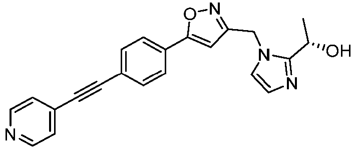 | (S)-1-(1-((5-(4-(pyridin-4-ylethynyl)phenyl)isoxazol -3-yl)methyl)-1H-imidazol -2-yl)ethan-1-ol |
| Example 11 | 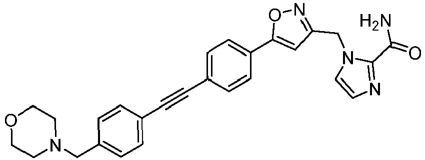 | 1-((5-(4-((4-(morpholino methyl)phenyl)ethynyl)ph enyl)isoxazol-3-yl)methyl )-1H-imidazol-2-carboxa mide |
| Example 12 | 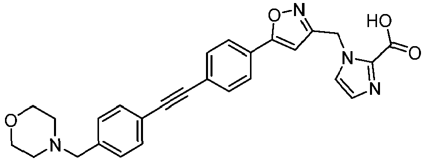 | 1-((5-(4-((4-(morpholino methyl)phenyl)ethynyl)ph enyl)isoxazol-3-yl)methyl )-1H-imidazol-2-carboxylic acid |
| Example 13 | 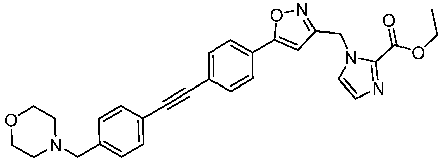 | Ethyl 1-((5-(4-((4-(morpholino methyl)phenyl)ethynyl) phenyl)isoxazol-3-yl)methyl )-1H-imidazol-2-carboxylate |
| Example 14 | 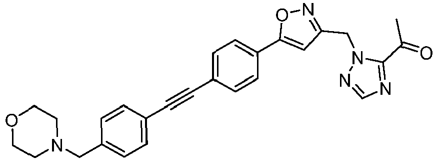 | 1-(1-((5-(4-((4-(morpholin omethyl)phenyl)ethynyl) phenyl)isoxazol-3-yl)methyl )-1H-1,2,4-triazol-5-yl) ethan-1-one |
| Example 15 | 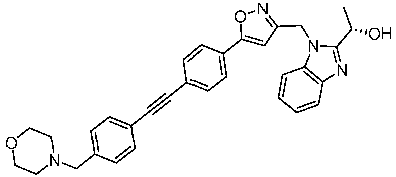 | (S)-1-(1-((5-(4-((4-(morph olinomethyl)phenyl)ethyn yl)phenyl)isoxazol-3-yl) methyl)-1H-benzo[d]imidazol -2-yl)ethan-1-ol |
| Example 16 | 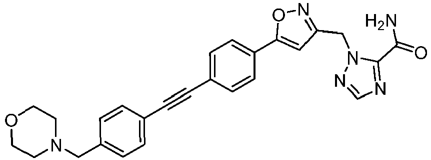 | 1-((5-(4-((4-(morpholino methyl)phenyl)ethynyl) phenyl)isoxazol-3-yl)methyl )-1H-1,2,4-triazol-5-carboxamide |
| Example 17 | 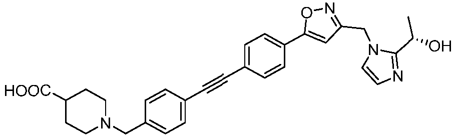 | (S)-1-(4-((4-(3-((2-(1-hydr oxyethyl)-1H-imidazol-1-yl)methyl)isoxazol-5-yl) phenyl)ethynyl)benzyl) piperidin-4-carboxylic acid |
| Example 18 | 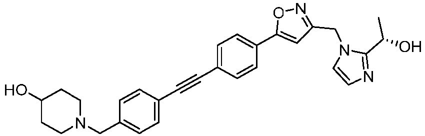 | (S)-1-(4-((4-(3-((2-(1-hydr oxyethyl)-1H-imidazol-1-yl)methyl)isoxazol-5-yl) phenyl)ethynyl)benzyl)pipe ridin-4-ol |
| Example 19 | 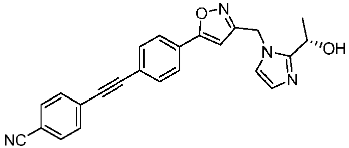 | (S)-4-((4-(3-((2-(1-hydrox yethyl)-1H-imidazol-1-yl) methyl)isoxazol-5-yl) phenyl)ethynyl)benzonitrile |
| Example 20 | 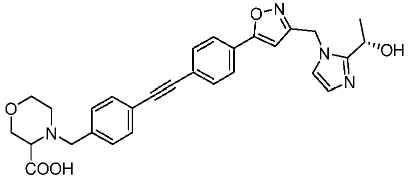 | 4-(4-((4-(3-((2-((S)-1-hydr oxyethyl)-1H-imidazol-1-yl)methyl)isoxazol-5-yl) phenyl)ethynyl)benzyl) morpholin-3-carboxylic acid |
| Example 21 | 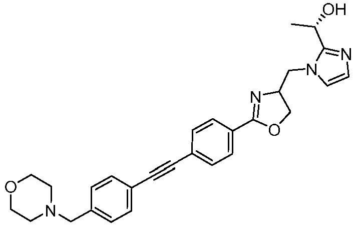 | (1S)-1-(1-((2-(4-((4-(morp holinomethyl)phenyl)ethy nyl)phenyl)-4,5-dihydroxa zol-4-yl)methyl)-1H-imidazol -2-yl)ethan-1-ol |
| Example 22 | 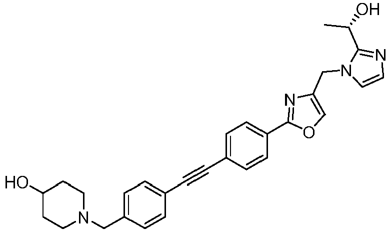 | (S)-1-(4-((4-(4-((2-(1-hydr oxyethyl)-1H-imidazol-1-yl)methyl)oxazol-2-yl)phe nyl)ethynyl)benzyl)piperidin -4-ol |
| Example 23 | 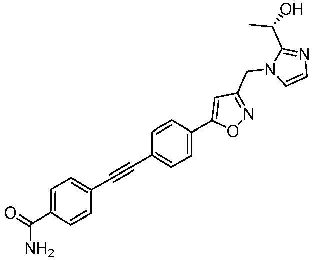 | (S)-4-((4-(3-((2-(1-hydrox yethyl)-1H-imidazol-1-yl) methyl)isoxazol-5-yl)phen yl)ethynyl)benzamide |
| Example 24 | 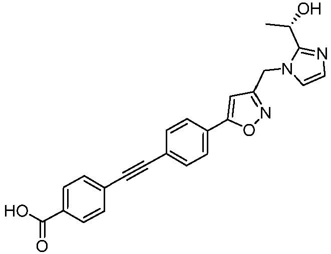 | (S)-4-((4-(3-((2-(1-hydrox yethyl)-1H-imidazol-1-yl) methyl)isoxazol-5-yl)phen yl)ethynyl)benzoic acid |
| Example 25 | 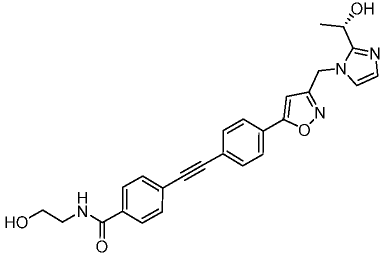 | (S)-N-(2-hydroxyethyl)-4-((4-(3-((2-(1-hydroxyethyl )-1H-imidazol-1-yl)methyl )isoxazol-5-yl)phenyl)eth ynyl)benzamide |
| Example 26 | 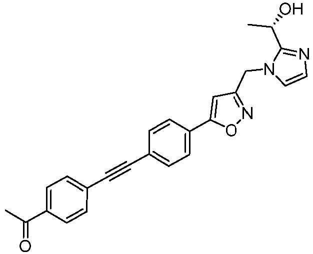 | (S)-1-(4-((4-(3-((2-(1-hydr oxyethyl)-1H-imidazol-1-yl)methyl)isoxazol-5-yl) phenyl)ethynyl)phenyl)ethan -1-one |
| Example 27 | 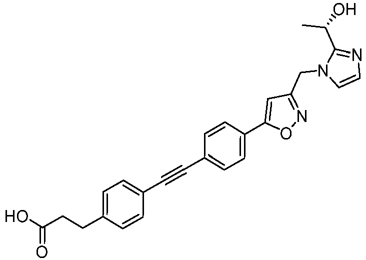 | (S)-3-(4-((4-(3-((2-(1-hydr oxyethyl)-1H-imidazol-1-yl)methyl)isoxazol-5-yl)p henyl)ethynyl)phenyl)pro panoic acid |
| Example 28 | 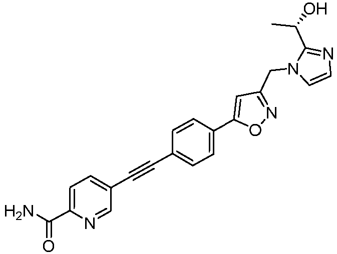 | (S)-5-((4-(3-((2-(1-hydrox yethyl)-1H-imidazol-1-yl) methyl)isoxazol-5-yl)phen yl)ethynyl)picolinamide |
| Example 29 | 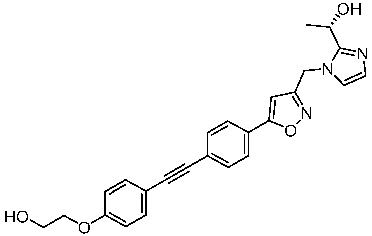 | (S)-1-(1-((5-(4-((4-(2-hydr oxyethoxy)phenyl)ethynyl )phenyl)isoxazol-3-yl) methyl)-1H-imidazol-2-yl) ethan-1-ol |
| Example 30 | 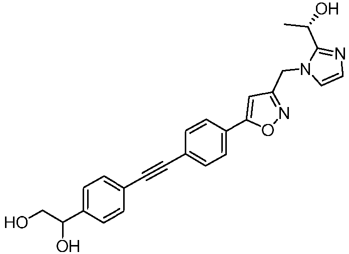 | 1-(4-((4-(3-((2-((S)-1-hydr oxyethyl)-1H-imidazol-1-yl)methyl)isoxazol-5-yl) phenyl)ethynyl)phenyl) ethane-1,2-diol |
| Example 31 | 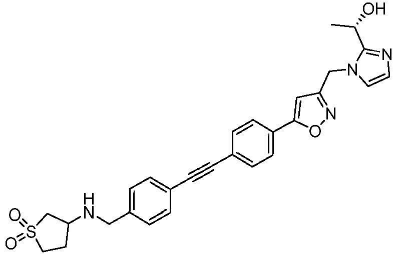 | 3-((4-((4-(3-((2-((S)-1-hyd roxyethyl))-1H-imidazol-1 -yl)methyl)isoxazol-5-yl) phenyl)ethynyl)benzyl) amino)tetrahydrothiophene 1,1-dioxide |
| Example 32 | 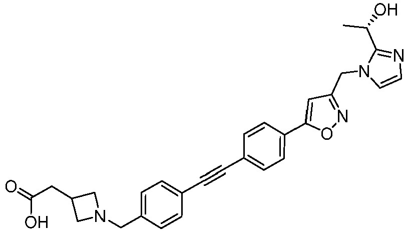 | (S)-2-(1-(4-((4-(3-((2-(1-hydroxyethyl)-1H-imidazol -1-yl)methyl)isoxazol-5-yl )phenyl)ethynyl)benzyl) azetidin-3-yl)acetic acid |
| Example 33 | 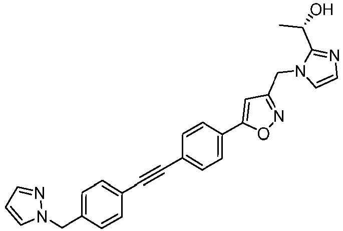 | (S)-1-(1-((5-(4-((4-((1H-pyrazol-1-yl)methyl)phenyl )ethynyl)phenyl)isoxazol-3-yl)methyl)-1H-imidazol -2-yl)ethan-1-ol |
| Example 34 | 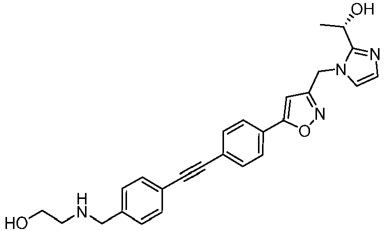 | (S)-2-((4-((4-(3-((2-(1-hyd roxyethyl)-1H-imidazol-1-yl)methyl)isoxazol-5-yl) phenyl)ethynyl)benzyl) amino)ethan-1-ol |
| Example 35 | 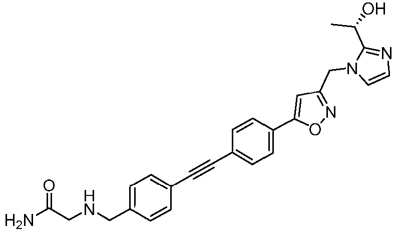 | (S)-2-((4-((4-(3-((2-(1-hydroxyethyl)-1H-imidazol-1-yl)methyl)isoxazol-5-yl) phenyl)ethynyl)benzyl) amino)acetamide |
| Example 36 | 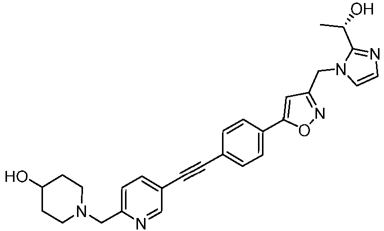 | (S)-1-((5-((4-(3-((2-(1-hyd roxyethyl)-1H-imidazol-1-yl)methyl)isoxazol-5-yl) phenyl)ethynyl)pyridin-2-yl )methyl)piperidin-4-ol |
| Example 37 | 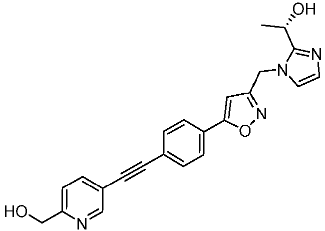 | (S)-1-(1-((5-(4-((6-(hydro xymethyl)pyridin-3-yl) ethynyl)phenyl)isoxazol-3-yl )methyl)-1H-imidazol-2-yl )ethan-1-ol |
| Example 38 | 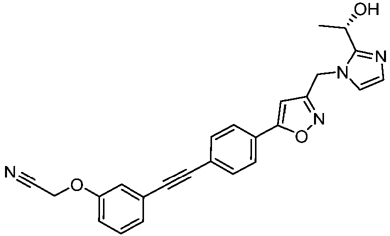 | (S)-2-(3-((4-(3-((2-(1-hydr oxyethyl)-1H-imidazol-1-yl)methyl)isoxazol-5-yl)p henyl)ethynyl)phenoxy)ac etonitrile |
| Example 39 | 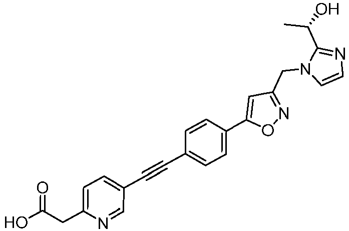 | (S)-2-(5-((4-(3-((2-(1-hydr oxyethyl)-1H-imidazol-1-yl)methyl)isoxazol-5-yl)p henyl)ethynyl)pyridin-2-yl )acetic acid |
| Example 40 | 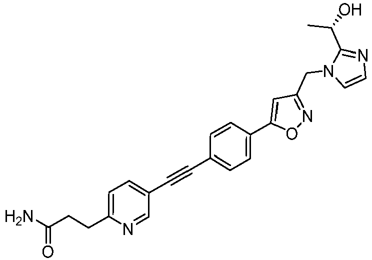 | (S)-3-(5-((4-(3-((2-(1-hydr oxyethyl)-1H-imidazol-1-yl)methyl)isoxazol-5-yl)p henyl)ethynyl)pyridin-2-yl )propanamide |
| Example 41 | 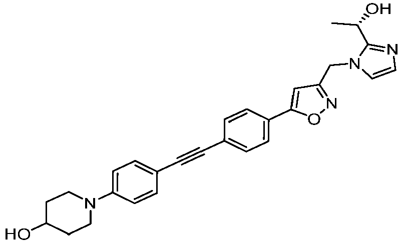 | (S)-1-(4-((4-(3-((2-(1-hydr oxyethyl)-1H-imidazol-1-yl)methyl)isoxazol-5-yl) phenyl)ethynyl)phenyl) piperidin-4-ol |
| Example 42 | 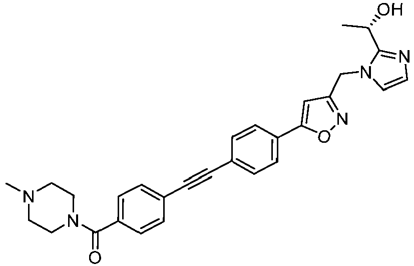 | (S)-(4-((4-(3-((2-(1-hydro xyethyl)-1H-imidazol-1-yl )methyl)isoxazol-5-yl) phenyl)ethynyl)phenyl)(4-methylpiperazin-1-yl)methanone |
| Example 43 | 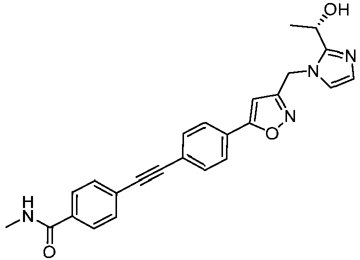 | (S)-4-((4-(3-((2-(1-hydrox yethyl)-1H-imidazol-1-yl) methyl)isoxazol-5-yl) phenyl)ethynyl)-N-methylbenz amide |
| Example 44 | 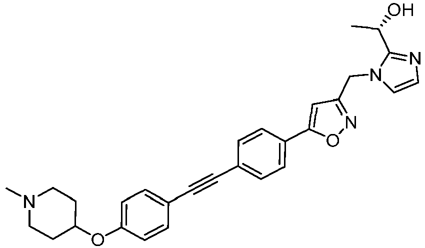 | (S)-1-(1-((5-(4-((4-((1-met hylpiperidin-4-yl)oxy)phe nyl)ethynyl)phenyl)isoxaz ol-3-yl)methyl)-1H-imidazol -2-yl)ethan-1-ol |
| Example 45 | 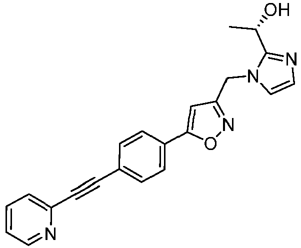 | (S)-1-(1-((5-(4-(pyridin-2-ylethynyl)phenyl)isoxazol -3-yl)methyl)-1H-imidazol -2-yl)ethan-1-ol |
| Example 46 | 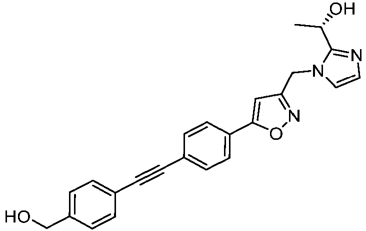 | (S)-1-(1-((5-(4-((4-(hydro xymethyl)phenyl)ethynyl) phenyl)isoxazol-3-yl)meth yl)-1H-imidazol-2-yl)ethan -1-ol |
| Example 47 | 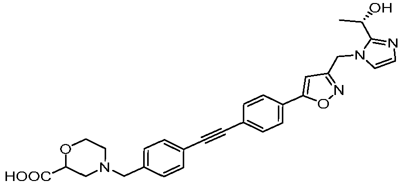 | 4-(4-((4-(3-((2-((S)-1-hydr oxyethyl)-1H-imidazol-1-yl)methyl)isoxazol-5-yl) phenyl)ethynyl)benzyl)mor pholin-2-carboxylic acid |
| Example 48 | 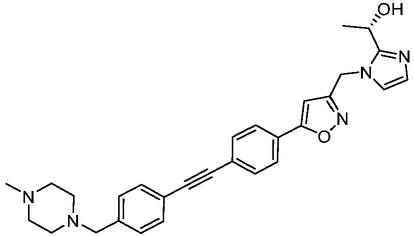 | (S)-1-(1-((5-(4-((4-((4-met hylpiperazin-1-yl)methyl) phenyl)ethynyl)phenyl)iso xazol-3-yl)methyl)-1H-im idazol-2-yl)ethan-1-ol |
| Example 49 | 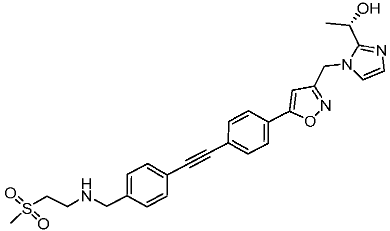 | (S)-1-(1-((5-(4-((4-(((2-(m ethylsulfonyl)ethyl)amino )methyl)phenyl)ethynyl) phenyl)isoxazol-3-yl)methyl )-1H-imidazol-2-yl)ethan -1-ol |
| Example 50 | 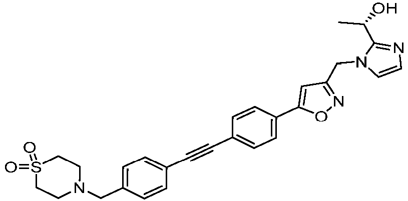 | (S)-4-(4-((4-(3-((2-(1-hydr oxyethyl)-1H-imidazol-1-yl)methyl)isoxazol-5-yl) phenyl)ethynyl)benzyl)thio morpholin-1,1-dioxide |
| Example 51 | 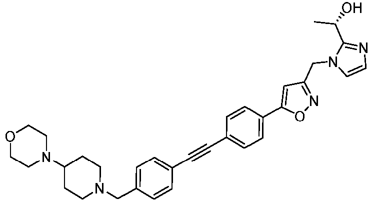 | (S)-1-(1-((5-(4-((4-((4-mo rpholinopiperidin-1-yl)me thyl)phenyl)ethynyl)phenyl )isoxazol-3-yl)methyl)-1 H-imidazol-2-yl)ethan-1-ol |
| Example 52 | 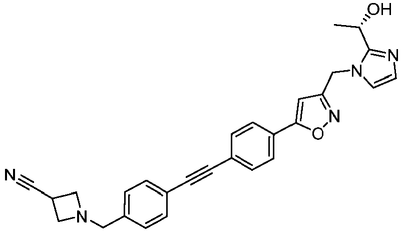 | (S)-1-(4-((4-(3-((2-(1-hydr oxyethyl)-1H-imidazol-1-yl)methyl)isoxazol-5-yl) phenyl)ethynyl)benzyl)azet idin-3-carbonitrile |
| Example 53 | 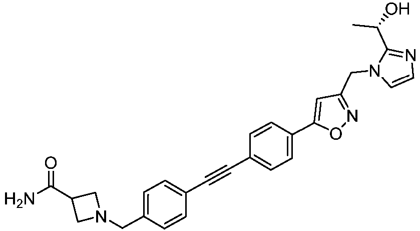 | (S)-1-(4-((4-(3-((2-(1-hydr oxyethyl)-1H-imidazol-1-yl)methyl)isoxazol-5-yl) phenyl)ethynyl)benzyl)azet idin-3-carboxamide |
| Example 54 | 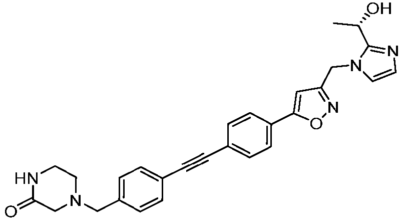 | (S)-4-(4-((4-(3-((2-(1-hydr oxyethyl)-1H-imidazol-1-yl)methyl)isoxazol-5-yl) phenyl)ethynyl)benzyl)pipe razin-2-one |
| Example 55 | 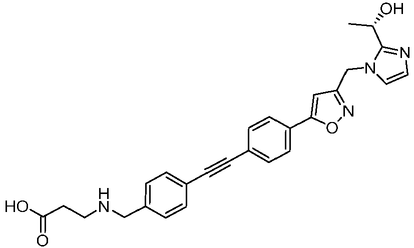 | (S)-3-((4-((4-(3-((2-(1-hyd roxyethyl)-1H-imidazol-1-yl)methyl)isoxazol-5-yl) phenyl)ethynyl)benzyl)ami no)propanoic acid |
| Example 56 | 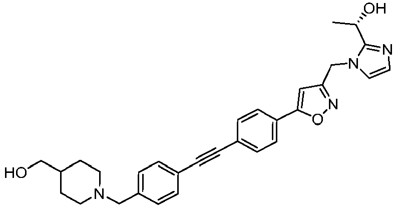 | (S)-1-(1-((5-(4-((4-((4-(hy droxymethyl)piperidin-1-yl )methyl)phenyl)ethynyl)p henyl)isoxazol-3-yl)methyl )-1H-imidazol-2-yl)ethan -1-ol |
| Example 57 | 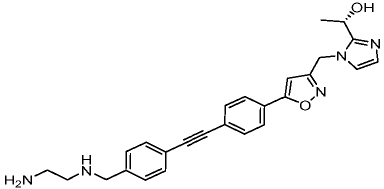 | (S)-1-(1-((5-(4-((4-((2-ami noethyl)amino)methyl)phe nyl)ethynyl)phenyl)isoxaz ol-3-yl)methyl)-1H-imida zol-2-yl)ethan-1-ol |
| Example 58 | 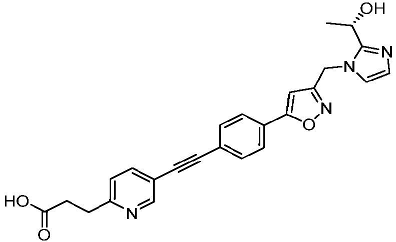 | (S)-3-(5-((4-(3-((2-(1-hydr oxyethyl)-1H-imidazol-1-yl)methyl)isoxazol-5-yl) phenyl)ethynyl)pyridin-2-yl )propanoic acid |
| Example 59 | 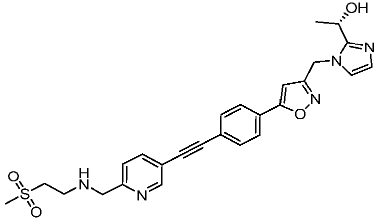 | (S)-1-(1-((5-(4-((6-(((2-(m ethylsulfonyl)ethyl)amino )methyl)pyridin-3-yl)ethy nyl)phenyl)isoxazol-3-yl) methyl)-1H-imidazol-2-yl )ethan-1-ol |
| Example 60 | 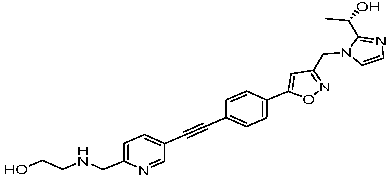 | (S)-2-(((5-((4-(3-((2-(1-hy droxyethyl))-1H-imidazol-1-yl)methyl)isoxazol-5-yl) phenyl)ethynyl)pyridin-2-yl)methyl)amino)ethan-1-ol |
| Example 61 | 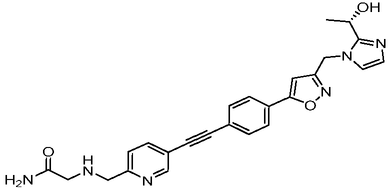 | (S)-2-(((5-((4-(3-((2-(1-hy droxyethyl))-1H-imidazol-1-yl)methyl)isoxazol-5-yl) phenyl)ethynyl)pyridin-2-yl)methyl)amino)acetamide |
| Example 62 | 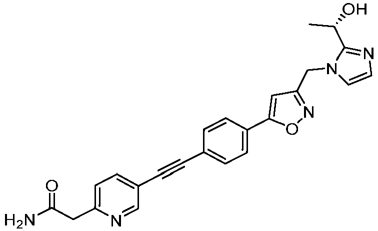 | (S)-2-(5-((4-(3-((2-(1-hydr oxyethyl)-1H-imidazol-1-yl)methyl)isoxazol-5-yl) phenyl)ethynyl)pyridin-2-yl )acetamide |
| Example 63 | 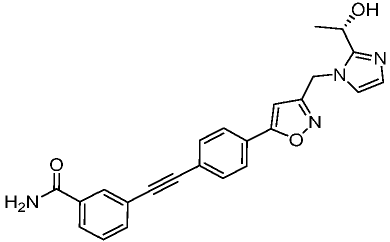 | (S)-3-((4-(3-((2-(1-hydrox yethyl)-1H-imidazol-1-yl) methyl)isoxazol-5-yl)phen yl)ethynyl)benzamide |
| Example 64 | 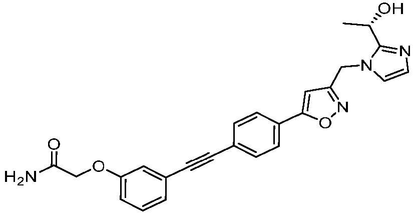 | (S)-2-(3-((4-(3-((2-(1-hydr oxyethyl)-1H-imidazol-1-yl)methyl)isoxazol-5-yl) phenyl)ethynyl)phenoxy) acetamide |
| Example 65 | 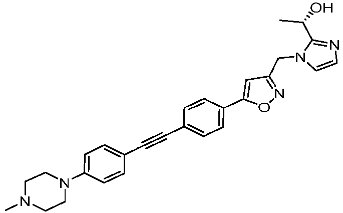 | (S)-1-(1-((5-(4-((4-(4-met hylpiperazin-1-yl)phenyl) ethynyl)phenyl)isoxazol-3 -yl)methyl)-1H-imidazol-2-yl)ethan-1-ol |
| Example 66 | 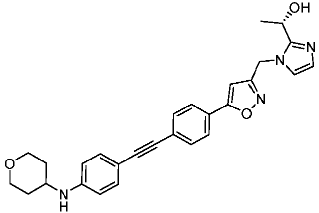 | (S)-1-(1-((5-(4-((4-((tetrah ydro-2H-pyran-4-yl)amino )phenyl)ethynyl)phenyl) isoxazol-3-yl)methyl)-1H-imidazol-2-yl)ethan-1-ol |
| Example 67 | 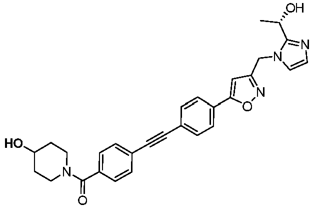 | (S)-(4-((4-(3-((2-(1-hydro xyethyl)-1H-imidazol-1-yl )methyl)isoxazol-5-yl)phe nyl)ethynyl)phenyl)(4-hyd roxypiperidin-1-yl)methanone |
| Example 68 | 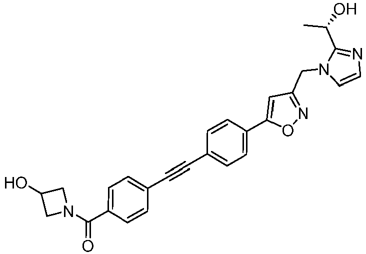 | (S)-(3-hydroxyazetidin-1-yl)(4-((4-(3-((2-(1-hydrox yethyl))-1H-imidazol-1-yl )methyl)isoxazol-5-yl)phe nyl)ethynyl)phenyl)metha none |
| Example 69 | 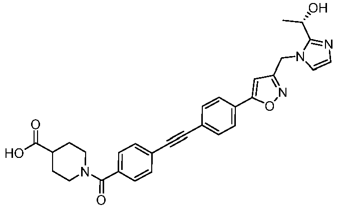 | (S)-1-(4-((4-(3-((2-(1-hydr oxyethyl)-1H-imidazol-1-yl)methyl)isoxazol-5-yl) phenyl)ethynyl)benzoyl)pip eridin-4-carboxylic acid |
| Example 70 | 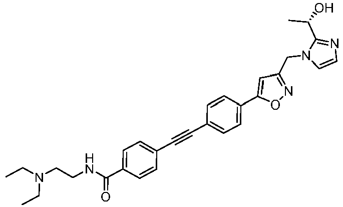 | (S)-N-(2-(diethylamino) ethyl)-4-((4-(3-((2-(1-hydro xyethyl))-1H-imidazol-1-yl)methyl)isoxazol-5-yl) phenyl)ethynyl)benzamide |
| Example 71 | 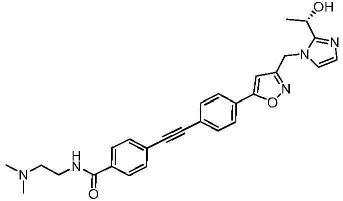 | (S)-N-(2-(dimethylamino) ethyl)-4-((4-(3-((2-(1-hydr oxyethyl))-1H-imidazol-1-yl)methyl)isoxazol-5-yl)p henyl)ethynyl)benzamide |
| Example 72 | 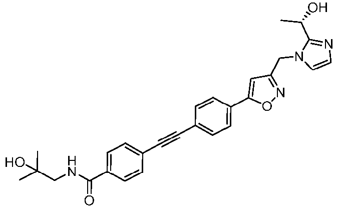 | (S)-N-(2-hydroxy-2-meth ylpropyl)-4-((4-(3-((2-(1-hydroxyethyl))-1H-imidazol -1-yl)methyl)isoxazol-5-yl) )phenyl)ethynyl)benzamide |
| Example 73 | 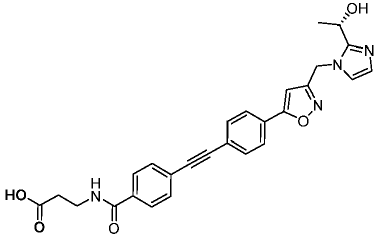 | (S)-3-(4-((4-(3-((2-(1-hydr oxyethyl)-1H-imidazol-1-yl)methyl)isoxazol-5-yl) phenyl)ethynyl)benzamido) propanoic acid |
| Example 74 | 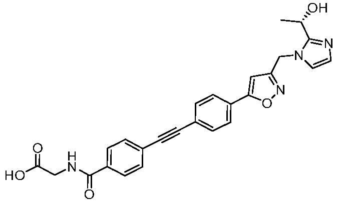 | (S)-(4-((4-(3-((2-(1-hydro xyethyl)-1H-imidazol-1-yl )methyl)isoxazol-5-yl)phe nyl)ethynyl)benzoyl)glycinate |
| Example 75 | 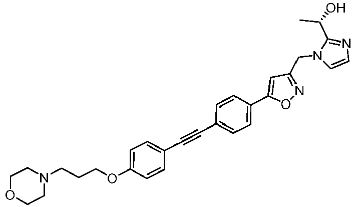 | (S)-1-(1-((5-(4-((4-(3-mor pholinopropoxy)phenyl) ethynyl)phenyl)isoxazol-3-yl )methyl)-1H-imidazol-2-yl)ethan-1-ol |
| Example 76 | 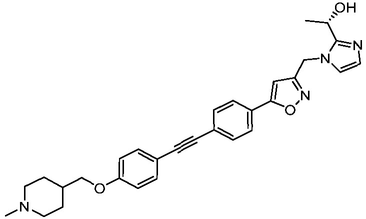 | (S)-1-(1-((5-(4-((4-((1-met hylpiperidin-4-yl)methoxy )phenyl)ethynyl)phenyl)is oxazol-3-yl)methyl)-1H-imidazol-2-yl)ethan-1-ol |
| Example 77 | 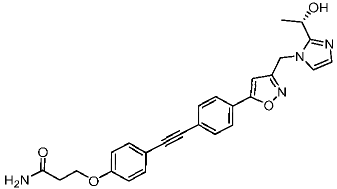 | (S)-3-(4-((4-(3-((2-(1-hydr oxyethyl)-1H-imidazol-1-yl)methyl)isoxazol-5-yl) phenyl)ethynyl)phenoxy) propanamide |
| Example 78 | 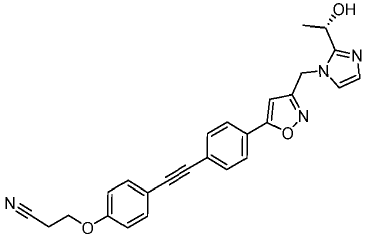 | (S)-3-(4-((4-(3-((2-(1-hydr oxyethyl)-1H-imidazol-1-yl)methyl)isoxazol-5-yl) phenyl)ethynyl)phenoxy) propanenitrile |
| Example 79 | 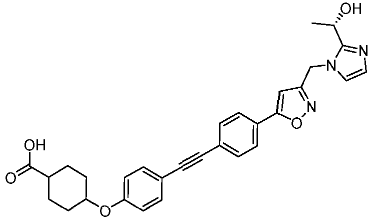 | (S)-4-(4-((4-(3-((2-(1-hydr oxyethyl)-1H-imidazol-1-yl)methyl)isoxazol-5-yl) phenyl)ethynyl)phenoxy) cyclohexane-1-carboxylic acid |
| Example 80 | 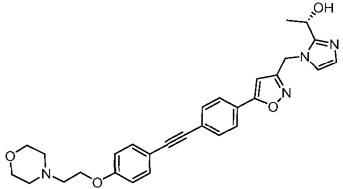 | (S)-1-(1-((5-(4-((4-(2-mor pholinoethoxy)phenyl)eth ynyl)phenyl)isoxazol-3-yl )methyl)-1H-imidazol-2-yl )ethan-1-ol |
| Example 81 | 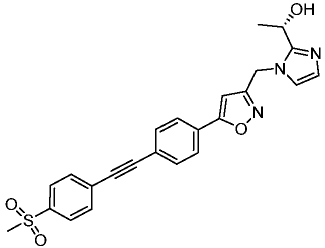 | (S)-1-(1-((5-(4-((4-(methy lsulfonyl)phenyl)ethynyl) phenyl)isoxazol-3-yl)meth yl)-1H-imidazol-2-yl)ethan -1-ol |
| Example 82 | 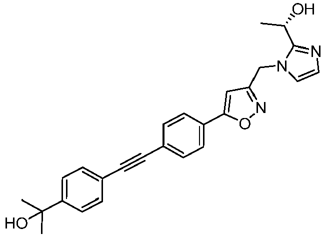 | (S)-2-(4-((4-(3-((2-(1-hydr oxyethyl)-1H-imidazol-1-yl)methyl)isoxazol-5-yl) phenyl)ethynyl)phenyl)pro pan-2-ol |
| Example 83 | 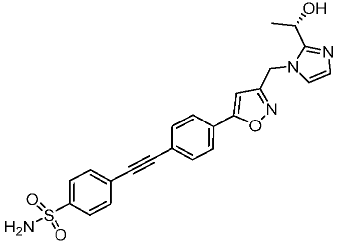 | (S)-4-((4-(3-((2-(1-hydrox yethyl)-1H-imidazol-1-yl) methyl)isoxazol-5-yl)phen yl)ethynyl)benzenesulfona mide |
| Example 84 | 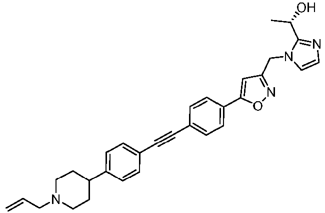 | (S)-1-(1-((5-(4-((4-(1-allyl piperidin-4-yl)phenyl)ethy nyl)phenyl)isoxazol-3-yl) methyl)-1H-imidazol-2-yl )ethan-1-ol |
| Example 85 | 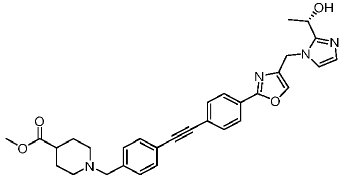 | Methyl (S)-1-(4-((4-(4-((2-(1-hydr oxyethyl)-1H-imidazol-1-yl)methyl)oxazol-2-yl)phe nyl)ethynyl)benzyl)piperi din-4-carboxylate |
| Example 86 | 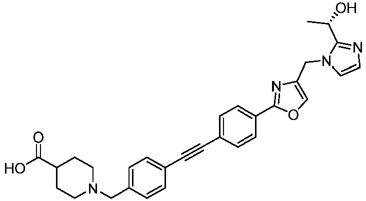 | (S)-1-(4-((4-(4-((2-(1-hydr oxyethyl)-1H-imidazol-1-yl)methyl)oxazol-2-yl)phe nyl)ethynyl)benzyl) piperidin-4-carboxylic acid |
| Example 87 | 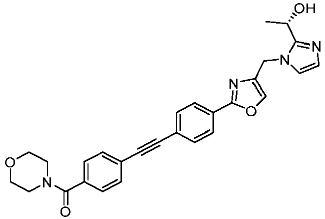 | (S)-(4-((4-(4-((2-(1-hydro xyethyl)-1H-imidazol-1-yl )methyl)oxazol-2-yl)phen yl)ethynyl)phenyl)(morph olinyl)methanone |
| Example 88 | 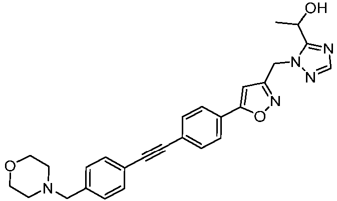 | 1-(1-((5-(4-((4-(morpholin omethyl)phenyl)ethynyl) phenyl)isoxazol-3-yl)methyl )-1H-1,2,4-triazol-5-yl) ethan-1-ol |
| Example 89 | 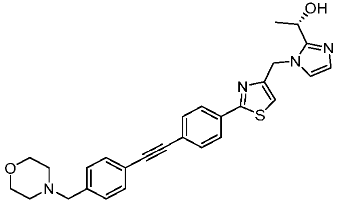 | (S)-1-(1-((2-(4-((4-(morph olinomethyl)phenyl)ethyn yl)phenyl)thiazol-4-yl)met hyl)-1H-imidazol-2-yl)ethan -1-ol |
| Example 90 | 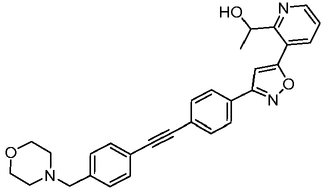 | 1-(3-(3-(4-((4-(morpholin omethyl)phenyl)ethynyl) phenyl)isoxazol-5-yl)pyridin -2-yl)ethan-1-ol |
| Example 91 | 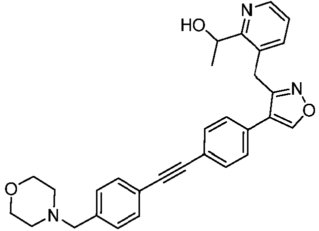 | 1-(3-((4-(4-((4-(morpholin omethyl)phenyl)ethynyl) phenyl)isoxazol-3-yl)methyl )pyridin-2-yl)ethan-1-ol |
| Example 92 | 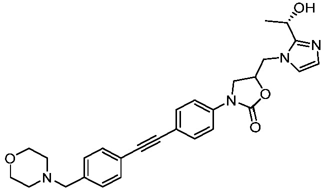 | 5-((2-((S)-1-hydroxyethyl) -1H-imidazol-1-yl)methyl )-3-(4-((4-(morpholinylme thyl)phenyl)ethynyl)phenyl )oxazolidin-2-one |
| Example 93 | 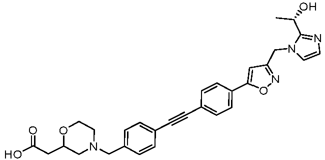 | 2-(4-(4-((4-(3-((2-((S)-1-hydroxyethyl)-1H-imidazol -1-yl)methyl)isoxazol-5-yl )phenyl)ethynyl)benzyl) morpholin-2-yl)acetic acid |
| Example 94 | 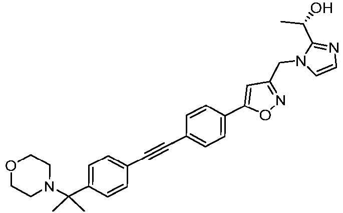 | (S)-1-(1-((5-(4-((4-(2-mor pholinoprop-2-yl)phenyl) ethynyl)phenyl)isoxazol-3-yl)methyl)-1H-imidazol-2 -yl)ethan-1-ol |
| Example 95 | 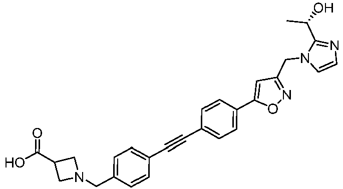 | (S)-1-(4-((4-(3-((2-(1-hydr oxyethyl)-1H-imidazol-1-yl)methyl)isoxazol-5-yl) phenyl)ethynyl)benzyl)azet idin-3-carboxylic acid |
| Example 96 | 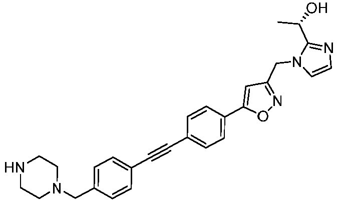 | (S)-1-(1-((5-(4-((4-(pipera zin-1-ylmethyl)phenyl)eth ynyl)phenyl)isoxazol-3-yl )methyl)-1H-imidazol-2-yl)ethan-1-ol |
| Example 97 | 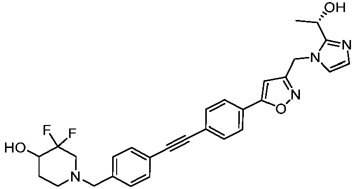 | 3,3-Difluoro-1-(4-((4-(3-(( 2-((S)-1-hydroxyethyl)-1H -imidazol-1-yl)methyl)is oxazol-5-yl)phenyl)ethynyl )benzyl)piperidin-4-ol |
| Example 98 | 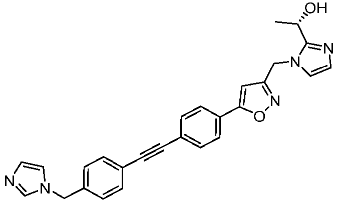 | (S)-1-(1-((5-(4-((4-((1H-imidazol-1-yl)methyl)phen yl)ethynyl)phenyl)isoxazol -3-yl)methyl)-1H-imidazol -2-yl)ethan-1-ol |
| Example 99 | 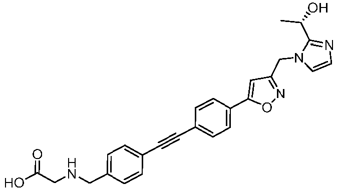 | (S)-(4-((4-(3-((2-(1-hydro xyethyl)-1H-imidazol-1-yl )methyl)isoxazol-5-yl) phenyl)ethynyl)benzyl)glycinate |
| Example 100 | 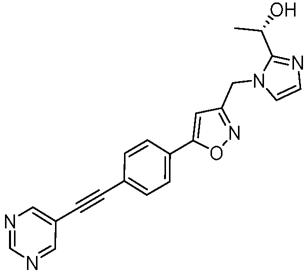 | (S)-1-(1-((5-(4-(pyrimidin -5-ylethynyl)phenyl)isoxa zol-3-yl)methyl)-1H-imid azol-2-yl)ethan-1-ol |
| Example 101 | 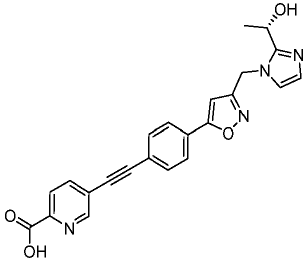 | (S)-5-((4-(3-((2-(1-hydrox yethyl)-1H-imidazol-1-yl) methyl)isoxazol-5-yl)phen yl)ethynyl)picolinic acid |
| Example 102 | 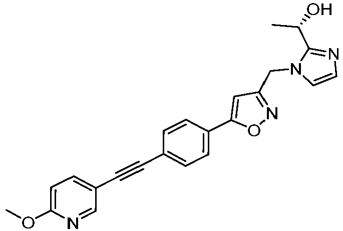 | (S)-1-(1-((5-(4-((6-methox ypyridin-3-yl)ethynyl)phe nyl)isoxazol-3-yl)methyl)-1H-imidazol-2-yl)ethan-1-ol |
| Example 103 | 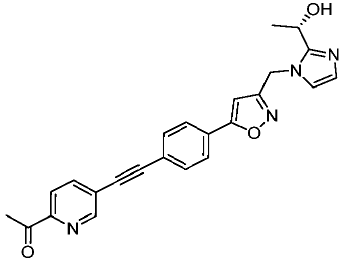 | (S)-1-(5-((4-(3-((2-(1-hydr oxyethyl)-1H-imidazol-1-yl)methyl)isoxazol-5-yl)p henyl)ethynyl)pyridin-2-yl )ethan-1-one |
| Example 104 | 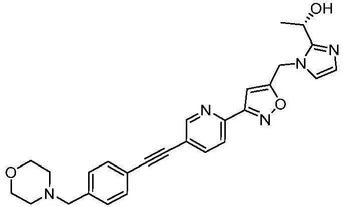 | (S)-1-(1-((3-(5-((4-(morph olinomethyl)phenyl)ethyn yl)pyridin-2-yl)isoxazol-5 -yl)methyl)-1H-imidazol-2-yl)ethan-1-ol |
| Example 105 | 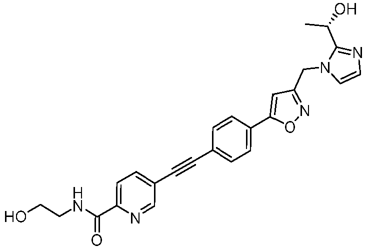 | (S)-N-(2-hydroxyethyl)-5-((4-(3-((2-(1-hydroxyethyl )-1H-imidazol-1-yl)methyl )isoxazol-5-yl)phenyl)eth ynyl)picolinamide |
| Example 106 | 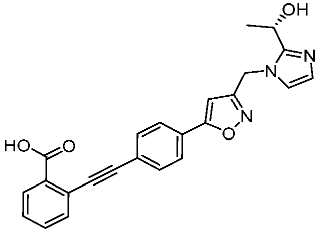 | (S)-2-((4-(3-((2-(1-hydrox yethyl)-1H-imidazol-1-yl) methyl)isoxazol-5-yl)phen yl)ethynyl)benzoic acid |
| Example 107 | 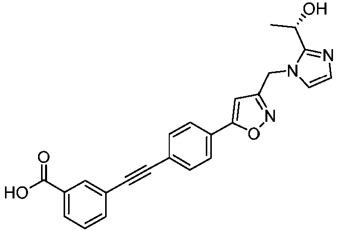 | (S)-3-((4-(3-((2-(1-hydrox yethyl)-1H-imidazol-1-yl) methyl)isoxazol-5-yl)phen yl)ethynyl)benzoic acid |
| Example 108 | 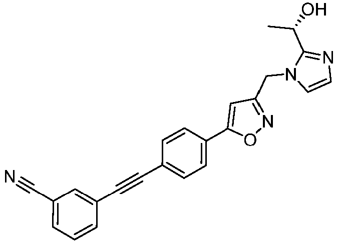 | (S)-3-((4-(3-((2-(1-hydrox yethyl)-1H-imidazol-1-yl) methyl)isoxazol-5-yl)phen yl)ethynyl)benzonitrile |
| Example 109 | 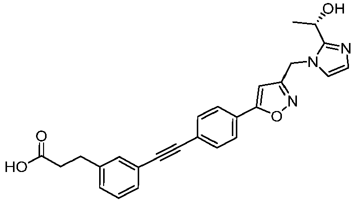 | (S)-3-(3-((4-(3-((2-(1-hydr oxyethyl)-1H-imidazol-1-yl)methyl)isoxazol-5-yl) phenyl)ethynyl)phenyl) propanoic acid |
| Example 110 | 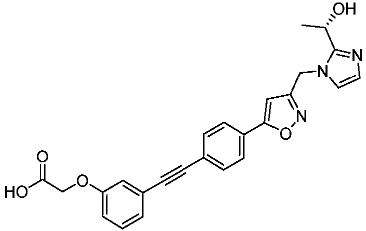 | (S)-2-(3-((4-(3-((2-(1-hydr oxyethyl)-1H-imidazol-1-yl)methyl)isoxazol-5-yl)p henyl)ethynyl)phenoxy)ac etic acid |
| Example 111 | 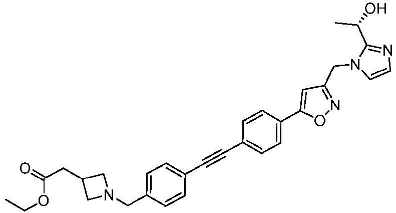 | Ethyl (S)-2-(1-(4-((4-(3-((2-(1-h ydroxyethyl)-1H-imidazol -1-yl)methyl)isoxazol-5-yl )phenyl)ethynyl)benzyl)az etidin-3-yl)acetate |
| Example 112 | 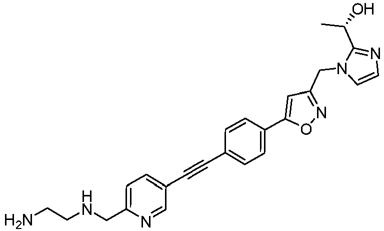 | (S)-1-(1-((5-(4-((6-((2-ami noethyl)amino)methyl)pyr idin-3-yl)ethynyl)phenyl)i soxazol-3-yl)methyl)-1H-i midazol-2-yl)ethan-1-ol |
| Example 113 | 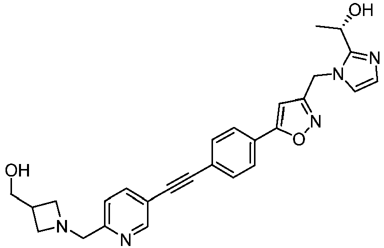 | (S)-1-(1-((5-(4-((6-((3-(hy droxymethyl)azetidin-1-yl )methyl)pyridin-3-yl)ethy nyl)phenyl)isoxazol-3-yl) methyl)-1H-imidazol-2-yl )ethan-1-ol |
| Example 114 | 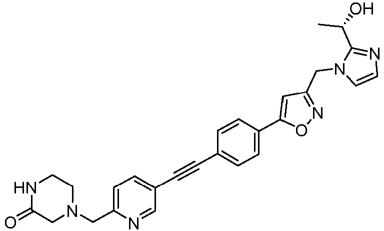 | (S)-4-((5-((4-(3-((2-(1-hyd roxyethyl)-1H-imidazol-1-yl)methyl)isoxazol-5-yl)p henyl)ethynyl)pyridin-2-yl )methyl)piperazin-2-one |
| Example 115 | 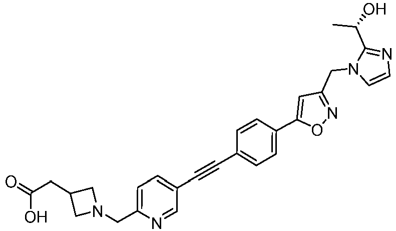 | (S)-2-(1-((5-((4-(3-((2-(1-hydroxyethyl))-1H-imidaz ol-1-yl)methyl)isoxazol-5-yl)phenyl)ethynyl)pyridin-2-yl)methyl)azetidin-3-yl) acetic acid |
| Example 116 | 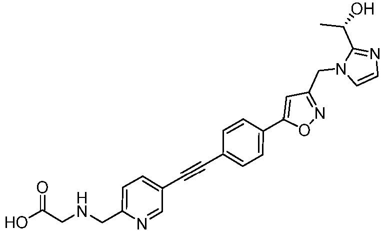 | (S)-((5-((4-(3-((2-(1-hydro xyethyl)-1H-imidazol-1-yl )methyl)isoxazol-5-yl)phe nyl)ethynyl)pyridin-2-yl) methyl)glycinate |
| Example 117 | 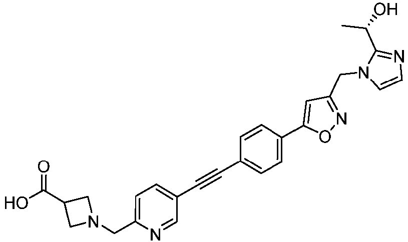 | (S)-1-((5-((4-(3-((2-(1-hyd roxyethyl)-1H-imidazol-1-yl)methyl)isoxazol-5-yl)p henyl)ethynyl)pyridin-2-yl )methyl)azetidin-3-carbox ylic acid |
| Example 118 | 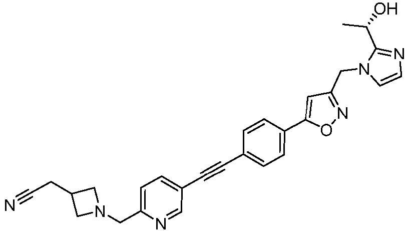 | (S)-2-(1-((5-((4-(3-((2-(1-hydroxyethyl))-1H-imidaz ol-1-yl)methyl)isoxazol-5-yl)phenyl)ethynyl)pyridin-2-yl)methyl)azetidin-3-yl) acetonitrile |
| Example 119 | 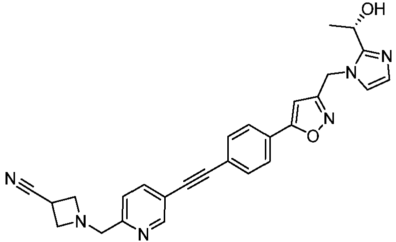 | (S)-1-((5-((4-(3-((2-(1-hyd roxyethyl)-1H-imidazol-1-yl)methyl)isoxazol-5-yl)p henyl)ethynyl)pyridin-2-yl )methyl)azetidin-3-carbon itrile |
| Example 120 | 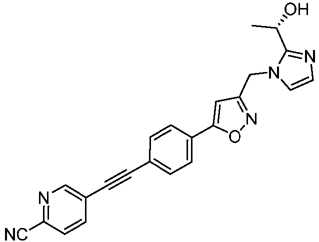 | (S)-5-((4-(3-((2-(1-hydrox yethyl)-1H-imidazol-1-yl) methyl)isoxazol-5-yl)phen yl)ethynyl)picolinonitrile |
| Example 121 | 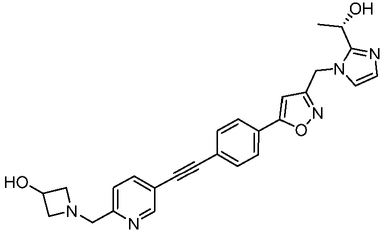 | (S)-1-((5-((4-(3-((2-(1-hyd roxyethyl)-1H-imidazol-1-yl)methyl)isoxazol-5-yl)p henyl)ethynyl)pyridin-2-yl )methyl)azetidin-3-ol |
| Example 122 | 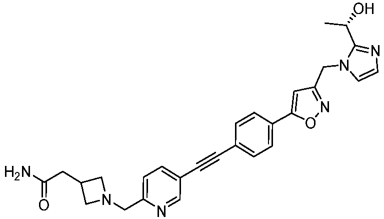 | (S)-2-(1-((5-((4-(3-((2-(1-hydroxyethyl))-1H-imidaz ol-1-yl)methyl)isoxazol-5-yl)phenyl)ethynyl)pyridin-2-yl)methyl)azetidin-3-yl) acetamide |
| Example 123 | 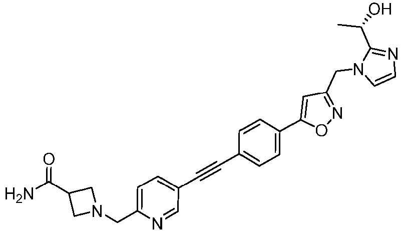 | (S)-1-((5-((4-(3-((2-(1-hyd roxyethyl)-1H-imidazol-1-yl)methyl)isoxazol-5-yl)p henyl)ethynyl)pyridin-2-yl )methyl)azetidin-3-carbox amide |

or a stereoisomer, tautomer or pharmaceutically acceptable salt thereof.

Note: If there is a difference between a drawn structure and the name given to that structure, the drawn structure shall prevail.

Furthermore, the present disclosure provides a pharmaceutical composition comprising an effective amount of the compound represaented by general formula (A-I), (I), (II-1), (II-2), (II-3), (II-4), (II-5), (III-1) or (III-2) or a stereoisomer, tautomer or pharmaceutically acceptable salt thereof, and a pharmaceutically acceptable carrier.

The present disclosure provides use of the compound represented by general formula (A-I), (I), (II-1), (II-2), (II-3), (II-4), (II-5), (III-1) or (III-2) or a stereoisomer, tautomer or pharmaceutically acceptable salt thereof, or the pharmaceutical composition thereof in the manufacture of an LPXC inhibitor.

The present disclosure also provides use of the compound represented by general formula (A-I), (I), (II-1), (II-2), (II-3), (II-4), (II-5), (III-1) or (III-2) or a stereoisomer, tautomer or pharmaceutically acceptable salt thereof, or the pharmaceutical composition thereof in the manufacture of a medicament for treating a disease mediated by LPXC, wherein the disease mediated by LPXC is preferably a bacterial infection caused by a Gram-negative bacterium; wherein the disease mediated by LPXC is a bacterial infection caused by a Gram-negative bacterium selected from the group consisting of *Escherichia coli, Pseudomonas aeruginosa, Proteus, Shigella dysenteriae, Klebsiella Pneumoniae, Brucella, Salmonella typhi, Acinetobacter, Yersinia, Legionella pneumophila, Bordetella pertussis, Shigella, Pasteurella, Vibrio cholerae,* and *Neisseria meningitidis.*

The present disclosure also provides use of the compound represented by general formula (A-I), (I), (II-1), (II-2), (II-3), (II-4), (II-5), (III-1) or (III-2) or a stereoisomer, tautomer or pharmaceutically acceptable salt thereof, or the pharmaceutical composition thereof in the manufacture of a medicament for treating a bacterial infection caused by a Gram-negative bacterium.

The present disclosure provides use of the compound represented by general formula (A-I), (I), (II-1), (II-2), (II-3), (II-4), (II-5), (III-1) or (III-2) or a stereoisomer, tautomer or pharmaceutically acceptable salt thereof, or the pharmaceutical composition thereof in the manufacture of a medicament for treating a bacterial infection caused by a Gram-negative bacterium selected from the group consisting of *Escherichia coli, Pseudomonas aeruginosa, Proteus, Shigella dysenteriae, Klebsiella Pneumoniae, Brucella, Salmonella typhi, Acinetobacter, Yersinia, Legionella pneumophila, Bordetella pertussis, Shigella, Pasteurella, Vibrio cholerae,* and *Neisseria meningitidis.*

The present disclosure also provides a method for treating a disease mediated by LPXC, comprising administering the compound represented by general formula (A-I), (I), (II-1), (II-2), (II-3), (II-4), (II-5), (III-1) or (III-2) or a stereoisomer, tautomer or pharmaceutically acceptable salt thereof, or the pharmaceutical composition thereof to a subject in need thereof. Preferably, the disease mediated by LPXC is a bacterial infection caused by a Gram-negative bacterium; more preferably, the Gram-negative bacterium is selected from the groupa consisting of *Escherichia coli, Pseudomonas aeruginosa, Proteus, Shigella dysenteriae, Klebsiella Pneumoniae, Brucella, Salmonella typhi, Acinetobacter, Yersinia, Legionella pneumophila, Bordetella pertussis, Shigella, Pasteurella, Vibrio cholerae,* and *Neisseria meningitidis.*

### Detailed description of the invention

Unless otherwise stated, some terms used in the description and claims of the present disclosure are defined as follows:

"Alkyl" as a group or a part of a group refers to a C₁-C₂₀ straight chain or a branched aliphatic hydrocarbon group. It is preferably C₁-C₁₀ alkyl, more preferably C₁-C₆ alkyl, or C₁-C₄ alkyl. Examples of alkyl include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. Alkyl may be substituted or unsubstituted.

"Alkenyl" refers to alkyl as defined above consisting of at least two carbon atoms and at least one carbon-carbon double bond. Representative examples of alkenyl include, but are not limited to, ethenyl, 1-propenyl, 2-propenyl, 1-, 2- or 3-butenyl and the like. It is preferably C₂-C₄ alkenyl. Alkenyl may be optionally substituted or unsubstituted.

"Cycloalkyl" refers to non-aromatic cyclic alkyl, including monocyclic, polycyclic, fused, bridged and spirocyclic rings, preferably a 5- to 7-membered monocyclic ring or a 7- to 10-membered bicyclic or tricyclic ring. Examples of "cycloalkyl" include, but are not limited to, cyclopropyl, cyclopentyl, cyclobutyl, cyclohexane. Cycloalkyl may be substituted or unsubstituted. It is preferably C₃-C₇ cycloalkyl, C₃-C₆ cycloalkyl or C₅-C₇ cycloalkyl.

"Spirocycloalkyl" refers to an all-carbon polycyclic group with 5 to 18 members and two or more cyclic structures, in which the single rings share one carbon atom (called a spiro atom) with each other, and the ring may contain one or more double bonds, but none of the rings have an aromatic system with fully conjugated π electrons. It is preferably 6- to 14-membered, more preferably 7 to 10-membered. According to the number of shared spiro atoms between the rings, the spirocycloalkyl is divided into monospiro, bispiro or polyspiro cycloalkyl, preferably monospiro and bispiro cycloalkyl, preferably 4-membered/5-membered, 4-membered/6-membered, 5-membered/5-membered or 5-membered/6-membered. Non-limiting examples of "spirocycloalkyl" include, but are not limited to, spiro[4.5]decyl, spiro[4.4]nonyl, spiro[3.5]nonyl, and spiro[2.4]heptyl.

"Fused cycloalkyl" refers to an all-carbon polycyclic group with 5 to 18 members and two or more cyclic structures, in which one or more rings may contain one or more double bonds, but none of the rings have an aromatic system with fully conjugated π electrons, preferably 6- to 12-membered, and more preferably 7- to 10-membered. According to the number of consituent rings, fused cycloalkyl can be divided into bicyclic, tricyclic, tetracyclic or polycyclic fused cycloalkyl, preferably bicyclic or tricyclic, more preferably 5-membered/5-membered or 5-membered/6-membered bicyclic alkyl. Non-limiting examples of "fused cycloalkyl" include, but are not limited to, bicyclo[3.1.0]hexyl, bicyclo[3.2.0]hept-1-enyl, bicyclo[3.2.0]heptyl, decalinyl or tetradecahydrophenanthyl.

"Bridged cycloalkyl" refers to an all-carbon polycyclic group with 5 to 18 members and two or more cyclic structures which share two carbon atoms that are not directly connected to each other, in which one or more rings may contain one or more double bonds, but none of the rings have an aromatic system with fully conjugated π electrons, preferably 6- to 12-membered, more preferably 7- to 10-membered. It is preferably 6- to 14-membered, more preferably 7- to 10-membered. According to the number of constituent rings, bridged cycloalkyl can be divided into bicyclic, tricyclic, tetracyclic or polycyclic bridged cycloalkyl, preferably bicyclic, tricyclic or tetracyclic, more preferably bicyclic or tricyclic. Non-limiting examples of "bridged cycloalkyl" include, but are not limited to, (1s,4s)-bicyclo[2.2.1]heptyl, bicyclo[3.2.1]octyl, (1s,5s)-bicyclo[3.3.1]nonyl, bicyclo[2.2.2]octyl, (1r,5r)-bicyclo[3.3.2]decyl.

"Heterocyclyl", "heterocycloalkyl", "heterocycle" or "heterocyclic" are used interchangeably in this application and all refer to non-aromatic heterocyclyl, in which one or more ring atoms are selected from the group consisting of heteroatoms of nitrogen, oxygen and S(O)ᵣ (where r is selected from 0, 1 or 2), including monocyclic, polycyclic, fused, bridged and spirocyclic rings. It is preferably a 5- to 7-membered monocyclic ring or a 7- to 10-membered bicyclic or tricyclic ring, which may contain 1, 2 or 3 atoms selected from the group consisting of nitrogen, oxygen, and/or sulfur. Examples of "heterocyclyl" include, but are not limited to, morpholinyl, oxetanyl, azetidinyl, thiomorpholinyl, tetrahydrofuranyl, tetrahydropyranyl, 1,1-dioxo-thiomorpholinyl, piperidinyl, 2-oxo-piperidinyl, pyrrolidinyl, 2-oxo-pyrrolidinyl, piperazin-2-one, 8-oxa-3-aza-bicyclo[3.2.1]octyl, piperazinyl, hexahydropyrimidine, 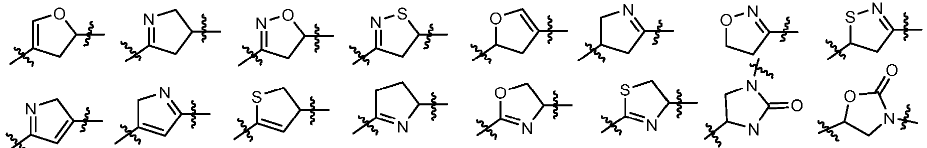 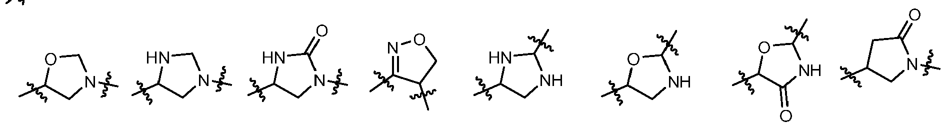 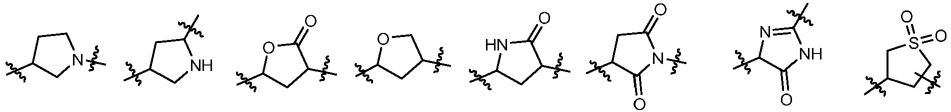 or

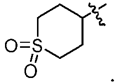

Heterocyclyl may be substituted or unsubstituted.

"Spiroheterocyclyl" refers to a polycyclic group with 5 to 18 members and two or more cyclic structures, in which the single rings share one atom with each other, and the ring contains one or more double bonds, but none of the rings have an aromatic system with fully conjugated π electrons, wherein one or more ring atoms are selected from the group consisting of heteroatoms of nitrogen, oxygen, and S(O)ᵣ (where r is selected from 0, 1, or 2), and the remaining ring atoms are carbon. It is preferably 6- to 14-memebered, more preferably 7 to 10-membered. According to the number of shared spiro atoms between the rings, the spirocycloalkyl is divided into monospiroheterocyclyl, bispiroheterocyclyl or polyspiroheterocyclyl, preferably monospiroheterocyclyl and bispiroheterocyclyl. It is more preferably a 4-membered/4-membered, 4-membered/5-membered, 4-membered/6-membered, 5-membered/5-membered or 5-membered/6-membered monospiroheterocyclyl. Non-limiting examples of "spiroheterocyclyl" include, but are not limited to, 1,7-dioxaspiro[4.5]decyl, 2-oxa-7-azaspiro[4.4]nonyl, 7-oxaspiro[3.5]nonyl, or 5-oxaspiro[2.4]heptyl.

"Fused heterocyclyl" refers to a polycyclic group containing two or more cyclic structures sharing a pair of atoms with each other, in which one or more rings may contain one or more double bonds, but none of the rings have an aromatic system with fully conjugated π electrons, wherein one or more ring atoms are selected from the group consisting of heteroatoms of nitrogen, oxygen, and S(O)ᵣ (where r is selected from 0, 1, or 2), and the remaining ring atoms are carbon. It is preferably 6- to 14-membered, more preferably 7 to 10-membered. According to the number of constituent rings, fused heterocyclyl can be divided into bicyclic, tricyclic, tetracyclic or polycyclic fused heterocyclyls, preferably bicyclic or tricyclic, more preferably 5-membered/5-membered or 5-membered/6-membered bicyclic fused heterocyclyl. Non-limiting examples of "fused heterocyclyl" include, but are not limited to, octahydropyrro[3,4-c]pyrrolyl, octahydro-1H-isoindolyl, 3-azabicyclo[3.1.0]hexyl, octahydrobenzo[b][1,4]dioxine.

"Bridged heterocyclyl" refers to a polycyclic group with 5 to 14 members, or 5 to 18 members, containing two or more cyclic structures which share two atoms that are not directly connected to each other, in which one or more rings may contain one or more double bonds, but none of the rings have an aromatic system with fully conjugated π electrons, wherein one or more ring atoms are selected from the group consisting of heteroatoms of nitrogen, oxygen, and S(O)ᵣ (where r is selected from 0, 1, or 2), and the remaining ring atoms are carbon. It is preferably 6-to 14-memebered, more preferably 7- to 10-memebered. According to the number of constituent rings, bridged heterocyclyl can be divided into bicyclic, tricyclic, tetracyclic or polycyclic bridged heterocyclyl, preferably bicyclic, tricyclic or tetracyclic, more preferably bicyclic or tricyclic. Non-limiting examples of "bridged heterocyclyl" include, but are not limited to, 2-azabicyclo[2.2.1]heptyl, 2-azabicyclo[2.2.2]octyl, or 2-azabicyclo[3.3.2]decyl.

"Aryl" refers to a carbocyclic aromatic system containing one or two rings, wherein the rings may be connected together in a fused manner. The term "aryl" includes monocyclic or bicyclic aryl, such as aromatic groups of phenyl, naphthyl, and tetrahydronaphthyl. Preferably aryl is C₆-C₁₀ aryl, more preferably phenyl and naphthyl, and most preferably naphthyl. Aryl may be substituted or unsubstituted.

"Heteroaryl" refers to an aromatic 5- to 6-membered monocyclic ring or 8- to 10-membered bicyclic ring, which may contain 1 to 4 atoms selected from the group consisting of nitrogen, oxygen and/or sulfur. Preferably heteroaryl is C₆-C₁₀ heteroaryl containing 1 to 3 heteroatoms selected from the group consisting of nitrogen, oxygen and sulfur. Examples of "heteroaryl" include, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothienyl, benzimidazolyl, indolyl, isoindolyl, 1,3-dioxo-isoindolyl, quinolyl, indazolyl, benzisothiazolyl, benzoxazolyl, benzisoxazolyl, isothiazolyl, 1H-1,2,4-triazolyl, 4H-1,2,4-triazolyl, pyridyl, pyrimidinyl, pyrazin-2(1H)-onyl, pyrimidin-4(3H)-onyl, pyridazin-3(2H)-onyl, 1H-indolyl, 1H-benzo[d]imidazolyl, 1H-pyrrolo[2,3-c]pyridyl, 3H-imidazo[4,5-c]pyridyl, isoquinolinyl, quinazolinyl, 2H-isoindolyl, furan[3,2-b]pyridyl, furan[2,3-c]pyridyl, thieno[2,3-c]pyridyl, benzofuryl, benzo[b]thienyl, 1H-pyrrolo[3,2-b]pyridyl, or 2H-pyrrolo[3,4-c]pyridyl. Heteroaryl may be substituted or unsubstituted.

"Alkoxy" refers to a group of (alkyl-O-), wherein alkyl is as defined herein. C₁-C₆ or C₁-C₄ alkoxy are preferred. Examples of alkoxy include, but are not limited to, methoxy, ethoxy, n-propoxy, isopropoxy, n-butoxy, isobutoxy, tert-butoxy, etc.

"Nitro" refers to -NO₂.

"Hydroxy" refers to -OH.

"Halogen" refers to fluorine, chlorine, bromine and iodine.

"Amino" refers to -NH₂.

"Cyano" refers to -CN.

"Benzyl" refers to -CH₂-phenyl.

"Carboxyl" refers to -C(O)OH.

"Carboxylate" refers to -C(O)O-alkyl or -C(O)O-cycloalkyl, wherein alkyl and cycloalkyl are as defined above.

"Hydroxyalkyl" refers to hydroxyl-substituted alkyl, wherein alkyl is as defined above.

"Aminoalkyl" refers to amino-substituted alkyl, wherein alkyl is as defined above.

"Haloalkyl" refers to halogen-substituted alkyl, wherein alkyl is as defined above.

"Haloalkoxy" refers to halogen-substituted alkoxy, wherein alkoxy is as defined above.

"DMSO" refers to dimethyl sulfoxide.

"BOC" refers to tert-butoxycarbonyl.

"Bn" refers to benzyl.

"THP" refers to 2-tetrahydropyranyl.

"TFA" refers to trifluoroacetic acid.

"Ts" refers to p-toluenesulfonyl.

"Leaving group" is an atom or functional group that is separated from a large molecule in a chemical reaction, and is a term used in nucleophilic substitution reactions and elimination reactions. In a nucleophilic substitution reaction, the reactant attacked by the nucleophile is called a substrate, and the atom or atomic group with a pair of electrons that breaks away from the substrate molecule is called a leaving group. Groups that are easy to accept electrons and have strong ability to withstand negative charges are good leaving groups. When the pKa of the conjugated acid of the leaving group is small, the leaving group is more likely to break away from other molecules. The reason is that when the pKa of the conjugate acid of the leaving group is small, the corresponding leaving group does not need to combine with other atoms, and the tendency to exist in the form of anion (or electrically neutral leaving group) increases. Common leaving groups include, but are not limited to, halogen, methanesulfonyl, -OTs or -OH.

"Substituted" means that one or more hydrogen atoms in a group, preferably up to 5, more preferably 1 to 3 hydrogen atoms, are independently substituted with a corresponding number of substituents. It goes without saying that the substituents are only in their possible chemical positions, and those skilled in the art are able to determine (either experimentally or theoretically) possible or impossible substitutions without making undue effort. For example, an amino or hydroxyl group with a free hydrogen may be unstable when combined with a carbon atom with an unsaturated (e.g., olefinic) bond.

"Substitution" or "substituted" mentioned in this specification, unless otherwise specified, means that the group can be substituted by one or more groups selected from the group consisting of: alkyl, alkoxy, alkylthio, alkylamino, halogen, sulfhydryl, hydroxyl, nitro, cyano, cycloalkyl, heterocyclyl, aryl, heteroaryl, cycloalkoxy, heterocycloalkoxy, cycloalkylthio, heterocycloalkylthio, amino, haloalkyl, hydroxyalkyl, carboxyl, carboxylate, -C(O)R₆, -C(O)OR₆, -OC(O)R₆, -NR₇R₈, -C(O)NR₇R₈, -SO₂NR₇R₈, -SO₂R₆, -NR₇C(O)R₈, -NR₁₂R₁₃, -C(O)NR₁₂R₁₃, -SO₂NR₁₂R₁₃ and -NR₁₂C(O)R₁₃;
R₆ is selected from the group consisting of a hydrogen atom, alkyl, cycloalkyl, heterocyclyl, aryl and heteroaryl, wherein the alkyl, cycloalkyl, heterocyclyl, aryl or heteroaryl is optionally further substituted with one or more substituents selected from the group consisting of hydroxyl, halogen, nitro, cyano, alkyl, alkoxy, haloalkyl, haloalkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
R₇, R₈, R₁₂ and R₁₃ are each independently selected from the group consisting of a hydrogen atom, hydroxyl, halogen, alkyl, alkoxy, cycloalkyl, heterocyclyl, aryl and heteroaryl, wherein the alkyl, alkoxy, cycloalkyl, heterocyclyl, aryl or heteroaryl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂R₉, -SO₂NR₁₀R₁₁ and -NR₁₀C(O)R₁₁;
alternatively, R₇ and R₈ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
alternatively, R₁₂ and R₁₃ together with the atom to which they are connected form 4- to 8-membered heterocyclyl, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, alkyl, alkoxy, cycloalkyl, heterocyclyl, aryl, heteroaryl, =O, -C(O)R₉, -C(O)OR₉, -OC(O)R₉, -NR₁₀R₁₁, -C(O)NR₁₀R₁₁, -SO₂NR₁₀R₁₁ and -NR₁₀C(O)R₁₁;
R₉, R₁₀ and R₁₁ are each independently selected from the group consisting of a hydrogen atom, alkyl, amino, cycloalkyl, heterocyclyl, aryl and heteroaryl, wherein the alkyl, cycloalkyl, heterocyclyl, aryl or heteroaryl is optionally further substituted with one or more substituents selected from the group consisting of hydroxyl, halogen, nitro, amino, cyano, alkyl, alkoxy, cycloalkyl, heterocyclyl, aryl, heteroaryl, carboxyl, and a carboxylate group.

"Pharmaceutically acceptable salt" refers to certain salts of the above compounds that can maintain their original biological activity and are suitable for medical use. The pharmaceutically acceptable salt of the compound represented by general formula (A-I) may be a metal salt or an amine salt formed with a suitable acid.

"Pharmaceutical composition" refers to a mixture of one or more compounds described herein, or physiologically pharmaceutically acceptable salts or prodrugs thereof, and other chemical components, as well as other components such as physiologically pharmaceutically acceptable carriers. The purpose of pharmaceutical compositions is to facilitate administration to living organisms and facilitate the absorption of active ingredients to exert biological activity.

### Synthetic methods of compounds of the present disclosure

**In** order to achieve the purpose of the present disclosure, the present disclosure adopts the following technical solutions:

The present disclosure provides a method for producing a compound represented by general formula (A-I) or a stereoisomer, tautomer or pharmaceutically acceptable salt thereof, comprising:

### Method One:

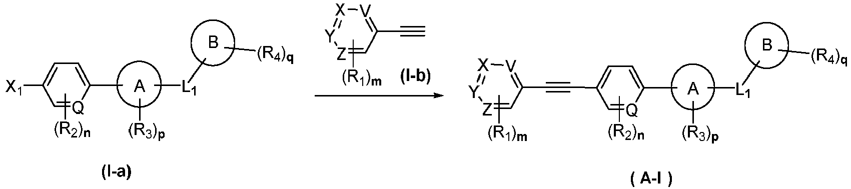

subjecting a compound represented by general formula (I-a) and a compound represented by general formula (I-b) to a coupling reaction under the action of a catalyst, and optionally further performing one or more steps of deprotection, hydrolysis, reduction, reductive amination or acid-amine condensation reaction to obtain the compound represented by general formula (A-I);
wherein:
   X₁ is halogen;
   ring A, ring B, X, Y, Z, V, Q, R₁-R₄, L₁, n, m, p, and q are as defined in the general formula (A-I).

### Method Two:

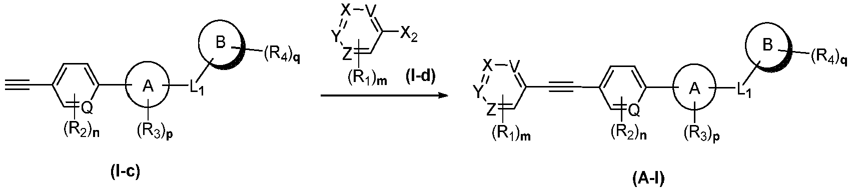

subjecting a compound represented by general formula (I-c) and a compound represented by (I-d) to a coupling reaction under the action of a catalyst, and optionally further performing one or more steps of deprotection, hydrolysis, reduction, reductive amination or acid-amine condensation reaction to obtain the compound represented by general formula (A-I);
wherein:
   X₂ is halogen;
   ring A, ring B, X, Y, Z, V, Q, R₁-R₄, L₁, n, m, p, and q are as defined in the general formula (A-I).

### Method Three:

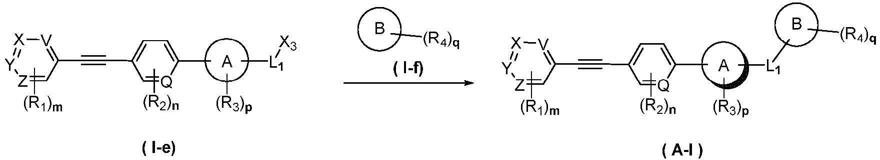

subjecting a compound represented by general formula (I-e) and a compound represented by (I-f) to a substitution reaction under the action of an alkaline reagent, and optionally further performing a deprotection reaction to obtain the compound represented by general formula (A-I);
wherein:
   X₃ is halogen;
   ring A, ring B, X, Y, Z, V, Q, R₁-R₄, L₁, n, m, p, and q are as defined in the general formula (A-I).

### BRIEF DESCRIPTION OF DRAWINGS

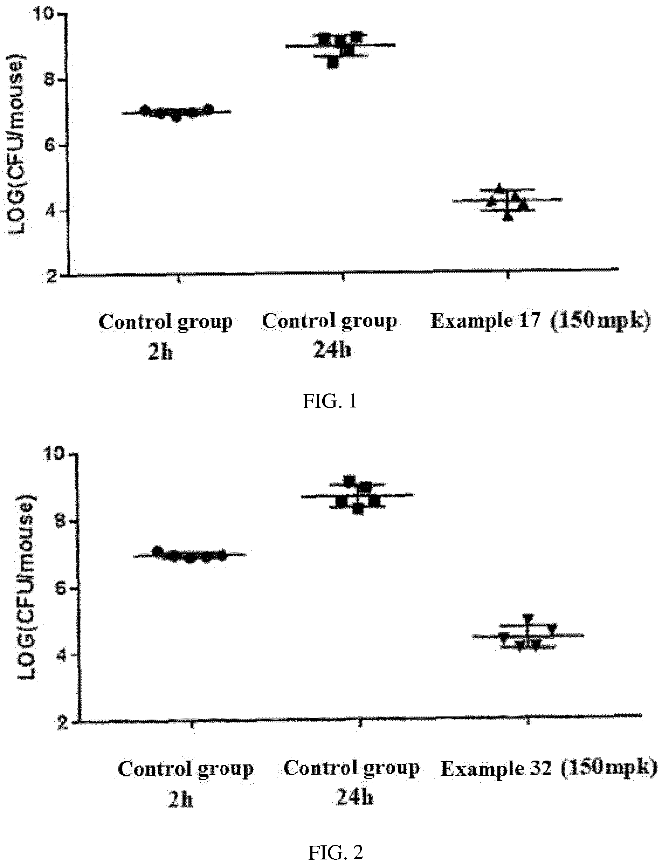

FIG. 1 shows changes in the log value of the total amount of Klebsiella Pneumoniae ATCC 51504 bacteria in the lungs after treatment with the compound of Example 17 of the present disclosure in Test Example 4.
FIG. 2 shows changes in the log value of the total amount of Klebsiella Pneumoniae ATCC 51504 bacteria in the lungs after treatment with the compound of Example 32 of the present disclosure in Test Example 4.

### DETAILED DESCRIPTION

The following examples are used to further describe the present disclosure, but these examples do not limit the scope of the present disclosure.

### Example

The examples provide the preparation of representative compounds represented by formula (A-I) and relevant structure identification data. It must be noted that the following examples are used to illustrate the present disclosure rather than limit the present disclosure. ¹H NMR spectrum was measured by Bruker instrument (400 MHz), and the chemical shift is expressed in ppm. Tetramethylsilane was used as internal standard (0.00 ppm). ¹H NMR is expressed as in a way of: s=singlet, d=doublet, t=triplet, m=multiplet, br=broadened, dd=doublet of doublet, and dt=doublet of triplet. The unit for a coupling constant provided is Hz.

Mass spectrum was measured by an LC/MS instrument, and ionization was carried out in a manner of ESI or APCI.

A Yantai Huanghai HSGF254 or Qingdao GF254 silica gel plate was used as a thin-layer chromatography silica gel plate. The silica gel plate used in thin-layer chromatography (TLC) has a specification of 0.15 mm-0.2 mm, and the silica gel plate used in thin-layer chromatography for separating and purifying products has a specification of 0.4 mm-0.5 mm.

In column chromatography, Yantai Huanghai 200-300 mesh silica gel was generally used as a carrier.

In the following examples, unless otherwise indicated, all temperatures are expressed in degree Celsius. Unless otherwise indicated, various starting materials and reagents were either commercially available or synthesized according to known methods, and commercially available materials and reagents were used directly without further purification. Unless otherwise specified, commercially available manufacturers include but are not limited to Aldrich Chemical Company, ABCR GmbH & Co.KG, Acros Organics, Guangzan Chemical Technology Co., Ltd., and Jingyan Chemical Technology Co., Ltd.

CD₃OD: Deuterated methanol.

CDCl₃: Deuterated chloroform.

DMSO-*d*₆: Deuterated dimethyl sulfoxide.

Argon gas atmosphere means that a reaction bottle is connected to an argon gas balloon with a volume of about 1 L.

In the examples, unless otherwise specified, the solution in the reaction refers to an aqueous solution.

The compounds were purified using a silica gel column chromatography eluent system and thin layer chromatography, wherein the eluent system was selected from the group consisting of A: petroleum ether and ethyl acetate system, B: dichloromethane and methanol system, and C: dichloromethane and ethyl acetate system; wherein the volume ratio of the solvents varies according to the polarity of the compound, and a small amount of acidic or alkaline reagents such as acetic acid, triethylamine, etc. can be added for adjustment.

### Example 1

### (S)-1-(1-((2-(4-((4-(morpholinomethyl)phenyl)ethynyl)phenyl)oxazol-4-yl)methyl)-1H-imidazol-2-yl)ethan-1-ol

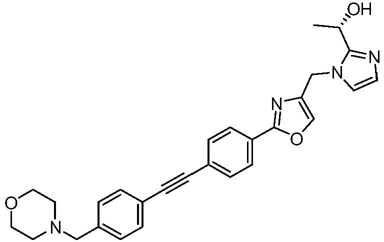

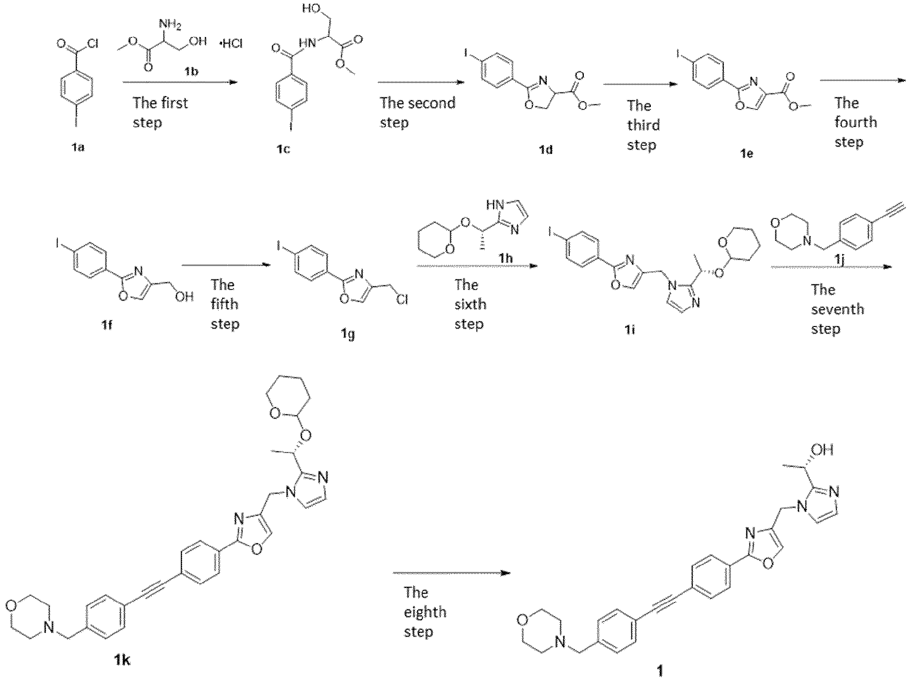

### The first step

### Methyl (4-iodobenzoyl)serine

Methyl serine hydrochloride **1b** (10.6 g, 68.13 mmol) and triethylamine (34.47 g, 340.66 mmol) were added into dichloromethane (230 mL). The mixture was then added with 4-iodobenzoyl chloride **1a** (19.97 g, 74.95 mmol) at 0°C, and warmed up to room temperature for 3 hours of reaction. The reaction solution was then added with water, and extracted with dichloromethane (30 mL×2). The aqueous layer was removed. The combined organic phase was washed with sodium bicarbonate aqueous solution followed by saturated sodium chloride solution, dried over anhydrous sodium sulfate, filtered, and concentrated under reduced pressure. The obtained residue was purified by silica gel column chromatography (eluent: System A) to obtain methyl (4-iodobenzoyl)serine **1c** (14 g) with a yield of 58.86%.
MS m/z (ESI): 350.0 [M+1]

### The second step

### Methyl 2-(4-iodophenyl)-4,5-dihydroxazol-4-carboxylate

Methyl (4-iodobenzoyl)serine **1c** (7 g, 20.05 mmol) and (methoxycarbonylsulfonyl)triethylammonium hydroxide (5.26 g, 22.06 mmol) were added to tetrahydrofuran (65 mL). The mixture was heated to reflux for 5 hours of reaction. After the reaction was completed, the reaction mixture was concentrated under reduced pressure, and the obtained residue was purified by silica gel column chromatography (eluent: System A) to obtain methyl 2-(4-iodophenyl)-4,5-dihydroxazol-4-carboxylate **1d** (5.75 g) with a yield of 86.61%.
MS m/z (ESI): 332.0 [M+1]

### The third step

### Methyl 2-(4-iodophenyl)oxazol-4-carboxylate

Methyl 2-(4-iodophenyl)-4,5-dihydroxazol-4-carboxylate **1d** (2 g, 6.04 mmol), 70% tert-butyl hydroperoxide aqueous solution (2.33 g, 18.12 mmol), 1,8-diazabicyclo[5.4.0]undec-7-ene (1.84 g, 12.08 mmol) and iodine (153.30 mg, 604.04 µmol) were added to tetrahydrofuran (50 mL) and heated to 60°C for 4 hours of reaction. The reaction solution was added with sodium thiosulfate aqueous solution, and extracted with ethyl acetate (30 mL×2). The aqueous layer was removed. The combined organic phase was washed with saturated sodium chloride solution (30 mL × 2), dried over anhydrous sodium sulfate, filtered, and concentrated under reduced pressure. The obtained residue was purified by silica gel column chromatography (eluent: System A) to obtain methyl 2-(4-iodophenyl)oxazol-4-carboxylate **1e** (550 mg) with a yield of 27.67%.
MS m/z (ESI): 330.0 [M+1]

### The fourth step

### (2-(4-Iodophenyl)oxazol-4-yl)methanol

Methyl 2-(4-iodophenyl)oxazol-4-carboxylate **1e** (850 mg, 2.58 mmol) was added to tetrahydrofuran (15 mL), and dropwise added with diisobutylaluminum hydride (1.0 M, 7.75 mL) at 0°C. The mixture was continued to react at 0°C for 2 hours, added with sodium potassium tartrate aqueous solution, then added with ethyl acetate, and stirred vigorously at room temperature for 3 hours. The reaction solution was extracted with ethyl acetate (30 mL×2). The aqueous layer was removed. The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain (2-(4-iodophenyl)oxazol-4-yl)methanol **1f** (650 mg) with a yield of 83.58%.
MS m/z (ESI): 302.0 [M+1]

¹H NMR (400 MHz, Chloroform-d) δ 7.75 (d, *J =* 8.5 Hz, 2H), 7.70 (d, *J =* 8.5 Hz, 2H), 7.59 (s, 1H), 4.62 (s, 2H).

### The fifth step

### 4-(Chloromethyl)-2-(4-iodophenyl)oxazole

At 0°C, (2-(4-iodophenyl)oxazol-4-yl)methanol 1f (660 mg, 2.19 mmol) was added to dichloromethane (10 mL), added with triethylamine (332.73 mg, 3.29 mmol, 457.05 µL), and then slowly added with methanesulfonyl chloride (251.11 mg, 2.19 mmol) dropwise for reaction at room temperature overnight. After the reaction was completed, the reaction mixture was extracted with dichloromethane and concentrated under reduced pressure. The obtained residue was purified by silica gel column chromatography (eluent: System A) to obtain 4-(chloromethyl)-2-(4-iodophenyl)oxazole **1g** (330 mg) with a yield of 47.11%.
MS m/z (ESI): 319.8 [M+1]

### The sixth step

### 2-(4-Iodophenyl)-4-((2-((1S)-1-((tetrahydro-2H-pyran-2-yl)oxy)ethyl)-1H-imidazol-1-y l)methyl)oxazole

2-((1S)-1-((tetrahydro-2H-pyran-2-yl)oxy)ethyl)-1H-imidazole **1h** (61.42 mg, 312.96 µmol, prepared according to patent WO 2018216822) was added to acetonitrile (10 mL), and then added with cesium carbonate (611.82 mg, 1.88 mmol) and 4-(chloromethyl)-2-(4-iodophenyl)oxazole **1g** (100 mg, 312.96 µmol). The mixture was heated to 100°C for 4 hours of reaction. The reaction solution was extracted with ethyl acetate (30 mL×2). The aqueous layer was removed. The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 2-(4-iodophenyl)-4-((2-((1S)-1-((tetrahydro-2H -pyran-2-yl)oxy)ethyl)-1H-imidazol-1-yl)methyl)oxazole **1i** (140 mg) with a yield of 93.2%.
MS m/z (ESI): 480.1 [M+1]

¹H NMR (400 MHz, Chloroform-d) δ 7.74 (dd, *J =* 8.6, 2.0 Hz, 2H), 7.66 (dd, *J =* 8.5, 1.7 Hz, 2H), 7.44 (d, *J =* 37.7 Hz, 1H), 7.01 (d, *J =* 1.3 Hz, 2H), 5.06-5.37 (m, 3H), 4.36-4.73 (m, 1H), 3.71-3.90 (m, 1H), 3.42 (tdd, *J =* 15.4, 6.6, 3.1 Hz, 1H), 1.68-1.89 (m, 2H), 1.59 (dd, *J =* 6.7, 5.1 Hz, 3H), 1.36-1.50 (m, 4H).

### The seventh step

### 4-(4-((4-(4-((2-((1S)-1-((tetrahydro-2H-pyran-2-yl)oxy)ethyl))-1H-imidazol-1-yl)methy l)oxazol-2-yl)phenyl)ethynyl)benzyl)morpholine

At room temperature, 2-(4-iodophenyl)-4-((2-((1S)-1-((tetrahydro-2H -pyran-2-yl)oxy)ethyl)-1H-imidazol-1-yl)methyl)oxazole **1i** (75 mg, 156.47 µmol), 4-(4-ethynylbenzyl)morpholine **1j** (31.49 mg, 156.47 µmol, prepared according to patent WO 2018208987), bis(triphenylphosphine)palladium dichloride (4.39 mg, 6.26 µmol), tetrabutylammonium bromide (50.44 mg, 156.47 µmol) and piperidine (39.97 mg, 469.42 µmol) were added to 1.5 mL of water. The mixture was heated to 70°C for 14 hours of reaction. The reaction solution was extracted with ethyl acetate (30 mL×2). The aqueous layer was removed. The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 4-(4-((4-(4-((2-((1S))-1-((tetrahydro-2H-pyran-2-yl)oxy)ethyl)-1H-imidazol-1-yl)methyl)oxazol-2-yl)phenyl)ethynyl)benzyl)morpholine **1k** (66 mg) with a yield of 76.32%.
MS m/z (ESI): 553.3 [M+1]

### The eighth step

### (S)-1-(1-((2-(4-((4-(morpholinomethyl)phenyl)ethynyl)phenyl)oxazol-4-yl)methyl)-1H-imidazol-2-yl)ethan-1-ol

4-(4-((4-(4-((2-((1S))-1-((tetrahydro-2H-pyran-2-yl)oxy)ethyl)-1H-imidazol-1-yl)methy l)oxazol-2-yl)phenyl)ethynyl)benzyl)morpholine **1k** (66 mg, 119.42 µmol) and trifluoroacetic acid (0.1 mL) were added to dichloromethane (2 mL) for 30 min of reaction at room temperature. After the reaction was completed, the reaction mixture was concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((2-(4-((4-(morpholinomethyl)phenyl)ethynyl) phenyl)oxazol-4-yl)methyl)-1H-imidazol-2-yl)ethan-1-ol 1 (10 mg) with a yield of 12.85%.
MS m/z (ESI): 469.2 [M+1]

¹H NMR (400 MHz, DMSO-*d*₆) δ 8.39 (s, 1H), 8.00 (d, *J =* 8.1 Hz, 2H), 7.71 (dd, *J* = 16.6, 8.1 Hz, 5H), 7.64 (s, 1H), 7.56 (d, *J =* 7.9 Hz, 2H), 6.33-6.43 (m, 1H), 5.76 (s, 2H), 5.49 (s, 2H), 5.35 (dd, *J =* 14.5, 6.6 Hz, 1H), 3.14 (d, *J =* 19.2 Hz, 4H), 2.42 (m, 4H), 1.56 (d, *J =* 6.6 Hz, 3H).

### Example 2

### (S)-1-(1-((5-(4-((4-(morpholinomethyl)phenyl)ethynyl)phenyl)isoxazol-3-yl)methyl)-1 H-imidazol-2-yl)ethan-1-ol

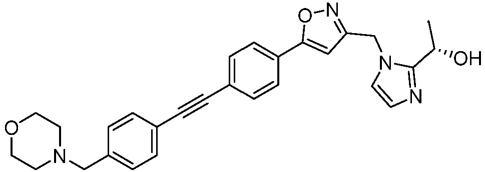

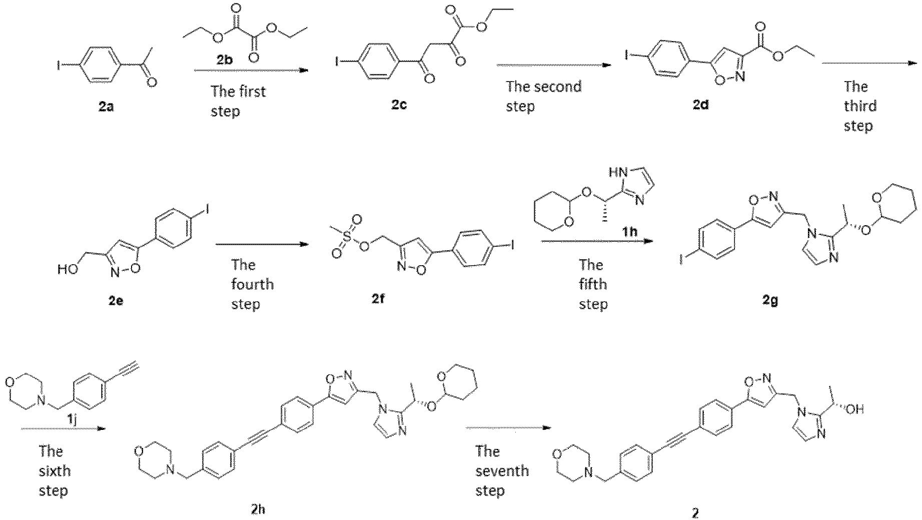

### The first step

### Ethyl 4-(4-iodophenyl)-2,4-dioxobutyrate

Sodium hydride (1.63 g, 40.64 mmol, 60% purity) was added to toluene (20 mL), added with 1-(4-iodophenyl)ethan-1-one **2a** (5 g, 20.32 mmol), heated to 50°C, dropwise added with the solution of diethyl oxalate **2b** (4.45 g, 30.48 mmol) in toluene, and heated to 50°C for 2 h of reaction. The reacrion mixture was cooled, poured into ice water, adjusted to acidity with 1M hydrochloric acid, and extracted with ethyl acetate (100 mL×2). The combined organic phase was washed with saturated brine (100 mL×3), dried over anhydrous sodium sulfate, and concentrated under reduced pressure. The obtained residue was purified by silica gel column chromatography (eluent: System A) to obtain ethyl 4-(4-iodophenyl)-2,4-dioxobutyrate **2c** (2.8 g) with a yield of 39.81%.
MS m/z (ESI): 347.0 [M+1]

### The second step

### Ethyl 5-(4-iodophenyl)isoxazol-3-carboxylate

Ethyl 4-(4-iodophenyl)-2,4-dioxobutyrate **2c** (2.8 g, 8.09 mmol) and hydroxylamine hydrochloride (1.69 g, 24.27 mmol) were added to ethanol (25 mL), and heated to reflux for 1 hour. After the reaction was completed, the reaction mixture was concentrated under reduced pressure, and dissolved in ethyl acetate (100 mL). The organic phase was washed with saturated brine (100 mL), dried over anhydrous sodium sulfate, and concentrated under reduced pressure. The obtained residue was purified by silica gel column chromatography (eluent: System A) to obtain ethyl 5-(4-iodophenyl)isoxazol-3-carboxylate **2d** (2.3 g) with a yield of 82.86%.
MS m/z (ESI): 343.8 [M+1]

### The third step

### (5-(4-Iodophenyl)isoxazol-3-yl)methanol

Ethyl 5-(4-iodophenyl)isoxazol-3-carboxylate **2d** (1.20 g, 3.50 mmol) was added to methanol (25 mL), added with sodium borohydride (198.46 mg, 5.25 mmol) in batches, and heated to 80°C for 4 hours of reaction. After the reaction was completed, the reaction mixture was added with ice water to quench the reaction, and extracted with ethyl acetate (50 mL×2). The organic phase was washed with saturated brine (50 mL), dried over anhydrous sodium sulfate, and concentrated under reduced pressure to obtain (5-(4-iodophenyl)isoxazol-3-yl)methanol **2e** (0.36 g) with a yield of 34.19%.
MS m/z (ESI): 302.0 [M+1]

### The fourth step

### Methyl (5-(4-iodophenyl)isoxazol-3-yl)methanesulfonate

(5-(4-Iodophenyl)isoxazol-3-yl)methanol **2e** (0.36 g, 1.20 mmol) and triethylamine (241.99 mg, 2.39 mmol, 333.31 µL) were added to dichloromethane (5 mL), cooled to 0°C, added with methanesulfonyl chloride (205.45 mg, 1.79 mmol) dropwise, and warmed up to room temperature for 4 hours of reaction. After the reaction was completed, the reaction mixture was added with water to quench the reaction, and extracted with dichloromethane (50 mL×3). The organic phases were combined and concentrated under reduced pressure. The obtained residue was purified by silica gel column chromatography (eluent: System A) to obtain methyl (5-(4-iodophenyl)isoxazol-3-yl)methanesulfonate **2f** (0.45 g) with a yield of 99.26%.
MS m/z (ESI): 379.8 [M+1]

### The fifth step

### 5-(4-Iodophenyl)-3-((2-((1S)-1-((tetrahydro-2H-pyran-2-yl)oxy)ethyl)-1H-imidazol-1-y l)methyl)isoxazole

2-((1S)-1-((tetrahydro-2H-pyran-2-yl)oxy)ethyl)-1H-imidazole **1h** (100 mg, 509.57 µmol) was added to N,N-dimethylformamide (2 mL), cooled to 0°C, added with sodium hydride (50.96 mg, 764.35 µmol, 60% purity) in batches, heated to room temperature for 1 hour of reaction, and added with methyl (5-(4-iodophenyl)isoxazol-3-yl)methanesulfonate **2f** (193.21 mg, 509.57 µmol) for 4 h of reaction at room temperature. After the reaction was completed, the reaction mixture was added with water to quench the reaction, and extracted with ethyl acetate (10 mL×3). The organic phases were combined and concentrated under reduced pressure. The obtained residue was purified by silica gel column chromatography (eluent: System A) to obtain 5-(4-iodophenyl)-3-((2-((1S)-1-((tetrahydro-2H-pyran-2-yl)oxy)ethyl)-1H-imidazol-1-yl)methyl)i soxazole **2g** (0.12 g) with a yield of 49.13%.
MS m/z (ESI): 480.1 [M+1]

### The sixth step

### 4-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)morpholine

4-(4-Ethynylbenzyl)morpholine **1j** (60 mg, 298.12 µmol), 5-(4-iodophenyl)-3-((2-((1S)-1-((tetrahydro-2H-pyran-2-yl)oxy)ethyl)-1H-imidazol-1-yl)methyl)i soxazole **2g** (142.89 mg, 298.12 µmol), triethylamine (90.50 mg, 894.35 µmol), bis(triphenylphosphine)palladium dichloride (68.86 mg, 59.62 µmol) and cuprous iodide (11.39 mg, 59.62 µmol) were added to N,N-dimethylformamide (2 mL), and the system was replaced with argon gas for reaction at room temperature for 3 hours. After the reaction was completed, the reaction mixture was added with water to quench the reaction, and extracted with ethyl acetate (10 mL×3). The organic phases were combined and concentrated under reduced pressure. The obtained residue was purified by silica gel column chromatography (eluent: System B) to obtain 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)morpholine **2h** (75 mg) with a yield of 45.52%.
MS m/z (ESI): 553.3 [M+1]

### The seventh step

### (S)-1-(1-((5-(4-((4-(morpholinomethyl)phenyl)ethynyl)phenyl)isoxazol-3-yl)methyl)-1 H-imidazol-2-yl)ethan-1-ol

4-(4-((4-(3-((2-((1S))-1-((tetrahydro-2H-pyran-2-yl)oxy)ethyl)-1H-imidazole-1-yl)meth yl)isoxazol-5-yl)phenyl)ethynyl)benzyl)morpholine **2h** (70 mg, 126.66 µmol) was dissolved in dioxane (2 mL), and then added with 1 mL of 4M hydrochloric acid in dioxane solution for 4 hours of reaction at room temperature. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-(morpholinomethyl)phenyl)ethynyl)phenyl)isoxazol-3-yl)methyl)-1H-imidazo l-2-yl)ethan-1-ol **2** (5 mg) with a yield of 6.16%.
MS m/z (ESI): 469.0 [M+1]

### Example 3

### (S)-1-(1-((3-(4-((4-(morpholinomethyl)phenyl)ethynyl)phenyl)isoxazol-5-yl)methyl)-1 H-imidazol-2-yl)ethan-1-ol

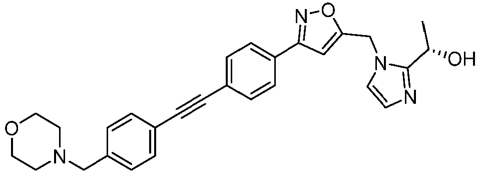

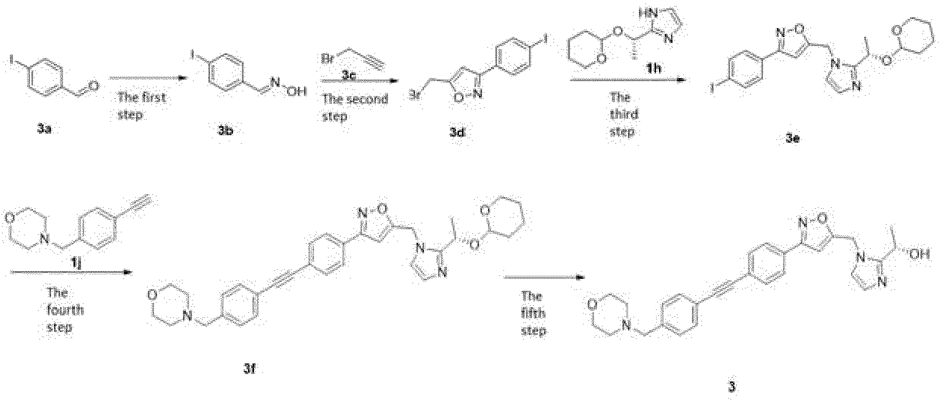

### The first step

### (E)-4-iodobenzaldehyde oxime

Hydroxylamine hydrochloride (5.99 g, 86.20 mmol), potassium carbonate (11.9 g, 86.20 mmol), 4-iodobenzaldehyde **3a** (10 g, 43.10 mmol) and water (80 mL) were sequentially added to ethanol (80 mL) for reaction at room temperature overnight. After the reaction was completed, the reaction mixture was extracted with ethyl acetate (100 mL×3). The organic phases were combined and concentrated under reduced pressure. The obtained residue was purified by silica gel column chromatography (eluent: System A) to obtain (E)-4-iodobenzaldehyde oxime **3b** (8 g) with a yield of 75.14%.
MS m/z (ESI): 247.8 [M+1]

### The second step

### 5-(Bromomethyl)-3-(4-iodophenyl)isoxazole

(E)-4-iodobenzaldehyde oxime **3b** (0.5 g, 2.02 mmol), 3-bromoprop-1-yne **3c** (288.93 mg, 2.43 mmol) and triethylamine (204.81 mg, 2.02 mmol, 0.3 mL) were dissolved in dichloromethane (6 mL), slowly added with sodium hypochlorite solution (7.5 mL) dropwise in an ice bath, and stirred at room temperature for 12 hours. After the reaction was completed, the reaction mixture was added with water to quench the reaction, and extracted with dichloromethane (50 mL×3). The organic phases were combined and concentrated under reduced pressure. The obtained residue was purified by silica gel column chromatography (eluent: System A) to obtain 5-(bromomethyl)-3-(4-iodophenyl)isoxazole **3d** (220 mg) with a yield of 29.86%.
MS m/z (ESI): 363.7 [M+1]

### The third step

### 3-(4-Iodophenyl)-5-((2-((1S)-1-((tetrahydro-2H-pyran-2-yl)oxy)ethyl)-1H-imidazol-1-y l)methyl)isoxazole

2-((1S)-1-((tetrahydro-2H-pyran-2-yl)oxy)ethyl)-1H-imidazole **1h** (120 mg, 611.48 µmol),potassium carbonate (211.28 mg, 1.53 mmol) and 5-(bromomethyl)-3-(4-iodophenyl)isoxazole **3d** (222.56 mg, 611.48 µmol) were added to acetonitrile (3 mL), and heated to 70°C for 7 hours of reaction. After the reaction was completed, the reaction mixture was added with water to quench the reaction, and extracted with ethyl acetate (50 mL×3). The organic phases were combined and concentrated under reduced pressure. The obtained residue was purified by silica gel column chromatography (eluent: System A) to obtain

3-(4-iodophenyl)-5-((2-((1S)-1-((tetrahydro-2H-pyran-2-yl)oxy)ethyl)-1H-imidazol-1-yl)methyl)i soxazole **3e** (0.2 g) with a yield of 68.24%.
MS m/z (ESI): 480.1 [M+1]

### The fourth step

### 4-(4-((4-(5-((2-((1S))-1-((tetrahydro-2H-pyran-2-yl)oxy)ethyl)-1H-imidazol-1-yl)methy l)isoxazol-3-yl)phenyl)ethynyl)benzyl)morpholine

4-(4-Ethynylbenzyl)morpholine **1j** (80 mg, 397.49 µmol), 3-(4-iodophenyl)-5-((2-((1S)-1-((tetrahydro-2H-pyran-2-yl)oxy)ethyl)-1H-imidazol-1-yl)methyl)i soxazole **3e** (0.2 g, 417.27 µmol), triethylamine (120.67 mg, 1.19 mmol), bis(triphenylphosphine)palladium dichloride (91.82 mg, 79.50 µmol) and cuprous iodide (15.18 mg, 79.50 µmol) were sequentially added to N,N-dimethylformamide (2 mL), and the system was replaced with argon gas three times for reaction at room temperature overnight. The reaction mixture was added with water to quench the reaction, and extracted with ethyl acetate (50 mL×3). The organic phases were combined and concentrated under reduced pressure. The obtained residue was purified by silica gel column chromatography (eluent: System B) to obtain 4-(4-((4-(5-((2-((1S))-1-((tetrahydro-2H-pyran-2-yl)oxy)ethyl)-1H-imidazol-1-yl)methyl)isoxazo l-3-yl)phenyl)ethynyl)benzyl)morpholine **3f** (60 mg) with a yield of 27.31%.
MS m/z (ESI): 553.3 [M+1]

### The fifth step

### (S)-1-(1-((3-(4-((4-(morpholinomethyl)phenyl)ethynyl)phenyl)isoxazol-5-yl)methyl)-1 H-imidazol-2-yl)ethan-1-ol

4-(4-((4-(5-((2-((1S))-1-((tetrahydro-2H-pyran-2-yl)oxy)ethyl)-1H-imidazol-1-yl)methy l)isoxazol-3-yl)phenyl)ethynyl)benzyl)morpholine **3f** (60 mg, 108.57 µmol) was dissolved in dioxane (2 mL), and then added with 0.5 mL of 4M hydrochloric acid in dioxane solution for 4 hours of reaction. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((3-(4-((4-(morpholinomethyl)phenyl)ethynyl)phenyl)isoxazol-5-yl)methyl)-1H-imidazo l-2-yl)ethan-1-ol **3** (15 mg) with a yield of 23.18%.
MS m/z (ESI): 469.0 [M+1]

### Example 4

### (S)-1-(1-((3-(4-((4-(morpholinomethyl)phenyl)ethynyl)phenyl)-4,5-dihydroisoxazol-5-y l)methyl)-1H-imidazol-2-yl)ethan-1-ol

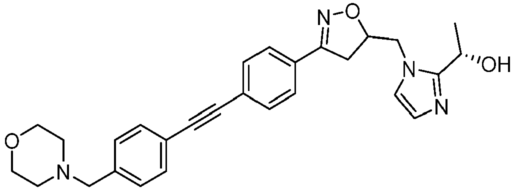

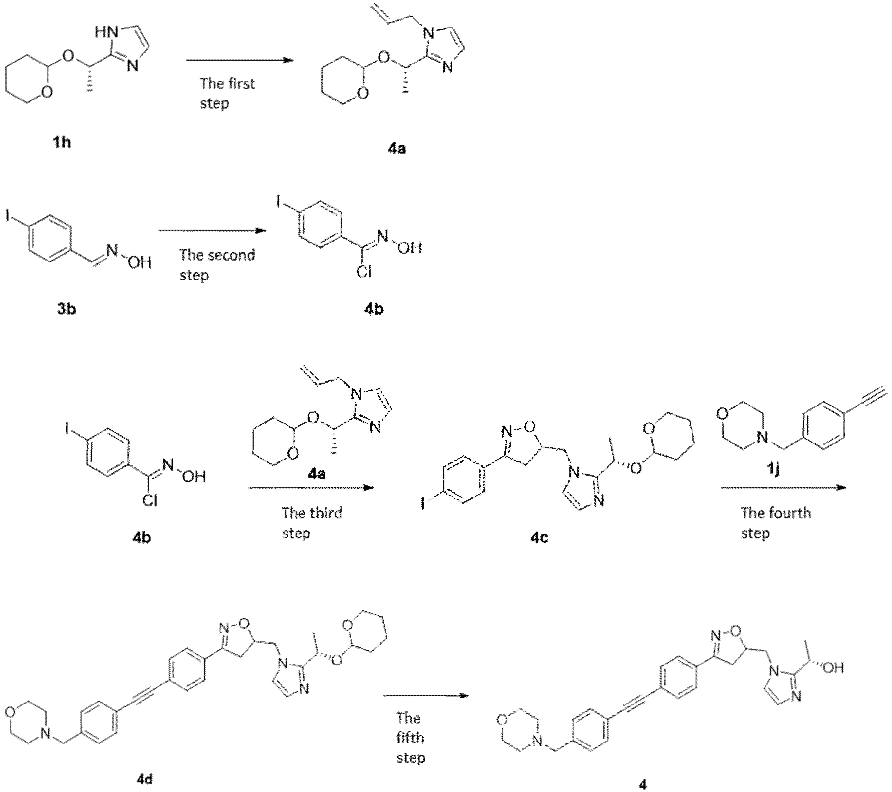

### The first step

### 1-Allyl-2-((1S)-1-((tetrahydro-2H-pyran-2-yl)oxy)ethyl)-1H-imidazole

2-((1S)-1-((tetrahydro-2H-pyran-2-yl)oxy)ethyl)-1H-imidazole **1h** (300 mg, 1.53 mmol) was added to tetrahydrofuran (5 mL), cooled to 0°C, added with sodium hydride (67.26 mg, 1.68 mmol) in batches for 1 h of reaction at room temperature, and then added with 3-bromoprop-1-ene (203.43 mg, 1.68 mmol) for 4 h of reaction at room temperature. After the reaction was completed, the reaction mixture was added with water to quench the reaction and extracted with ethyl acetate (50 mL×3). The organic phases were combined, dried over anhydrous sodium sulfate, and filtered. The filtrate was collected, concentrated under reduced pressure, and separated by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 1-allyl-2-((1S)-1-((tetrahydro-2H-pyran-2-yl)oxy)ethyl)-1H-imidazole **4a** (0.25 g) with a yield of 69.21%.
MS m/z (ESI): 237.1 [M+1]

### The second step

### (Z)-N-Hydroxy-4-iodobenzimidyl chloride

(E)-4-iodobenzaldehyde oxime **3b** (200 mg, 809.61 µmol) was dissolved in N,N-dimethylformamide (2 mL), slowly added with chlorosuccinimide (118.92 mg, 890.57 µmol),and stirred for reaction at room temperature overnight. After the reaction was completed, (Z)-N-hydroxy-4-iodobenzimidyl chloride **4b** was obtained. The reaction solution was directly used in the next reaction without purification.

### The third step

### 3-(4-Iodophenyl)-5-((2-((1S)-1-((tetrahydro-2H-pyran-2-yl)oxy)ethyl)-1H-imidazol-1-y l)methyl)-4,5-dihydroisoxazole

1-Allyl-2-((1S)-1-((tetrahydro-2H-pyran-2-yl)oxy)ethyl)-1H-imidazole **4a** (167.91 mg, 710.54 µmol) and triethylamine (143.80 mg, 1.42 mmol, 0.2 mL) were sequentially added to N,N-dimethylformamide (4 mL), and added with the above reaction solution of (Z)-N-hydroxy-4-iodobenzimidyl chloride **4b** dropwise for 4 h of reaction at room temperature. After the reaction was completed, the reaction mixture was added with water to quench the reaction and extracted with ethyl acetate (50 mL×3). The organic phases were combined and concentrated under reduced pressure. The obtained residue was purified by silica gel column chromatography (eluent: System A) to obtain 3-(4-iodophenyl)-5-((2-((1S)-1-((tetrahydro-2H-pyran-2-yl)oxy)ethyl)-1H-imidazol-1-yl)methyl) -4,5-dihydroisoxazole **4c** (120 mg) with a yield of 35.09%.
MS m/z (ESI): 481.8 [M+1]

### The fourth step

### 4-(4-((4-(5-((2-((1S))-1-((tetrahydro-2H-pyran-2-yl)oxy)ethyl)-1H-imidazol-1-yl)methy l)-4,5-dihydroisoxazol-3-yl)phenyl)ethynyl)benzyl)morpholine

4-(4-Ethynylbenzyl)morpholine **1j** (50 mg, 248.43 µmol), 3-(4-iodophenyl)-5-((2-((1S)-1-((tetrahydro-2H-pyran-2-yl)oxy)ethyl)-1H-imidazol-1-yl)methyl) -4,5-dihydroisoxazole **4c** (120 mg, 249.31 µmol), triethylamine (75.42 mg, 745.29 µmol), bis(triphenylphosphine)palladium dichloride (57.39 mg, 49.69 µmol),and cuprous iodide (9.49 mg, 49.69 µmol) were sequentially added to N,N-dimethylformamide (1 mL), and the system was replaced with argon gas three times for reaction at room temperature overnight. After the reaction was completed, the reaction mixture was added with water to quench the reaction and extracted with ethyl acetate (50 mL×3). The organic phases were combined and concentrated under reduced pressure. The obtained residue was purified by silica gel column chromatography (eluent: System B) to obtain 4-(4-((4-(5-((2-((1S))-1-((tetrahydro-2H-pyran-2-yl)oxy)ethyl)-1H-imidazol-1-yl)methyl)-4,5-dih ydroisoxazol-3-yl)phenyl)ethynyl)benzyl)morpholine **4d** (30 mg) with a yield of 21.77%.
MS m/z (ESI): 555.0 [M+1]

### The fifth step

### (S)-1-(1-((3-(4-((4-(morpholinomethyl)phenyl)ethynyl)phenyl)-4,5-dihydroisoxazol-5-y l)methyl)-1H-imidazol-2-yl)ethan-1-ol

4-(4-((4-(5-((2-((1S))-1-((tetrahydro-2H-pyran-2-yl)oxy)ethyl)-1H-imidazol-1-yl)methy l)-4,5-dihydroisoxazol-3-yl)phenyl)ethynyl)benzyl)morpholine **4d** (30 mg, 54.09 µmol) was dissolved in dichloromethane (2 mL), and added with trifluoroacetic acid (6.17 mg, 54.09 µmol, 0.3 mL) for 4 hours of reaction at room temperature. After the reaction was completed, the reaction mixture was concentrated under reduced pressure and separated by preparative liquid chromotography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((3-(4-((4-(morpholinomethyl)phenyl)ethynyl)phenyl)-4,5-dihydroisoxazol-5-yl)methyl) -1H-imidazol-2-yl)ethan-1-ol **4** (10 mg) with a yield of 26.88%.
MS m/z (ESI): 471.0 [M+1]

### Example 5

### (S)-1-(1-((5-(4-((4-morpholinophenyl))ethynyl)phenyl)isoxazol-3-yl)methyl)-1H-imida zol-2-yl)ethanol-1-ol

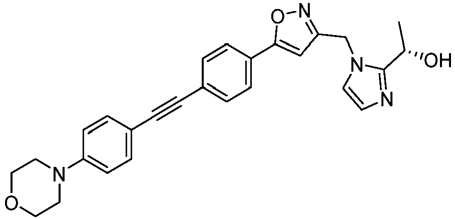

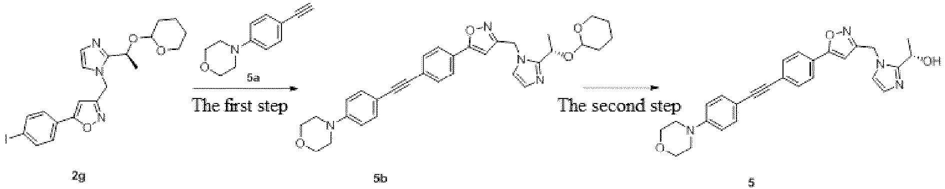

### The first step

### 4-(4-((4-(3-((2-((1S)-1-((tetrahydro-2H-pyran-2-yl)oxy)ethyl))-1H-imidazol-1-yl)methy l)isoxazol-5-yl)phenyl)ethynyl)phenyl)morpholine

4-(4-ethynylphenyl)morpholine **5a** (10 mg, 53.41 µmol), 5-(4-iodophenyl)-3-((2-((1S)-1-((tetrahydro-2H-pyran-2-yl)oxy)ethyl)-1H-imidazol-1-yl)methyl)i soxazole **2g** (25.60 mg, 53.41 µmol),cuprous iodide (1.02 mg, 5.34 µmol), bis(triphenylphosphine)palladium dichloride (6.17 mg, 5.34 µmol) and triethylamine (16.21 mg, 160.22 µmol) were sequentially added to N,N-dimethylformamide (1 mL), and the system was replaced with argon gas three times and stirred at room temperature for 1 hour. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 4-(4-((4-(3-((2-((1S)-1-((tetrahydro-2H-pyran-2-yl)oxy)ethyl))-1H-imidazol-1-yl)methyl)isoxazo l-5-yl)phenyl)ethynyl)phenyl)morpholine **5b** (15 mg) with a yield of 52.14%.
MS m/z (ESI): 539.0 [M+1]

### The second step

### (S)-1-(1-((5-(4-((4-morpholinophenyl))ethynyl)phenyl)isoxazol-3-yl)methyl)-1H-imida zol-2-yl)ethan-1-ol

4-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenyl)morpholine **5b** (10 mg, 18.57 µmol) was added to a mixed solution of dichloromethane (4 mL) and trifluoroacetic acid (1 mL), and stirred continuously for 3 hours at room temperature. The mixture was filtered under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-morpholinophenyl))ethynyl)phenyl)isoxazol-3-yl)methyl)-1H-imidazol-2-yl)e than-1-ol 5 (4.4 mg) with a yield of 39.6%.
MS m/z (ESI): 455.0 [M+1]

### Example 6

### (S)-(4-((4-(3-((2-(1-hydroxyethyl)-1H-imidazol-1-yl)methyl)isoxazol-5-yl)phenyl)ethyn yl)phenyl)(morpholino)methanone

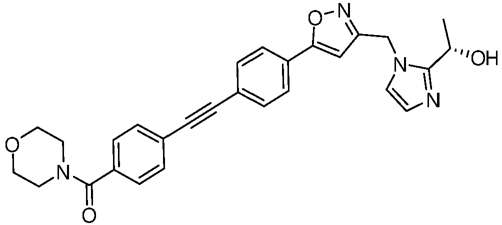

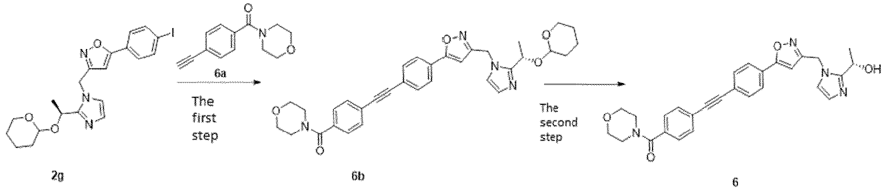

### The first step

### Morpholine (4-((4-(3-((2-((1S))-1-((tetrahydro-2H-pyran-2-yl)oxy)ethyl) -1H-imidazol-1-yl)methyl)isoxazol-5-yl)phenyl)ethynyl)phenyl)methanone

(4-Ethynylphenyl)(morpholino)methanone **6a** (85.56 mg, 397.49 µmol), 5-(4-iodophenyl)-3-((2-((1S)-1-((tetrahydro-2H-pyran-2-yl)oxy)ethyl)-1H-imidazol-1-yl)methyl)i soxazole **2g** (190.52 mg, 397.49 µmol), triethylamine (120.67 mg, 1.19 mmol), bis(triphenylphosphine)palladium dichloride (91.82 mg, 79.50 µmol) and cuprous iodide (15.18 mg, 79.50 µmol) were dissolved in N,N-dimethylformamide (2.5 mL), and the system was replaced with argon gas three times for reaction at room temperature overnight. After the reaction was completed, the reaction mixture was added with water to quench the reaction, and extracted with ethyl acetate (50 ml × 3). The organic phases were combined and concentrated under reduced pressure. The obtained residue was purified by silica gel column chromatography (eluent: System B) to obtain morpholine (4-((4-(3-((2-((1S))-1-((tetrahydro-2H-pyran-2-yl)oxy)ethyl)-1H-imidazol-1-yl)methyl)isoxazol-5-yl)phenyl)ethynyl)phenyl)methanone **6b** (100 mg) with a yield of 44.4%.
MS m/z (ESI): 567.3 [M+1]

### The second step

### (S)-(4-((4-(3-((2-(1-hydroxyethyl)-1H-imidazol-1-yl)methyl)isoxazol-5-yl)phenyl)ethyn yl)phenyl)(morpholino)methanone

Morpholine (4-((4-(3-((2-((1S))-1-((tetrahydro-2H-pyran-2-yl)oxy)ethyl)-1H-imidazol-1-yl)methyl)isoxazol-5-yl)phenyl)ethynyl)phenyl)methanone **6b** (100 mg, 176.48 µmol) was dissolved in dioxane (2 mL), and slowly added with 0.5 mL of 4M hydrochloric acid in dioxane solution for reaction at room temperature for 4 hours. After the reaction was completed, the reaction mixture was filtered under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-(4-((4-(3-((2-(1-hydroxyethyl)-1H-imidazol-1-yl)methyl)isoxazol-5-yl)phenyl)ethynyl)pheny l)(morpholino)methanone **6** (15 mg) with a yield of 13.81%.
MS m/z (ESI): 483.2 [M+1]

### Example 7

### (S)-1-(1-((5-(4-(phenylethynyl)phenyl)isoxazol-3-yl)methyl)-1H-imidazol-2-yl)ethan-1-ol

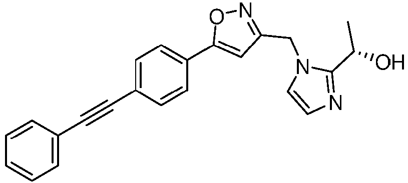

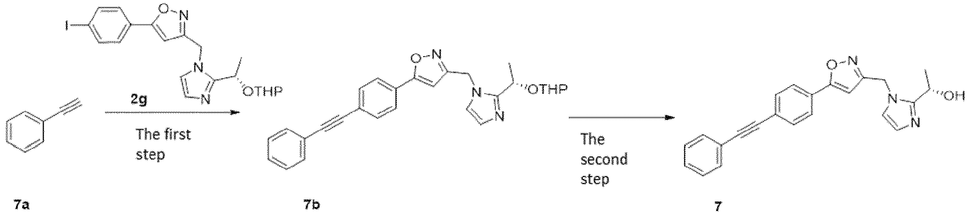

### The first step

### 5-(4-(Phenylethynyl)phenyl)-3-((2-((1S)-1-((tetrahydro-2H-pyran-2-yl)oxy)ethyl)-1H-i midazol-1-yl)methyl)isoxazole

5-(4-Iodophenyl)-3-((2-((1S)-1-((tetrahydro-2H-pyran-2-yl)oxy)ethyl)-1H-imidazol-1-y l)methyl)isoxazole **2g** (234.65 mg, 489.56 µmol), acetylenebenzene **7a** (50 mg, 489.56 µmol), allylpalladium chloride dimer (17.91 mg, 48.96 µmol), tri-tert-butyl phosphine (99.05 mg, 48.96 µmol, 10% purity) and triethylenediamine (164.74 mg, 1.47 mmol) were sequentially added to acetonitrile (4 mL), and the system was replaced with argon gas three times and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 5-(4-(phenylethynyl)phenyl)-3-((2-((1S) -1-((tetrahydro-2H-pyran-2-yl)oxy)ethyl)-1H -imidazol-1-yl)methyl)isoxazole **7b** (100 mg) with a yield of 45.04%.
MS m/z (ESI): 454.2 [M+1]

### The second step

### (S)-1-(1-((5-(4-(phenylethynyl)phenyl)isoxazol-3-yl)methyl)-1H-imidazol-2-yl)ethan-1-ol

5-(4-(Phenylethynyl)phenyl)-3-((2-((1S)-1-((tetrahydro-2H-pyran-2-yl)oxy)ethyl)-1H-i midazol-1-yl)methyl)isoxazole **7b** (100 mg, 220.49 µmol) was added to a mixed solvent of dichloromethane (3 mL) and trifluoroacetic acid (0.5 mL), and continuously stirred at room temperature for 3 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-(phenylethynyl)phenyl)isoxazol-3-yl)methyl)-1H-imidazol-2-yl)ethan-1-ol 7 (70 mg) with a yield of 62.91%.
MS m/z (ESI): 370.1 [M+1]

¹H NMR (400 MHz, DMSO-*d*₆) *δ* 7.92 (d, *J* = 8.4 Hz, 2H), 7.77 (d, *J* = 1.6 Hz, 1H), 7.73 (d, *J =* 8.4 Hz, 2H), 7.67 (d, *J =* 1.6 Hz, 1H), 7.61 - 7.57 (m, 2H), 7.50 - 7.42 (m, 3H), 7.19 (s, 1H), 6.39 (br, 1H), 5.72 (s, 2H), 5.24 (q, *J =* 6.8 Hz, 1H), 1.50 (d, *J =* 6.4 Hz, 3H).

### Example 8

### (S)-1-(1-((5-(4-((3-(2-methoxyethoxy)phenyl)ethynyl)phenyl)isoxazol-3-yl)methyl)-1H -imidazol-2-yl)ethan-1-ol

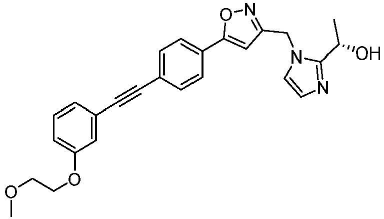

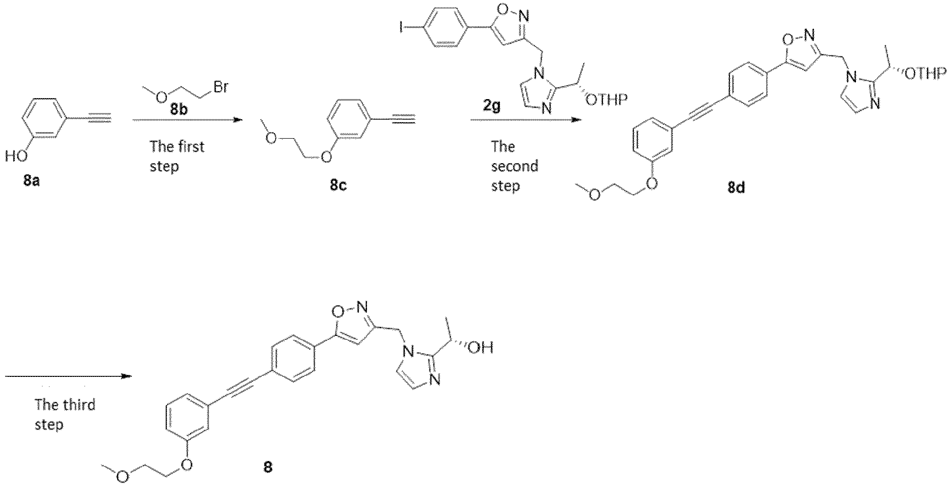

### The first step

### 1-Ethynyl-3-(2-methoxyethoxy)benzene

3-Ethynylphenol **8a** (500 mg, 4.23 mmol), 1-bromo-2-methoxyethane **8b** (705.94 mg, 5.08 mmol) and potassium carbonate (701.95 mg, 5.08 mmol) were sequentially added to acetone (2 mL), and the system was replaced with argon gas and heated to 110°C for 2 h of reaction under microwave. After the reaction was completed, the reaction mixture was added with water (20 mL) to quench the reaction, and extracted with dichloromethane (10 mL×3). The combined organic phase was washed with saturated brine (20 mL), dried over anhydrous sodium sulfate, and concentrated under reduced pressure. The obtained residue was purified by silica gel column chromatography (eluent: System B) to obtain 1-ethynyl-3-(2-methoxyethoxy)benzene **8c** (380 mg) with a yield of 50.95%.
MS m/z (ESI): 177.0 [M+1]

### The second step

### 5-(4-((3-(2-Methoxyethoxy)phenyl)ethynyl)phenyl)-3-((2-((1S)-1-((tetrahydro-2H-pyra n-2-yl)oxy)ethyl)-1H-imidazol-1-yl)methyl)isoxazole

1-Ethynyl-3-(2-methoxyethoxy)benzene **8c** (50 mg, 283.75 µmol), 5-(4-iodophenyl)-3-((2-((1S)-1-((tetrahydro-2H-pyran-2-yl)oxy)ethyl)-1H-imidazol-1-yl)methyl)i soxazole **2g** (136.00 mg, 283.75 µmol),cuprous iodide (5.42 mg, 28.38 µmol), bis(triphenylphosphine)palladium dichloride (32.77 mg, 28.38 µmol) and triethylamine (143.56 mg, 1.42 mmol) were sequentially added to N,N-dimethylformamide (2 mL), and the system was replaced with argon gas three times and stirred at room temperature for 16 hours. After the reaction was completed, the reaction mixture was concentrated under reduced pressure. The obtained residue was purified by silica gel column chromatography (eluent: System B) to obtain 5-(4-((3-(2-methoxyethoxy)phenyl)ethynyl)phenyl)-3-((2-((1S)-1-((tetrahydro-2H-pyran-2-yl)ox y)ethyl)-1H-imidazol-1-yl)methyl)isoxazole **8d** (30 mg) with a yield of 20.04%.
MS m/z (ESI): 527.9 [M+1]

### The third step

### (S)-1-(1-((5-(4-((3-(2-methoxyethoxy)phenyl)ethynyl)phenyl)isoxazol-3-yl)methyl)-1H -imidazol-2-yl)ethan-1-ol

5-(4-((3-(2-Methoxyethoxy)phenyl)ethynyl)phenyl)-3-((2-((1S)-1-((tetrahydro-2H-pyra n-2-yl)oxy)ethyl)-1H-imidazol-1-yl)methyl)isoxazole **8d** (60 mg, 113.72 µmol) and trifluoroacetic acid (12.97 mg, 113.72 µmol, 0.5 mL) were sequentially added to dichloromethane (1 mL), and stirred at room temperature for 3 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((3-(2-methoxyethoxy)phenyl)ethynyl)phenyl)isoxazol-3-yl)methyl)-1H-imidazol -2-yl)ethan-1-ol **8** (3.8 mg, 6.13 µmol) with a yield of 5.39%.
MS m/z (ESI): 444.2 [M+1]

### Example 9

### (S)-1-(1-((5-(4-(pyridin-3-ylethynyl)phenyl)isoxazol-3-yl)methyl)-1H-imidazol-2-yl)eth an-1-ol

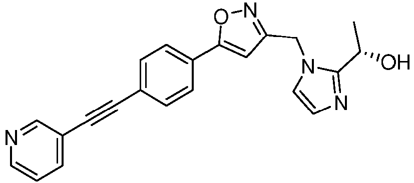

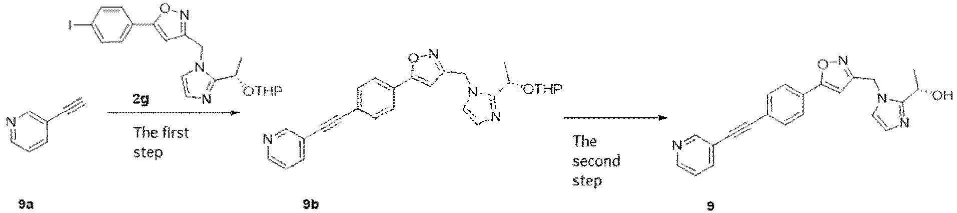

### The first step

### 5-(4-(Pyridin-3-ylethynyl)phenyl)-3-((2-((1S)-1-((tetrahydro-2H-pyran-2-yl)oxy)ethyl)-1H-imidazol-1)-yl)methyl)isoxazole

3-Ethynylpyridine **9a** (20 mg, 193.95 µmol), 5-(4-iodophenyl)-3-((2-((1S)-1-((tetrahydro-2H-pyran-2-yl)oxy)ethyl)-1H-imidazol-1-yl)methyl)i soxazole **2g** (92.96 mg, 193.95 µmol), allylpalladium chloride dimer (7.08 mg, 19.39 µmol), triethylene diamine (43.51 mg, 387.89 µmol) and tri-tert-butylphosphine (3.92 mg, 19.39 µmol, 10% toluene solution) were sequentially added to acetonitrile (2 mL), and the system was replaced with argon gas three times for reaction at room temperature for 12 hours. After the reaction was completed, the reaction mixture was concentrated under reduced pressure. The obtained residue was purified by silica gel column chromatography (eluent: System B) to obtain 5-(4-(pyridin-3-ylethynyl)phenyl)-3-((2-((1S)-1-((tetrahydro-2H-pyran-2-yl)oxy)ethyl)-1H-imida zol-1)-yl)methyl)isoxazole **9b** (60 mg) with a yield of 68.06%.
MS m/z (ESI): 455.2 [M+1]

### The second step

### (S)-1-(1-((5-(4-(pyridin-3-ylethynyl)phenyl)isoxazol-3-yl)methyl)-1H-imidazol-2-yl)eth an-1-ol

5-(4-(Pyridin-3-ylethynyl)phenyl)-3-((2-((1S)-1-((tetrahydro-2H-pyran-2-yl)oxy)ethyl)-1H-imidazol-1)-yl)methyl)isoxazole **9b** (60 mg, 132.01 µmol) was dissolved in 4M hydrochloric acid in dioxane (3 mL) and stirred at room temperature for 4 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-(pyridin-3-ylethynyl)phenyl)isoxazol-3-yl)methyl)-1H-imidazol-2-yl)ethan-1-ol 9 (20 mg) with a yield of 31.12%.
MS m/z (ESI): 371.2 [M+1]

### Example 10

### (S)-1-(1-((5-(4-(pyridin-4-ylethynyl)phenyl)isoxazol-3-yl)methyl)-1H-imidazol-2-yl)eth an-1-ol

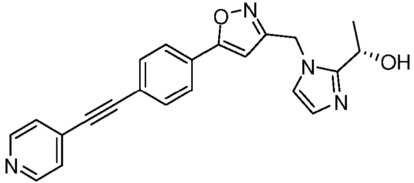

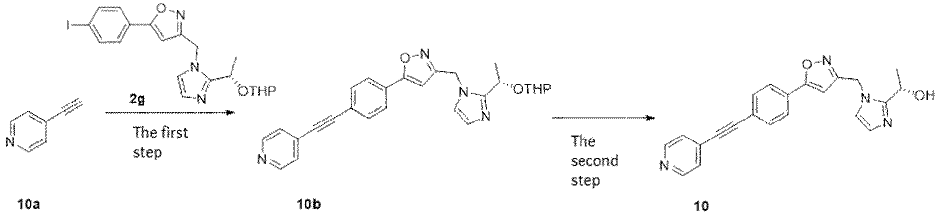

### The first step

### 5-(4-(Pyridin-4-ylethynyl)phenyl)-3-((2-((1S)-1-((tetrahydro-2H-pyran-2-yl)oxy)ethyl)-1H-imidazol-1)-yl)methyl)isoxazole

4-Ethynylpyridine **10a** (20 mg, 193.95 µmol), 5-(4-iodophenyl)-3-((2-((1S)-1-((tetrahydro-2H-pyran-2-yl)oxy)ethyl)-1H-imidazol-1-yl)methyl)i soxazole 2g (92.96 mg, 193.95 µmol), allylpalladium chloride dimer (7.08 mg, 19.39 µmol), triethylene diamine (43.51 mg, 387.89 µmol) and tri-tert-butylphosphine (3.92 mg, 19.39 µmol) were sequentially added to acetonitrile (2 mL), and the system was replaced with argon gas three times for reaction at room temperature for 12 hours. After the reaction was completed, the reaction mixture was concentrated under reduced pressure. The obtained residue was purified by silica gel column chromatography (eluent: System B) to obtain 5-(4-(pyridin-4-ylethynyl)phenyl)-3-((2-((1S)-1-((tetrahydro-2H-pyran-2-yl)oxy)ethyl)-1H-imida zol-1)-yl)methyl)isoxazole **10b** (80 mg) with a yield of 90.75%.
MS m/z (ESI): 455.1 [M+1]

### The second step

### (S)-1-(1-((5-(4-(pyridin-4-ylethynyl)phenyl)isoxazol-3-yl)methyl)-1H-imidazol-2-yl)eth an-1-ol

5-(4-(Pyridin-4-yl-ethynyl)phenyl)-3-((2-((1S)-1-((tetrahydro-2H-pyran-2-yl)oxy)ethyl) -1H-imidazol-1)-yl)methyl)isoxazole **10b** (80 mg, 176.01 µmol) was dissolved in dioxane (2 mL), and added with 4M hydrochloric acid in dioxane (1 mL) dropwise for reaction at room temperature for 4 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-(pyridin-4-ylethynyl)phenyl)isoxazol-3-yl)methyl)-1H-imidazol-2-yl)ethan-1-ol **10** (20 mg) with a yield of 21.77%.
MS m/z (ESI): 371.1 [M+1]

### Example 11

### 1-((5-(4-((4-(Morpholinomethyl)phenyl)ethynyl)phenyl)isoxazol-3-yl)methyl)-1H-imid azol-2-carboxamide

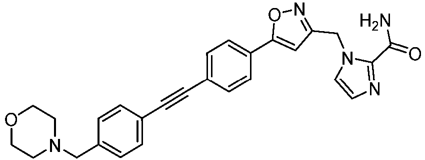

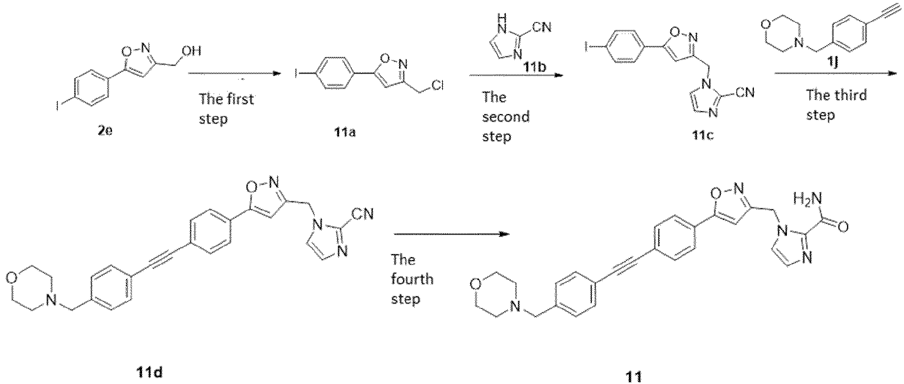

### The first step

### 3-(Chloromethyl)-5-(4-iodophenyl)isoxazole

(5-(4-Iodophenyl)isoxazol-3-yl)methanol **2e** (1.0 g, 3.32 mmol) and triethylamine (840.23 mg, 8.30 mmol) were sequentially added to dichloromethane (20 mL), added with methanesulfonyl chloride (684.84 mg, 5.98 mmol) in an ice-water bath, and continuously stirred at room temperature for 12 hours. The reaction mixture was added with dichloromethane (30 mL) and water (15 mL) for liquid separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 3-(chloromethyl)-5-(4-iodophenyl)isoxazole **11a** (800 mg) with a yield of 75.38%.
MS m/z (ESI): 320.0 [M+1]

### The second step

### 1-((5-(4-Iodophenyl)isoxazol-3-yl)methyl)-1H-imidazol-2-nitrile

1H-imidazol-2-nitrile **11b** (150 mg, 1.61 mmol), 3-(chloromethyl)-5-(4-iodophenyl)isoxazole **11a** (514.88 mg, 1.61 mmol), sodium hydride (83.83 mg, 2.10 mmol, 60% purity) were sequentially added to N,N-dimethylformamide (3 mL), and the system was replaced with argon gas three times and stirred at room temperature for 12 hours. After the reaction was completed, the reaction mixture was added with ethyl acetate (30 mL) and water (15 mL) for layer separation. The aqueous phase was extracted with ethyl acetate (30 mL × 2). The combined organic phase was washed with saturated sodium chloride solution (30 mL × 2), dried over anhydrous sodium sulfate, filtered, and concentrated under reduced pressure. The obtained residue was purified by silica gel column chromatography (eluent: System A) to obtain 1-((5-(4-iodophenyl)isoxazol-3-yl)methyl)-1H-imidazol-2-nitrile **11c** (600 mg) with a yield of 98.99%.
MS m/z (ESI): 377.0 [M+1]

### The third step

### 1-((5-(4-((4-(Morpholinomethyl)phenyl)ethynyl)phenyl)isoxazol-3-yl)methyl)-1H-imid azol-2-nitrile

1-((5-(4-Iodophenyl)isoxazol-3-yl)methyl)-1H-imidazol-2-nitrile **11c** (50 mg, 132.93 µmol),4-(4-ethynylbenzyl)morpholine **1j** (26.75 mg, 132.93 µmol),cuprous iodide (5.08 mg, 26.59 µmol),bis(triphenylphosphine)palladium dichloride (30.71 mg, 26.59 µmol),and triethylamine (40.35 mg, 398.78 µmol) were sequentially added to N,N-dimethylformamide (1 mL), and the system was replaced with argon gas three times and continuously stirred at room temperature for 1 hour. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 1-((5-(4-((4-(morpholinomethyl)phenyl)ethynyl)phenyl)isoxazol-3-yl)methyl)-1H-imidazol-2-nit rile **11d** (40 mg) with a yield of 66.95%.
MS m/z (ESI): 450.2 [M+1]

### The fourth step

### 1-((5-(4-((4-(Morpholinomethyl)phenyl)ethynyl)phenyl)isoxazol-3-yl)methyl)-1H-imid azol-2-carboxamide

1-((5-(4-((4-(Morpholinomethyl)phenyl)ethynyl)phenyl)isoxazol-3-yl)methyl)-1H-imid azol-2-nitrile **11d** (30 mg, 66.74 µmol) was dissolved in dimethyl sulfoxide (1 mL), slowly added with 5 M sodium hydroxide aqueous solution (1 mL) followed by hydrogen peroxide (0.5 mL), and continuously stirred at room temperature for 1 hour. After the reaction was completed, the reaxtion mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B : CH₃CN) to obtain 1-((5-(4-((4-(morpholinomethyl)phenyl)ethynyl)phenyl)isoxazol-3-yl)methyl)-1H-imidazol-2-car boxamide 11 (8.0 mg) with a yield of 19.68%.
MS m/z (ESI): 468.1 [M+1]

### Example 12

### 1-((5-(4-((4-(Morpholinomethyl)phenyl)ethynyl)phenyl)isoxazol-3-yl)methyl)-1H-imid azol-2-carboxylic acid

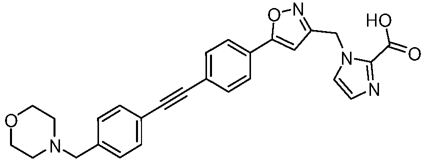

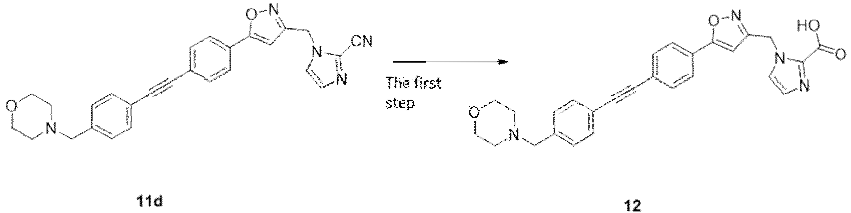

### The first step

### 1-((5-(4-((4-(Morpholinomethyl)phenyl)ethynyl)phenyl)isoxazol-3-yl)methyl)-1H-imid azol-2-carboxylic acid

1-((5-(4-((4-(Morpholinomethyl)phenyl)ethynyl)phenyl)isoxazol-3-yl)methyl)-1H-imid azol-2-nitrile **11d** (50 mg, 111.23 µmol) was dissolved in dioxane (0.5 mL), slowly added with water (0.5 mL) followed by concentrated sulfuric acid (1 mL), heated to 100°C, and continuously stirred for 2 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 1-((5-(4-((4-(morpholinomethyl)phenyl)ethynyl)phenyl)isoxazol-3-yl)methyl)-1H-imidazol-2-car boxylic acid **12** (3.2 mg) with a yield of 5.51%.
MS m/z (ESI): 486.2 [M+18]

### Example 13

### Ethyl

### 1-((5-(4-((4-(morpholinomethyl)phenyl)ethynyl)phenyl)isoxazol-3-yl)methyl)-1H-imidazol-2-car boxylate

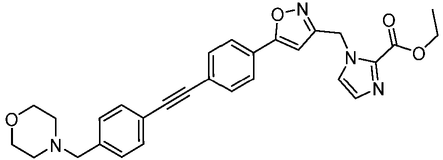

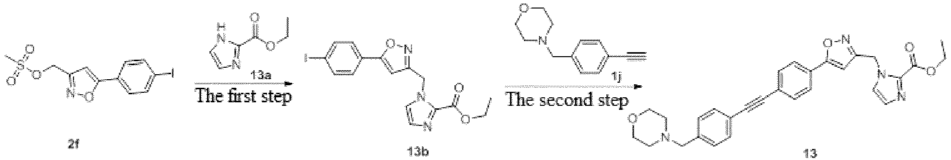

### The first step

### Ethyl 1-((5-(4-iodophenyl)isoxazol-3-yl)methyl)-1H-imidazol-2-carboxylate

Ethyl 1H-imidazol-2-carboxylate **13a** (100 mg, 713.57 µmol), methyl (5-(4-iodophenyl)isoxazol-3-yl)methanesulfonate **2f** (270.57 mg, 713.57 µmol),and sodium hydride (37.12 mg, 928.00 µmol, 60% purity) were sequentially added to N,N-dimethylformamide (3 mL), and the system was replaced with argon gas three times and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain ethyl 1-((5-(4-iodophenyl)isoxazol-3-yl)methyl)-1H-imidazol-2-carboxylate **13b** (70 mg) with a yield of 23.18%.
MS m/z (ESI): 424.0 [M+1]

### The second step

### Ethyl 1-((5-(4-((4-(morpholinomethyl)phenyl)ethynyl)phenyl)isoxazol-3-yl)methyl)-1H-imidazol-2-car boxylate

Ethyl 1-((5-(4-iodophenyl)isoxazol-3-yl)methyl)-1H-imidazol-2-carboxylate **13b** (70 mg, 165.40 µmol), 4-(4-ethynylbenzyl)morpholine **1j** (33.29 mg, 165.40 µmol),cuprous iodide (6.32 mg, 33.08 µmol),bis(triphenylphosphine)palladium dichloride (38.21 mg, 33.08 µmol) and triethylamine (50.21 mg, 496.21 µmol) were sequentially added to N,N-dimethylformamide (2 mL), and the system was replaced with argon gas three times and continuously stirred at room temperature for 1 hour. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain ethyl 1-((5-(4-((4-(morpholinomethyl)phenyl)ethynyl)phenyl)isoxazol-3-yl)methyl)-1H-imidazol-2-car boxylate **13** (3.2 mg) with a yield of 3.07%.
MS m/z (ESI): 497.2 [M+1]

### Example 14

### 1-(1-((5-(4-((4-(Morpholinomethyl)phenyl)ethynyl)phenyl)isoxazol-3-yl)methyl)-1H-1, 2,4-triazol-5-yl)ethan-1-one

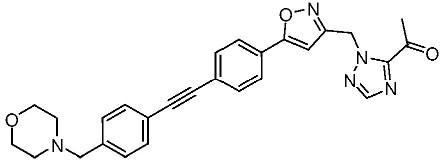

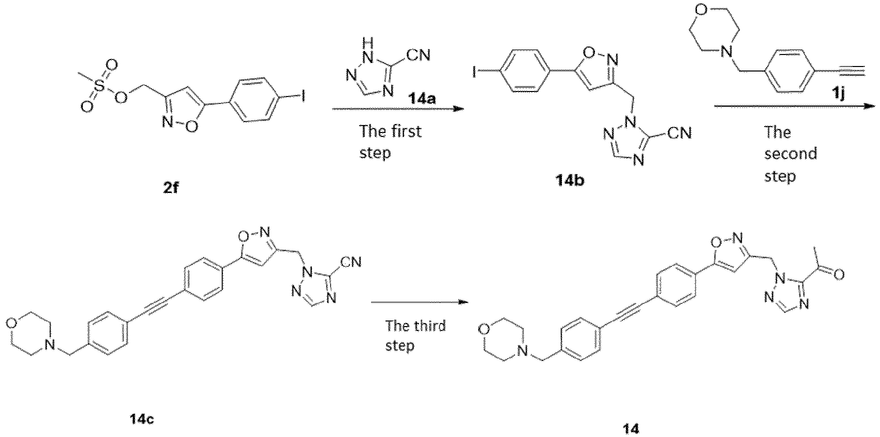

### The first step

### 1-((5-(4-Iodophenyl)isoxazol-3-yl)methyl)-1H-1,2,4-triazol-5-carbonitrile

1H-1,2,4-triazol-5-nitrile **14a** (100 mg, 1.06 mmol), methyl (5-(4-iodophenyl)isoxazol-3-yl)methanesulfonate **2f** (403.05 mg, 1.06 mmol) and potassium carbonate (293.83 mg, 2.13 mmol) were sequentially added to acetonitrile (5 mL), and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 1-((5-(4-iodophenyl)isoxazol-3-yl)methyl)-1H-1,2,4-triazol-5-carbonitrile **14b** (200 mg) with a yield of 49.89%.
MS m/z (ESI): 378.0 [M+1]

### The second step

### 1-((5-(4-((4-(Morpholinomethyl)phenyl)ethynyl)phenyl)isoxazol-3-yl)methyl)-1H-1,2,4 -triazol-5-carbonitrile

1-((5-(4-Iodophenyl)isoxazol-3-yl)methyl)-1H-1,2,4-triazol-5-carbonitrile **14b** (100.00 mg, 265.15 µmol), 4-(4-ethynylbenzyl)morpholine **1j** (53.37 mg, 265.15 µmol),cuprous iodide (10.13 mg, 53.03 µmol),bis(triphenylphosphine)palladium dichloride (61.25 mg, 53.03 µmol), and triethylamine (80.49 mg, 795.46 µmol) were sequentially added to N,N-dimethylformamide (1 mL), and the system was replaced with argon gas three times and continuously stirred at room temperature for 1 hour. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 1-((5-(4-((4-(morpholinomethyl)phenyl)ethynyl)phenyl)isoxazol-3-yl)methyl)-1H-1,2,4-triazol-5 -carbonitrile **14c** (80 mg) with a yield of 66.97 %.
MS m/z (ESI): 451.0 [M+1]

### The third step

### 1-(1-((5-(4-((4-(Morpholinomethyl)phenyl)ethynyl)phenyl)isoxazol-3-yl)methyl)-1H-1, 2,4-triazol-5-yl)ethan-1-one

Under the protection of nitrogen, 1-((5-(4-((4-(morpholinomethyl)phenyl)ethynyl)phenyl)isoxazol-3-yl)methyl)-1H-1,2,4-triazol-5 -carbonitrile **14c** (20 mg, 44.40 µmol) was added to tetrahydrofuran (1 mL), dropwise added with methyl magnesium bromide (3 M, 29.60 µL) in an ice-water bath, and continuously stirred for 3 hours. After the reaction was completed, the reaction mixture was added with 2 M dilute hydrochloric acid (10 mL), stirred at room temperature for 1 hour, and extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 1-(1-((5-(4-((4-(morpholinomethyl)phenyl)ethynyl)phenyl)isoxazol-3-yl)methyl)-1H-1,2,4-triazo l-5-yl)ethan-1-one **14** (2.8 mg) with a yield of 9.76%.
MS m/z (ESI): 468.2 [M+1]

### Example 15

### (S)-1-(1-((5-(4-((4-(Morpholinomethyl)phenyl)ethynyl)phenyl)isoxazol-3-yl)methyl)-1 H-benzo[d]imidazol-2-yl)ethan-1-ol

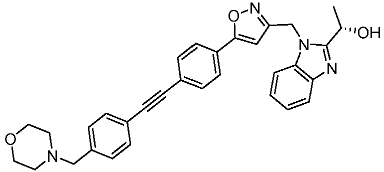

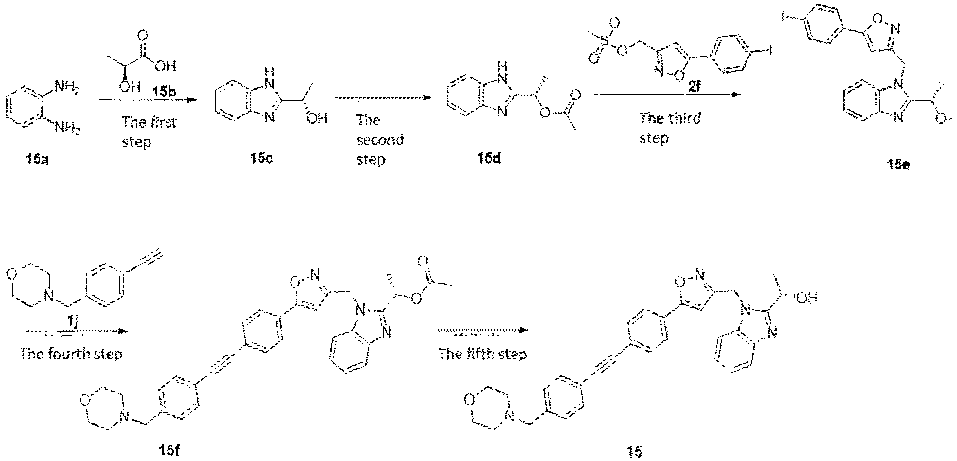

### The first step

### (S)-1-(1H-benzo[d]imidazol-2-yl)ethan-1-ol

Benzene-1,2-diamine **15a** (50 mg, 462.36 µmol) and (S)-2-hydroxypropanoic acid **15b** (124.95 mg, 1.39 mmol) were sequentially added to 6M hydrochloric acid (1 mL), heated to 110°C, and continuously stirred for 4 hours. After the reaction was completed, the reaction mixture was neutralized to pH = 8 with sodium hydroxide solution, filtered with suction, and dried under vacuum to obtain (S)-1-(1H-benzo[d]imidazol-2-yl)ethan-1-ol **15c** (60 mg) with a yield of 80.01%.
MS m/z (ESI): 163.0 [M+1]

### The second step

### (S)-1-(1H-benzo[d]imidazol-2-yl)ethyl acetate

(S)-1-(1H-benzo[d]imidazol-2-yl)ethan-1-ol 15c (50 mg, 308.28 µmol) was added to acetic anhydride (0.5 mL), heated to 130°C, and continuously stirred for 2 hours. After the reaction was completed, the reaction mixture was added with water to quench the reaction, extracted with dichloromethane (2 mL×3), and dried over anhydrous sodium sulfate to obtain (S)-1-(1H-benzo[d]imidazol-2-yl)ethyl acetate **15d** (50 mg) with a yield of 79.42%, which was directly used in the next reaction without purification.
MS m/z (ESI): 205.0 [M+1]

### The third step

### (S)-1-(1-((5-(4-iodophenyl))isoxazol-3-yl)methyl)-1H-benzo[d]imidazol-2-yl)ethyl acetate

(S)-1-(1H-benzo[d]imidazol-2-yl)ethyl acetate **15d** (600 mg, 2.94 mmol), methyl (5-(4-iodophenyl)isoxazol-3-yl)methanesulfonate **2f** (1.67 g, 4.41 mmol) and potassium carbonate (2.03 g, 14.69 mmol) were sequentially added to N,N-dimethylformamide (1 mL), heated to 65°C, and continuously stirred for 16 hours. After the reaction was completed, the reaction mixture was concentrated under reduced pressure, and added with water and dichloromethane for layer separation. The aqueous phase was extracted with dichloromethane. The combined organic phase was dried over anhydrous sodium sulfate and concentrated under reduced pressure. The obtained residue was purified by silica gel column chromatography (eluent: System A) to obtain (S)-1-(1-((5-(4-iodophenyl))isoxazol-3-yl)methyl)-1H-benzo[d]imidazol-2-yl)ethyl acetate **15e** (80 mg) with a yield of 5.59%.
MS m/z (ESI): 487.8 [M+1]

### The fourth step

### (S)-1-(1-((5-(4-((4-(morpholinomethyl)phenyl)ethynyl)phenyl)isoxazol-3-yl)methyl)-1 H-benzo[d]imidazol-2-yl)ethyl acetate

(S)-1-(1-((5-(4-Iodophenyl))isoxazol-3-yl)methyl)-1H-benzo[d]imidazol-2-yl)ethyl acetate **15e** (20 mg, 41.04 µmol),4-(4-ethynylbenzyl)morpholine **1j** (8.26 mg, 41.04 µmol), allylpalladium chloride dimer (375.43 µg, 1.03 µmol),tri-tert-butyl phosphine (830.39 µg, 4.10 µmol) and triethylenediamine (9.21 mg, 82.09 µmol) were sequentially added to acetonitrile (1 mL), and continuously stirred at room temperature for 16 hours. After the reaction was completed, tehr eaction mixture was concentrated under reduced pressure, added with 6 mL of ethyl acetate, filtered, and concentrated under reduced pressure. The obtained residue was purified by silica gel column chromatography (eluent: System B) to obtain (S)-1-(1-((5-(4-((4-(morpholinomethyl)phenyl)ethynyl)phenyl)isoxazol-3-yl)methyl)-1H-benzo[d ]imidazol-2-yl)ethyl acetate **15f** (15 mg) with a yield of 65.19%.
MS m/z (ESI): 561.0 [M+1]

### The fifth step

### (S)-1-(1-((5-(4-((4-(morpholinomethyl)phenyl)ethynyl)phenyl)isoxazol-3-yl)methyl)-1 H-benzo[d]imidazol-2-yl)ethan-1-ol

(S)-1-(1-((5-(4-((4-(morpholinomethyl)phenyl)ethynyl)phenyl)isoxazol-3-yl)methyl)-1 H-benzo[d]imidazol-2-yl)ethyl acetate **15f** (100 mg, 178.37 µmol) and potassium carbonate (29.58 mg, 214.04 µmol) were sequentially added to methanol, and continuously stirred at room temperature for 16 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-(morpholinomethyl)phenyl)ethynyl)phenyl)isoxazol-3-yl)methyl)-1H-benzo[d ]imidazol-2-yl)ethan-1-ol **15** (10 mg) with a yield of 7.98%.
MS m/z (ESI): 519.0 [M+1]

### Example 16

### 1-((5-(4-((4-(Morpholinomethyl)phenyl)ethynyl)phenyl)isoxazol-3-yl)methyl)-1H-1,2,4 -triazol-5-carboxamide

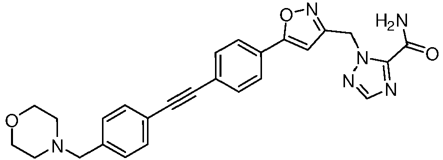

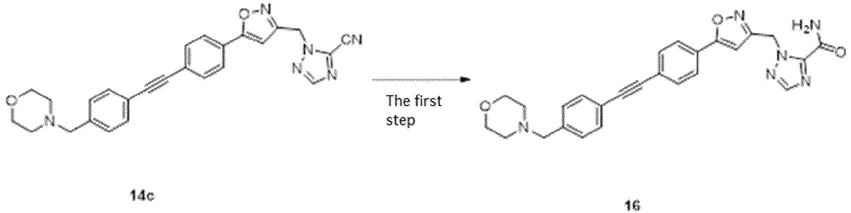

### The first step

### 1-((5-(4-((4-(Morpholinomethyl)phenyl)ethynyl)phenyl)isoxazol-3-yl)methyl)-1H-1,2,4 -triazol-5-carboxamide

1-((5-(4-((4-(Morpholinomethyl)phenyl)ethynyl)phenyl)isoxazol-3-yl)methyl)-1H-1,2,4 -triazol-5-carbonitrile **14c** (20 mg, 44.40 µmol) was dissolved in dimethyl sulfoxide (1 mL), slowly added with 5 M sodium hydroxide aqueous solution (1 mL) followed by hydrogen peroxide (0.5 mL), and continuously stirred at room temperature for 1 hour. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 1-((5-(4-((4-(morpholinomethyl)phenyl)ethynyl)phenyl)isoxazol-3-yl)methyl)-1H-1,2,4-triazol-5 -carboxamide **16** (1.9 mg) with a yield of 6.61%.
MS m/z (ESI): 469.1 [M+1]

### Example 17

### (S)-1-(4-((4-(3-((2-(1-hydroxyethyl)-1H-imidazol-1-yl)methyl)isoxazol-5-yl)phenyl)eth ynyl)benzyl)piperidin-4-carboxylic acid

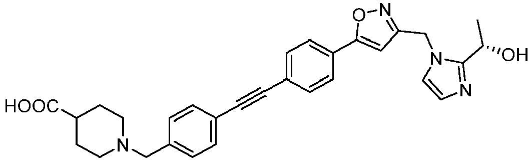

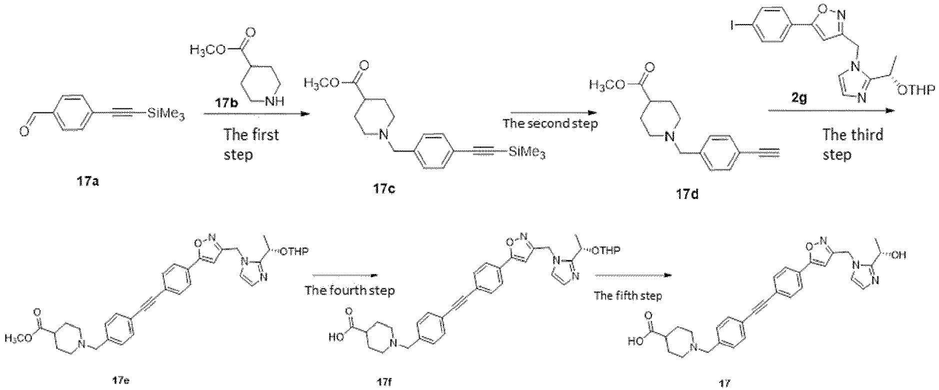

### The first step

### Methyl 1-(4-((trimethylsilyl)ethynyl)benzyl)piperidin-4-carboxylate

4-((Trimethylsilyl)ethynyl)benzaldehyde **17a** (200 mg, 988.51 µmol), methyl piperidin-4-carboxylate **17b** (283.08 mg, 1.98 mmol) and acetic acid (0.4 mL) were dissolved in 1,2-dichloroethane (4 mL), stirred for 30 minutes, added with sodium triacetoxyborohydride (628.52 mg, 2.97 mmol), and continuously stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL × 2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain methyl 1-(4-((trimethylsilyl)ethynyl)benzyl)piperidin-4-carboxylate **17c** (250 mg) with a yield of 76.75%.
MS m/z (ESI): 330.0 [M+1]

### The second step

### Methyl 1-(4-ethynylbenzyl)piperidin-4-carboxylate

Methyl 1-(4-((trimethylsilyl)ethynyl)benzyl)piperidin-4-carboxylate **17c** (125 mg, 379.35 µmol) was dissolved in methanol (5 mL), added with potassium fluoride (44.08 mg, 758.71 µmol),and continuously stirred at room temperature for 4 hours. After the reaction was completed, the reaction mixture was concentrated to dryness under reduced pressure. The residue was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure to obtain methyl 1-(4-ethynylbenzyl)piperidin-4-carboxylate **17d** (90 mg) with a yield of 92.2%, which was used directly in the next reaction without purification.
MS m/z (ESI): 258.0 [M+1]

### The third step

### Methyl 1-(4-((4-(3-((2-((1S))-1-((tetrahydro-2H-pyran-2-yl)oxy)ethyl)-1H-imidazol-1-yl)methyl)isoxazo l-5-yl)phenyl)ethynyl)benzyl)piperidin-4-carboxylate

Methyl 1-(4-ethynylbenzyl)piperidin-4-carboxylate **17d** (90 mg, 349.75 µmol), 5-(4-iodophenyl)-3-((2-((1S)-1-((tetrahydro-2H-pyran-2-yl)oxy)ethyl)-1H-imidazol-1-yl)methyl)i soxazole **2g** (167.64 mg, 349.75 µmol),cuprous iodide (6.68 mg, 34.97 µmol), bis(triphenylphosphine)palladium dichloride (40.40 mg, 34.97 µmol) and triethylamine (176.96 mg, 1.75 mmol) were sequentially added to N,N-dimethylformamide (4 mL), and the system was replaced with argon gas three times and continuously stirred at room temperature for 1 hour. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain methyl 1-(4-((4-(3-((2-((1S))-1-((tetrahydro-2H-pyran-2-yl)oxy)ethyl)-1H-imidazol-1-yl)methyl)isoxazo l-5-yl)phenyl)ethynyl)benzyl)piperidin-4-carboxylate **17e** (60 mg) with a yield of 28.18%.
MS m/z (ESI): 609.0 [M+1]

### The fourth step

### 1-(4-((4-(3-((2-((1S))-1-((tetrahydro-2H-pyran-2-yl)oxy)ethyl)-1H-imidazol-1-yl)methy l)isoxazol-5-yl)phenyl)ethynyl)benzyl)piperidin-4-carboxylic acid

Methyl 1-(4-((4-(3-((2-((1S))-1-((tetrahydro-2H-pyran-2-yl)oxy)ethyl)-1H-imidazol-1-yl)methyl)isoxazo l-5-yl)phenyl)ethynyl)benzyl)piperidin-4-carboxylate **17e** (50 mg, 82.14 µmol),and lithium hydroxide monohydrate (10.34 mg, 246.42 µmol) were sequentially added to a mixed solvent of tetrahydrofuran (1 mL) and water (0.2 mL), and heated to 60°C for 4 hours of reaction. After the reaction was completed, the reaction mixture was adjusted to acidity with 2M hydrochloric acid, and added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 1-(4-((4-(3-((2-((1S))-1-((tetrahydro-2H-pyran-2-yl)oxy)ethyl)-1H-imidazol-1-yl)methyl)isoxazo l-5-yl)phenyl)ethynyl)benzyl)piperidin-4-carboxylic acid **17f** (40 mg) with a yield of 81.89%.
MS m/z (ESI): 595.1 [M+1]

### The fifth step

### (S)-1-(4-((4-(3-((2-(1-hydroxyethyl)-1H-imidazol-1-yl)methyl)isoxazol-5-yl)phenyl)eth ynyl)benzyl)piperidin-4-carboxylic acid

1-(4-((4-(3-((2-((1S))-1-((tetrahydro-2H-pyran-2-yl)oxy)ethyl)-1H-imidazol-1-yl)methy l)isoxazol-5-yl)phenyl)ethynyl)benzyl)piperidin-4-carboxylic acid **17f** (40 mg, 67.26 µmol) was added sequentially to a mixed solvent of dichloromethane (5 mL) and trifluoro acetic acid (0.5 mL), and continuously stirred at room temperature for 3 hours. After the reaction was completed, the reaction mixture was concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(4-((4-(3-((2-(1-hydroxyethyl)-1H-imidazol-1-yl)methyl)isoxazol-5-yl)phenyl)ethynyl)ben zyl)piperidin-4-carboxylic acid **17** (10 mg) with a yield of 23.8%.
MS m/z (ESI): 511.1 [M+1]

¹H NMR (400 MHz, DMSO-*d*₆) *δ* 12.13 (br, 1H), 7.89 (d, *J=* 8.0 Hz, 2H), 7.69 (d, *J=* 8.4 Hz, 2H), 7.54 (d, *J =* 8.4 Hz, 2H), 7.37 (d, *J =* 8.0 Hz, 2H), 7.19 (s, 1H), 7.01 (s, 1H), 6.84 (s 1H), 5.51 - 5.42 (m, 3H), 4.89 (penta, *J =* 6.0 Hz, 1H), 3.50 (br, 2H), 2.75 (d, *J =* 8.0 Hz, 2H), 2.21 (br, 1H), 2.01 (br, 2H), 1.79 (d, *J =* 11.2 Hz, 2H), 1.63 - 1.50 (m, 2H), 1.48 (d, *J =* 6.4 Hz, 3H).

### Example 18

### (S)-1-(4-((4-(3-((2-(1-hydroxyethyl)-1H-imidazol-1-yl)methyl)isoxazol-5-yl)phenyl)eth ynyl)benzyl)piperidin-4-ol

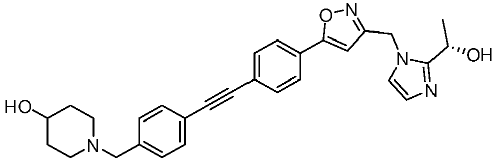

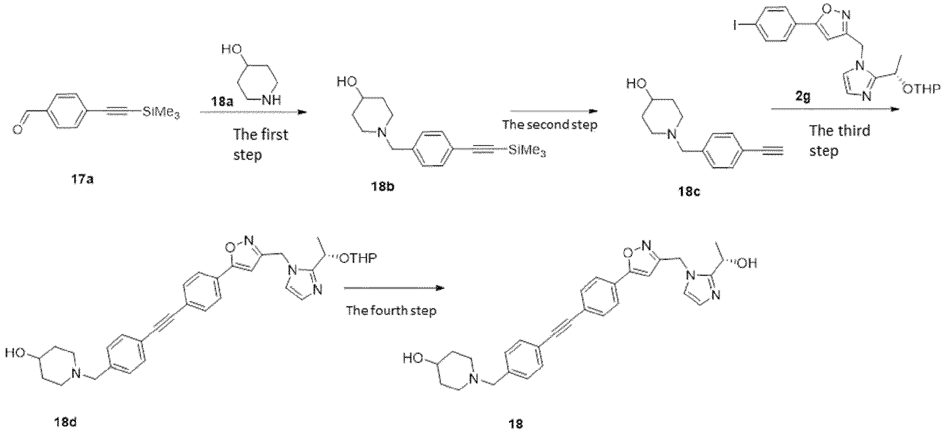

### The first step

### 1-(4-((Trimethylsilyl)ethynyl)benzyl)piperidin-4-ol

4-((Trimethylsilyl)ethynyl)benzaldehyde **17a** (200 mg, 988.51 µmol), piperidin-4-ol 18a (149.98 mg, 1.48 mmol) and acetic acid (0.5 mL) were dissolved in dichloroethane (5 mL), stirred for 30 minutes, added with sodium triacetoxyborohydride (628.52 mg, 2.97 mmol), and continuously stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 1-(4-((trimethylsilyl)ethynyl)benzyl)piperidin-4-ol **18b** (90 mg) with a yield of 31.67%.
MS m/z (ESI): 287.9 [M+1]

### The second step

### 1-(4-Ethynylbenzyl)piperidin-4-ol

1-(4-((Trimethylsilyl)ethynyl)benzyl)piperidin-4-ol **18b** (90 mg, 313.08 µmol) was dissolved in methanol (2 mL), added with potassium fluoride (72.76 mg, 1.25 mmol), and continuously stirred at room temperature for 4 hours. After the reaction was completed, the reaction mixture was concentrated to dryness under reduced pressure. The residue was added with ethyl acetate (30 mL) and water (15 mL) for layer separation. The aqueous phase was extracted with ethyl acetate (30 mL×2). The organic phase was washed with saturated sodium chloride solution (30 mL×2), dried over anhydrous sodium sulfate, filtered, and concentrated under reduced pressure to obtain 1-(4-ethynylbenzyl)piperidin-4-ol **18c** (50 mg) with a yield of 74.18%, which was used directly in the next reaction without purification.
MS m/z (ESI): 216.0 [M+1]

### The third step

### 1-(4-((4-(3-((2-((1S))-1-((tetrahydro-2H-pyran-2-yl)oxy)ethyl)-1H-imidazol-1-yl)methy l)isoxazol-5-yl)phenyl)ethynyl)benzyl)piperidin-4-ol

5-(4-Iodophenyl)-3-((2-((1S)-1-((tetrahydro-2H-pyran-2-yl)oxy)ethyl)-1H-imidazol-1-y l)methyl)isoxazole **2g** (100 mg, 208.63 µmol),1-(4-ethynylbenzyl)piperidin-4-ol **18c** (35.93 mg, 166.91 µmol),allylpalladium chloride dimer (7.63 mg, 20.86 µmol), tri-tert-butylphosphine (42.21 mg, 20.86 µmol) and triethylenediamine (70.21 mg, 625.90 µmol) were sequentially added to acetonitrile (2 mL), and the system was replaced with argon gas three times and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 1-(4-((4-(3-((2-((1S))-1-((tetrahydro-2H-pyran-2-yl)oxy)ethyl)-1H-imidazol-1-yl)methyl)isoxazo l-5-yl)phenyl)ethynyl)benzyl)piperidin-4-ol **18d** (40 mg) with a yield of 33.83%.
MS m/z (ESI): 567.0 [M+1]

### The fourth step

### (S)-1-(4-((4-(3-((2-(1-hydroxyethyl)-1H-imidazol-1-yl)methyl)isoxazol-5-yl)phenyl)eth ynyl)benzyl)piperidin-4-ol

1-(4-((4-(3-((2-((1S))-1-((tetrahydro-2H-pyran-2-yl)oxy)ethyl)-1H-imidazol-1-yl)methy l)isoxazol-5-yl)phenyl)ethynyl)benzyl)piperidin-4-ol **18d** (40 mg, 70.59 µmol) was added to a mixed solvent of dichloromethane (3 mL) and trifluoroacetic acid (1 mL), and continuously stirred at room temperature for 4 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(4-((4-(3-((2-(1-hydroxyethyl)-1H-imidazol-1-yl)methyl)isoxazol-5-yl)phenyl)ethynyl)ben zyl)piperidin-4-ol **18** (15.0 mg) with a yield of 35.09%.
MS m/z (ESI): 483.3 [M+1]

### Example 19

### (S)-4-((4-(3-((2-(1-hydroxyethyl)-1H-imidazol-1-yl)methyl)isoxazol-5-yl)phenyl)ethyn yl)benzonitrile

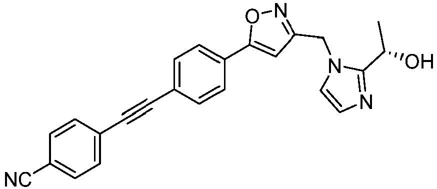

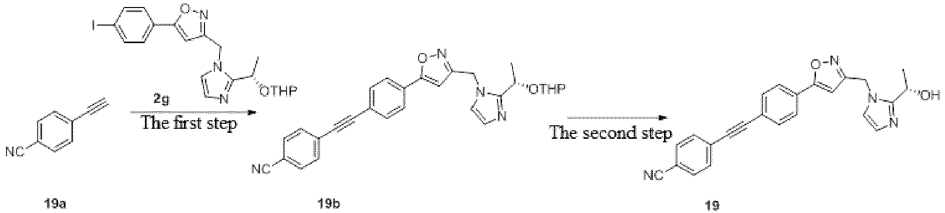

### The first step

### 4-((4-(3-((2-((1S)-1-((tetrahydro-2H-pyran-2-yl)oxy)ethyl)-1H-imidazol-1-yl)methyl)is oxazol-5-yl)phenyl)ethynyl)benzonitrile

5-(4-Iodophenyl)-3-((2-((1S)-1-((tetrahydro-2H-pyran-2-yl)oxy)ethyl)-1H-imidazol-1-y l)methyl)isoxazole **2g** (376.99 mg, 786.52 µmol),4-ethynylbenzonitrile **19a** (100 mg, 786.52 µmol),allylpalladium chloride dimer (28.78 mg, 78.65 µmol),tri-tert-butylphosphine (159.13 mg, 78.65 µmol) and triethylenediamine (264.67 mg, 2.36 mmol) were sequentially added to acetonitrile (5.0 mL), and the system was replaced with argon gas three times and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 4-((4-(3-((2-((1S)-1-((tetrahydro-2H-pyran-2-yl)oxy)ethyl)-1H-imidazol-1-yl)methyl)isoxazol-5-yl)phenyl)ethynyl)benzonitrile **19b** (320 mg) with a yield of 85.02%.
MS m/z (ESI): 478.9 [M+1]

### The second step

### (S)-4-((4-(3-((2-(1-hydroxyethyl)-1H-imidazol-1-yl)methyl)isoxazol-5-yl)phenyl)ethyn yl)benzonitrile

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzonitrile **19b** (30 mg, 62.69 µmol) was added to a mixed solvent of trifluoroacetic acid (0.5 mL) and dichloromethane (3 mL), and stirred at room temperature for 3 hours. The reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-4-((4-(3-((2-(1-hydroxyethyl)-1H-imidazol-1-yl)methyl)isoxazol-5-yl)phenyl)ethynyl)benzon itrile **19** (1.87 mg) with a yield of 5.67%.
MS m/z (ESI): 395.0 [M+1]

### Example 20

### 4-(4-((4-(3-((2-((S)-1-hydroxyethyl)-1H-imidazol-1-yl)methyl)isoxazol-5-yl)phenyl)eth ynyl)benzyl)morpholin-3-carboxylic acid

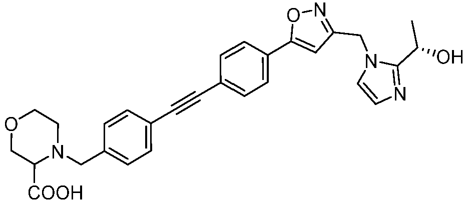

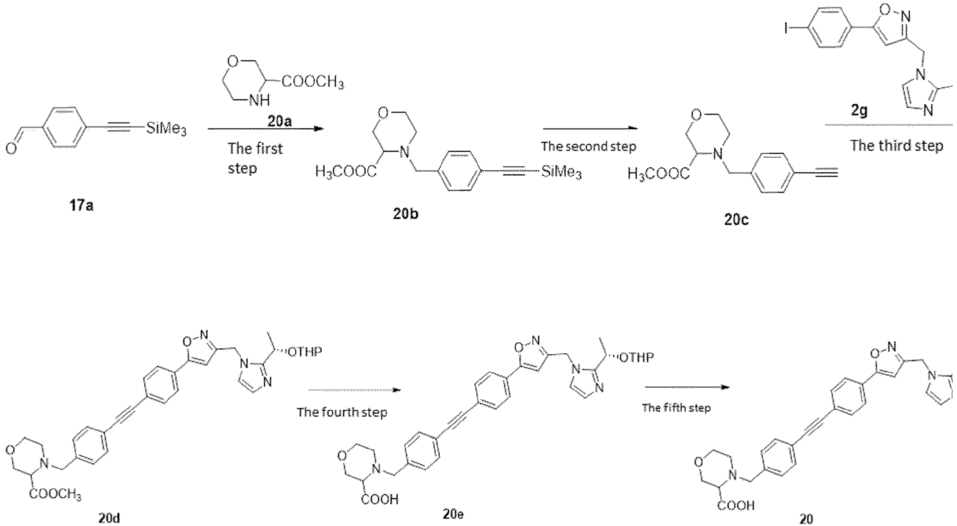

### The first step

### Methyl 4-(4-((trimethylsilyl)ethynyl)benzyl)morpholin-3-carboxylate

4-((Trimethylsilyl)ethynyl)benzaldehyde **17a** (1 g, 4.94 mmol), methyl morpholin-3-carboxylate **20a** (1.08 g, 5.93 mmol) and acetic acid (0.6 g, 24.71 mmol, 0.5 mL) were dissolved in dichloroethane (12 mL), stirred at room temperature for 1 hour, and slowly added with sodium triacetoxyborohydride (3.14 g, 14.83 mmol) for reaction at room temperature overnight. After the reaction was completed, the reaction mixture was added with water to quench the reaction, and extracted with dichloromethane (50ml×3). The organic phases were combined and concentrated under reduced pressure. The obtained residue was purified by silica gel column chromatography (eluent: System A) to obtain methyl 4-(4-((trimethylsilyl)ethynyl)benzyl)morpholin-3-carboxylate **20b** (0.9 g) with a yield of 54.93%.
MS m/z (ESI): 332.2 [M+1]

### The second step

### Methyl 4-(4-ethynylbenzyl)morpholin-3-carboxylate

Methyl 4-(4-((trimethylsilyl)ethynyl)benzyl)morpholin-3-carboxylate **20b** (0.9 g, 2.72 mmol) and tetrabutylammonium fluoride (851.87 mg, 3.26 mmol, 6 mL) were dissolved in tetrahydrofuran (10 mL) for 4 h of reaction at room temperature. After the reaction was completed, the reaction mixture was concentrated under reduced pressure. The obtained residue was purified by silica gel column chromatography (eluent: System A) to obtain methyl 4-(4-ethynylbenzyl)morpholin-3-carboxylate **20c** (0.45 g ) with a yield of 63.92%.
MS m/z (ESI): 260.1 [M+1]

### The third step

### Methyl 4-(4-((4-(3-((2-((1S)-1-((tetrahydro-2H-pyran-2-yl)oxy)ethyl)) -1H-imidazol-1-yl)methyl)isoxazol-5-yl)phenyl)ethynyl)benzyl)morpholin-3-carboxylate

Methyl 4-(4-ethynylbenzyl)morpholin-3-carboxylate **20c** (80 mg, 308.52 µmol), 5-(4-iodophenyl)-3-((2-((1S)-1-((tetrahydro-2H-pyran-2-yl)oxy)ethyl)-1H-imidazol-1-yl)methyl)i soxazole **2g** (147.88 mg, 308.52 µmol),allylpalladium chloride dimer (11.26 mg, 30.85 µmol), triethylenediamine (69.21 mg, 617.05 µmol) and tri-tert-butylphosphine (6.24 mg, 30.85 µmol) were sequentially added to acetonitrile (2 mL), and the system was replaced with argon gas three times for 12 h of reaction at room temperature. After the reaction was completed, the reaction mixture was concentrated under reduced pressure. The obtained residue was purified by silica gel column chromatography (eluent: System B) to obtain methyl 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)morpholin-3-carboxylate 20d (110 mg) with a yield of 58.38%.
MS m/z (ESI): 611.0 [M+1]

### The fourth step

### 4-(4-((4-(3-((2-((1S)-1-((tetrahydro-2H-pyran-2-yl)oxy)ethyl))-1H-imidazol-1-yl)methy l)isoxazol-5-yl)phenyl)ethynyl)benzyl)morpholin-3-carboxylic acid

Methyl 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)morpholin-3-carboxylate **20d** (105 mg, 171.93 µmol) was dissolved in a mixed solvent of tetrahydrofuran (2 ml) and water (0.5 mL), added with lithium hydroxide monohydrate (72.14 mg, 1.72 mmol) and heated to 70°C for reaction overnight. After the reaction was completed, the reaction mixture was adjusted to pH=3 with an appropriate amount of 2 M hydrochloric acid, washed with ethyl acetate (50 mL×3), concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 4-(4-((4-(3-((2-((1S)-1-((tetrahydro-2H-pyran-2-yl)oxy)ethyl))-1H-imidazol-1-yl)methyl)isoxazo l-5-yl)phenyl)ethynyl)benzyl)morpholin-3-carboxylic acid **20e** (90 mg) with a yield of 87.73%.
MS m/z (ESI): 597.3 [M+1]

### The fifth step

### 4-(4-((4-(3-((2-((S)-1-hydroxyethyl)-1H-imidazol-1-yl)methyl)isoxazol-5-yl)phenyl)eth ynyl)benzyl)morpholin-3-carboxylic acid

4-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)morpholin-3-carboxylic acid **20e** (90 mg, 150.84 µmol) was dissolved in dioxane (2 mL) and dropwise added with 4 M hydrochloric acid in dioxane solution (1 mL) for reaction at room temperature for 4 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 4-(4-((4-(3-((2-((S)-1-hydroxyethyl)-1H-imidazol-1-yl)methyl)isoxazol-5-yl)phenyl)ethynyl)ben zyl)morpholin-3-carboxylic acid **20** (20 mg) with a yield of 20.42%.
MS m/z (ESI): 513.2 [M+1]

### Example 21

### (1S)-1-(1-((2-(4-((4-(morpholinomethyl)phenyl)ethynyl)phenyl)-4,5-dihydrooxazol-4-yl )methyl)-1H-imidazol-2-yl)ethan-1-ol

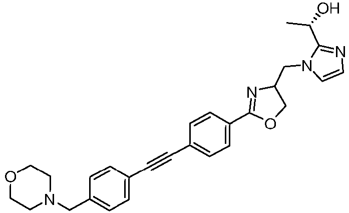

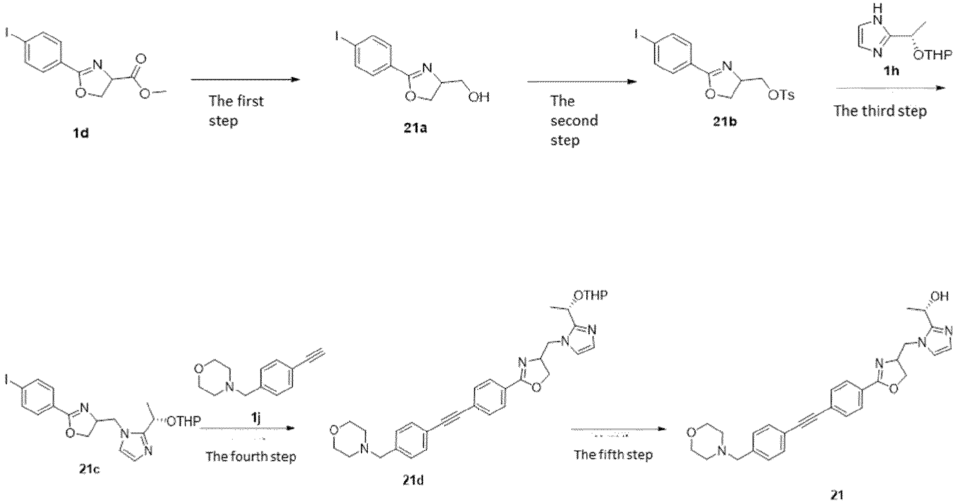

### The first step

### (2-(4-Iodophenyl)-4,5-dihydrooxazol-4-yl)methanol

Methyl 2-(4-iodophenyl)-4,5-dihydroxazol-4-carboxylate **1d** (3.5 g, 10.57 mmol) was added to tetrahydrofuran (80 mL), dropwise added with diisobutylaluminum hydride (1.0 M, 31.71 mL) at 0°C, continued to be stirred at 0°C for 2 hours, added with sodium potassium tartrate solution (50 mL) and ethyl acetate (50 mL), and stirred vigorously at room temperature for 3 hours. The reaction solution was extracted with ethyl acetate (30 mL×2). The aqueous layer was removed. The organic phases were combined, washed with saturated sodium chloride solution (30 mL×2), dried over anhydrous sodium sulfate, filtered, and concentrated under reduced pressure. The obtained residue was purified by silica gel column chromatography (eluent: System A) to obtain (2-(4-iodophenyl)-4,5-dihydrooxazol-4-yl)methanol 21a (2.5 g) with a yield of 78.03%.
MS m/z (ESI): 303.8 [M+1]

### The second step

### Methyl (2-(4-Iodophenyl)-4,5-dihydrooxazol-4-yl)4-methylbenzenesulfonate

(2-(4-Iodophenyl)-4,5-dihydrooxazol-4-yl)methanol **21a** (2.4 g, 7.92 mmol) was added to dichloromethane (60 mL), and slowly added with triethylamine (2.40 g, 23.75 mmol, 3.30 mL) dropwise at 0°C for reaction at room temperature overnight. The reaction solution was concentrated under reduced pressure. The obtained residue was purified by silica gel column chromatography (eluent: System A) to obtain methyl (2-(4-iodophenyl)-4,5-dihydrooxazol-4-yl)4-methylbenzenesulfonate **21b** (2.4 g) with a yield of 66.28%.
MS m/z (ESI): 457.7 [M+1]

### The third step

### 2-(4-Iodophenyl)-4-((2-((1S)-1-((tetrahydro-2H-pyran-2-yl)oxy)ethyl)-1H-imidazol-1-y l)methyl)-4,5-dihydrooxazole

2-((1S)-1-((tetrahydro-2H-pyran-2-yl)oxy)ethyl)-1H-imidazole **1h** (257.49 mg, 1.31 mmol) was added to N,N-dimethylformamide (10 mL), and then added with sodium hydride (56.88 mg, 1.31 mmol, 60% oil dispersion) followed by methyl 2-(4-iodophenyl)-4,5-dihydroxazol-4-yl)4-methylbenzenesulfonate **21b** (300 mg, 656.05 µmol). The reaction solution was heated to 70°C for 10 min of reaction. The reaction solution was cooled to 0°C, added with water (50 mL), and extracted with ethyl acetate (30 mL×2). The aqueous layer was removed. The organic phases were combined, and washed with saturated sodium chloride solution (30 mL×2), dried over anhydrous sodium sulfate, filtered, and concentrated under reduced pressure. The obtained residue was purified by silica gel column chromatography (eluent: System A) to obtain 2-(4-iodophenyl)-4-((2-((1S)-1-((tetrahydro-2H-pyran-2-yl)oxy)ethyl)-1H-imidazol-1-yl)methyl) -4,5-dihydrooxazole **21c** (130 mg) with a yield of 41.17%.
MS m/z (ESI): 481.8 [M+1]

### The fourth step

### 4-(4-((4-(4-((2-((1S)-1-((tetrahydro-2H-pyran-2-yl)oxy)ethyl)-1H-imidazol-1-yl)methyl )-4,5-dihydrooxazol-2-yl)phenyl)ethynyl)benzyl)morpholine

At room temperature, a mixture of 2-(4-iodophenyl)-4-((2-((1S)-1-((tetrahydro-2H-pyran-2-yl)oxy)ethyl)-1H-imidazol-1-yl)methyl) -4,5-dihydrooxazole **21c** (100 mg, 207.76 µmol) and 4-(4-ethynylbenzyl)morpholine **1j** (41.81 mg, 207.76 µmol) was sequentially added with bistriphenylphosphine palladium dichloride (5.83 mg, 8.31 µmol),tetrabutylammonium bromide (66.97 mg, 207.76 µmol) and piperidine (53.07 mg, 623.28 µmol). The reaction solution was heated to 70°C and stirred for 4 hours. The reaction solution was cooled to room temperature, and extracted with ethyl acetate (30 mL×2). The aqueous layer was removed. The organic phases were combined, washed with saturated sodium chloride solution (30 mL×2), dried over anhydrous sodium sulfate, filtered, and concentrated under reduced pressure to obtain 4-(4-((4-(4-((2-((1S)-1-((tetrahydro-2H-pyran-2-yl)oxy)ethyl)-1H-imidazol-1-yl)methyl)-4,5-dih ydrooxazol-2-yl)phenyl)ethynyl)benzyl)morpholine **21d** (100 mg) with a yield of 86.78%.
MS m/z (ESI): 555.3 [M+1]

### The fifth step

### (1S)-1-(1-((2-(4-((4-(morpholinomethyl)phenyl)ethynyl)phenyl)-4,5-dihydrooxazol-4-yl )methyl)-1H-imidazol-2-yl)ethan-1-ol

4-(4-((4-(4-((2-((1S))-1-((tetrahydro-2H-pyran-2-yl)oxy)ethyl)-1H-imidazol-1-yl)methy l)-4,5-dihydroxazol-2-yl)phenyl)ethynyl)benzyl)morpholine **21d** (100 mg, 180.28 µmol) was added to dichloromethane (2 mL), and added with trifluoroacetic acid (0.1 mL) for reaction at room temperature for 1 hour. The reaction solution was cooled to 0°C, adjusted to neutral pH with 1N sodium hydroxide solution, extracted with dichloromethane (30 mL×3), dried over anhydrous sodium sulfate, filtered, and concentrated under reduced pressure to obtain (1S)-1-(1-((2-(4-((4-(morpholinomethyl)phenyl)ethynyl)phenyl)-4,5-dihydrooxazol-4-yl)methyl)-1H-imidazol-2-yl)ethan-1-ol **21** (4 mg) with a yield of 4.15%.
MS m/z (ESI): 471.0 [M+1]

### Example 22

### (S)-1-(4-((4-(4-((2-(1-hydroxyethyl)-1H-imidazol-1-yl)methyl)oxazol-2-yl)phenyl)ethy nyl)benzyl)piperidin-4-ol

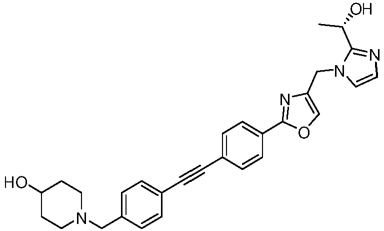

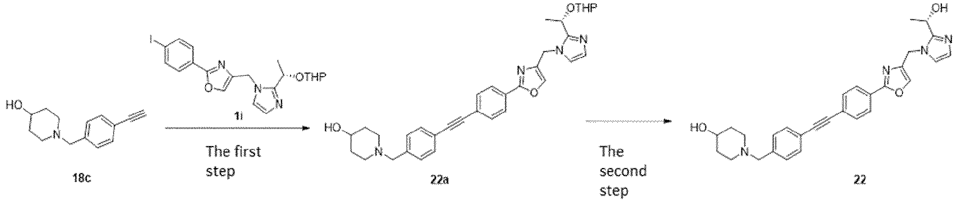

### The first step

### 1-(4-((4-(4-((2-((1S)-1-((tetrahydro-2H-pyran-2-yl)oxy)ethyl)-1H-imidazol-1-yl)methyl )oxazol-2-yl)phenyl)ethynyl)benzyl)piperidin-4-ol

At room temperature, an aqueous solution of 2-(4-iodophenyl)-4-((2-((1S)-1-((tetrahydro-2H-pyran-2-yl)oxy)ethyl)-1H-imidazol-1-yl)methyl) oxazole **1i** (60 mg, 125.18 µmol) and 1-(4-ethynylbenzyl)piperidin-4-ol 18c (26.95 mg, 125.18 µmol) was added with bistriphenylphosphine palladium dichloride (3.51 mg, 5.01 µmol), tetrabutylammonium bromide (40.35 mg, 125.18 µmol) and piperidine (31.98 mg, 375.54 µmol), heated to 70°C, and stirred for 5 hours. The reaction solution was cooled to room temperature, and extracted with ethyl acetate (30 mL×2). The aqueous layer was removed. The organic phases were combined, washed with saturated sodium chloride solution (30 mL×2), dried over anhydrous sodium sulfate, filtered, and concentrated under reduced pressure. The obtained residue was purified by silica gel column chromatography (eluent: System B) to obtain 1-(4-((4-(4-((2-((1S)-1-((tetrahydro-2H-pyran-2-yl)oxy)ethyl)-1H-imidazol-1-yl)methyl)oxazol-2 -yl)phenyl)ethynyl)benzyl)piperidin-4-ol **22a** (60 mg) with a yield of 84.58%.
MS m/z (ESI): 567.0 [M+1]

### The second step

### (S)-1-(4-((4-(4-((2-(1-hydroxyethyl)-1H-imidazol-1-yl)methyl)oxazol-2-yl)phenyl)ethy nyl)benzyl)piperidin-4-ol

1-(4-((4-(4-((2-((1S)-1-((tetrahydro-2H-pyran-2-yl)oxy)ethyl)-1H-imidazol-1-yl)methyl )oxazol-2-yl)phenyl)ethynyl)benzyl)piperidin-4-ol **22a** (60 mg, 105.88 µmol) was added to dichloromethane (2 mL), and added with trifluoroacetic acid (0.1 mL) for reaction at room temperature for 30 minutes. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(4-((4-(4-((2-(1-hydroxyethyl)-1H-imidazol-1-yl)methyl)oxazol-2-yl)phenyl)ethynyl)benzy l)piperidin-4-ol **22** (13 mg) with a yield of 20.07%.
MS m/z (ESI): 483.0 [M+1]

### Example 23

### (S)-4-((4-(3-((2-(1-hydroxyethyl)-1H-imidazol-1-yl)methyl)isoxazol-5-yl)phenyl)ethyn yl)benzamide

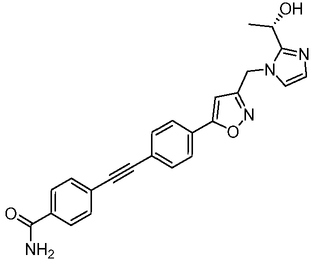

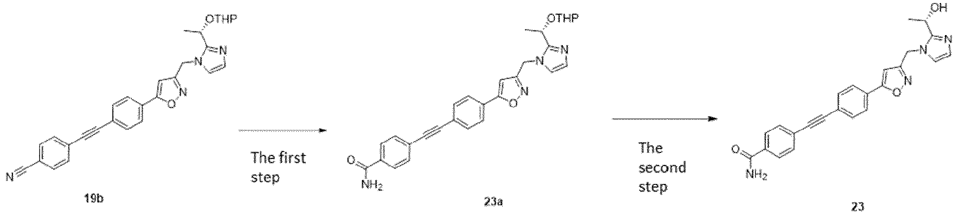

### The first step

### 4-((4-(3-((2-((1S)-1-((tetrahydro-2H-pyran-2-yl)oxy)ethyl)-1H-imidazol-1-yl)methyl)is oxazol-5-yl)phenyl)ethynyl)benzamide

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzonitrile **19b** (230 mg, 480.63 µmol) was added to dimethyl sulfoxide (5 mL), then added with 5 N sodium hydroxide aqueous solution (2 mL), added with hydrogen peroxide (1 mL) dropwise in an ice bath, warmed to room temperature, and continuously stirred for 1 hour. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated to dryness under reduced pressure to obtain 4-((4-(3-((2-((1S)-1-((tetrahydro-2H-pyran-2-yl)oxy)ethyl)-1H-imidazol-1-yl)methyl)isoxazol-5-yl)phenyl)ethynyl)benzamide **23a** (200 mg), which was used directly in the next reaction.
MS m/z (ESI): 496.9 [M+1]

### The second step

### (S)-4-((4-(3-((2-(1-hydroxyethyl)-1H-imidazol-1-yl)methyl)isoxazol-5-yl)phenyl)ethyn yl)benzamide

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzamide **23a** (180 mg, 362.50 µmol) was added to dichloromethane (10 mL), added with trifluoroacetic acid (1 mL), and continuously stirred at room temperature for 2 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-4-((4-(3-((2-(1-hydroxyethyl)-1H-imidazol-1-yl)methyl)isoxazol-5-yl)phenyl)ethynyl)benza mide 23 (89.96 mg) with a yield of 58.31%.
MS m/z (ESI): 413.0 [M+1]

¹H NMR (400 MHz, DMSO-*d*₆) *δ* 8.10 (s, 1H), 7.94 (d, *J =* 8.4 Hz, 4H), 7.80 (d, *J =* 2.0 Hz, 1H), 7.76 (d, *J =* 8.4 Hz, 2H), 7.72 (d, *J =* 2.0 Hz, 1H), 7.67 (d, *J =* 8.4 Hz, 2H), 7.51 (s, 1H), 7.21 (s, 1H), 6.44 (br, 1H), 5.73 (s, 2H), 5.26 (q, *J =* 6.8 Hz, 1H), 1.50 (d, *J =* 6.4 Hz, 3H).

### Example 24

### (S)-4-((4-(3-((2-(1-hydroxyethyl)-1H-imidazol-1-yl)methyl)isoxazol-5-yl)phenyl)ethyn yl)benzoic acid

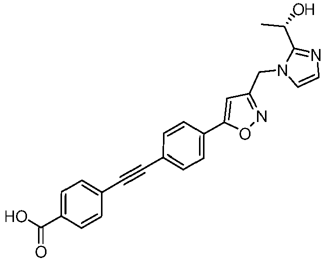

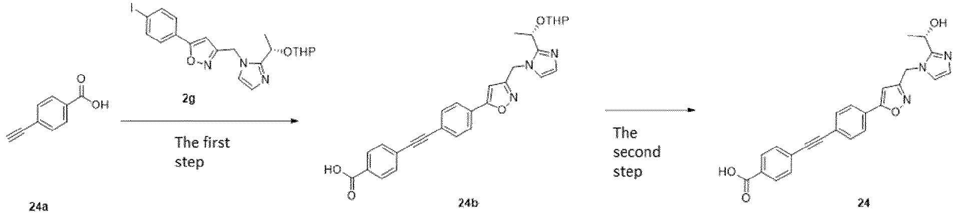

### The first step

### 4-((4-(3-((2-((1S)-1-((tetrahydro-2H-pyran-2-yl)oxy)ethyl)-1H-imidazol-1-yl)methyl)is oxazol-5-yl)phenyl)ethynyl)benzoic acid

4-Ethynylbenzoic acid 24a (25 mg, 171.07 µmol, commercially available), 5-(4-iodophenyl)-3-((2-((1S)-1-((tetrahydro-2H-pyran-2-yl)oxy)ethyl)-1H-imidazol-1-yl)methyl)i soxazole **2g** (82.0 mg, 171.07 µmol), allylpalladium(II) chloride dimer (6.24 mg, 17.11 µmol), triethylenediamine (38.4 mg, 342.13 µmol), and tri-tert-butylphosphine (10% toluene solution) (3.46 mg, 17.11 µmol) were sequentially added to acetonitrile (2 mL), and the system was replaced with argon gas three times for reaction at room temperature for 16 hours. After the reaction was completed, the reaction mixture was concentrated under reduced pressure. The obtained residue was purified by silica gel column chromatography (eluent: System B) to obtain 4-((4-(3-((2-((1S)-1-((tetrahydro-2H-pyran-2-yl)oxy)ethyl)-1H-imidazol-1-yl)methyl)isoxazol-5-yl)phenyl)ethynyl)benzoic acid **24b** (50 mg) with a yield of 58.75%.
MS m/z (ESI): 498.1 [M+1]

### The second step

### (S)-4-((4-(3-((2-(1-hydroxyethyl)-1H-imidazol-1-yl)methyl)isoxazol-5-yl)phenyl)ethyn yl)benzoic acid

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzoic acid **24b** (10 mg, 20.10 µmol) was dissolved in 4 N hydrochloric acid in dioxane solution (0.5 mL) for reaction at room temperature for 4 hours. After the reaction was completed, the reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-4-((4-(3-((2-(1-hydroxyethyl)-1H-imidazol-1-yl)methyl)isoxazol-5-yl)phenyl)ethynyl)benzoi c acid **24** (4 mg) with a yield of 36.34%.
MS m/z (ESI): 414.1[M+1]

### Example 25

### (S)-N-(2-Hydroxyethyl)-4-((4-(3-((2-(1-hydroxyethyl)-1H-imidazol-1-yl)methyl)isoxaz ol-5-yl)phenyl)ethynyl)benzamide

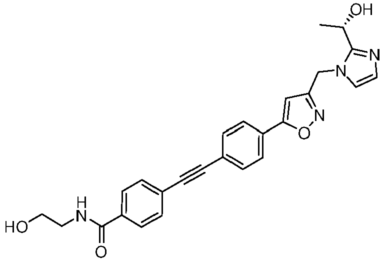

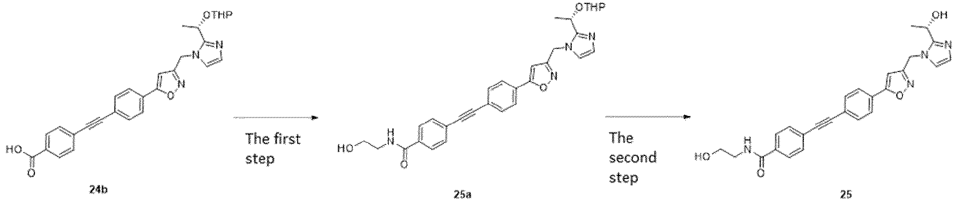

### The first step

### N-(2-Hydroxyethyl)-4-((4-(3-((2-((1S)-1-((tetrahydro-2H-pyran-2-yl)oxy)ethyl)-1H-imi dazol-1-yl)methyl)isoxazol-5-yl)phenyl)ethynyl)benzamide

2-Aminoethanol (6.14 mg, 100.49 µmol), 4-((4-(3-((2-((1S)-1-((tetrahydro-2H-pyran-2-yl)oxy)ethyl)-1H-imidazol-1-yl)methyl)isoxazol-5-yl)phenyl)ethynyl)benzoic acid **24b** (50 mg, 100.49 µmol), triethylamine (10.2 mg, 100.49 µmol), 1-(3-dimethylaminopropyl)-3-ethylcarbodiimide hydrochloride (28.9 mg, 150.74 µmol) and 1-hydroxybenzotriazole (20.4 mg, 150.74 µmol) were sequentially added to N,N-dimethylformamide (2 mL) for reaction at room temperature overnight. After the reaction was completed, the reaction mixture was extracted with ethyl acetate (50 mL×3). The organic phases were combined and concentrated under reduced pressure. The obtained residue was purified by silica gel column chromatography (eluent: System B) to obtain N-(2-hydroxyethyl)-4-((4-(3-((2-((1S)-1-((tetrahydro-2H-pyran-2-yl)oxy)ethyl)-1H-imidazol-1-yl )methyl)isoxazol-5-yl)phenyl)ethynyl)benzamide **25a** (40 mg) with a yield of 73.63%.
MS m/z (ESI): 541.1[M+1]

### The second step

### (S)-N-(2-hydroxyethyl)-4-((4-(3-((2-(1-hydroxyethyl)-1H-imidazol-1-yl)methyl)isoxaz ol-5-yl)phenyl)ethynyl)benzamide

N-(2-hydroxyethyl)-4-((4-(3-((2-((1S)-1-((tetrahydro-2H-pyran-2-yl)oxy)ethyl)-1H-imi dazol-1-yl)methyl)isoxazol-5-yl)phenyl)ethynyl)benzamide **25a** (40 mg, 73.99 µmol) was dissolved in dichloromethane (1 mL), and added with trifluoroacetic acid (0.5 mL) dropwise for reaction at room temperature for 4 hours. After the reaction was completed, the reaction mixture was concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-N-(2-hydroxyethyl)-4-((4-(3-((2-(1-hydroxyethyl)-1H-imidazol-1-yl)methyl)isoxazol-5-yl)ph enyl)ethynyl)benzamide **25** (5 mg) with a yield of 10.84%.
MS m/z (ESI): 457.3[M+1]

### Example 26

### (S)-1-(4-((4-(3-((2-(1-hydroxyethyl)-1H-imidazol-1-yl)methyl)isoxazol-5-yl)phenyl)eth ynyl)phenyl)ethan-1-one

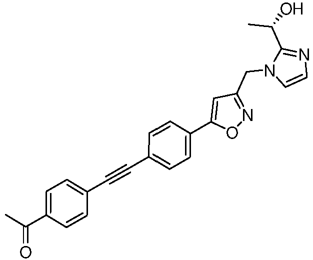

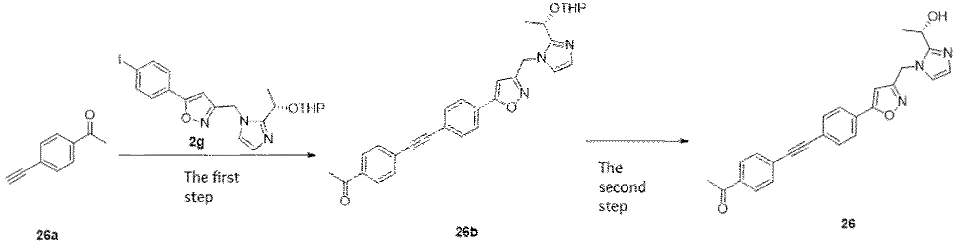

### The first step

### 1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenyl)ethan-1-one

5-(4-Iodophenyl)-3-((2-((1S)-1-((tetrahydro-2H-pyran-2-yl)oxy)ethyl)-1H-imidazol-1-y l)methyl)isoxazole **2g** (166 mg, 346.81 µmol), 1-(4-ethynylphenyl)ethan-1-one **26a** (50 mg, 346.81 µmol, commercially available), allylpalladium(II) chloride dimer (12.7 mg, 34.68 µmol), triethylenediamine (77.8 mg, 693.63 µmol) and tri-tert-butylphosphine (10% toluene solution) (7.02 mg, 34.68 µmol) were sequentially added to acetonitrile (2 mL), and the system was replaced with argon gas three times for reaction at room temperature for 12 hours. After the reaction was completed, the reaction mixture was concentrated under reduced pressure. The obtained residue was purified by silica gel column chromatography (eluent: System B) to obtain 1-(4-((4-(3-((2-((1S)-1-((tetrahydro-2H-pyran-2-yl)oxy)ethyl)-1H-imidazol-1-yl)methyl)isoxazol -5-yl)phenyl)ethynyl)phenyl)ethan-1-one **26b** (50 mg) with a yield of 29.09%.
MS m/z (ESI): 496.3 [M+1]

### The second step

### (S)-1-(4-((4-(3-((2-(1-hydroxyethyl)-1H-imidazol-1-yl)methyl)isoxazol-5-yl)phenyl)eth ynyl)phenyl)ethan-1-one

1-(4-((4-(3-((2-((1S))-1-((tetrahydro-2H-pyran-2-yl)oxy)ethyl)-1H-imidazol-1-yl)methy l)isoxazol-5-yl)phenyl)ethynyl)phenyl)ethan-1-one 26b (30 mg, 60.54 µmol) was dissolved in dichloromethane (2 mL), and added with trifluoroacetic acid (0.2 mL) dropwise for reaction at room temperature for 4 hours. After the reaction was completed, the reaction mixture was concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(4-((4-(3-((2-(1-hydroxyethyl)-1H-imidazol-1-yl)methyl)isoxazol-5-yl)phenyl)ethynyl)phe nyl)ethan-1-one **26** (10 mg) with a yield of 29.68%.
MS m/z (ESI): 412.1 [M+1]

### Example 27

### (S)-3-(4-((4-(3-((2-(1-hydroxyethyl)-1H-imidazol-1-yl)methyl)isoxazol-5-yl)phenyl)eth ynyl)phenyl)propanoic acid

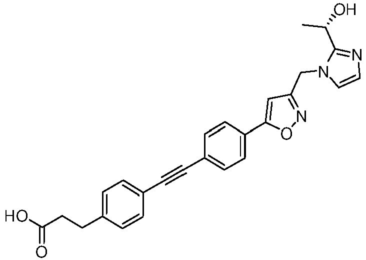

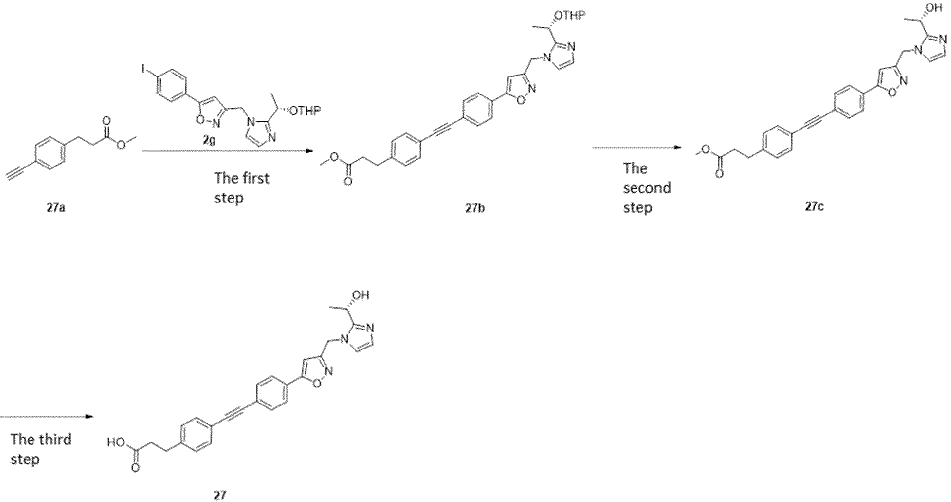

### The first step

### Methyl 3-(4-((4-(3-((2-((1S)-1-((tetrahydro-2H-pyran-2-yl)oxy)ethyl)-1H-imidazol-1-yl)methyl)isoxazol -5-yl)phenyl)ethynyl)phenyl)propanoate

5-(4-Iodophenyl)-3-((2-((1S)-1-((tetrahydro-2H-pyran-2-yl)oxy)ethyl)-1H-imidazol-1-y l)-methyl)isoxazole **2g** (50 mg, 104.32 µmol), methyl 3-(4-ethynylphenyl)propanoate 27a (39.27 mg, 208.63 µmol, prepared according to the published patent WO2010012650A), allylpalladium(II) chloride dimer (12.7 mg, 34.68 µmol), triethylenediamine (35.10 mg, 312.95 µmol) and tri-tert-butylphosphine (10% toluene solution) (42.21 mg, 20.86 µmol) were sequentially added into acetonitrile (5 mL), and the system was replaced with argon gas three times and continuously stirred at room temperature for 12 hours. The reaction solution was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain methyl 3-(4-((4-(3-((2-((1S)-1-((tetrahydro-2H-pyran-2-yl)oxy)ethyl)-1H-imidazol-1-yl)methyl)isoxazol -5-yl)phenyl)ethynyl)phenyl)propanoate **27b** (25 mg) with a yield of 44.41%.
MS m/z (ESI): 540.0 [M+1]

### The second step

### Methyl (S)-3-(4-((4-(3-((2-(1-hydroxyethyl)-1H-imidazol-1-yl)methyl)isoxazol-5-yl)phenyl)ethynyl)phenyl)propanoate

Methyl 3-(4-((4-(3-((2-((1S)-1-((tetrahydro-2H-pyran-2-yl)oxy)ethyl)-1H-imidazol-1-yl)methyl)isoxazol -5-yl)phenyl)ethynyl)phenyl)propanoate **27b** (25 mg, 46.33 µmol) was added to dichloromethane (3 mL), added with trifluoroacetic acid (1 mL), and continuously stirred at room temperature for 2 hours. After the reaction was completed, the reaction mixture was concentrated under reduced pressure to obtain methyl (S)-3-(4-((4-(3-((2-(1-hydroxyethyl)-1H-imidazol-1-yl)methyl)isoxazol-5-yl)phenyl)ethynyl)phe nyl)propanoate **27c** (20 mg), which was used directly in the next reaction.
MS m/z (ESI): 456.0 [M+1]

### The third step

### (S)-3-(4-((4-(3-((2-(1-hydroxyethyl)-1H-imidazol-1-yl)methyl)isoxazol-5-yl)phenyl)eth ynyl)phenyl)propanoic acid

Methyl (S)-3-(4-((4-(3-((2-(1-hydroxyethyl)-1H-imidazol-1-yl)methyl)isoxazol-5-yl)phenyl)ethynyl)phe nyl)propanoate **27c** (20 mg, 43.91 µmol) was added to a mixed solution of 2.5 N sodium hydroxide aqueous solution and tetrahydrofuran (4 mL, V:V=1:3), heated to 75°C, and continuously stirred for 3 hours. After the reaction was completed, the reaction solution was cooled to room temperature, concentrated under reduced pressure, and separated by preparative liquid chromatography (separation column AKZONOBEL Kromasil; 250×21.2 mm I.D.; 5 µm, 20 mL/min; mobile phase A: 0.05% TFA+H₂O, mobile phase B : CH₃CN) to obtain (S)-3-(4-((4-(3-((2-(1-hydroxyethyl)-1H-imidazol-1-yl)methyl)isoxazol-5-yl)phenyl)ethynyl)phe nyl)propanoic acid **27** (1.4 mg) with a yield of 6.50%.
MS m/z (ESI): 442.0 [M+1]

### Example 28

### (S)-5-((4-(3-((2-(1-Hydroxyethyl)-1H-imidazol-1-yl)methyl)isoxazol-5-yl)phenyl)ethyn yl)picolinamide

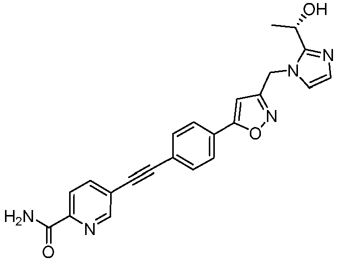

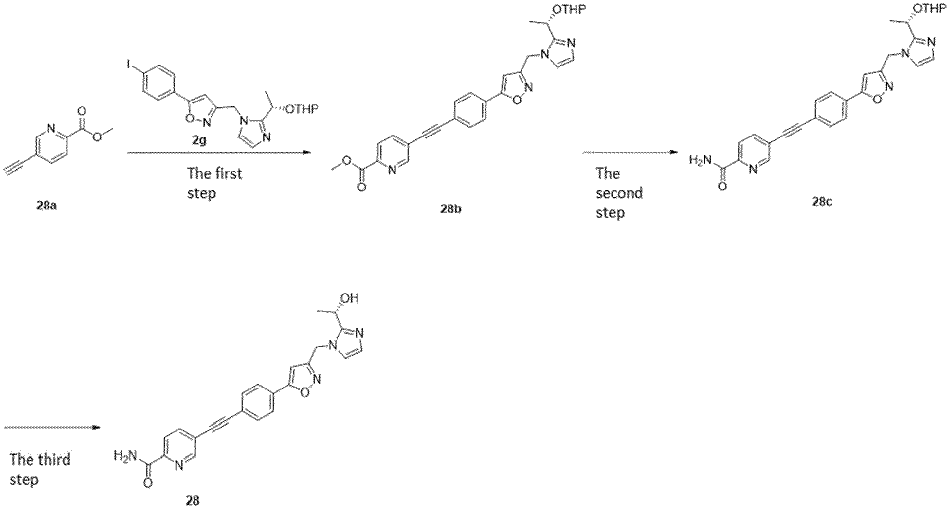

### The first step

### Methyl 5-((4-(3-((2-((1S)-1-((tetrahydro-2H-pyran-2-yl)oxy)ethyl)-1H-imidazol-1-yl)methyl)isoxazol-5-yl)phenyl)ethynyl)picolinate

5-(4-Iodophenyl)-3-((2-((1S)-1-((tetrahydro-2H-pyran-2-yl)oxy)ethyl)-1H-imidazol-1-y l)methyl)isoxazole **2g** (50 mg, 104.32 µmol), methyl 5-ethynylpicolinate **28a** (33.62 mg, 208.63 µmol, commercially available), allylpalladium(II) chloride dimer (3.82 mg, 10.43 µmol), tri-tert-butylphosphine (10% toluene solution) (2.11 mg, 10.43 µmol) and triethylenediamine (35.10 mg, 312.95 µmol) were sequentially added to acetonitrile (0.5 mL). The system was replaced with argon gas three times, and then the reaction solution was heated to 100°C and stirred for 16 hours. After the reaction was completed, the reaction mixture was concentrated under reduced pressure. The obtained residue was purified by silica gel column chromatography (eluent: System B) to obtain methyl 5-((4-(3-((2-((1S)-1-((tetrahydro-2H-pyran-2-yl)oxy)ethyl)-1H-imidazol-1-yl)methyl)isoxazol-5-yl)phenyl)ethynyl)picolinate **28b** (13 mg) with a yield of 24.31%.
MS m/z (ESI): 512.9 [M+1]

### The second step

### 5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinamide

Methyl 5-((4-(3-((2-((1S)-1-((tetrahydro-2H-pyran-2-yl)oxy)ethyl)-1H-imidazol-1-yl)methyl)isoxazol-5-yl)phenyl)ethynyl)picolinate **28b** (20 mg, 39.02 µmol) was added to a mixed solvent of ammonia (1 mL) and methanol (1 mL), and stirred at 25°C for 16 hours. After the reaction was completed, the reaction mixture was concentrated under reduced pressure. The residue was purified by preparative liquid chromatography (separating column AKZONOBEL Kromasil; 250×21.2 mm I.D.; 5 µm, 20 mL/min; mobile phase A: 0.05% TFA+H₂O, 1 mobile phase B: CH₃CN) to obtain 5-((4-(3-((2-((1S)-1-((tetrahydro-2H-pyran-2-yl)oxy)ethyl)-1H-imidazol-1-yl)methyl)isoxazol-5-yl)phenyl)ethynyl)picolinamide **28c** (12.5 mg) with a yield of 64.39%.
MS m/z (ESI): 498.2 [M+1]

### The third step

### (S)-5-((4-(3-((2-(1-hydroxyethyl)-1H-imidazol-1-yl)methyl)isoxazol-5-yl)phenyl)ethyn yl)picolinamide

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinamide **28c** (20 mg, 40.20 µmol) and ammonia (2.82 mg, 80.39 µmol) were sequentially added to methanol (0.5 mL), and stirred at room temperature for 16 hours. After the reaction was completed, the reaction mixture was concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-5-((4-(3-((2-(1-hydroxyethyl)-1H-imidazol-1-yl)methyl)isoxazol-5-yl)phenyl)ethynyl)picolin amide **28** (8 mg) with a yield of 35.85%.
MS m/z (ESI): 414.1 [M+1]

### Example 29

### (S)-1-(1-((5-(4-((4-(2-hydroxyethoxy)phenyl)ethynyl)phenyl)isoxazol-3-yl)methyl)-1H-imidazol-2-yl)ethan-1-ol

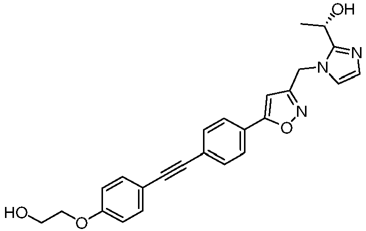

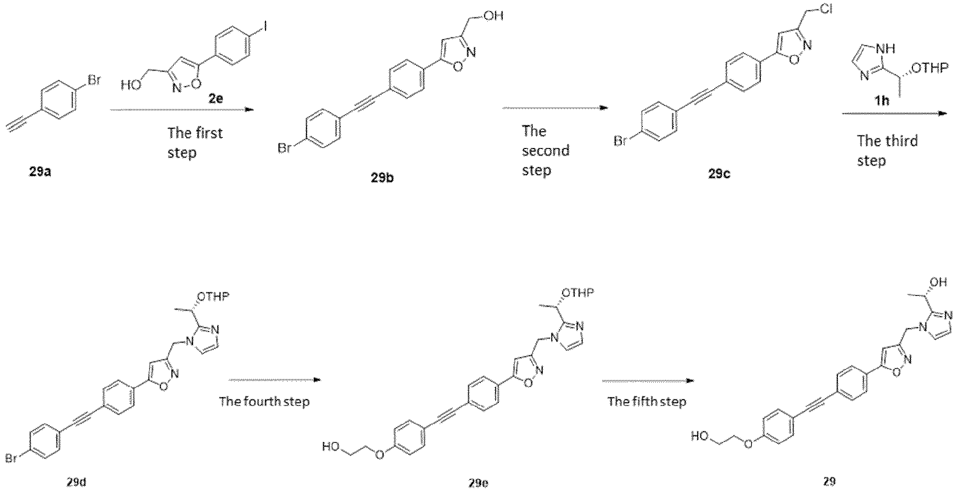

### The first step

### (5-(4-((4-Bromophenyl)ethynyl)phenyl)isoxazol-3-yl)methanol

(5-(4-Iodophenyl)isoxazol-3-yl)methanol **2e** (2 g, 6.64 mmol), 1-bromo-4-ethynylbenzene **29a** (1.32 g, 7.31 mmol), cuprous iodide (25.30 mg, 132.86 µmol), bistriphenylphosphine palladium dichloride (46.63 mg, 66.43 µmol) and triethylamine (2.08 g, 20.59 mmol, 2.86 mL) were sequentially added to tetrahydrofuran (5 mL), and the system was replaced with argon gas three times and stirred at room temperature for 16 hours. After the reaction was completed, the reaction mixture was concentrated under reduced pressure. The obtained residue was purified by silica gel column chromatography (eluent: System B) to obtain (5-(4-((4-bromophenyl)ethynyl)phenyl)isoxazol-3-yl)methanol **29b** (1.6 g) with a yield of 68.0%.
MS m/z (ESI): 353.8 [M+1]

### The second step

### 5-(4-((4-Bromophenyl)ethynyl)phenyl)-3-(chloromethyl)isoxazole

(5-(4-((4-Bromophenyl)ethynyl)phenyl)isoxazol-3-yl)methanol **29b** (1.25 g, 3.53 mmol) and triethylamine (714.22 mg, 7.06 mmol, 983.78 µL) were sequentially added to a mixed solvent of dichloromethane (4 mL) and N,N-dimethylformamide (1 mL), and the system was replaced with argon gas three times. The reaction solution was slowly added with methanesulfonyl chloride (4.04 g, 35.29 mmol, 2.73 mL) dropwise in an ice-water bath, heated to 65°C and stirred for 16 hours. After the reaction was completed, the reaction mixture was cooled to room temperature and concentrated under reduced pressure. The obtained residue was purified by silica gel column chromatography (eluent: System A) to obtain 5-(4-((4-bromophenyl)ethynyl)phenyl)-3-(chloromethyl)isoxazole **29c** (1.2 g) with a yield of 91.25%.
MS m/z (ESI): 371.8 [M+1]

### The third step

### 5-(4-((4-Bromophenyl)ethynyl)phenyl)-3-((2-((1S)-1-((tetrahydro-2H-pyran-2-yl)oxy)et hyl)-1H-imidazol-1-yl)methyl)isoxazole

2-((1S)-1-((Tetrahydro-2H-pyran-2-yl)oxy)ethyl)-1H-imidazole **1h** (347.58 mg, 1.77 mmol) and sodium hydride (50.26 mg, 1.93 mmol) were sequentially added to N,N-dimethylformamide (2 mL), and the system was replaced with argon gas three times. The reaction solution was stirred at 0°C for 0.5 hours, slowly dropwise added with 5-(4-((4-bromophenyl)ethynyl)phenyl)-3-(chloromethyl)isoxazole **29c** (600 mg, 1.61 mmol) in an ice-water bath, heated to 25°C and stirred for 16 hours. After the reaction was completed, the reaction mixture was added with water (20 mL) to quench the reaction, and filtered with suction. The solid was collected and dried under vacuum to obtain 5-(4-((4-bromophenyl)ethynyl)phenyl)-3-((2-((1S)-1-((tetrahydro-2H-pyran-2-yl)oxy)ethyl)-1H-i midazol-1-yl)methyl)isoxazole **29d** (700 mg) with a yield of 81.65%.
MS m/z (ESI): 531.8 [M+1]

### The fourth step

### 2-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenoxy)ethan-1-ol

5-(4-((4-Bromophenyl)ethynyl)phenyl)-3-((2-((1S)-1-((tetrahydro-2H-pyran-2-yl)oxy)et hyl)-1H-imidazol-1-yl)methyl)isoxazole **29d** (300 mg, 563.46 µmol), ethylene glycol (104.92 mg, 1.69 mmol, 94.27 µL), 5-di-tert-butylphosphine-1',3',5'-triphenyl-1'H-[1,4']dipyrazole (114.18 mg, 225.38 µmol) and cesium carbonate (550.76 mg, 1.69 mmol) were sequentially added to toluene (2 mL), and the system was replaced with argon gas three times. The reaction solution was slowly dropwise added with tris(dibenzylideneacetone)dipalladium (50.39 mg, 112.69 µmol), and stirred at 25°C for 16 hours. After the reaction was completed, the reaction mixture was added with water (20 mL) to quench the reaction, and extracted with ethyl acetate (20 mL×3). The combined organic phase was washed with saturated brine (20 mL), dried over anhydrous sodium sulfate, and concentrated under reduced pressure to obtain 2-(4-((4-(3-((2-((1S)-1-((tetrahydro-2H-pyran-2-yl)oxy)ethyl)-1H-imidazol-1-yl)methyl)isoxazol -5-yl)phenyl)ethynyl)phenoxy)ethan-1-ol **29e** (30 mg) with a yield of 10.37%.
MS m/z (ESI): 513.7 [M+1]

### The fifth step

### (S)-1-(1-((5-(4-((4-(2-hydroxyethoxy)phenyl)ethynyl)phenyl)isoxazol-3-yl)methyl)-1H-imidazol-2-yl)ethan-1-ol

2-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenoxy)ethan-1-ol **29e** (30 mg, 58.41 µmol) and trifluoroacetic acid (6.66 mg, 58.41 µmol) were sequentially added into dichloromethane (2 mL), and stirred at 25°C for 4 hours. After the reaction was completed, the reaction was concentrated under reduced pressure. The obtained residue was purified by thin layer chromatography (developing solvent: System B) to obtain (S)-1-(1-((5-(4-((4-(2-hydroxyethoxy)phenyl)ethynyl)phenyl)isoxazol-3-yl) methyl)-1H-imidazol-2-yl)ethan-1-ol **29** (10 mg) with a yield of 37.47%.
MS m/z (ESI): 429.9 [M+1]

### Example 30

### 1-(4-((4-(3-((2-((S)-1-hydroxyethyl)-1H-imidazol-1-yl)methyl)isoxazol-5-yl)phenyl)eth ynyl)phenyl)ethane-1,2-diol

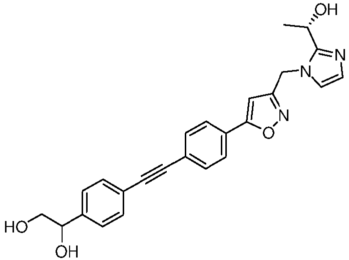

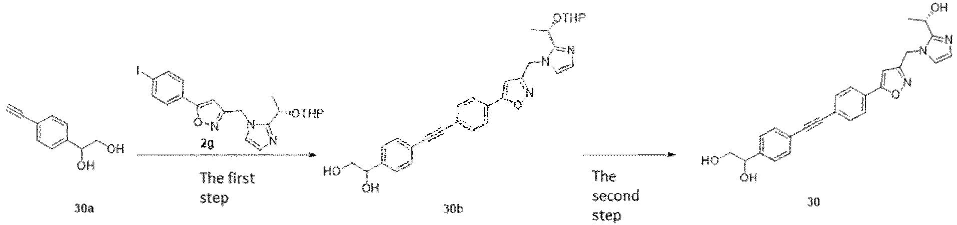

### The first step

### 1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenyl)ethane-1,2-diol

5-(4-Iodophenyl)-3-((2-((1S)-1-((tetrahydro-2H-pyran-2-yl)oxy)ethyl)-1H-imidazol-1-y l)methyl)isoxazole **2g** (50 mg, 104.32 µmol), 1-(4-ethynylphenyl)ethane-1,2-diol 30a (33.84 mg, 208.63 µmol, prepared according to the published patent WO2014142298A1), allylpalladium(II) chloride dimer (5.71 mg, 15.65 µmol), triethylenediamine (35.10 mg, 312.95 µmol), and tri-tert-butylphosphine (10% toluene solution) (42.21 mg, 20.86 µmol) were sequentially added to acetonitrile (5 mL), and the system was replaced with argon gas three times and continuously stirred at room temperature for 12 hours. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 1-(4-((4-(3-((2-((1S)-1-((tetrahydro-2H-pyran-2-yl)oxy)ethyl)-1H-imidazol-1-yl)methyl)isoxazol -5-yl)phenyl)ethynyl)phenyl)ethane-1,2-diol **30b** (40 mg) with a yield of 74.66%.
MS m/z (ESI): 514.2 [M+1]

### The second step

### 1-(4-((4-(3-((2-((S)-1-hydroxyethyl)-1H-imidazol-1-yl)methyl)isoxazol-5-yl)phenyl)eth ynyl)phenyl)ethane-1,2-diol

1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenyl)ethane-1,2-diol **30b** (40 mg, 77.88 µmol) and trifluoroacetic acid (0.5 mL) were sequentially added into dichloromethane (5 mL), and continuously stirred at room temperature for 12 hours. The reaction solution was concentrated under reduced pressure. The obtained residue was dissolved in methanol (5 mL), added with 2 N sodium hydroxide aqueous solution (1 mL), and continuously stirred at room temperature for 12 hours. After the reaction was completed, ther reactuion mixrture was concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 1-(4-((4-(3-((2-((S)-1-hydroxyethyl)-1H-imidazol-1-yl)methyl)isoxazol-5-yl)phenyl)ethynyl)phe nyl)ethane-1,2-diol **30** (20 mg) with a yield of 57.75%.
MS m/z (ESI): 430.1 [M+1]

### Example 31

### 3-((4-((4-(3-((2-((S)-1-hydroxyethyl))-1H-imidazol-1-yl)methyl)isoxazol-5-yl)phenyl)et hynyl)benzyl)amino)tetrahydrothiophene 1,1-dioxide

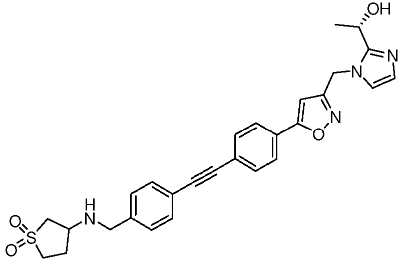

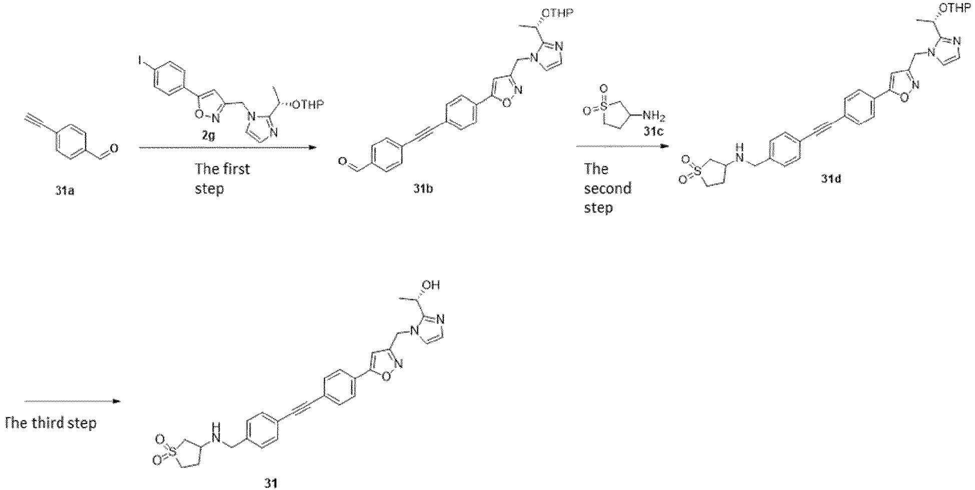

### The first step

### 4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde

4-Ethynylbenzaldehyde **31a** (40 mg, 307.35 µmol, commercially available), 5-(4-iodophenyl)-3-((2-((1S)-1-((tetrahydro-2H-pyran-2-yl)oxy)ethyl)-1H-imidazol-1-yl)methyl)i soxazole **2g** (147 mg, 307.35 µmol), allylpalladium(II) chloride dimer (11.2 mg, 30.74 µmol), triethylenediamine (69.0 mg, 614.71 µmol) and tri-tert-butylphosphine (10% toluene solution) (6.22 mg, 30.74 µmol) were dissolved in acetonitrile (2 mL), and the system was replaced with argon gas 3 times for reaction at room temperature for 16 hours. After the reaction was completed, the reaction mixture was concentrated under reduced pressure. The obtained residue was purified by silica gel column chromatography (eluent: System B) to obtain 4-((4-(3-((2-((1S)-1-((tetrahydro-2H-pyran-2-yl)oxy)ethyl)-1H-imidazol-1-yl)methyl)isoxazol-5-yl)phenyl)ethynyl)benzaldehyde **31b** (80 mg) with a yield of 54.05%.
MS m/z (ESI): 482.1 [M+1]

### The second step

### 3-((4-((4-(3-((2-((1S)-1-((tetrahydro-2H-pyran-2-yl)oxy)ethyl)-1H-imidazol-1-yl)methy l)isoxazol-5-yl)phenyl)ethynyl)benzyl)amino)tetrahydrothiophene 1,1-dioxide

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (50 mg, 103.83 µmol) and 3-aminotetrahydrothiophene 1,1-dioxide **31c** (21.39 mg, 124.60 µmol, commercially available) were dissolved in 1,2-dichloroethane (2 mL), added with acetic acid (0.2 mL), stirred at room temperature for 30 minutes, added with sodium acetate borohydride (66.02 mg, 311.50 µmol), and continuouly stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phases were washed with saturated sodium chloride solution (30 mL×2), dried over anhydrous sodium sulfate, filtered, and concentrated under reduced pressure. The obtained residue was purified by silica gel column chromatography (eluent: System B) to obtain 3-((4-((4-(3-((2-((1S))-1-((tetrahydro-2H-pyran-2-yl)oxy)ethyl)-1H-imidazol-1-yl)methyl)isoxaz ol-5-yl)phenyl)ethynyl)benzyl)amino)tetrahydrothiophene 1,1-dioxide **31d** (50 mg) with a yield of 80.16%.
MS m/z (ESI): 601.4 [M+1]

### The third step

### 3-((4-((4-(3-((2-((S)-1-hydroxyethyl))-1H-imidazol-1-yl)methyl)isoxazol-5-yl)phenyl)et hynyl)benzyl)amino)tetrahydrothiophene 1,1-dioxide

3-((4-((4-(3-((2-((1S))-1-((tetrahydro-2H-pyran-2-yl)oxy)ethyl)-1H-imidazol-1-yl)meth yl)isoxazol-5-yl)phenyl)ethynyl)benzyl)amino)tetrahydrothiophene 1,1-dioxide **31d** (50 mg, 83.23 µmol) was added to dichloromethane (5 mL), added with trifluoroacetic acid (0.5 mL), and continuously stirred at room temperature for 4 hours. The reaction solution was concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 3-((4-((4-(3-((2-((S)-1-hydroxyethyl))-1H-imidazol-1-yl)methyl)isoxazol-5-yl)phenyl)ethynyl)be nzyl)amino)tetrahydrothiophene 1,1-dioxide **31** (43.2 mg) with a yield of 79.42%.
MS m/z (ESI): 517.2 [M+1]

### Example 32

### (S)-2-(1-(4-((4-(3-((2-(1-hydroxyethyl)-1H-imidazol-1-yl)methyl)isoxazol-5-yl)phenyl) ethynyl)benzyl)azetidin-3-yl)acetic acid

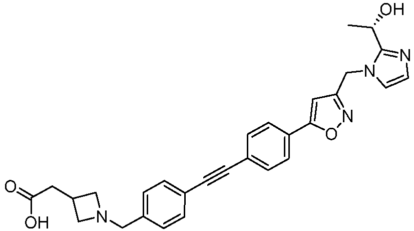

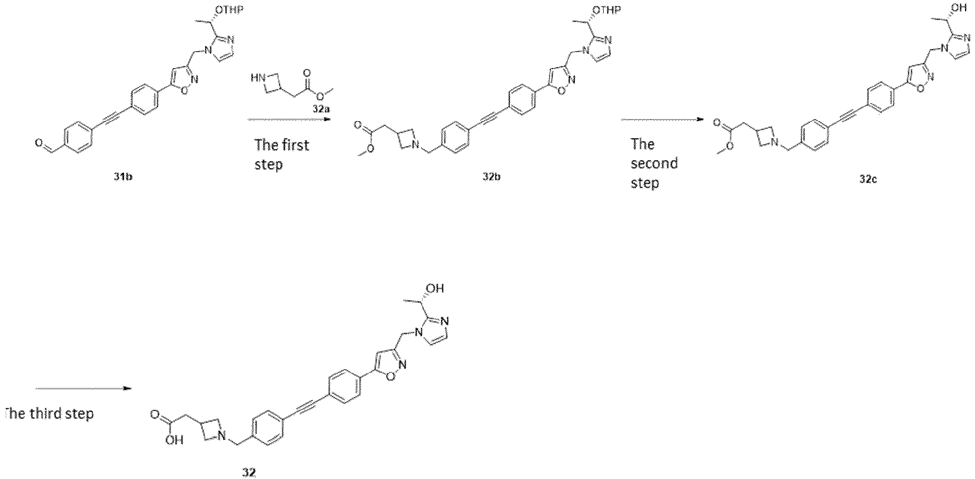

### The first step

### Methyl 2-(1-(4-((4-(3-((2-((1S)-1-((tetrahydro-2H-pyran-2-yl)oxy)ethyl)-1H-imidazol-1-yl)methyl)isoxa zol-5-yl)phenyl)ethynyl)benzyl)azetidin-3-yl)acetate

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (70 mg, 145.37 µmol), and methyl 2-(azetidin-3-yl)acetate **32a** (37.55 mg, 154.41 µmol, commercially available) were dissolved in 1,2-dichloroethane (5 mL), added with acetic acid (0.5 mL), stirred for 30 minutes, added with sodium acetate borohydride (92.43 mg, 436.10 µmol), and continuously stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phases were washed with saturated sodium chloride solution (30 mL×2), dried over anhydrous sodium sulfate, filtered, and concentrated under reduced pressure to obtain methyl 2-(1-(4-((4-(3-((2-((1S)-1-((tetrahydro-2H-pyran-2-yl)oxy)ethyl)-1H-imidazol-1-yl)methyl)isoxa zol-5-yl)phenyl)ethynyl)benzyl)azetidin-3-yl)acetate **32b** (80 mg), which was used directly in the next reaction.
MS m/z (ESI): 595.0 [M+1]

### The second step

### Methyl (S)-2-(1-(4-((4-(3-((2-(1-hydroxyethyl)-1H-imidazol-1-yl)methyl)isoxazol-5-yl)phenyl)ethynyl)b enzyl)azetidin-3-yl)acetate

Methyl 2-(1-(4-((4-(3-((2-((1S)-1-((tetrahydro-2H-pyran-2-yl)oxy)ethyl)-1H-imidazol-1-yl)methyl)isoxa zol-5-yl)phenyl)ethynyl)benzyl)azetidin-3-yl)acetate **32b** (80 mg, 134.52 µmol) was added with to dichloromethane (5 mL), added with trifluoroacetic acid (0.5 mL), and continuously stirred at room temperature for 2 hours. The reaction solution was concentrated under reduced pressure to obtain methyl (S)-2-(1-(4-((4-(3-((2-(1-hydroxyethyl)-1H-imidazol-1-yl)methyl)isoxazol-5-yl)phenyl)ethynyl)b enzyl)azetidin-3-yl)acetate **32c** (60 mg), which was used directly in the next reaction.
MS m/z (ESI): 511.2 [M+1]

### The third step

### (S)-2-(1-(4-((4-(3-((2-(1-hydroxyethyl)-1H-imidazol-1-yl)methyl)isoxazol-5-yl)phenyl) ethynyl)benzyl)azetidin-3-yl)acetic acid

Methyl (S)-2-(1-(4-((4-(3-((2-(1-hydroxyethyl)-1H-imidazol-1-yl)methyl) isoxazol-5-yl)phenyl)ethynyl)benzyl)azetidin-3-yl)acetate **32c** (60.0 mg, 117.51 µmol) was dissolved in a mixed solvent of tetrahydrofuran (2 mL) and methanol (2 mL), added with 2.5 N sodium hydroxide aqueous solution (1 mL), and stirred continuously for 4 hours at room temperature. The reaction mixture was adjusted to pH=6 with 1 N dilute hydrochloric acid, and concentrated to dryness under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-(1-(4-((4-(3-((2-(1-hydroxyethyl)-1H-imidazol-1-yl)methyl)isoxazol-5-yl)phenyl)ethynyl)b enzyl)azetidin-3-yl)acetic acid **32** (50.0 mg) with a yield of 68.29%.
MS m/z (ESI): 497.2 [M+1]

¹H NMR (400 MHz, CD₃OD) *δ* 7.78 (d, *J =* 8.4 Hz, 2H), 7.59 (d, *J =* 8.0 Hz, 2H), 7.49 (d, *J =* 8.0 Hz, 2H), 7.37 (d, *J =* 8.0 Hz, 2H), 7.16 (s, 1H), 6.94 (s, 1H), 6.75 (s, 1H), 5.53 (d, *J =* 16.0 Hz, 1H), 5.06 (q, *J =* 6.8 Hz, 1H), 4.43 (t, *J =* 8.0 Hz, 1H), 4.08 (dd, *J =* 9.2, 6.6 Hz, 1H), 3.78 (s, 2H), 3.31 (br, 1H), 2.79 - 2.61 (m, 4H), 2.33 (dd, *J =* 17.4, 6.6 Hz, 1H), 1.61 (d, *J =* 6.4 Hz, 3H).

### Example 33

### (S)-1-(1-((5-(4-((4-((1H-pyrazol-1-yl)methyl)phenyl)ethynyl)phenyl)isoxazol-3-yl)meth yl)-1H-imidazol-2-yl)ethan-1-ol

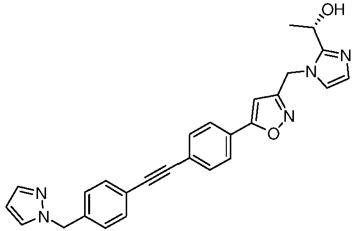

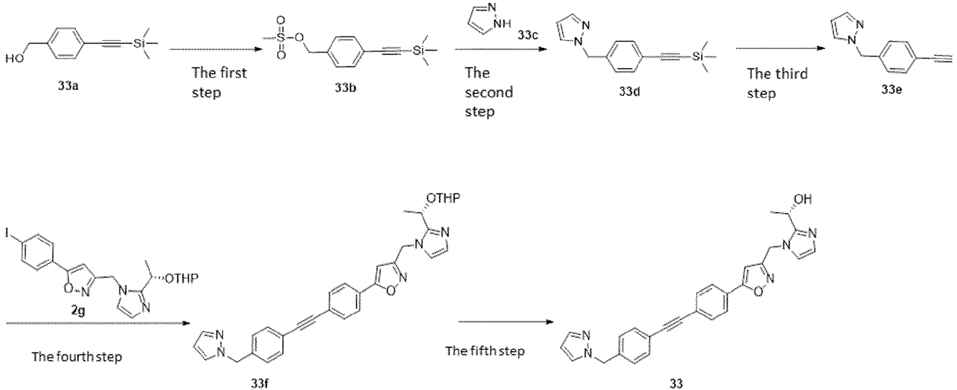

### The first step

### 4-((Trimethylsilyl)ethynyl)benzyl methanesulfonate

(4-((Trimethylsilyl)ethynyl)phenyl)methanol **33a** (500 mg, 2.45 mmol, commercially available) and triethylamine (742.81 mg, 7.34 mmol, 1.02 mL) were sequentially added to dichloromethane (10 mL), added with methylsulfonyl chloride (560.59 mg, 4.89 mmol, 378.77 µL) in an ice bath, and continuously stirred at room temperature for 12 hours. After the reaction was completed, the reaction mixture was added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 4-((trimethylsilyl)ethynyl)benzyl methanesulfonate **33b** (600 mg) with a yield of 60.77%.

### The second step

### 1-(4-((Trimethylsilyl)ethynyl)benzyl)-1H-pyrazole

4-((Trimethylsilyl)ethynyl)benzyl methanesulfonate **33b** (100 mg, 354.07 µmol), 1H-pyrazole **33c** (48.21 mg, 708.14 µmol, commercially available) and potassium carbonate (146.81 mg, 1.06 mmol) were sequentially added to N,N-dimethylformamide (2 mL), and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 1-(4-((trimethylsilyl)ethynyl)benzyl)-1H-pyrazole **33d** (80 mg) with a yield of 88.81%.
MS m/z (ESI): 255.0 [M+1]

### The third step

### 1-(4-Ethynylbenzyl)-1H-pyrazole

1-(4-((Trimethylsilyl)ethynyl)benzyl)-1H-pyrazole **33d** (80 mg, 314.46 µmol) and potassium fluoride (18.27 mg, 314.46 µmol) were sequentially added to methanol (2 mL), and continuously stirred at room temperature for 5 hours. The reaction solution was concentrated under reduced pressure. The obtained residue was purified by silica gel column chromatography (eluent: System B) to obtain 1-(4-ethynylbenzyl)-1H-pyrazole **33e** (30 mg) with a yield of 52.35%.
MS m/z (ESI): 183.0 [M+1]

### The fourth step

### 5-(4-((4-((1H-pyrazol-1-yl)methyl)phenyl)ethynyl)phenyl)-3-((2-((1S)-1-((tetrahydro-2 H-pyran-2-yl)oxy)ethyl)-1H-imidazol-1-yl)methyl)isoxazole

1-(4-Ethynylbenzyl)-1H-pyrazole **33e** (30.0 mg, 164.64 µmol), 5-(4-iodophenyl)-3-((2-((1S)-1-((tetrahydro-2H-pyran-2-yl)oxy)ethyl)-1H-imidazol-1-yl)methyl)i soxazole **2g** (71.02 mg, 148.17 µmol), allylpalladium(II) chloride dimer (12.02 mg, 32.93 µmol), triethylenediamine (55.40 mg, 493.91 µmol) and tri-tert-butylphosphine (10% toluene solution) (66.62 mg, 32.93 µmol) were sequentially added to acetonitrile (2 mL), and the system was replaced with argon gas three times and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 5-(4-((4-((1H-pyrazol-1-yl)methyl)phenyl)ethynyl)phenyl)-3-((2-((1S)-1-((tetrahydro-2H-pyran-2-yl)oxy)ethyl)-1H-imidazol-1-yl)methyl)isoxazole **33f** (50 mg) with a yield of 56.91%.
MS m/z (ESI): 534.2 [M+1]

### The fifth step

### (S)-1-(1-((5-(4-((4-((1H-pyrazol-1-yl)methyl)phenyl)ethynyl)phenyl)isoxazol-3-yl)meth yl)-1H-imidazol-2-yl)ethan-1-ol

5-(4-((4-((1H-pyrazol-1-yl)methyl)phenyl)ethynyl)phenyl)-3-((2-((1S)-1-((tetrahydro-2 H-pyran-2-yl)oxy)ethyl)-1H-imidazol-1-yl)methyl)isoxazole **33f** (50 mg, 93.70 µmol) was added to dichloromethane (5 mL), added with trifluoroacetic acid (0.5 mL), and continuously stirred at room temperature for 2 hours. The reaction solution was concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-((1H-pyrazol-1-yl)methyl)phenyl)ethynyl)phenyl)isoxazol-3-yl)methyl)-1H-i midazol-2-yl)ethan-1-ol 33 (22.9 mg) with a yield of 41.55%.
MS m/z (ESI): 450.1 [M+1]

### Example 34

### (S)-2-((4-((4-(3-((2-(1-hydroxyethyl)-1H-imidazol-1-yl)methyl)isoxazol-5-yl)phenyl)et hynyl)benzyl)amino)ethan-1-ol

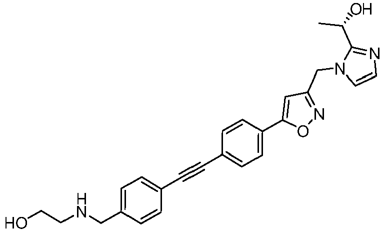

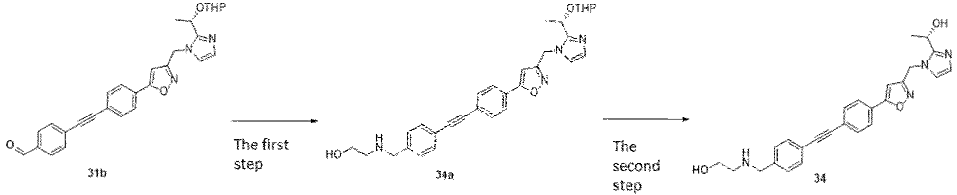

### The first step

### 2-((4-((4-(3-((2-((1S))-1-((tetrahydro-2H-pyran-2-yl)oxy)ethyl)-1H-imidazol-1-yl)meth yl)isoxazol-5-yl)phenyl)ethynyl)benzyl)amino)ethan-1-ol

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (100 mg, 207.67 µmol), and 2-aminoethanol (25.37 mg, 415.33 µmol) were dissolved in 1,2-dichloroethane (5 mL), added with acetic acid (0.5 mL), stirred for 30 minutes, added with sodium acetate borohydride (132.04 mg, 623.00 µmol), and continuously stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 2-((4-((4-(3-((2-((1S))-1-((tetrahydro-2H-pyran-2-yl)oxy)ethyl)-1H-imidazol-1-yl)methyl)isoxaz ol-5-yl)phenyl)ethynyl)benzyl)amino)ethan-1-ol **34a** (50 mg) with a yield of 45.72%.
MS m/z (ESI): 527.3 [M+1]

### The second step

### (S)-2-((4-((4-(3-((2-(1-hydroxyethyl)-1H-imidazol-1-yl)methyl)isoxazol-5-yl)phenyl)et hynyl)benzyl)amino)ethan-1-ol

2-((4-((4-(3-((2-((1S))-1-((tetrahydro-2H-pyran-2-yl)oxy)ethyl)-1H-imidazol-1-yl)meth yl)isoxazol-5-yl)phenyl)ethynyl)benzyl)amino)ethan-1-ol **34a** (50 mg, 94.94 µmol) was added to dichloromethane (5 mL), added with trifluoroacetic acid (0.5 mL), and continuously stirred at room temperature for 2 hours. The reaction solution was concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-((4-((4-(3-((2-(1-hydroxyethyl)-1H-imidazol-1-yl)methyl)isoxazol-5-yl)phenyl)ethynyl)ben zyl)amino)ethan-1-ol **34** (40.0 mg) with a yield of 66.47%.
MS m/z (ESI): 443.2 [M+1]

### Example 35

### (S)-2-((4-((4-(3-((2-(1-hydroxyethyl)-1H-imidazol-1-yl)methyl)isoxazol-5-yl)phenyl)et hynyl)benzyl)amino)acetamide

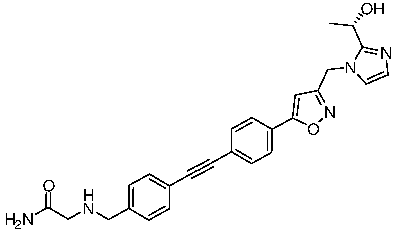

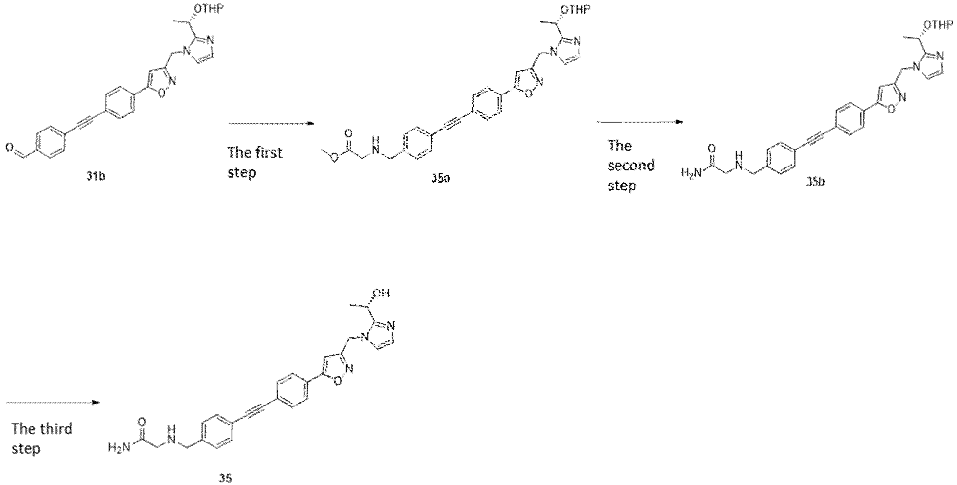

### The first step

### Methyl (4-((4-(3-((2-((1S)-1-((tetrahydro-2H-pyran-2-yl)oxy)ethyl) -1H-imidazol-1-yl)methyl)isoxazol-5-yl)phenyl)ethynyl)benzyl)glycinate

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (200 mg, 415.33 µmol) and methyl glycinate (104.29 mg, 830.67 µmol) were dissolved in 1,2-dichloroethane (20 mL), added with acetic acid (0.5 mL), stirred for 30 minutes, added with sodium acetate borohydride (264.08 mg, 1.25 mmol), and continuously stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain methyl (4-((4-(3-((2-((1S)-1-((tetrahydro-2H-pyran-2-yl)oxy)ethyl)-1H-imidazol-1-yl)methyl)isoxazol-5 -yl)phenyl)ethynyl)benzyl)glycinate **35a** (200 mg) with a yield of 86.82%.
MS m/z (ESI): 555.0 [M+1]

### The second step

### 2-((4-((4-(3-((2-((1S))-1-((tetrahydro-2H-pyran-2-yl)oxy)ethyl)-1H-imidazol-1-yl)meth yl)isoxazol-5-yl)phenyl)ethynyl)benzyl)amino)acetamide

Methyl (4-((4-(3-((2-((1S)-1-((tetrahydro-2H-pyran-2-yl)oxy)ethyl) -1H-imidazol-1-yl)methyl)isoxazol-5-yl)phenyl)ethynyl)benzyl)glycinate **35a** (50 mg, 90.15 µmol) and ammonia (3 mL) were sequentially added to ethanol (3 mL), and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure to obtain 2-((4-((4-(3-((2-((1S))-1-((tetrahydro-2H-pyran -2-yl)oxy)ethyl)-1H-imidazol-1-yl)methyl)isoxazol-5-yl)phenyl)ethynyl)benzyl)amino)acetamide **35b** (40 mg), which was used directly in the next reaction.
MS m/z (ESI): 540.0 [M+1]

### The third step

### (S)-2-((4-((4-(3-((2-(1-hydroxyethyl)-1H-imidazol-1-yl)methyl)isoxazol-5-yl)phenyl)et hynyl)benzyl)amino)acetamide

2-((4-((4-(3-((2-((1S))-1-((tetrahydro-2H-pyran-2-yl)oxy)ethyl)-1H-imidazol-1-yl)meth yl)isoxazol-5-yl)phenyl)ethynyl)benzyl)amino)acetamide **35b** (40 mg, 74.13 µmol) and trifluoroacetic acid (0.5 mL) were sequentially added to dichloromethane (2 mL), and continuously stirred at room temperature for 12 hours. The reaction solution was concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-((4-((4-(3-((2-(1-hydroxyethyl)-1H-imidazol-1-yl)methyl)isoxazol-5-yl)phenyl)ethynyl)ben zyl)amino)acetamide 35 (10.4 mg) with a yield of 22.42%.
MS m/z (ESI): 456.2 [M+1]

### Example 36

### (S)-1-((5-((4-(3-((2-(1-hydroxyethyl)-1H-imidazol-1-yl)methyl)isoxazol-5-yl)phenyl)et hynyl)pyridin-2-yl)methyl)piperidin-4-ol

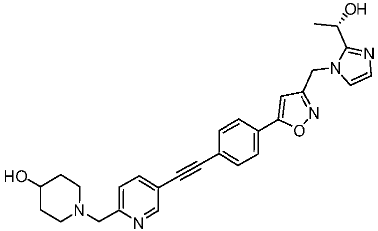

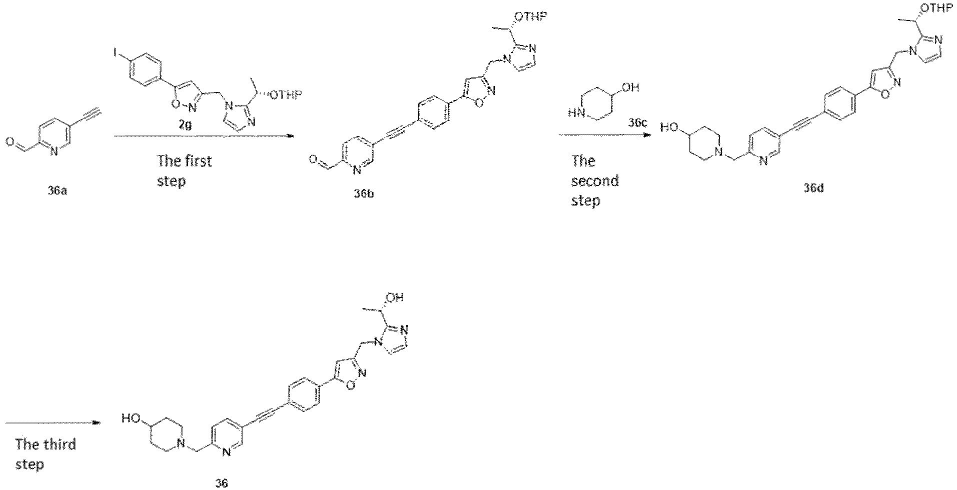

### The first step

### 5-((4-(3-((2-((1S)-1-((tetrahydro-2H-pyran-2-yl)oxy)ethyl)-1H-imidazol-1-yl)methyl)is oxazol-5 -yl)phenyl)ethynyl)picolinaldehyde

5-(4-Iodophenyl)-3-((2-((1S)-1-((tetrahydro-2H-pyran-2-yl)oxy)ethyl)-1H-imidazol-1-y l)methyl)isoxazole **2g** (200 mg, 417.27 µmol), 5-ethynylpicolinaldehyde **36a** (109.43 mg, 834.53 µmol), allylpalladium(II) chloride dimer (30.46 mg, 83.45 µmol), triethylenediamine (140.41 mg, 1.25 mmol) and tri-tert-butylphosphine (10% toluene solution) (168.84 mg, 83.45 µmol) were sequentially added to acetonitrile (2 mL), and the system was replaced with argon gas three times and continuously stirred at room temperature for 12 hours. After the reaction was completed, the reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain 5-((4-(3-((2-((1S)-1-((tetrahydro-2H-pyran-2-yl)oxy)ethyl)-1H-imidazol-1-yl)methyl)isoxazol-5 -yl)phenyl)ethynyl)picolinaldehyde **36b** (108 mg) with a yield of 53.64%.
MS m/z (ESI): 483.3 [M+1]

### The second step

### 1-((5-((4-(3-((2-((1S))-1-((tetrahydro-2H-pyran-2-yl)oxy)ethyl)-1H-imidazol-1-yl)meth yl)isoxazol-5-yl)phenyl)ethynyl)pyridin-2-yl)methyl)piperidin-4-ol

5-((4-(3-((2-((1S)-1-((tetrahydro-2H-pyran-2-yl)oxy)ethyl)-1H-imidazol-1-yl)methyl)is oxazol-5 -yl)phenyl)ethynyl)picolinaldehyde **36b** (50 mg, 103.62 µmol), and piperidin-4-ol **36c** (15.72 mg, 155.43 µmol) were dissolved in dichloromethane (0.5 mL), stirred for 30 minutes, added with sodium acetate borohydride (43.92 mg, 207.24 µmol), and continuously stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phases were washed with saturated sodium chloride solution (30 mL×2), dried over anhydrous sodium sulfate, filtered, and concentrated under reduced pressure. The obtained residue was purified by silica gel column chromatography (eluent: System B) to obtain 1-((5-((4-(3-((2-((1S))-1-((tetrahydro-2H-pyran-2-yl)oxy)ethyl)-1H-imidazol-1-yl)methyl)isoxaz ol-5-yl)phenyl)ethynyl)pyridin-2-yl)methyl)piperidin-4-ol **36d** (20 mg) with a yield of 34.00%.
MS m/z (ESI): 568.4 [M+1]

### The third step

### (S)-1-((5-((4-(3-((2-(1-hydroxyethyl)-1H-imidazol-1-yl)methyl)isoxazol-5-yl)phenyl)et hynyl)pyridin-2-yl)methyl)piperidin-4-ol

1-((5-((4-(3-((2-((1S))-1-((tetrahydro-2H-pyran-2-yl)oxy)ethyl)-1H-imidazol-1-yl)meth yl)isoxazol-5-yl)phenyl)ethynyl)pyridin-2-yl)methyl)piperidin-4-ol **36d** (20 mg, 35.23 µmol) and trifluoroacetic acid (12.05 mg, 105.69 µmol) were sequentially added to dichloromethane (2 mL), and continuously stirred at room temperature for 12 hours. The reaction solution was concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-((5-((4-(3-((2-(1-hydroxyethyl)-1H-imidazol-1-yl)methyl)isoxazol-5-yl)phenyl)ethynyl)pyr idin-2-yl)methyl)piperidin-4-ol 36 (10 mg) with a yield of 47.36%.
MS m/z (ESI): 484.3 [M+1]

### Example 37

### (S)-1-(1-((5-(4-((6-(hydroxymethyl)pyridin-3-yl)ethynyl)phenyl)isoxazol-3-yl)methyl)-1H-imidazol-2-yl)ethan-1-ol

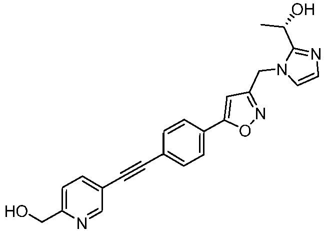

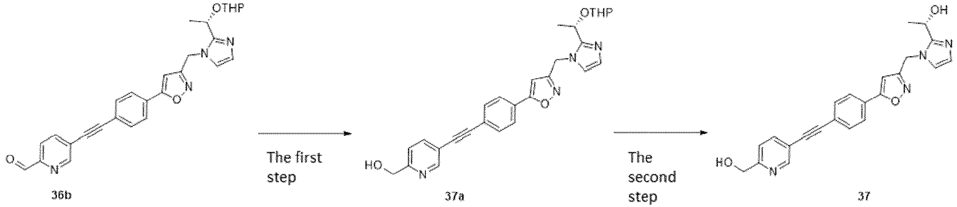

### The first step

### (5-((4-(3-((2-((1S)-1-((tetrahydro-2H-pyran-2-yl)oxy)ethyl)-1H-imidazol-1-yl)methyl)is oxazol-5-yl)phenyl)ethynyl)pyridin-2-yl)methanol

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) was dissolved in dichloromethane (0.5 mL), added with sodium acetate borohydride (39.53 mg, 186.52 µmol), and continuously stirred at room temperature for 12 hours. After the reaction was completed, the reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phases were washed with saturated sodium chloride solution (30 mL×2), dried over anhydrous sodium sulfate, filtered, and concentrated under reduced pressure to obtain (5-((4-(3-((2-((1S)-1-((tetrahydro-2H-pyran-2-yl)oxy)ethyl)-1H-imidazol-1-yl)methyl)isoxazol-5 -yl)phenyl)ethynyl)pyridin-2-yl)methanol **37a** (20 mg), which was used directly in the next reaction.
MS m/z (ESI): 485.0 [M+1]

### The second step

### (S)-1-(1-((5-(4-((6-(hydroxymethyl)pyridin-3-yl)ethynyl)phenyl)isoxazol-3-yl)methyl)-1H-imidazol-2-yl)ethan-1-ol

(5-((4-(3-((2-((1S)-1-((tetrahydro-2H-pyran-2-yl)oxy)ethyl)-1H-imidazol-1-yl)methyl)is oxazol-5-yl)phenyl)ethynyl)pyridin-2-yl)methanol **37a** (10 mg, 20.64 µmol) and trifluoroacetic acid (4.71 mg, 41.28 µmol) were sequentially added to dichloromethane (2 mL), and continuously stirred at room temperature for 4 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((6-(hydroxymethyl)pyridin-3-yl)ethynyl)phenyl)isoxazol-3-yl)methyl)-1H-imida zol-2-yl)ethan-1-ol 37 (4.90 mg) with a yield of 44.77%.
MS m/z (ESI): 401.3 [M+1]

### Example 38

### (S)-2-(3-((4-(3-((2-(1-hydroxyethyl)-1H-imidazol-1-yl)methyl)isoxazol-5-yl)phenyl)eth ynyl)phenoxy)acetonitrile

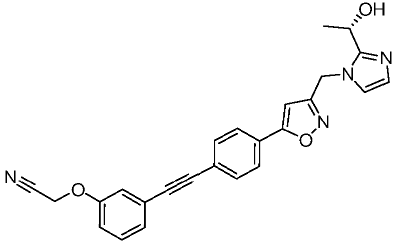

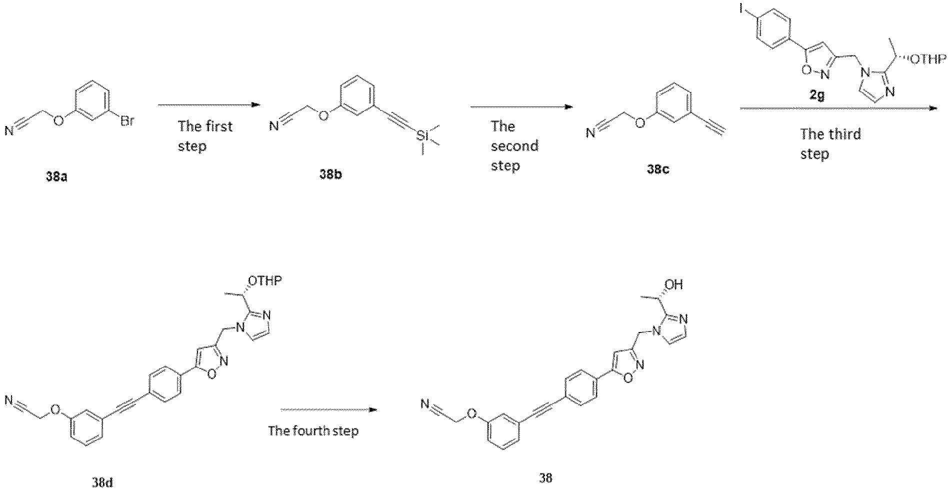

### The first step

### 2-(3-((Trimethylsilyl)ethynyl)phenoxy)acetonitrile

2-(3-Bromophenoxy)acetonitrile **38a** (50 mg, 235.80 µmol, prepared according to the published patent WO2000034258A2), (trimethylsilyl)acetylene (27.79 mg, 282.96 µmol, 39.99 µL), allylpalladium(II) chloride dimer (8.63 mg, 23.58 µmol), tri-tert-butylphosphine (10% toluene solution) (4.77 mg, 23.58 µmol) and triethylenediamine (79.35 mg, 707.40 µmol) were sequentially added into acetonitrile (1 mL), and the system was replaced with argon gas three times. The reaction solution was heated to 100°C and stirred for 16 hours. After the reaction was completed, the reaction mixture was concentrated under reduced pressure. The obtained residue was purified by silica gel column chromatography (eluent: System B) to obtain 2-(3-((trimethylsilyl)ethynyl)phenoxy)acetonitrile **38b** (35 mg) with a yield of 64.72%.
MS m/z (ESI): 229.9 [M+1]

### The second step

### 2-(3-Ethynylphenoxy)acetonitrile

2-(3-((Trimethylsilyl)ethynyl)phenoxy)acetonitrile **38b** (70 mg, 305.21 µmol) and potassium fluoride (53.20 mg, 915.63 µmol) were sequentially added to methanol (1 mL), and continuously stirred at 25°C for 16 hours. After the reaction was completed, the reaction solution was concentrated under reduced pressure to obtain 2-(3-ethynylphenoxy)acetonitrile **38c** (47 mg), which was directly used for the next reaction.
MS m/z (ESI): 157.9 [M+1]

### The third step

### 2-(3-((4-(3-((2-((1S)-1-((tetrahydro-2H-pyran-2-yl)oxy)ethyl)-1H-imidazol-1-yl)methyl )isoxazol-5-yl)phenyl)ethynyl)phenoxy)acetonitrile

2-(3-Ethynylphenoxy)acetonitrile **38c** (50 mg, 318.13 µmol), 5-(4-iodophenyl)-3-((2-((1S)-1-((tetrahydro-2H-pyran-2-yl)oxy)ethyl)-1H-imidazol-1-yl)methyl)i soxazole **2g** (76.24 mg, 159.07 µmol), allylpalladium(II) chloride dimer (11.64 mg, 31.81 µmol), tri-tert-butylphosphine (10% toluene solution) (6.44 mg, 31.81 µmol) and triethylenediamine (107.06 mg, 954.39 µmol) were sequentially added to acetonitrile (1 mL), and the system was replaced with argon gas three times and continuously stirred at 25°C for 16 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 2-(3-((4-(3-((2-((1S)-1-((tetrahydro-2H-pyran-2-yl)oxy)ethyl)-1H-imidazol-1-yl)methyl)isoxazol -5-yl)phenyl)ethynyl)phenoxy)acetonitrile **38d** (10 mg) with a yield of 6.18%.
MS m/z (ESI): 509.0 [M+1]

### The fourth step

### (S)-2-(3-((4-(3-((2-(1-hydroxyethyl)-1H-imidazol-1-yl)methyl)isoxazol-5-yl)phenyl)eth ynyl)phenoxy)acetonitrile

Trifluoroacetic acid (6.73 mg, 58.99 µmol) was slowly dropwise added to 2-(3-((4-(3-((2-((1S)-1-((tetrahydro-2H-pyran-2-yl)oxy)ethyl)-1H-imidazol-1-yl)methyl)isoxazol -5-yl)phenyl)ethynyl)phenoxy)acetonitrile **38d** (10 mg, 19.66 µmol) in dichloromethane solution (0.5 mL), and stirred continuously at 25°C for 16 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-(3-((4-(3-((2-(1-hydroxyethyl)-1H-imidazol-1-yl)methyl)isoxazol-5-yl)phenyl)ethynyl)phe noxy)acetonitrile **38** (5.4 mg) with a yield of 51%.
MS m/z (ESI): 424.9 [M+1]

### Example 39

### (S)-2-(5-((4-(3-((2-(1-hydroxyethyl)-1H-imidazol-1-yl)methyl)isoxazol-5-yl)phenyl)eth ynyl)pyridin-2-yl)acetic acid

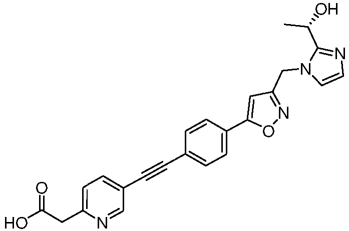

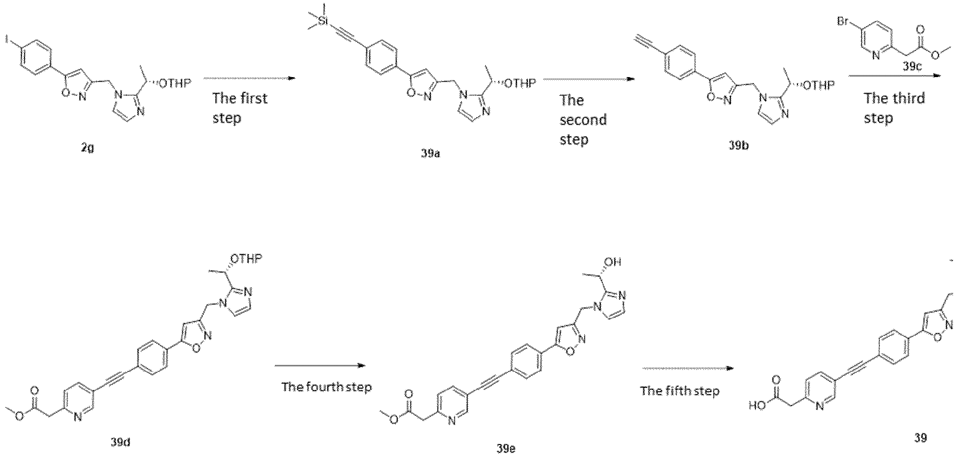

### The first step

### 3-((2-((1S)-1-((tetrahydro-2H-pyran-2-yl)oxy)ethyl)-1H-imidazol-1-yl)methyl)-5-(4-((tr imethylsilyl)ethynyl)phenyl)isoxazole

5-(4-Iodophenyl)-3-((2-((1S)-1-((tetrahydro-2H-pyran-2-yl)oxy)ethyl)-1H-imidazol-1-y l)methyl)isoxazole **2g** (250 mg, 521.58 µmol), (trimethylsilyl)acetylene (204.92 mg, 2.09 mmol, 294.84 µL), allylpalladium(II) chloride dimer (28.56 mg, 78.24 µmol), triethylenediamine (117.01 mg, 1.04 mmol) and tri-tert-butylphosphine (10% toluene solution) (211.05 mg, 104.32 µmol) were sequentially added to acetonitrile (4.75 mL), and the system was replaced with argon gas three times and stirred continuously at room temperature for 12 hours. After the reaction was completed, the reaction mixture was concentrated under reduced pressure. The obtained residue was purified by silica gel column chromatography (eluent: System B) to obtain 3-((2-((1S)-1-((tetrahydro-2H-pyran-2-yl)oxy)ethyl)-1H-imidazol-1-yl)methyl)-5-(4-((trimethylsi lyl)ethynyl)phenyl)isoxazole **39a** ( 200 mg) with a yield of 85.28%.
MS m/z (ESI): 450.3 [M+1]

### The second step

### 5-(4-Ethynylphenyl)-3-((2-((1S)-1-((tetrahydro-2H-pyran-2-yl)oxy)ethyl)-1H-imidazol-1-yl)methyl)isoxazole

3-((2-((1S)-1-((tetrahydro-2H-pyran-2-yl)oxy)ethyl)-1H-imidazol-1-yl)methyl)-5-(4-((tr imethylsilyl)ethynyl)phenyl)isoxazole **39a** (200 mg, 444.82 µmol) and potassium fluoride (77.53 mg, 1.33 mmol) were sequentially added to methanol (5 mL), and continuously stirred at room temperature for 12 hours. After the reaction was completed, the reaction solution was concentrated under reduced pressure. The obtained residue was purified by silica gel column chromatography (eluent: System B) to obtain 5-(4-ethynylphenyl)-3-((2-((1S)-1-((tetrahydro-2H-pyran-2-yl)oxy)ethyl)-1H-imidazol-1-yl)meth yl)isoxazole **39b** (150 mg) with a yield of 89.34 %.
MS m/z (ESI): 378.2 [M+1]

### The third step

### Methyl 2-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)acetate

5-(4-Ethynylphenyl)-3-((2-((1S)-1-((tetrahydro-2H-pyran-2-yl)oxy)ethyl)-1H-imidazol-1-yl)methyl)isoxazole **39b** (100 mg, 264.95 µmol), methyl 2-(5-bromopyridin-2-yl)acetate 39c (30.48 mg, 132.47 µmol, commercially available), allylpalladium(II) chloride dimer (19.34 mg, 52.99 µmol), triethylenediamine (89.16 mg, 794.84 µmol) and tri-tert-butylphosphine (10% toluene solution) (107.21 mg, 52.99 µmol, 10% purity) were sequentially added to acetonitrile (2 mL), and the system was replaced with argon gas three times and stirred continuously at room temperature for 12 hours. After the reaction was completed, the reaction mixture was concentrated under reduced pressure. The obtained residue was purified by silica gel column chromatography (eluent: System B) to obtain methyl 2-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)acetate **39d** (12 mg) with a yield of 8.60%.
MS m/z (ESI): 527.3 [M+1]

### The fourth step

### Methyl (S)-2-(5-((4-(3-((2-(1-hydroxyethyl)-1H-imidazol-1-yl)methyl)isoxazol-5-yl)phenyl)ethynyl)pyri din-2-yl)acetate

Methyl 2-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)acetate **39d** (12 mg, 22.79 µmol) and trifluoroacetic acid (5.20 mg, 45.58 µmol) were sequentially added into dichloromethane (1 mL), and continuously stirred at room temperature for 12 hours. After the reaction was completed, the reaction mixture was concentrated under reduced pressure to obtain methyl (S)-2-(5-((4-(3-((2-(1-hydroxyethyl)-1H-imidazol-1-yl)methyl)isoxazol-5-yl)phenyl)ethynyl)pyri din-2-yl)acetate **39e** (10 mg), which was used directly in the next reaction.
MS m/z (ESI): 443.3 [M+1]

### The fifth step

### (S)-2-(5-((4-(3-((2-(1-hydroxyethyl)-1H-imidazol-1-yl)methyl)isoxazol-5-yl)phenyl)eth ynyl)pyridin-2-yl)acetic acid

Methyl (S)-2-(5-((4-(3-((2-(1-hydroxyethyl)-1H-imidazol-1-yl)methyl)isoxazol-5-yl)phenyl)ethynyl)pyri din-2-yl)acetate **39e** (10 mg, 22.60 µmol) was added to a mixed solvent of methanol (2 mL) and tetrahydrofuran (2 mL), then added with 2.5 N sodium hydroxide aqueous solution (1 mL), and stirred continuously for 12 hours at room temperature. The reaction solution was adjusted to pH=5 with 1 N dilute hydrochloric acid, and concentrated under reduced pressure. The residue was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, concentrated under reduced pressure, and the residue was purified by preparative liquid chromatography (separation column AKZONOBEL Kromasil; 250×21.2 mm I.D.; 5 µm, 20 mL/min; mobile phase A: 0.05%TFA+H₂O, mobile phase B: CH₃CN) to obtain (S)-2-(5-((4-(3-((2-(1-hydroxyethyl)-1H-imidazol-1-yl)methyl)isoxazol-5-yl)phenyl)ethynyl)pyri din-2-yl)acetic acid **39** (1.8 mg) with a yield of 13.21%.
MS m/z (ESI): 429.0 [M+1]

¹H NMR (400 MHz, DMSO-*d₆*) *δ* 8.72 (br, 1H), 8.03 - 7.94 (m, 3H), 7.82 - 7.73 (m, 4H), 7.44 (dd, *J =* 26.4, 8.0 Hz, 1H), 7.22 (s, 1H), 5.76 (s, 2H), 5.29 (q, *J =* 6.4 Hz, 1H), 3.84 (s, 1H), 2.55 (d, *J =* 6.4 Hz, 2H), 1.52 (d, *J =* 6.4 Hz, 3H).

### Example 40

### (S)-3-(5-((4-(3-((2-(1-hydroxyethyl)-1H-imidazol-1-yl)methyl)isoxazol-5-yl)phenyl)eth ynyl)pyridin-2-yl)propanamide

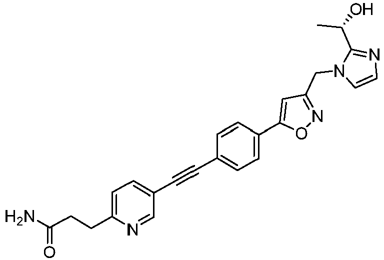

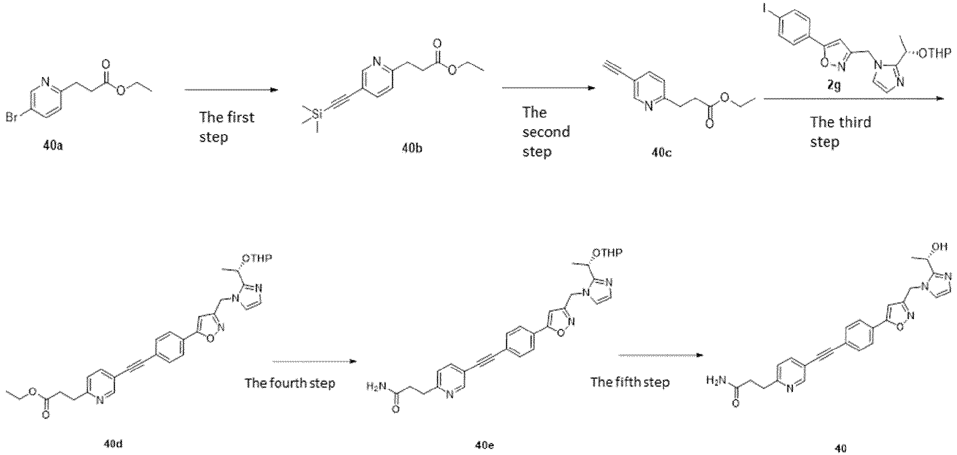

### The first step

### Ethyl 3-(5-((trimethylsilyl)ethynyl)pyridin-2-yl)propanoate

Ethyl 3-(5-bromopyridin-2-yl)propanoate **40a** (200 mg, 774.86 µmol, commercially available), (trimethylsilyl)acetylene (456.63 mg, 4.65 mmol, 657.02 µL), allylpalladium(II) chloride dimer (56.56 mg, 154.97 µmol), triethylenediamine (260.75 mg, 2.32 mmol), and tri-tert-butylphosphine (10% toluene solution) (313.54 mg, 154.97 µmol) were sequentially added to acetonitrile (2.53 mL), and the system was replaced with argon gas three times. The reaction solution was heated to 100°C, stirred for 6 hours, and added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure to dryness. The obtained residue was purified by silica gel column chromatography (eluent: System B) to obtain ethyl 3-(5-((trimethylsilyl)ethynyl)pyridin-2-yl)propanoate **40b** (180 mg) with a yield of 84.34%.
MS m/z (ESI): 276.2 [M+1]

### The second step

### Ethyl 3-(5-ethynylpyridin-2-yl)propanoate

Ethyl 3-(5-((trimethylsilyl)ethynyl)pyridin-2-yl)propanoate **40b** (180 mg, 653.55 µmol) and potassium fluoride (75.94 mg, 1.31 mmol) were sequentially added to methanol (2 mL), and continuously stirred at room temperature for 12 hours. After the reaction was completed, the reaction solution was concentrated under reduced pressure. The obtained residue was purified by silica gel column chromatography (eluent: System B) to obtain ethyl 3-(5-ethynylpyridin-2-yl)propanoate **40c** ( 108 mg) with a yield of 81.31%.
MS m/z (ESI): 204.2 [M+1]

### The third step

### Ethyl 3-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)propanoate

5-(4-Iodophenyl)-3-((2-((1S)-1-((tetrahydro-2H-pyran-2-yl)oxy)ethyl)-1H-imidazol-1-y l)methyl)isoxazole **2g** (170 mg, 354.68 µmol), ethyl 3-(5-ethynylpyridin-2-yl)propanoate **40c** (108.13 mg, 532.01 µmol), allylpalladium(II) chloride dimer (25.89 mg, 70.94 µmol), triethylenediamine (119.35 mg, 1.06 mmol) and tri-tert-butylphosphine (10% toluene solution) (143.52 mg, 70.94 µmol) were sequentially added to acetonitrile (2 mL), and the system was replaced with argon gas three times and continuously stirred at room temperature for 12 hours. After the reaction was completed, the reaction mixture was concentrated under reduced pressure. The obtained residue was purified by silica gel column chromatography (eluent: System B) to obtain ethyl 3-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)propanoate **40d** (30 mg) with a yield of 15.25%.
MS m/z (ESI): 555.0 [M+1]

### The fourth step

### 3-(5-((4-(3-((2-((1S)-1-((tetrahydro-2H-pyran-2-yl)oxy)ethyl)-1H-imidazol-1-yl)methyl )isoxazol-5-yl)phenyl)ethynyl)pyridin-2-yl)propanamide

Ethyl 3-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)propanoate **40d** (30 mg, 54.09 µmol) and ammonia (18.96 mg, 540.90 µmol) were sequentially added to ethanol (0.5 mL), and continuously stirred at room temperature for 12 hours. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure to obtain 3-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)propanamide **40e** (20 mg), which was used directly in the next reaction.
MS m/z (ESI): 526.3 [M+1]

### The fifth step

### (S)-3-(5-((4-(3-((2-(1-hydroxyethyl)-1H-imidazol-1-yl)methyl)isoxazol-5-yl)phenyl)eth ynyl)pyridin-2-yl)propanamide

3-(5-((4-(3-((2-((1S)-1-((tetrahydro-2H-pyran-2-yl)oxy)ethyl)-1H-imidazol-1-yl)methyl )isoxazol-5-yl)phenyl)ethynyl)pyridin-2-yl)propanamide **40e** (20 mg, 38.05 µmol) and trifluoroacetic acid (13.02 mg, 114.16 µmol) were sequentially added to dichloromethane (2 mL), and stirred continuously at room temperature for 12 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-3-(5-((4-(3-((2-(1-hydroxyethyl)-1H-imidazol-1-yl)methyl)isoxazol-5-yl)phenyl)ethynyl)pyri din-2-yl)propanamide **40** (4.20 mg) with a yield of 19.45%.
MS m/z (ESI): 442.2 [M+1]

### Example 41

### (S)-1-(4-((4-(3-((2-(1-hydroxyethyl)-1H-imidazol-1-yl)methyl)isoxazol-5-yl)phenyl)eth ynyl)phenyl)piperidin-4-ol

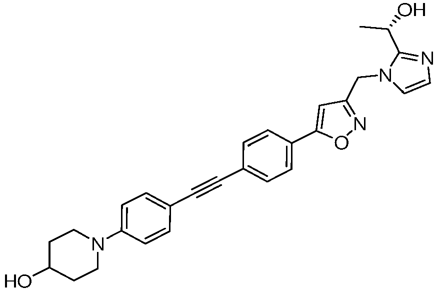

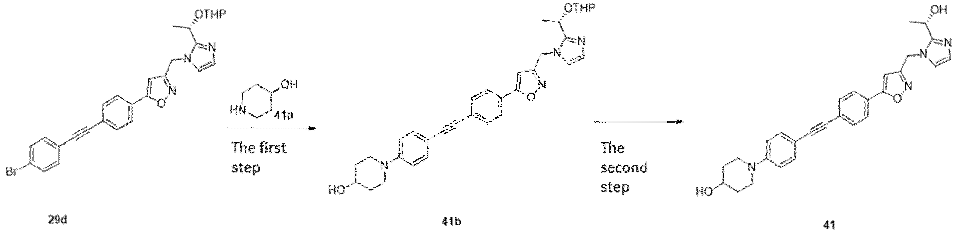

### The first step

### 1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenyl)piperidin-4-ol

5-(4-((4-Bromophenyl)ethynyl)phenyl)-3-((2-((1S)-1-((tetrahydro-2H-pyran-2-yl)oxy)et hyl)-1H-imidazol-1-yl)methyl)isoxazole **29d** (50 mg, 281.73 µmol), 4-hydroxypiperidine **41a** (28.50 mg, 9.39 µmol, commercially available), sodium tert-butoxide (27.07 mg, 281.73 µmol) and methanesulfonic acid (2-dicyclohexylphosphino-2',6'-diisopropoxy-1,1'-biphenyl)(2-amino-1,1'-biphenyl-2-yl)palladi um(II) (7.86 mg, 9.39 µmol) were sequentially added to 1,4-dioxane (1 mL). The system was replaced with argon gas three times, heated to 100°C and stirred for 16 hours. After the reaction was completed, the reaction mixture was concentrated under reduced pressure. The obtained residue was purified by silica gel column chromatography (eluent: System B) to obtain 1-(4-((4-(3-((2-((1S)-1-((tetrahydro-2H-pyran-2-yl)oxy)ethyl)-1H-imidazol-1-yl)methyl)isoxazol -5-yl)phenyl)ethynyl)phenyl)piperidin-4-ol **41b** (10 mg) with a yield of 19.27%.
MS m/z (ESI): 553.0 [M+1]

### The second step

### (S)-1-(4-((4-(3-((2-(1-hydroxyethyl)-1H-imidazol-1-yl)methyl)isoxazol-5-yl)phenyl)eth ynyl)phenyl)piperidin-4-ol

1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)phenyl)piperidin-4-ol **41b** (10 mg, 18.09 µmol) was added to dichloromethane (0.5 mL), slowly added with trifluoroacetic acid (2.06 mg, 18.09 µmol) dropwise, and stirred at 25°C for 16 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(4-((4-(3-((2-(1-hydroxyethyl)-1H-imidazol-1-yl)methyl)isoxazol-5-yl)phenyl)ethynyl)phe nyl)piperidin-4-ol **41** (7 mg) with a yield of 63.88%.
MS m/z (ESI): 469.0 [M+1]

### Example 42

### (S)-(4-((4-(3-((2-(1-hydroxyethyl)-1H-imidazol-1-yl)methyl)isoxazol-5-yl)phenyl)ethyn yl)phenyl)(4-methylpiperazin-1-yl)methanone

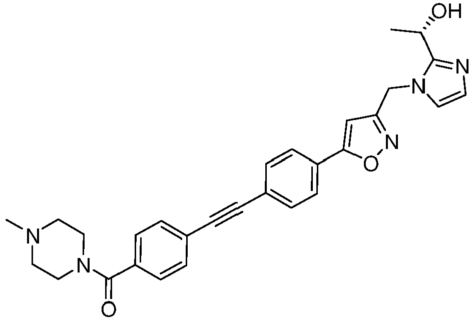

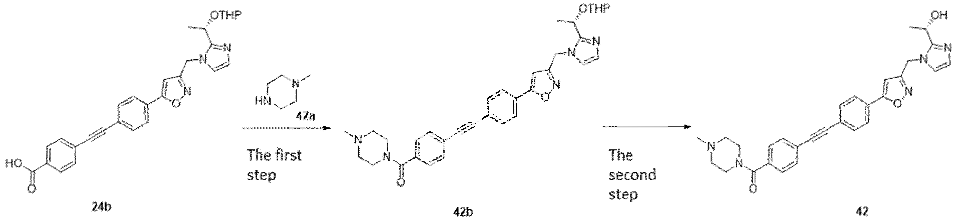

### The first step

### (4-Methylpiperazin-1-yl)(4-((4-(3-((2-((1S)-1-((tetrahydro-2H-pyran-2-yl)oxy)ethyl)-1 H-imidazol-1-yl)methyl)isoxazol-5-yl)phenyl)ethynyl)phenyl)methanone

1-Methylpiperazine **42a** (10 mg, 99.84 µmol, commercially available), 4-((4-(3-((2-((1S))-1-((tetrahydro-2H-pyran-2)-yl)oxy)ethyl)-1H-imidazol-1-yl)methyl)isoxazol-5-yl)phenyl)ethynyl)benzoic acid **24b** (49.7 mg, 99.84 µmol), O-(7-azabenzotriazol-1-yl)-N,N,N',N'-tetramethylurea hexafluorophosphate (75.9 mg, 199.68 µmol) and N,N-diisopropylethylamine (38.7 mg, 299.52 µmol) were sequentially added to dimethyl sulfoxide (2 mL) for reaction at room temperature for 4 hours. After the reaction was completed, the reaction mixture was added with water (50 mL) to quench the reaction, and extracted with ethyl acetate (50 mL×3). The organic phases were combined and concentrated under reduced pressure. The obtained residue was purified by silica gel column chromatography (eluent: System B) to obtain (4-methylpiperazin-1-yl)(4-((4-(3-((2-((1S)-1-((tetrahydro-2H-pyran-2-yl)oxy)ethyl)-1H-imidazo l-1-yl)methyl)isoxazol-5-yl)phenyl)ethynyl)phenyl)methanone **42b** (40 mg) with a yield of 69.11%.
MS m/z (ESI): 580.1 [M+1]

### The second step

### (S)-(4-((4-(3-((2-(1-hydroxyethyl)-1H-imidazol-1-yl)methyl)isoxazol-5-yl)phenyl)ethyn yl)phenyl)(4-methylpiperazin-1-yl)methanone

(4-Methylpiperazin-1-yl)(4-((4-(3-((2-((1S)-1-((tetrahydro-2H-pyran-2-yl)oxy)ethyl)-1 H-imidazol-1-yl)methyl)isoxazol-5-yl)phenyl)ethynyl)phenyl)methanone **42b** (40 mg, 69.00 µmol) was dissolved in dichloromethane (1 mL), and added with trifluoroacetic acid (0.2 mL) dropwise for reaction at room temperature for 3 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-(4-((4-(3-((2-(1-hydroxyethyl)-1H-imidazol-1-yl)methyl)isoxazol-5-yl)phenyl)ethynyl)pheny l)(4-methylpiperazin-1-yl)methanone **42** (10 mg) with a yield of 22.58%.
MS m/z (ESI): 496.1[M+1]

### Example 43

### (S)-4-((4-(3-((2-(1-hydroxyethyl)-1H-imidazol-1-yl)methyl)isoxazol-5-yl)phenyl)ethyn yl)-N-methylbenzamide

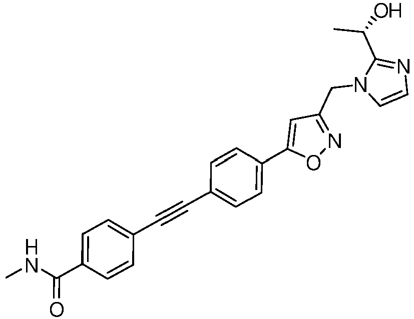

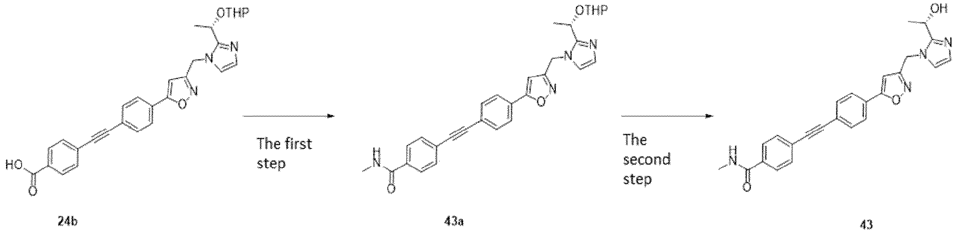

### The first step

### N-Methyl-4-((4-(3-((2-((1S)-1-((tetrahydro-2H-pyran-2-yl)oxy)ethyl)-1H-imidazol-1-yl )methyl)isoxazol-5-yl)phenyl)ethynyl)benzamide

Methylamine gas was introduced into a solution of 4-((4-(3-((2-((1S)-1-((tetrahydro-2H-pyran-2-yl)oxy)ethyl)-1H-imidazol-1-yl)methyl)isoxazol-5-yl)phenyl)ethynyl)benzoic acid 24b (50 mg, 100.49 µmol), triethylamine (30.5 mg, 301.48 µmol), 1-(3-dimethylaminopropyl)-3-ethylcarbodiimide hydrochloride (28.9 mg, 150.74 µmol) and 1-hydroxybenzotriazole (20.4 mg, 150.74 µmol) in N,N-dimethylformamide (5 mL) for reaction at room temperature for 24 hours. After the reaction was completed, the reaction mixture was added with water (50 mL) to quench the reaction, and extracted with ethyl acetate (50 mL×3). The organic phases were combined and concentrated under reduced pressure. The obtained residue was purified by silica gel column chromatography (eluent: System B) to obtain N-methyl-4-((4-(3-((2-((1S)-1-((tetrahydro-2H-pyran-2-yl)oxy)ethyl)-1H-imidazol-1-yl)methyl)i soxazol-5-yl)phenyl)ethynyl)benzamide **43a** (10 mg) with a yield of 19.49%.
MS m/z (ESI): 511.3 [M+1]

### The second step

### (S)-4-((4-(3-((2-(1-hydroxyethyl)-1H-imidazol-1-yl)methyl)isoxazol-5-yl)phenyl)ethyn yl)-N-methylbenzamide

N-methyl-4-((4-(3-((2-((1S)-1-((tetrahydro-2H-pyran-2-yl)oxy)ethyl)-1H-imidazol-1-yl )methyl)isoxazol-5-yl)phenyl)ethynyl)benzamide **43a** (10 mg, 19.59 µmol) was dissolved in dichloromethane (1 mL), and added with trifluoroacetic acid (0.2 mL) for reaction at room temperature for 4 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-4-((4-(3-((2-(1-hydroxyethyl)-1H-imidazol-1-yl)methyl)isoxazol-5-yl)phenyl)ethynyl)-N-met hylbenzamide **43** (840.00 µg) with a yield of 7.54%.
MS m/z (ESI): 427.3[M+1]

### Example 44

### (S)-1-(1-((5-(4-((4-((1-methylpiperidin-4-yl)oxy)phenyl)ethynyl)phenyl)isoxazol-3-yl) methyl)-1H-imidazol-2-yl)ethan-1-ol

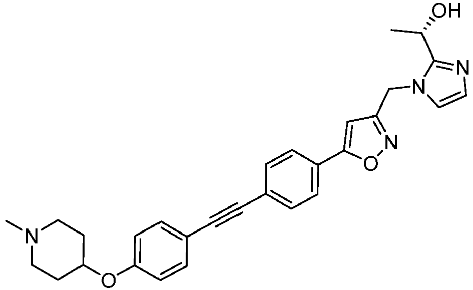

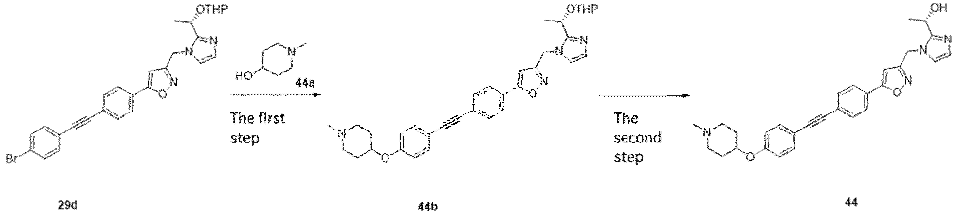

### The first step

### 5-(4-((4-((1-Methylpiperidin-4-yl)oxy)phenyl)ethynyl)phenyl)-3-((2-((1S)-1-((tetrahydr o-2H-pyran-2-yl)oxy)ethyl)-1H-imidazol-1-yl)methyl)isoxazole

5-(4-((4-Bromophenyl)ethynyl)phenyl)-3-((2-((1S)-1-((tetrahydro-2H-pyran-2-yl)oxy)et hyl)-1H-imidazol-1-yl)methyl)isoxazole **29d** (50 mg, 93.91 µmol), 1-methylpiperidin-4-ol **44a** (32.45 mg, 281.73 µmol, 33.11 µL), tris(dibenzylidene-BASEacetone)dipalladium (8.40 mg, 18.78 µmol), 5-di-tert-butylphosphine-1',3',5'-triphenyl-1'H-[1,4']dipyrazole (19.03 mg, 37.56 µmol) and cesium carbonate (91.79 mg, 281.73 µmol) were sequentially added to toluene (0.5 mL). The system was replaced with argon gas three times, heated to 100°C and stirred for 16 hours. After the reaction was completed, the reaction mixture was concentrated under reduced pressure. The obtained residue was purified by silica gel column chromatography (eluent: System B) to obtain 5-(4-((4-((1-methylpiperidin-4-yl)oxy)phenyl)ethynyl)phenyl)-3-((2-((1S)-1-((tetrahydro-2H-pyr an-2-yl)oxy)ethyl)-1H-imidazol-1-yl)methyl)isoxazole **44b** (20 mg) with a yield of 37.58%.
MS m/z (ESI): 567.0 [M+1]

### The second step

### (S)-1-(1-((5-(4-((4-((1-Methylpiperidin-4-yl)oxy)phenyl)ethynyl)phenyl)isoxazol-3-yl) methyl)-1H-imidazol-2-yl)ethan-1-ol

5-(4-((4-((1-methylpiperidin-4-yl)oxy)phenyl)ethynyl)phenyl)-3-((2-((1S)-1-((tetrahydr o-2H-pyran-2-yl)oxy)ethyl)-1H-imidazol-1-yl)methyl)isoxazole **44b** (20 mg, 35.29 µmol) was added to dichloromethane (0.5 mL), slowly added with trifluoroacetic acid (12.07 mg, 105.88 µmol) dropwise, and stirred at 25°C for 16 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-((1-methylpiperidin-4-yl)oxy)phenyl)ethynyl)phenyl)isoxazol-3-yl)methyl)-1 H-imidazol-2-yl)ethan-1-ol 44 (10 mg) with yield of 55.49%.
MS m/z (ESI): 484.0 [M+1]

### Example 45

### (S)-1-(1-((5-(4-(Pyridin-2-ylethynyl)phenyl)isoxazol-3-yl)methyl)-1H-imidazol-2-yl)et han-1-ol

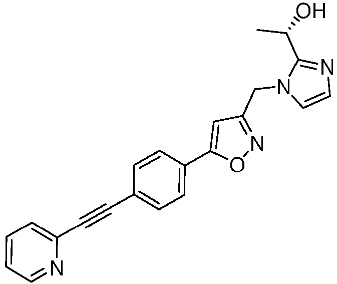

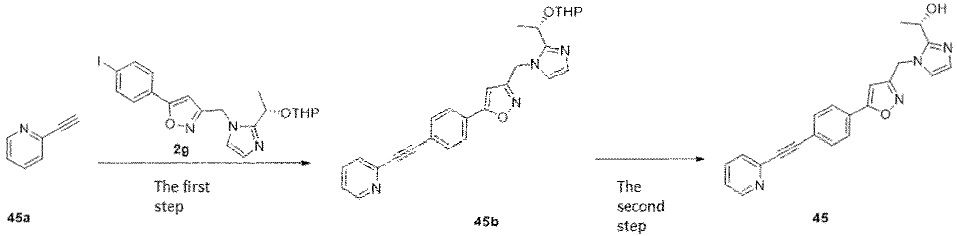

### The first step

### 5-(4-(Pyridin-2-ylethynyl)phenyl)-3-((2-((1S)-1-((tetrahydro-2H-pyran-2-yl)oxy)ethyl)-1H-imidazol-1-yl)methyl)isoxazole

2-Ethynylpyridine **45a** (150.00 mg, 1.45 mmol, 146.91 µL), 5-(4-iodophenyl)-3-((2-((1S)-1-((tetrahydro-2H-pyran)-2-yl)oxy)ethyl)-1H-imidazol-1-yl)methyl )isoxazole **2g** (464.80 mg, 969.73 µmol), allylpalladium(II) chloride dimer (35.48 mg, 96.97 µmol), tri-tert-butylphosphine (19.62 mg, 96.97 µmol) and triethylenediamine (326.33 mg, 2.91 mmol) were sequentially added to acetonitrile (1 mL), and the system was replaced with argon gas three times and stirred at 25°C for 16 hours. After the reaction was completed, the reaction was concentrated under reduced pressure. The obtained residue was purified by silica gel column chromatography (eluent: System A) to obtain 5-(4-(pyridin-2-ylethynyl)phenyl)-3-((2-((1S)-1-((tetrahydro-2H-pyran-2-yl)oxy)ethyl)-1H-imida zol-1-yl)methyl)isoxazole **45b** (320 mg) with a yield of 72.60%.
MS m/z (ESI): 455.0 [M+1]

### The second step

### (S)-1-(1-((5-(4-(pyridin-2-ylethynyl)phenyl)isoxazol-3-yl)methyl)-1H-imidazol-2-yl)eth an-1-ol

5-(4-(Pyridin-2-ylethynyl)phenyl)-3-((2-((1S)-1-((tetrahydro-2H-pyran-2-yl)oxy)ethyl)-1H-imidazol-1-yl)methyl)isoxazole **45b** (320 mg, 704.04 µmol) was added to dichloromethane (1 mL), slowly added with trifluoroacetic acid (80.28 mg, 704.04 µmol, 2 mL) dropwise, and stirred at 25°C for 4 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-(pyridin-2-ylethynyl)phenyl)isoxazol-3-yl)methyl)-1H-imidazol-2-yl)ethan-1-ol 45 (10 mg) with a yield of 2.58%.
MS m/z (ESI): 371.0 [M+1]

### Example 46

### (S)-1-(1-((5-(4-((4-(hydroxymethyl)phenyl)ethynyl)phenyl)isoxazol-3-yl)methyl)-1H-i midazol-2-yl)ethan-1-ol

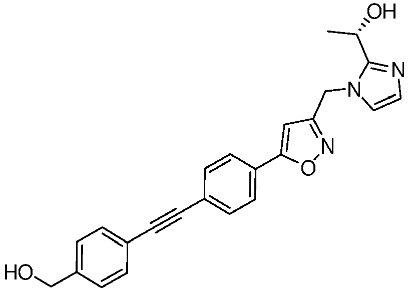

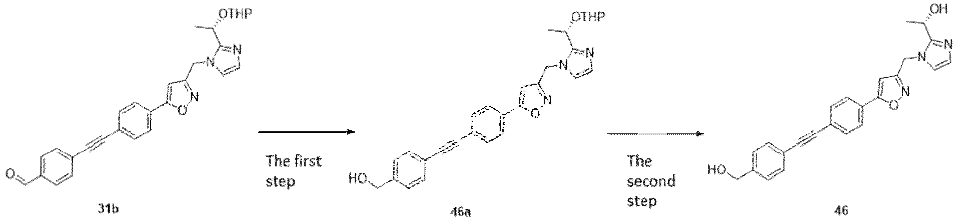

### The first step

### (4-((4-(3-((2-((1S)-1-((tetrahydro-2H-pyran-2-yl)oxy)ethyl)-1H-imidazol-1-yl)methyl)is oxazol-5-yl)phenyl)ethynyl)phenyl)methanol

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (20 mg, 41.53 µmol) was added to 1,2-dichloroethane (3 mL), stirred for 30 minutes, added with sodium cyanoborohydride (2.61 mg, 41.53 µmol), and stirred continuously at room temperature for 12 hours. The reaction mixrture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The organic phase was combined, washed with saturated sodium chloride solution (30 mL×2), dried over anhydrous sodium sulfate, filtered, and concentrated under reduced pressure. The obtained residue was purified by silica gel column chromatography (eluent: System A) to obtain (4-((4-(3-((2-((1S)-1-((tetrahydro-2H-pyran-2-yl)oxy)ethyl)-1H-imidazol-1-yl)methyl)isoxazol-5 -yl)phenyl)ethynyl)phenyl)methanol **46a** (10 mg) with a yield of 48.55%.
MS m/z (ESI): 484.0 [M+1]

### The second step

### (S)-1-(1-((5-(4-((4-(hydroxymethyl)phenyl)ethynyl)phenyl)isoxazol-3-yl)methyl)-1H-i midazol-2-yl)ethan-1-ol

(4-((4-(3-((2-((1S))-1-((tetrahydro-2H-pyran-2-yl)oxy)ethyl)-1H-imidazol-1-yl)methyl)i soxazol-5-yl)phenyl)ethynyl)phenyl)methanol **46a** (10 mg, 20.68 µmol) and trifluoroacetic acid (0.2 mL) were sequentially added to dichloromethane (5 mL), and stirred continuously at room temperature for 12 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-(hydroxymethyl)phenyl)ethynyl)phenyl)isoxazol-3-yl)methyl)-1H-imidazol-2 -yl)ethan-1-ol **46** (2.0 mg) with a yield of 21.79%.
MS m/z (ESI): 400.1 [M+1]

### Example 47

### 4-(4-((4-(3-((2-((S)-1-hydroxyethyl)-1H-imidazol-1-yl)methyl)isoxazol-5-yl)phenyl)eth ynyl)benzyl)morpholin-2-carboxylic acid

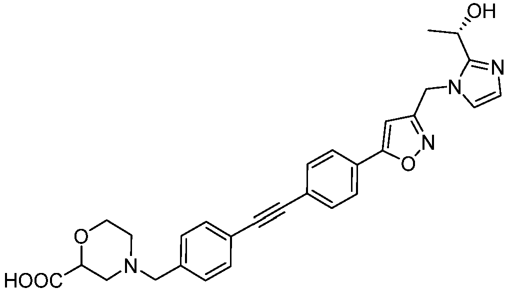

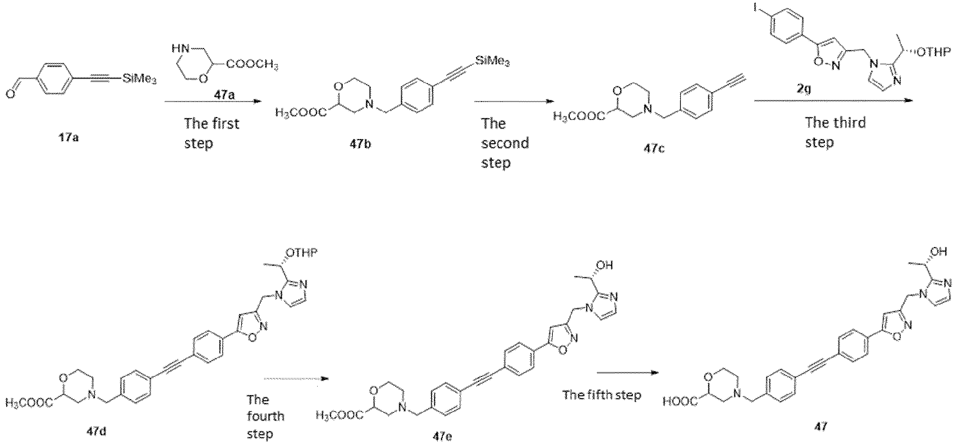

### The first step

### Methyl 4-(4-((trimethylsilyl)ethynyl)benzyl)morpholin-2-carboxylate

4-((Trimethylsilyl)ethynyl)benzaldehyde **17a** (200 mg, 988.51 µmol) and methyl morpholin-2-carboxylate **47a** (269.30 mg, 1.48 mmol) were dissolved in 1,2-dichloroethane (5 mL), then added with (0.4 mL), stirred for 30 minutes, added with sodium acetoxyborohydride (628.52 mg, 2.97 mmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain methyl 4-(4-((trimethylsilyl)ethynyl)benzyl)morpholin-2-carboxylate **47b** (120 mg) with a yield of 36.62%.
MS m/z (ESI): 331.2 [M+1]

### The second step

### Methyl 4-(4-ethynylbenzyl)morpholin-2-carboxylate

Methyl 4-(4-((trimethylsilyl)ethynyl)benzyl)morpholin-2-carboxylate **47b** (120 mg, 362.01 µmol) and potassium fluoride (105.16 mg, 1.81 mmol) were sequentially added to methanol (3 mL), and continuously stirred at room temperature for 12 hours. The system was concentrated under reduced pressure. The obtained residue was purified by silica gel column chromatography (eluent: System A) to obtain methyl 4-(4-ethynylbenzyl)morpholin-2-carboxylate **47c** (90 mg) with a yield of 95.88%.
MS m/z (ESI): 260.1 [M+1]

### The third step

### Methyl 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)morpholin-2-carboxylate

Methyl 4-(4-ethynylbenzyl)morpholin-2-carboxylate **47c** (50 mg, 192.83 µmol), 5-(4-iodophenyl)-3-((2-((1S)-1-((tetrahydro-2H-pyran-2-yl)oxy)ethyl)-1H-imidazol-1-yl)methyl)i soxazole **2g** (101.67 mg, 212.11 µmol), allylpalladium chloride dimer (7.06 mg, 19.28 µmol), tri-tert-butylphosphine (39.01 mg, 19.28 µmol, 10% toluene solution) and triethylenediamine (64.89 mg, 578.48 µmol) were sequentially added to acetonitrile (2 mL), and the system was replaced with argon gas gas three times and continuously stirred at room temperature for 12 hours. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System B) to obtain methyl 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)morpholin-2-carboxylate **47d** (60 mg) with a yield of 50.95%.
MS m/z (ESI): 611.3 [M+1]

### The fourth step

### Methyl 4-(4-((4-(3-((2-((S)-1-hydroxyethyl)-1H-imidazol-1-yl)methyl)isoxazol-5-yl)phenyl)ethynyl)ben zyl)morpholin-2-carboxylate

Methyl 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)morpholin-2-carboxylate **47d** (50 mg, 81.87 µmol) was added to dichloromethane (3 mL), stirred, added with trifluoroacetic acid (0.5 mL), and continuously stirred at room temperature for 2 hours. The reaction solution was concentrated under reduced pressure to obtain methyl 4-(4-((4-(3-((2-((S)-1-hydroxyethyl)-1H-imidazol-1-yl)methyl)isoxazol-5-yl)phenyl)ethynyl)ben zyl)morpholin-2-carboxylate **47e** (30 mg), which was used directly in the next reaction.
MS m/z (ESI): 527.2 [M+1]

### The fifth step

### 4-(4-((4-(3-((2-((S)-1-hydroxyethyl)-1H-imidazol-1-yl)methyl)isoxazol-5-yl)phenyl)eth ynyl)benzyl)morpholin-2-carboxylic acid

Methyl 4-(4-((4-(3-((2-((S)-1-hydroxyethyl)-1H-imidazol-1-yl)methyl)isoxazol-5-yl)phenyl)ethynyl)ben zyl)morpholin-2-carboxylate **47e** (30 mg, 56.97 µmol) and lithium hydroxide monohydrate (7.17 mg, 170.91 µmol) were sequentially added to tetrahydrofuran (3 mL), and continuously stirred at room temperature for 12 hours. The system was adjusted to pH 5 with 2 N dilute hydrochloric acid, and concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+ H₂O, mobile phase B: CH₃CN) to obtain 4-(4-((4-(3-((2-((S)-1-hydroxyethyl)-1H-imidazol-1-yl)methyl)isoxazol-5-yl)phenyl)ethynyl)ben zyl)morpholin-2-carboxylic acid **47** (20 mg) with a yield of 56.03%.
MS m/z (ESI): 512.9 [M+1]

### Example 48

### (S)-1-(1-((5-(4-((4-((4-methylpiperazin-1-yl)methyl)phenyl)ethynyl)phenyl)isoxazol-3-yl)methyl)-1H-imidazol-2-yl)ethan-1-ol

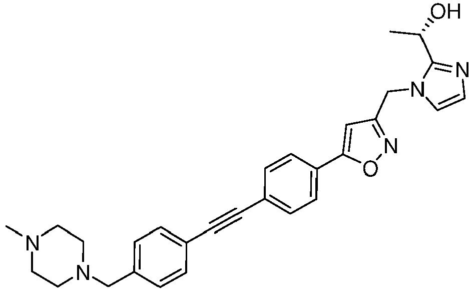

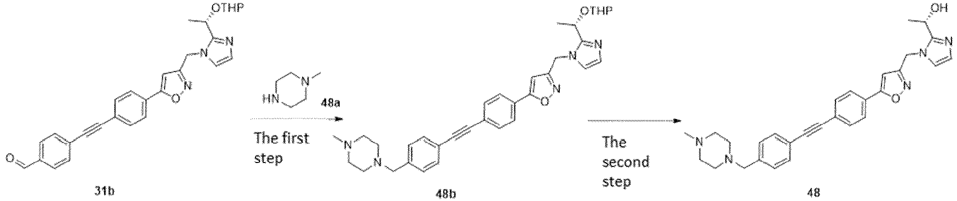

### The first step

### 5-(4-((4-((4-Methylpiperazin-1-yl)methyl)phenyl)ethynyl)phenyl)-3-((2-((1S)-1-((tetrah ydro-2H-pyran-2-yl)oxy)ethyl)-1H-imidazol-1-yl)methyl)isoxazole

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (25 mg, 51.92 µmol) and 1-methylpiperazine **48a** (7.80 mg, 77.87 µmol, commercially available) were dissolved in methanol (2 mL), added with acetic acid (0.2 mL), stirred for 30 minutes, added with sodium cyanoborohydride (9.79 mg, 155.75 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 5-(4-((4-((4-methylpiperazin-1-yl)methyl)phenyl)ethynyl)phenyl)-3-((2-((1S)-1-((tetrahydro-2H-pyran-2-yl)oxy)ethyl)-1H-imidazol-1-yl)methyl)isoxazole **48b** (25 mg) with a yield of 85.12%.
MS m/z (ESI): 566.7 [M+1]

### The second step

### (S)-1-(1-((5-(4-((4-((4-methylpiperazin-1-yl)methyl)phenyl)ethynyl)phenyl)isoxazol-3-yl)methyl)-1H-imidazol-2-yl)ethan-1-ol

5-(4-((4-((4-Methylpiperazin-1-yl)methyl)phenyl)ethynyl)phenyl)-3-((2-((1S)-1-((tetrah ydro-2H-pyran-2-yl)oxy)ethyl)-1H-imidazol-1-yl)methyl)isoxazole **48b** (25 mg, 44.19 µmol) was added to dichloromethane (5 mL), added with trifluoroacetic acid (0.5 mL), and continuously stirred at room temperature for 4 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-((4-methylpiperazin-1-yl)methyl)phenyl)ethynyl)phenyl)isoxazol-3-yl)methyl )-1H-imidazol-2-yl)ethan-1-ol **48** (18.0 mg) with a yield of 67.29%.
MS m/z (ESI): 482.0 [M+1]

### Example 49

### (S)-1-(1-((5-(4-((4-(((2-(methylsulfonyl)ethyl)amino)methyl)phenyl)ethynyl)phenyl)iso xazol-3-yl)methyl)-1H-imidazol-2-yl)ethan-1-ol

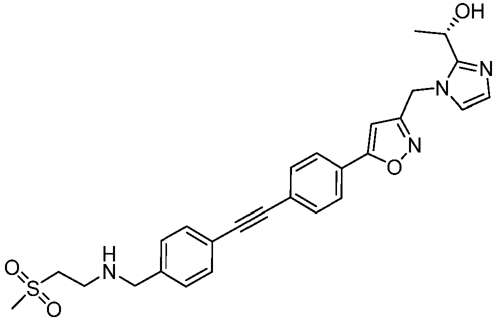

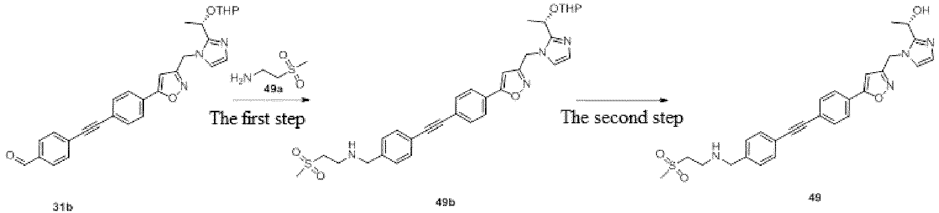

### The first step

### 2-(Methylsulfonyl)-N-(4-((4-(3-((2-((1S)-1-((tetrahydro-2H-pyran-2-yl)oxy)ethyl)-1H-i midazol-1-yl)methyl)isoxazol-5-yl)phenyl)ethynyl)benzyl)ethan-1-amine

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (25 mg, 51.92 µmol) and 2-(methylsulfonyl)ethan-1-amine **49a** (7.67 mg, 62.30 µmol, commercially available) were dissolved in 1,2-dichloroethane (2 mL), added with acetic acid (0.2 mL), stirred for 30 minutes, then added with sodium acetate borohydride (33.01 mg, 155.75 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 2-(methylsulfonyl)-N-(4-((4-(3-((2-((1S)-1-((tetrahydro-2H-pyran-2-yl)oxy)ethyl)-1H-imidazol-1 -yl)methyl)isoxazol-5-yl)phenyl)ethynyl)benzyl)ethan-1-amine **49b** (25 mg) with a yield of 81.80%.
MS m/z (ESI): 589.2 [M+1]

### The second step

### (S)-1-(1-((5-(4-((4-(((2-(methylsulfonyl)ethyl)amino)methyl)phenyl)ethynyl)phenyl)iso xazol-3-yl)methyl)-1H-imidazol-2-yl)ethan-1-ol

2-(Methylsulfonyl)-N-(4-((4-(3-((2-((1S)-1-((tetrahydro-2H-pyran-2-yl)oxy)ethyl)-1H-i midazol-1-yl)methyl)isoxazol-5-yl)phenyl)ethynyl)benzyl)ethan-1-amine **49b** (25 mg, 42.47 µmol) was added to dichloromethane (3 mL), then added with trifluoroacetic acid (0.3 mL), and continuously stirred at room temperature for 4 hours. The reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-(((2-(methylsulfonyl)ethyl)amino)methyl)phenyl)ethynyl)phenyl)isoxazol-3-y l)methyl)-1H-imidazol-2-yl)ethan-1-ol **49** (19 mg) with a yield of 68.85%.
MS m/z (ESI): 505.2 [M+1]

### Example 50

### (S)-4-(4-((4-(3-((2-(1-hydroxyethyl)-1H-imidazol-1-yl)methyl)isoxazol-5-yl)phenyl)eth ynyl)benzyl)thiomorpholine 1,1-dioxide

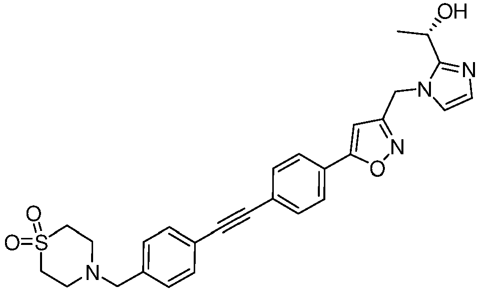

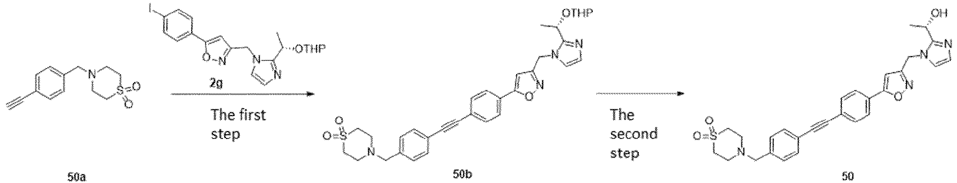

### The first step

### 4-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)thiomorpholine 1,1-dioxide

5-(4-Iodophenyl)-3-((2-((1S)-1-((tetrahydro-2H-pyran-2-yl)oxy)ethyl)-1H-imidazol-1-y l)methyl)isoxazole **2g** (30 mg, 62.59 µmol), 4-(4-ethynylbenzyl)thiomorpholine 1,1-dioxide **50a** (23.41 mg, 93.88 µmol, prepared according to the published patent WO 2017093544), allylpalladium(II) chloride dimer (2.29 mg, 6.26 µmol), tri-tert-butylphosphine (12.66 mg, 6.26 µmol, 10% toluene solution) and triethylenediamine (21.06 mg, 187.77 µmol) were sequentially added to acetonitrile (5 mL), and the system was replaced with argon gas three times and stirred continuously at room temperature for 12 hours. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over sodium sulfate, filtered, and concentrated under reduced pressure. The obtained residue was purified by silica gel column chromatography (eluent: System A) to obtain 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)thiomorpholine 1,1-dioxide **50b** (30 mg) with a yield of 79.79%.
MS m/z (ESI): 601.4 [M+1]

### The second step

### (S)-4-(4-((4-(3-((2-(1-hydroxyethyl)-1H-imidazol-1-yl)methyl)isoxazol-5-yl)phenyl)eth ynyl)benzyl)thiomorpholine 1,1-dioxide

4-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)thiomorpholine 1,1-dioxide **50b** (30 mg, 49.94 µmol) was added to dichloromethane (5 mL), added with trifluoroacetic acid (0.5 mL), and continuously stirred at room temperature for 4 hours. The reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-4-(4-((4-(3-((2-(1-hydroxyethyl)-1H-imidazol-1-yl)methyl)isoxazol-5-yl)phenyl)ethynyl)ben zyl)thiomorpholine 1,1-dioxide **50** (3.3 mg) with a yield of 9.95%.
MS m/z (ESI): 517.0 [M+1]

### Example 51

### (S)-1-(1-((5-(4-((4-((4-morpholinopiperidin-1-yl)methyl)phenyl)ethynyl)phenyl)isoxazo 1-3-yl)methyl)-1H-imidazol-2-yl)ethan-1-ol

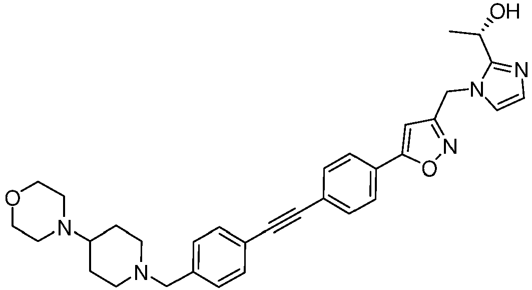

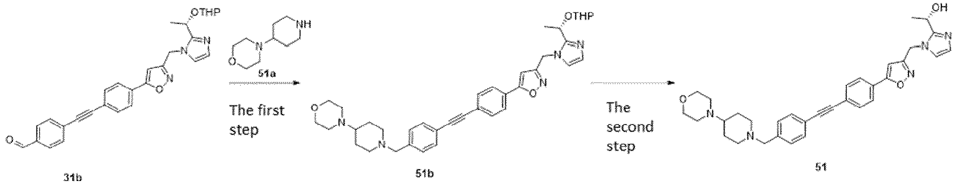

### The first step

### 4-(1-(4-((4-(3-((2-((1S)-1-((tetrahydro-2H-pyran-2-yl)oxy)ethyl)-1H-imidazol-1-yl)met hyl)isoxazol-5-yl)phenyl)ethynyl)benzyl)piperidin-4-yl)morpholine

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (50 mg, 103.83 µmol) and 4-(piperidin-4-yl)morpholine **51a** (35.36 mg, 207.67 µmol, commercially available) were dissolved in 1,2-dichloroethane (5 mL), added with acetic acid (0.2 mL), stirred for 30 minutes, then added with sodium acetate borohydride (66.02 mg, 311.50 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 4-(1-(4-((4-(3-((2-((1S)-1-((tetrahydro-2H-pyran-2-yl)oxy)ethyl)-1H-imidazol-1-yl)methyl)isoxa zol-5-yl)phenyl)ethynyl)benzyl)piperidin-4-yl)morpholine **51b** (50 mg) with a yield of 75.74%.
MS m/z (ESI): 636.4 [M+1]

### The second step

### (S)-1-(1-((5-(4-((4-((4-morpholinopiperidin-1-yl)methyl)phenyl)ethynyl)phenyl)isoxazo 1-3-yl)methyl)-1H-imidazol-2-yl)ethan-1-ol

4-(1-(4-((4-(3-((2-((1S))-1-(tetrahydro-2H-pyran-2-yl)oxy)ethyl)-1H-imidazol-1-yl)met hyl)isoxazol-5-yl)phenyl)ethynyl)benzyl)piperidin-4-yl)morpholine **51b** (50 mg, 78.64 µmol) was added to dichloromethane (5 mL), then added with trifluoroacetic acid (0.2 mL), and continuously stirred at room temperature for 2 hours. The reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-((4-morpholinopiperidin-1-yl)methyl)phenyl)ethynyl)phenyl)isoxazol-3-yl)me thyl)-1H-imidazol-2-yl)ethan-1-ol **51** (50.0 mg) with a yield of 76.79%.
MS m/z (ESI): 552.3 [M+1]

### Example 52

### (S)-1-(4-((4-(3-((2-(1-hydroxyethyl)-1H-imidazol-1-yl)methyl)isoxazol-5-yl)phenyl)eth ynyl)benzyl)azetidin-3-carbonitrile

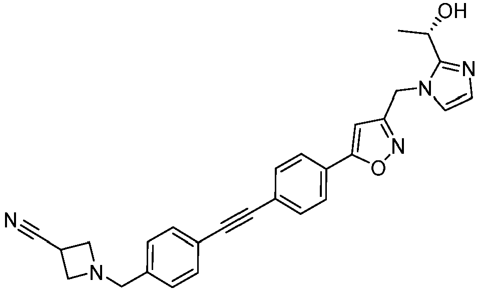

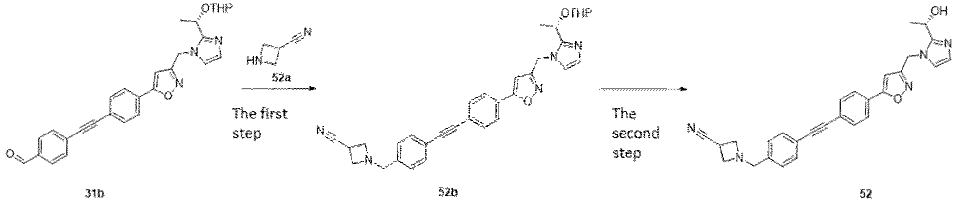

### The first step

### 1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)azetidin-3-carbonitrile

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (50 mg, 103.83 µmol) and azetidin-3-carbonitrile **52a** (24.62 mg, 207.67 µmol, commercially available) were dissolved in 1,2-dichloroethane (5 mL), added with acetic acid (0.2 mL), stirred for 30 minutes, then added with sodium acetate borohydride (66.02 mg, 311.50 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 1-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)azetidin-3-carbonitrile **52b** (50 mg) with a yield of 87.93%.
MS m/z (ESI): 548.3 [M+1]

### The second step

### (S)-1-(4-((4-(3-((2-(1-hydroxyethyl)-1H-imidazol-1-yl)methyl)isoxazol-5-yl)phenyl)eth ynyl)benzyl)azetidin-3-carbonitrile

1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)azetidin-3-carbonitrile **52b** (50 mg, 91.30 µmol) was added to dichloromethane (5 mL), then added with trifluoroacetic acid (0.2 mL), and continuously stirred at room temperature for 2 hours. The reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(4-((4-(3-((2-(1-hydroxyethyl)-1H-imidazol-1-yl)methyl)isoxazol-5-yl)phenyl)ethynyl)ben zyl)azetidin-3-carbonitrile 52 (50.1 mg) with a yield of 87.13%.
MS m/z (ESI): 464.0 [M+1]

### Example 53

### (S)-1-(4-((4-(3-((2-(1-hydroxyethyl)-1H-imidazol-1-yl)methyl)isoxazol-5-yl)phenyl)eth ynyl)benzyl)azetidin-3-carboxamide

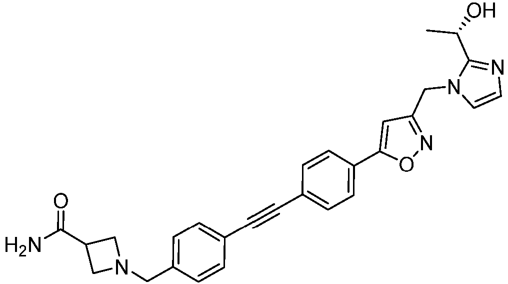

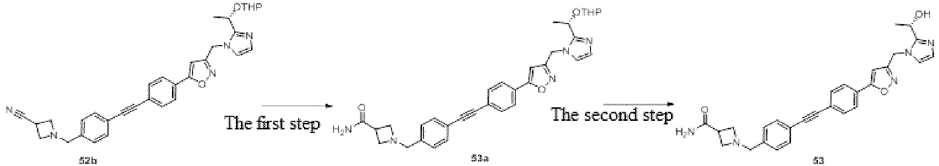

### The first step

### 1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)azetidin-3-carboxamide

1-(4-((4-(3-((2-((1S))-1-((tetrahydro-2H-pyran-2-yl)oxy)ethyl)-1H-imidazol-1-yl)methy l)isoxazol-5-yl)phenyl)ethynyl)benzyl)azetidin-3-carbonitrile **52b** (50 mg, 91.30 µmol) was added to dimethyl sulfoxide (1 mL), added with 2.5 M sodium hydroxide aqueous solution (1 mL) and hydrogen peroxide (0.3 mL) in an ice bath, and continuously stirred at room temperature for 0.5 hours. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated to dryness under reduced pressure to obtain 1-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)azetidin-3-carboxamide **53a** (40 mg), which was used directly in the next reaction.
MS m/z (ESI): 566.3 [M+1]

### The second step

### (S)-1-(4-((4-(3-((2-(1-hydroxyethyl)-1H-imidazol-1-yl)methyl)isoxazol-5-yl)phenyl)eth ynyl)benzyl)azetidin-3-carboxamide

1-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)azetidin-3-carboxamide 53a (40 mg, 70.71 µmol) was added to dichloromethane (5 mL), then added with trifluoroacetic acid (0.2 mL), and continuously stirred at room temperature for 2 hours. The reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(4-((4-(3-((2-(1-hydroxyethyl)-1H-imidazol-1-yl)methyl)isoxazol-5-yl)phenyl)ethynyl)ben zyl)azetidin-3-carboxamide **53** (25.9 mg) with a yield of 55.35%.
MS m/z (ESI): 482.2 [M+1]

### Example 54

### (S)-4-(4-((4-(3-((2-(1-hydroxyethyl)-1H-imidazol-1-yl)methyl)isoxazol-5-yl)phenyl)eth ynyl)benzyl)piperazin-2-one

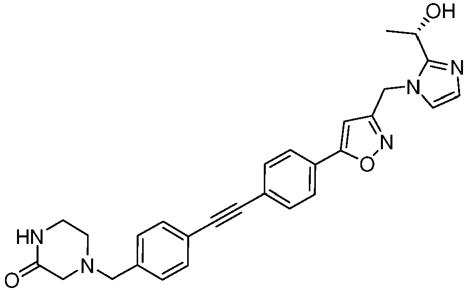

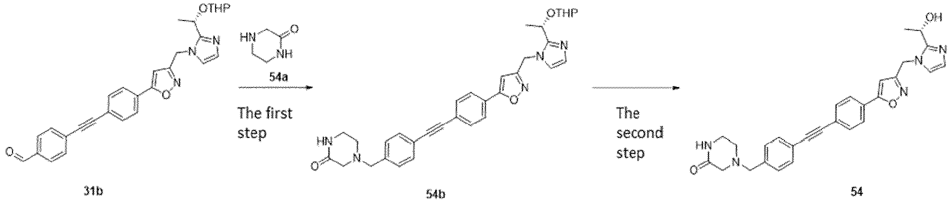

### The first step

### 4-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)piperazin-2-one

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (50 mg, 103.83 µmol) and piperazin-2-one **54a** (15.59 mg, 155.75 µmol, commercially available) were dissolved in 1,2-dichloroethane (5 mL), added with acetic acid (0.2 mL), stirred for 30 minutes, then added with sodium acetate borohydride (66.02 mg, 311.50 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 4-(4-((4-(3-((2-((1S)-1-((tetrahydro-2H-pyran-2-yl)oxy)ethyl)-1H-imidazol-1-yl)methyl)isoxazol -5-yl)phenyl)ethynyl)benzyl)piperazin-2-one **54b** (50 mg) with a yield of 85.13%.
MS m/z (ESI): 566.3 [M+1]

### The second step

### (S)-4-(4-((4-(3-((2-(1-hydroxyethyl)-1H-imidazol-1-yl)methyl)isoxazol-5-yl)phenyl)eth ynyl)benzyl)piperazin-2-one

4-(4-((4-(3-((2-((1S)-1-((tetrahydro-2H-pyran-2-yl)oxy)ethyl)-1H-imidazol-1-yl)methyl )isoxazol-5-yl)phenyl)ethynyl)benzyl)piperazin-2-one **54b** (50 mg, 88.39 µmol) was added to dichloromethane (5 mL), then added with trifluoroacetic acid (0.5 mL), and stirred continuously at room temperature for 2 hours. The reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-4-(4-((4-(3-((2-(1-hydroxyethyl)-1H-imidazol-1-yl)methyl)isoxazol-5-yl)phenyl)ethynyl)ben zyl)piperazin-2-one **54** (26.1 mg) with a yield of 47.15%.
MS m/z (ESI): 482.2 [M+1]

### Example 55

### (S)-3-((4-((4-(3-((2-(1-hydroxyethyl)-1H-imidazol-1-yl)methyl)isoxazol-5-yl)phenyl)et hynyl)benzyl)amino)propanoic acid

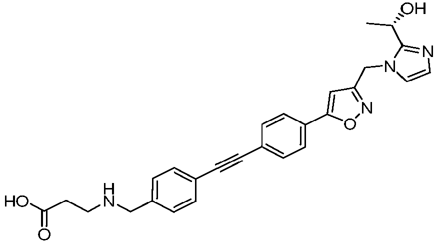

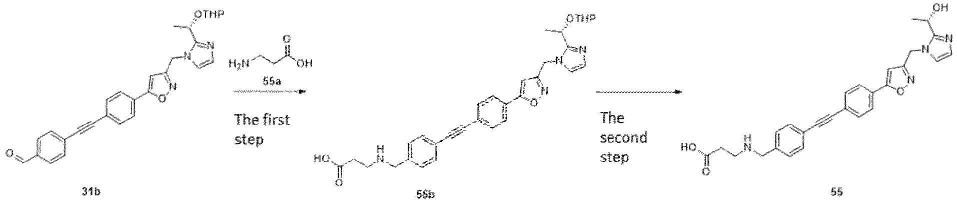

### The first step

### 3-((4-((4-(3-((2-((1S)-1-((tetrahydro-2H-pyran-2-yl)oxy)ethyl)-1H-imidazol-1-yl)methy l)isoxazol-5-yl)phenyl)ethynyl)benzyl)amino)propanoic acid

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (50 mg, 103.83 µmol) and 3-aminopropanoic acid 55a (13.88 mg, 155.75 µmol) were dissolved in 1,2-dichloroethane (5 mL), added with acetic acid (0.2 mL), stirred for 30 minutes, then added with sodium acetate borohydride (66.02 mg, 311.50 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain 3-((4-((4-(3-((2-((1S)-1-((tetrahydro-2H-pyran-2-yl)oxy)ethyl)-1H-imidazol-1-yl)methyl)isoxazo l-5-yl)phenyl)ethynyl)benzyl)amino)propanoic acid **55b** (50 mg) with a yield of 86.82%.
MS m/z (ESI): 555.3 [M+1]

### The second step

### (S)-3-((4-((4-(3-((2-(1-hydroxyethyl)-1H-imidazol-1-yl)methyl)isoxazol-5-yl)phenyl)et hynyl)benzyl)amino)propanoic acid

3-((4-((4-(3-((2-((1S)-1-((tetrahydro-2H-pyran-2-yl)oxy)ethyl)-1H-imidazol-1-yl)methy l)isoxazol-5-yl)phenyl)ethynyl)benzyl)amino)propanoic acid **55b** (50 mg, 90.15 µmol) was added to dichloromethane (5 mL), then added with trifluoroacetic acid (0.5 mL), and stirred continuously for 2 hours at room temperature. The reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-3-((4-((4-(3-((2-(1-hydroxyethyl)-1H-imidazol-1-yl)methyl)isoxazol-5-yl)phenyl)ethynyl)ben zyl)amino)propanoic acid **55** (23.0 mg) with a yield of 39.28%.
MS m/z (ESI): 471.0 [M+1]

### Example 56

### (S)-1-(1-((5-(4-((4-((4-(hydroxymethyl)piperidin-1-yl)methyl)phenyl)ethynyl)phenyl)is oxazol-3-yl)methyl)-1H-imidazol-2-yl)ethan-1-ol

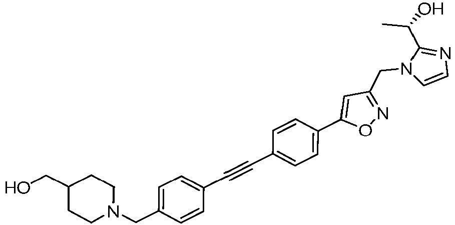

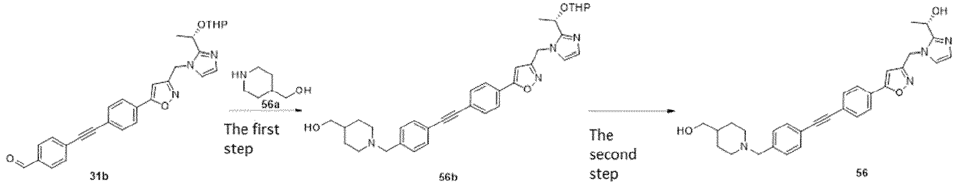

### The first step

### (1-(4-((4-(3-((2-((1S))-1-((tetrahydro-2H-pyran-2-yl)oxy)ethyl)-1H-imidazol-1-yl)meth yl)isoxazol-5-yl)phenyl)ethynyl)benzyl)piperidin-4-yl)methanol

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (75 mg, 155.75 µmol) and piperidin-4-ylmethanol 56a (42.84 mg, 186.90 µmol, commercially available) were dissolved in 1, 2-dichloroethane (5 mL), added with acetic acid (0.5 mL), stirred for 30 minutes, then added with sodium acetate borohydride (99.03 mg, 467.25 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain (1-(4-((4-(3-((2-((1S))-1-((tetrahydro-2H-pyran-2-yl)oxy)ethyl)-1H-imidazol-1-yl)methyl)isoxaz ol-5-yl)phenyl)ethynyl)benzyl)piperidin-4-yl)methanol **56b** (65 mg) with a yield of 71.87%.
MS m/z (ESI): 581.3 [M+1]

### The second step

### (S)-1-(1-((5-(4-((4-((4-(hydroxymethyl)piperidin-1-yl)methyl)phenyl)ethynyl)phenyl)is oxazol-3-yl)methyl)-1H-imidazol-2-yl)ethan-1-ol

(1-(4-((4-(3-((2-((1S))-1-((tetrahydro-2H-pyran-2-yl)oxy)ethyl)-1H-imidazol-1-yl)meth yl)isoxazol-5-yl)phenyl)ethynyl)benzyl)piperidin-4-yl)methanol **56b** (65 mg, 111.93 µmol) and trifluoroacetic acid (0.5 mL) were sequentially added into dichloromethane (5 mL), and continuously stirred at room temperature for 12 hours. The reaction solution was concentrated under reduced pressure. The obtained residue was added to methanol (2 mL), added with 5 M sodium hydroxide aqueous solution (2 mL), and continuously stirred for 12 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-((4-(hydroxymethyl)piperidin-1-yl)methyl)phenyl)ethynyl)phenyl)isoxazol-3-yl)methyl)-1H-imidazol-2-yl)ethan-1-ol **56** (20.5 mg) with a yield of 28.49%.
MS m/z (ESI): 497.0 [M+1]

### Example 57

### (S)-1-(1-((5-(4-((4-(((2-aminoethyl)amino)methyl)phenyl)ethynyl)phenyl)isoxazol-3-yl) methyl)-1H-imidazol-2-yl)ethan-1-ol

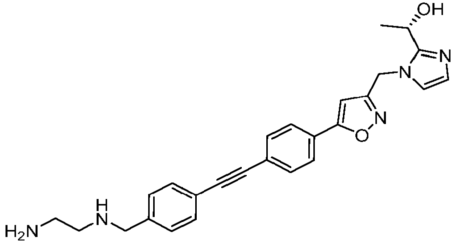

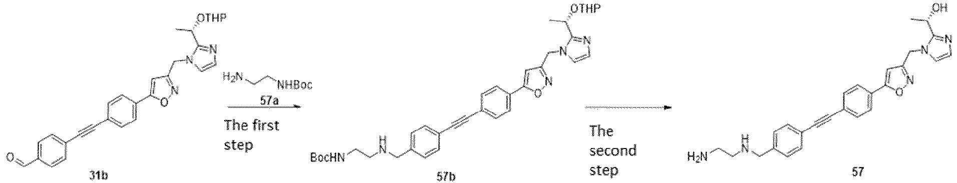

### The first step

### Tert-butyl (2-((4-((4-(3-((2-((1S))-1-((tetrahydro-2H-pyran-2-yl)oxy)ethyl)-1H-imidazol-1-yl)methyl)isoxaz ol-5-yl)phenyl)ethynyl)benzyl)amino)ethyl)carbamate

4-((4-(3-((2-((1S)-1-((tetrahydro-2H-pyran-2-yl)oxy)ethyl)-1H-imidazol-1-yl)methyl)is oxazol-5-yl)phenyl)ethynyl)benzaldehyde **31b** (65 mg, 134.98 µmol) and tert-butyl (2-aminoethyl)carbamate **57a** (32.44 mg, 202.47 µmol, commercially available) were dissolved in 1,2-dichloroethane (20 mL), added with acetic acid (0.5 mL), stirred for 30 minutes, then added with sodium acetate borohydride (85.83 mg, 404.95 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure. The obtained residue was purified by silica gel column chromatography (eluent: System A) to obtain tert-butyl (2-((4-((4-(3-((2-((1S))-1-((tetrahydro-2H-pyran-2-yl)oxy)ethyl)-1H-imidazol-1-yl)methyl)isoxaz ol-5-yl)phenyl)ethynyl)benzyl)amino)ethyl)carbamate **57b** (65 mg) with a yield of 76.95%.
MS m/z (ESI): 626.0 [M+1]

### The second step

### (S)-1-(1-((5-(4-((4-(((2-aminoethyl)amino)methyl)phenyl)ethynyl)phenyl)isoxazol-3-yl) methyl)-1H-imidazol-2-yl)ethan-1-ol

Tert-butyl (2-((4-((4-(3-((2-((1S))-1-((tetrahydro-2H-pyran-2-yl)oxy)ethyl)-1H-imidazol-1-yl)methyl)isoxaz ol-5-yl)phenyl)ethynyl)benzyl)amino)ethyl)carbamate **57b** (65.00 mg, 103.87 µmol) and trifluoroacetic acid (1 mL) were sequentially added to dichloromethane (3 mL), and stirred continuously at room temperature for 12 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((4-(((2-aminoethyl)amino)methyl)phenyl)ethynyl)phenyl)isoxazol-3-yl)methyl)-1H-imidazol-2-yl)ethan-1-ol **57** (20 mg) with a yield of 31.95%.
MS m/z (ESI): 442.3 [M+1]

### Example 58

### (S)-3-(5-((4-(3-((2-(1-hydroxyethyl)-1H-imidazol-1-yl)methyl)isoxazol-5-yl)phenyl)eth ynyl)pyridin-2-yl)propanoic acid

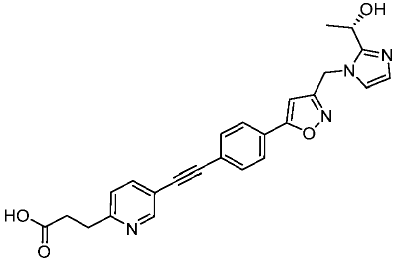

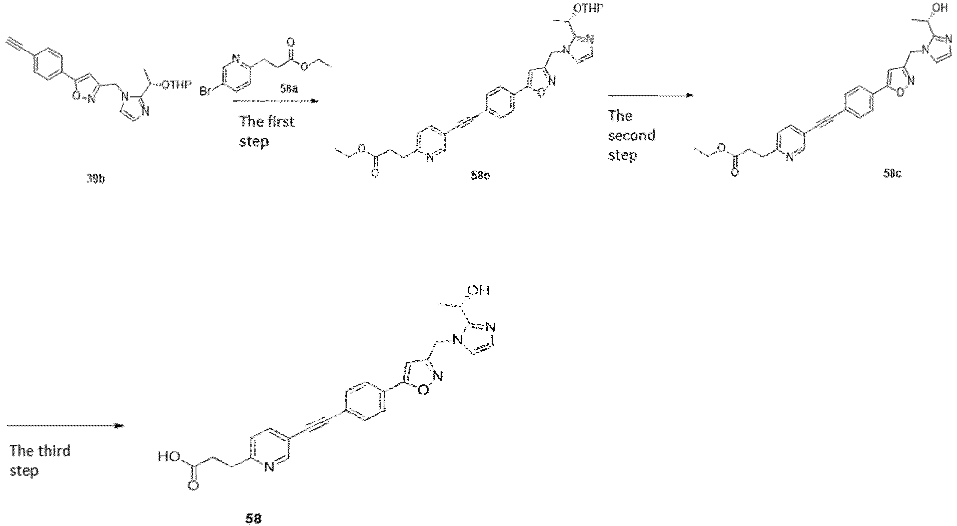

### The first step

### Ethyl 3-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)propanoate

5-(4-Ethynylphenyl)-3-((2-((1S)-1-((tetrahydro-2H-pyran-2-yl)oxy)ethyl)-1H-imidazol-1-yl)methyl)isoxazole **39b** (100 mg, 264.95 µmol), ethyl 3-(5-bromopyridin-2-yl)propanoate **58a** (34.19 mg, 132.47 µmol, prepared according to the published patent WO 2017221100), allylpalladium(II) chloride dimer (19.34 mg, 52.99 µmol), triethylenediamine (89.16 mg, 794.84 µmol) and tri-tert-butylphosphine (107.21 mg, 52.99 µmol, 10% toluene solution) were sequentially added to acetonitrile (2 mL), and the system was replaced with argon gas three times and stirred continuously at room temperature for 12 hours. After the reaction was completed, the reaction was concentrated under reduced pressure. The obtained residue was purified by silica gel column chromatography (eluent: System B) to obtain ethyl 3-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)propanoate **58b** (20 mg) with a yield of 13.61%.
MS m/z (ESI): 555.4 [M+1]

### The second step

### Ethyl (S)-3-(5-((4-(3-((2-(1-hydroxyethyl)-1H-imidazol-1-yl)methyl)isoxazol-5-yl)phenyl)ethynyl)pyri din-2-yl)propanoate

Ethyl 3-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)propanoate **58b** (20 mg, 36.06 µmol) and trifluoroacetic acid (8.22 mg, 72.12 µmol) were sequentially added to dichloromethane (1 mL), and continuously stirred at room temperature for 12 hours. The reaction solution was concentrated under reduced pressure to obtain ethyl (S)-3-(5-((4-(3-((2-(1-hydroxyethyl)-1H-imidazol-1-yl)methyl)isoxazol-5-yl)phenyl)ethynyl)pyri din-2-yl)propanoate **58c** (10 mg) with a yield of 58.94%.
MS m/z (ESI): 471.3 [M+1]

### The third step

### (S)-3-(5-((4-(3-((2-(1-hydroxyethyl)-1H-imidazol-1-yl)methyl)isoxazol-5-yl)phenyl)eth ynyl)pyridin-2-yl)propanoic acid

Ethyl (S)-3-(5-((4-(3-((2-(1-hydroxyethyl)-1H-imidazol-1-yl)methyl)isoxazol-5-yl)phenyl)ethynyl)pyri din-2-yl)propanoate **58c** (10 mg, 21.25 µmol) was added to a mixed solution of methanol (2 mL) and tetrahydrofuran (2 mL), then added with 2.5 M sodium hydroxide aqueous solution (1 mL), and stirred continuously at room temperature for 12 hours. The reaction solution was adjusted to pH 5 with 1 M dilute hydrochloric acid, and concentrated under reduced pressure. The residue was added with ethyl acetate (30 mL) and water (15 mL) for layer separation. The aqueous phase was extracted with ethyl acetate (30 mL × 2). The combined organic phase was washed with saturated sodium chloride solution (30 mL×2), dried over anhydrous sodium sulfate, filtered, and concentrated under reduced pressure. The residue was purified by preparative liquid chromatography (separation column AKZONOBEL Kromasil; 250×21.2 mm I.D.; 5 µm, 20 mL/min; mobile phase A: 0.05%TFA+H₂O, mobile phase B: CH₃CN) to obtain (S)-3-(5-((4-(3-((2-(1-hydroxyethyl)-1H-imidazol-1-yl)methyl)isoxazol-5-yl)phenyl)ethynyl)pyri din-2-yl)propanoic acid **58** (2.5 mg) with a yield of 18.18%.
MS m/z (ESI): 443.2 [M+1]

### Example 59

### (S)-1-(1-((5-(4-((6-(((2-(methylsulfonyl)ethyl)amino)methyl)pyridin-3-yl)ethynyl)phen yl)isoxazol-3-yl)methyl)-1H-imidazol-2-yl)ethan-1-ol

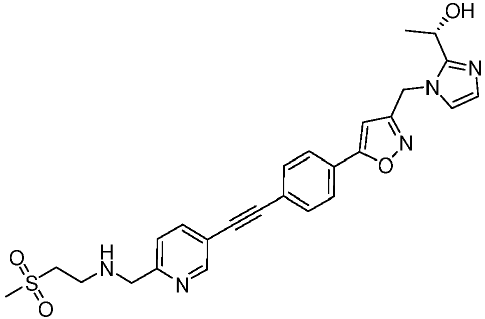

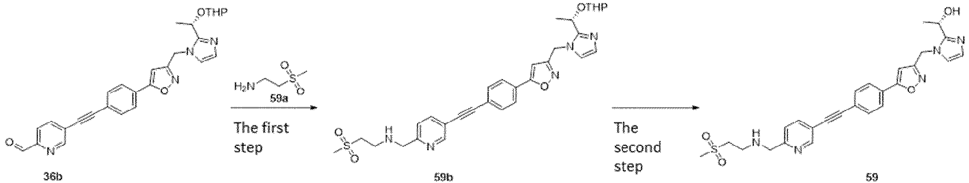

### The first step

### 2-(Methylsulfonyl)-N-((5-((4-(3-((2-((1S))-1-((tetrahydro-2H-pyran-2-yl)oxy)ethyl)-1H -imidazol-1-yl)methyl)isoxazol-5-yl)phenyl)ethynyl)pyridin-2-yl)methyl)ethan-1-amine

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde 36b (50 mg, 103.62 µmol), and 2-(methylsulfonyl)ethan-1-amine **59a** (19.15 mg, 155.43 µmol, commercially available) were dissolved in dichloromethane (0.5 mL), stirred for 30 minutes, added with sodium acetate borohydride (43.92 mg, 207.24 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain (S)-1-(1-(((5-(4-((6-(((2-(methylsulfonyl)ethyl)amino)methyl)pyridin-3-yl)ethynyl)phenyl)isoxaz ol-3-yl)methyl)-1H-imidazol-2-yl)ethan-1-ol **59b** (20 mg), which was used directly in the next reaction.
MS m/z (ESI): 590.3 [M+1]

### The second step

### (S)-1-(1-((5-(4-((6-(((2-(methylsulfonyl)ethyl)amino)methyl)pyridin-3-yl)ethynyl)phen yl)isoxazol-3-yl)methyl)-1H-imidazol-2-yl)ethan-1-ol

(S)-1-(1-(((5-(4-((6-(((2-(methylsulfonyl)ethyl)amino)methyl)pyridin-3-yl)ethynyl)phen yl)isoxazol-3-yl)methyl)-1H-imidazol-2-yl)ethan-1-ol **59b** (20 mg, 33.92 µmol) was added to dichloromethane (2 mL), added with trifluoroacetic acid (0.5 mL), and stirred continuously at room temperature for 3 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((6-(((2-(methylsulfonyl)ethyl)amino)methyl)pyridin-3-yl)ethynyl)phenyl)isoxazo 1-3-yl)methyl)-1H-imidazol-2-yl)ethan-1-ol **59** (12 mg) with a yield of 52.94%.
MS m/z (ESI): 505.9 [M+1]

### Example 60

### (S)-2-(((5-((4-(3-((2-(1-hydroxyethyl))-1H-imidazol-1-yl)methyl)isoxazol-5-yl)phenyl) ethynyl)pyridin-2-yl)methyl)amino)ethan-1-ol

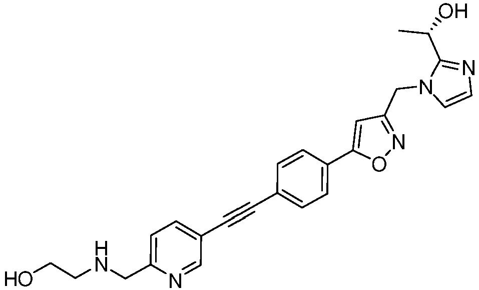

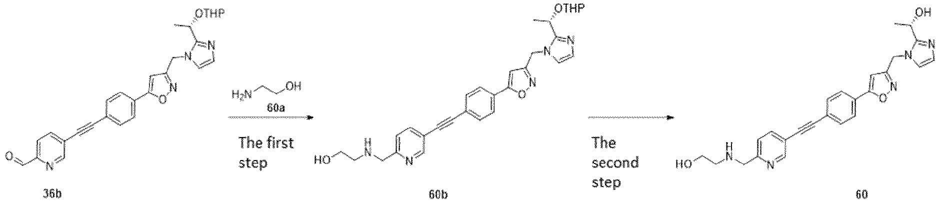

### The first step

### 2-(((5-((4-(3-((2-((1S))-1-((tetrahydro-2H-pyran-2-yl)oxy)ethyl)-1H-imidazol-1-yl)met hyl)isoxazol-5-yl)phenyl)ethynyl)pyridin-2-yl)methyl)amino)ethan-1-ol

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol), and 2-aminoethan-1-ol **60a** (9.49 mg, 155.43 µmol, commercially available) were dissolved in dichloromethane (0.5 mL), stirred for 30 minutes, added with sodium acetate borohydride (65.88 mg, 310.86 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain 2-(((5-((4-(3-((2-((1S))-1-((tetrahydro-2H-pyran -2-yl)oxy)ethyl)-1H-imidazol-1-yl)methyl)isoxazol-5-yl)phenyl)ethynyl)pyridin-2-yl)methyl)ami no)ethan-1-ol **60b** (20 mg), which was used directly in the next reaction.
MS m/z (ESI): 528.3 [M+1]

### The second step

### (S)-2-(((5-((4-(3-((2-(1-hydroxyethyl))-1H-imidazol-1-yl)methyl)isoxazol-5-yl)phenyl) ethynyl)pyridin- 2-yl)methyl)amino)ethan-1-ol

2-(((5-((4-(3-((2-((1S))-1-((tetrahydro-2H-pyran-2-yl)oxy)ethyl))-1H-imidazol-1-yl)met hyl)isoxazol-5-yl)phenyl)ethynyl)pyridin-2-yl)methyl)amino)ethan-1-ol **60b** (20 mg, 37.91 µmol) and trifluoroacetic acid (8.64 mg, 75.81 µmol) were sequentially added to dichloromethane (2 mL), and stirred continuously for 4 hours at room temperature. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-(((5-((4-(3-((2-(1-hydroxyethyl))-1H-imidazol-1-yl)methyl)isoxazol-5-yl)phenyl)ethynyl)p yridin-2-yl)methyl)amino)ethan-1-ol **60** (12.75 mg) with a yield of 57.31%.
MS m/z (ESI): 444.3 [M+1]

### Example 61

### (S)-2-(((5-((4-(3-((2-(1-hydroxyethyl))-1H-imidazol-1-yl)methyl)isoxazol-5-yl)phenyl) ethynyl)pyridin-2-yl)methyl)amino)acetamide

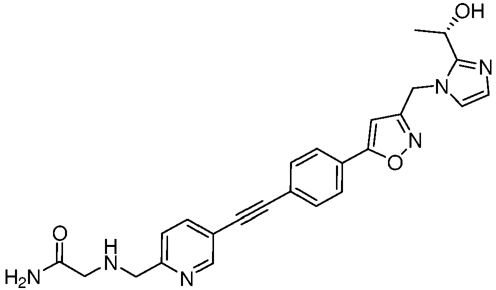

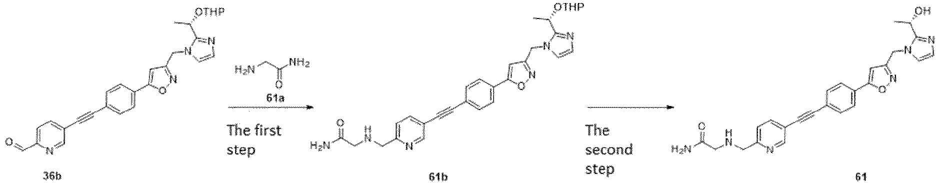

### The first step

### 2-(((5-((4-(3-((2-((1S))-1-((tetrahydro-2H-pyran-2-yl)oxy)ethyl)-1H-imidazol-1-yl)met hyl)isoxazol-5-yl)phenyl)ethynyl)pyridin-2-yl)methyl)amino)acetamide

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) and 2-aminoacetamide **61a** (11.51 mg, 155.43 µmol) were dissolved in dichloromethane (0.5 mL), stirred at room temperature for 30 minutes, added with sodium acetate borohydride (39.53 mg, 186.52 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain 2-(((5-((4-(3-((2-((1S))-1-((tetrahydro-2H-pyran-2-yl)oxy)ethyl)-1H-imidazol-1-yl)methyl)isoxaz ol-5-yl)phenyl)ethynyl)pyridin-2-yl)methyl)amino)acetamide **61b** (20 mg), which was used directly in the next reaction.
MS m/z (ESI): 541.0 [M+1]

### The second step

### (S)-2-(((5-((4-(3-((2-(1-hydroxyethyl))-1H-imidazol-1-yl)methyl)isoxazol-5-yl)phenyl) ethynyl)pyridin-2-yl)methyl)amino)acetamide

2-(((5-((4-(3-((2-((1S))-1-((tetrahydro-2H-pyran-2-yl)oxy)ethyl)-1H-imidazol-1-yl)met hyl)isoxazol-5-yl)phenyl)ethynyl)pyridin-2-yl)methyl)amino)acetamide **61b** (20 mg, 37.00 µmol) and trifluoroacetic acid (8.44 mg, 73.99 µmol) were sequentially added to dichloromethane (2 mL), and stirred continuously at room temperature for 4 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-(((5-((4-(3-((2-(1-hydroxyethyl))-1H-imidazol-1-yl)methyl)isoxazol-5-yl)phenyl)ethynyl)p yridin-2-yl)methyl)amino)acetamide **61** (5.50 mg) with a yield of 3.45%.
MS m/z (ESI): 457.3 [M+1]

### Example 62

### (S)-2-(5-((4-(3-((2-(1-hydroxyethyl)-1H-imidazol-1-yl)methyl)isoxazol-5-yl)phenyl)eth ynyl)pyridin-2-yl)acetamide

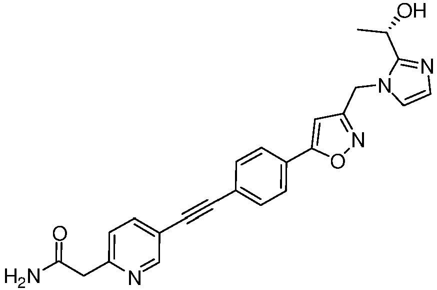

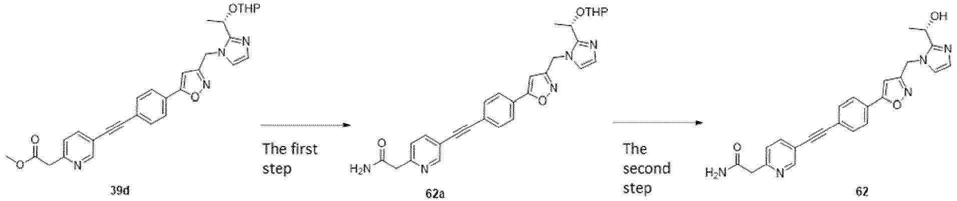

### The first step

### 2-(5-((4-(3-((2-((1S)-1-((tetrahydro-2H-pyran-2-yl)oxy)ethyl)-1H-imidazol-1-yl)methyl )isoxazol-5-yl)phenyl)ethynyl)pyridin-2-yl)acetamide

Methyl 2-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)acetate 39d (45 mg, 85.46 µmol) and ammonia solution (29.95 mg, 854.57 µmol) were sequentially added to ethanol (1 mL), and continuously stirred at room temperature for 12 hours. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure to obtain 2-(5-((4-(3-((2-((1S)-1-((tetrahydro-2H-pyran-2-yl)oxy)ethyl)-1H-imidazol-1-yl)methyl)isoxazol -5-yl)phenyl)ethynyl)pyridin-2-yl)acetamide **62a** (40 mg), which was used directly in the next reaction.
MS m/z (ESI): 512.0 [M+1]

### The second step

### (S)-2-(5-((4-(3-((2-(1-hydroxyethyl)-1H-imidazol-1-yl)methyl)isoxazol-5-yl)phenyl)eth ynyl)pyridin-2-yl)acetamide

2-(5-((4-(3-((2-((1S)-1-((tetrahydro-2H-pyran-2-yl)oxy)ethyl)-1H-imidazol-1-yl)methyl )isoxazol-5-yl)phenyl)ethynyl)pyridin-2-yl)acetamide **62a** (40 mg, 78.19 µmol) and trifluoroacetic acid (8.92 mg, 78.19 µmol) were sequentially added to dichloromethane (1 mL), and continuously stirred at room temperature for 12 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-(5-((4-(3-((2-(1-hydroxyethyl)-1H-imidazol-1-yl)methyl)isoxazol-5-yl)phenyl)ethynyl)pyri din-2-yl)acetamide **62** (19.0 mg) with a yield of 44.43%.
MS m/z (ESI): 428.1 [M+1]

### Example 63

### (S)-3-((4-(3-((2-(1-hydroxyethyl)-1H-imidazol-1-yl)methyl)isoxazol-5-yl)phenyl)ethyn yl)benzamide

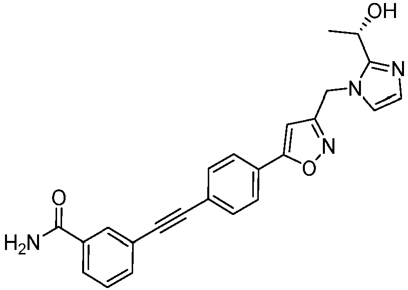

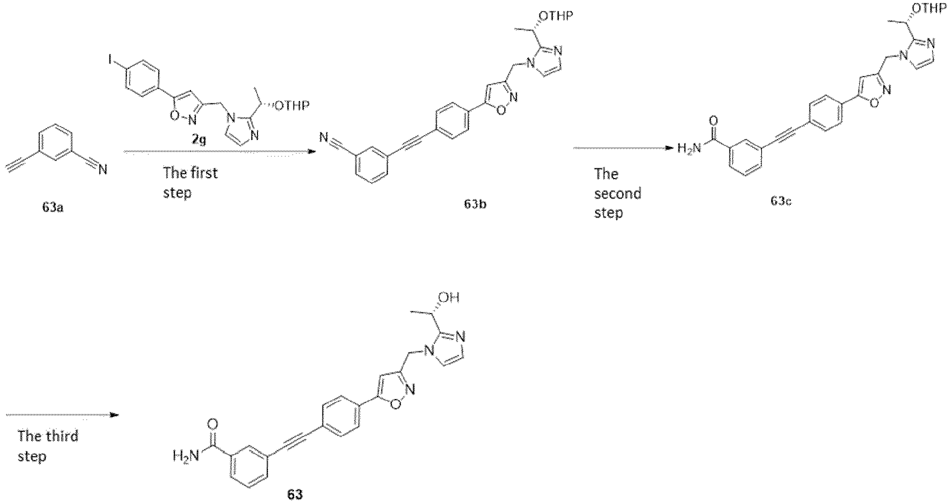

### The first step

### 3-((4-(3-((2-((1S)-1-((tetrahydro-2H-pyran-2-yl)oxy)ethyl)-1H-imidazol-1-yl)methyl)is oxazol-5-yl)phenyl)ethynyl)benzonitrile

3-Ethylbenzonitrile **63a** (50 mg, 393.26 µmol), 5-(4-iodophenyl)-3-((2-((1S)-1-((tetrahydro-2H-pyran-2-yl)oxy)ethyl)-1H-imidazol-1-yl)methyl)i soxazole **2g** (94.25 mg, 196.63 µmol), allylpalladium(II) chloride dimer (14.39 mg, 39.33 µmol), tri-tert-butylphosphine (7.96 mg, 39.33 µmol, 10% toluene solution) and triethylenediamine (132.34 mg, 1.18 mmol) were sequentially added to acetonitrile (0.5 mL), and the system was replaced with argon gas three times and stirred at 25°C for 16 hours. After the reaction was completed, the reaction was concentrated under reduced pressure, and the residue was purified by column chromatography (eluent: System B) to obtain 3-((4-(3-((2-((1S)-1-((tetrahydro-2H-pyran-2-yl)oxy)ethyl)-1H-imidazol-1-yl)methyl)isoxazol-5-yl)phenyl)ethynyl)benzonitrile **63b** (40 mg) with a yield of 21.26%.
MS m/z (ESI): 479.0 [M+1]

### The second step

### 3-((4-(3-((2-((1S)-1-((tetrahydro-2H-pyran-2-yl)oxy)ethyl)-1H-imidazol-1-yl)methyl)is oxazol-5 -yl)phenyl)ethynyl)benzamide

3-((4-(3-((2-((1S)-1-((tetrahydro-2H-pyran-2-yl)oxy)ethyl)-1H-imidazol-1-yl)methyl)is oxazol-5-yl)phenyl)ethynyl)benzonitrile **63b** (20 mg, 41.79 µmol) and sodium hydroxide (2.01 mg, 50.15 µmol) were sequentially added to dimethyl sulfoxide (0.5 mL), slowly added with hydrogen peroxide (0.5 mL) dropwise in a water bath, then warmed to 25°C and stirred for 0.5 hours. After the reaction was completed, the reaction mixture was concentrated under reduced pressure to obtain 3-((4-(3-((2-((1S)-1-((tetrahydro-2H-pyran-2-yl)oxy)ethyl)-1H-imidazol-1-yl)methyl)isoxazol-5-yl)phenyl)ethynyl)benzamide **63c** (20 mg), which was used directly in the next reaction.
MS m/z (ESI): 497.0 [M+1]

### The third step

### (S)-3-((4-(3-((2-(1-hydroxyethyl)-1H-imidazol-1-yl)methyl)isoxazol-5-yl)phenyl)ethyn yl)benzamide

3-((4-(3-((2-((1S)-1-((tetrahydro-2H-pyran-2-yl)oxy)ethyl)-1H-imidazol-1-yl)methyl)is oxazol-5-yl)phenyl)ethynyl)benzamide **63c** (20 mg, 40.28 µmol) was added to dichloromethane (0.5 mL), slowly added with trifluoroacetic acid (13.78 mg, 120.83 µmol) dropwise, and stirred at 25°C for 16 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-3-((4-(3-((2-(1-hydroxyethyl)-1H-imidazol-1-yl)methyl)isoxazol-5-yl)phenyl)ethynyl)benza mide **63** (6 mg) with a yield of 26.82%.
MS m/z (ESI): 413.0 [M+1]

### Example 64

### (S)-2-(3-((4-(3-((2-(1-hydroxyethyl)-1H-imidazol-1-yl)methyl)isoxazol-5-yl)phenyl)eth ynyl)phenoxy)acetamide

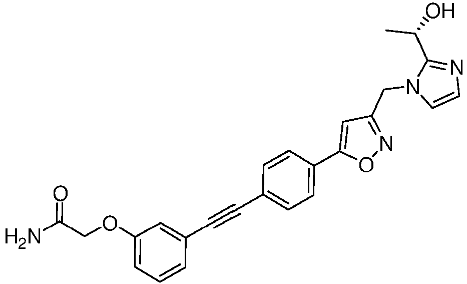

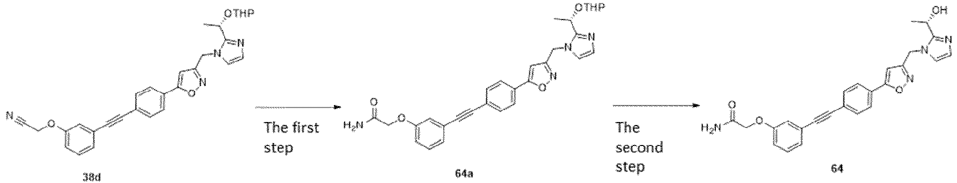

### The first step

### 2-(3-((4-(3-((2-((1S)-1-((tetrahydro-2H-pyran-2-yl)oxy)ethyl)-1H-imidazol-1-yl)methyl )isoxazol-5-yl)phenyl)ethynyl)phenoxy)acetamide

2-(4-((4-(3-((2-((1S))-1-((tetrahydro-2H-pyran-2-yl)oxy)ethyl)-1H-imidazol-1-yl)methy l)isoxazol-5-yl)phenyl)ethynyl)phenoxy)acetonitrile **38d** (50 mg, 98.32 µmol) and sodium hydroxide (11.80 mg, 294.95 µmol) were sequentially added to dimethyl sulfoxide (0.5 mL), slowly added with hydrogen peroxide (0.5 mL) dropwise in a water bath, then warmed to room temperature, and stirred for 2 hours. After the reaction was completed, the reaction mixture was concentrated under reduced pressure to obtain 2-(3-((4-(3-((2-((1S)-1-((tetrahydro-2H-pyran-2-yl)oxy)ethyl)-1H-imidazol-1-yl)methyl)isoxazol -5-yl)phenyl)ethynyl)phenoxy)acetamide **64a** (51 mg), which was directly used in the next reaction.
MS m/z (ESI): 527.9 [M+1]

### The second step

### (S)-2-(3-((4-(3-((2-(1-hydroxyethyl)-1H-imidazol-1-yl)methyl)isoxazol-5-yl)phenyl)eth ynyl)phenoxy)acetamide

2-(3-((4-(3-((2-((1S)-1-((tetrahydro-2H-pyran-2-yl)oxy)ethyl)-1H-imidazol-1-yl)methyl )isoxazol-5-yl)phenyl)ethynyl)phenoxy)acetamid **64a** (50 mg, 94.95 µmol) was added to dichloromethane (1 mL), slowly added with trifluoroacetic acid (32.48 mg, 284.86 µmol) dropwise, and stirred at 25°C for 16 hours. After the reaction was completed, the reaction mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-(3-((4-(3-((2-(1-hydroxyethyl)-1H-imidazol-1-yl)methyl)isoxazol-5-yl)phenyl)ethynyl)phe noxy)acetamide **64** (9.4 mg) with a yield of 16.01%.
MS m/z (ESI): 442.9 [M+1]

Examples 65-110 were synthesized according to the synthesis method of Examples 1-64 of the present disclosure. The structure and characterization data are as shown in the following table:

| Exa mple No. | Structure | Name | MS m/z (ESI) |
|---|---|---|---|
| 65 | 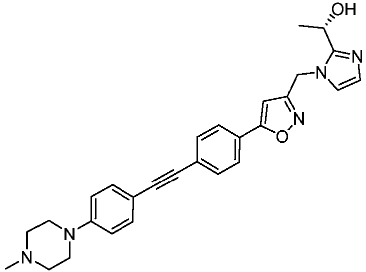 | (S)-1-(1-((5-(4-((4-(4-methylp iperazin-1-yl)phenyl)ethynyl) phenyl)isoxazol-3-yl)methyl)-1H-imidazol-2-yl)ethan-1-ol | 486.0 [M+19] |
| 66 | 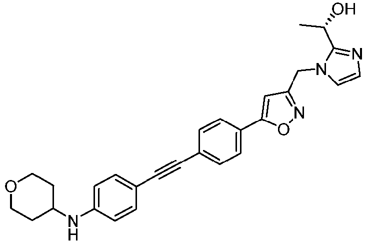 | (S)-1-(1-((5-(4-((4-((tetrahydr o-2H-pyran-4-yl)amino)pheny l)ethynyl)phenyl)isoxazol-3-yl )methyl)-1H-imidazol-2-yl) ethan-1-ol | 487.0 [M+19] |
| 67 | 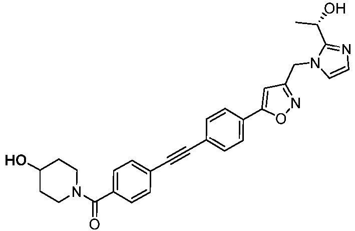 | (S)-(4-((4-(3-((2-(1-hydroxyet hyl)-1H-imidazol-1-yl)methyl )isoxazol-5-yl)phenyl)ethynyl) phenyl)(4-hydroxypiperidin-1-yl)methanone | 497.3 [M+1] |
| 68 | 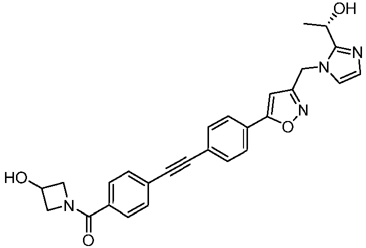 | (S)-(3-hydroxyazetidin-1-yl)(4 -((4-(3-((2-(1-hydroxyethyl)-1H-imidazol-1-yl)methyl)isoxa zol-5-yl)phenyl)ethynyl)phenyl )methanone | 469.3 [M+1] |
| 69 | 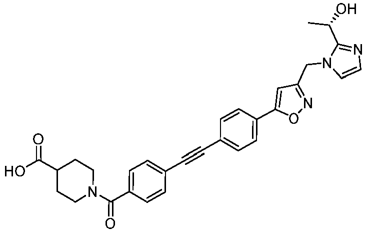 | (S)-1-(4-((4-(3-((2-(1-hydroxy ethyl)-1H-imidazol-1-yl)meth yl)isoxazol-5-yl)phenyl)ethyn yl)benzoyl)piperidin-4-carboxylic acid | 525.3 [M+1] |
| 70 | 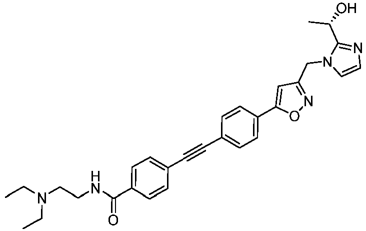 | (S)-N-(2-(diethylamino)ethyl) -4-((4-(3-((2-(1-hydroxyethyl) -1H-imidazol-1-yl)methyl)iso xazol-5-yl)phenyl)ethynyl)ben zamide | 512.3 [M+1] |
| 71 | 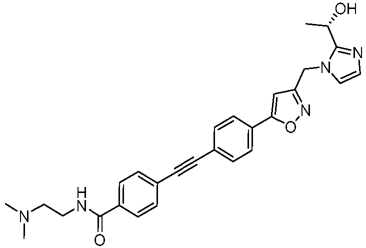 | (S)-N-(2-(dimethylamino)ethyl )-4-((4-(3-((2-(1-hydroxyethyl )-1H-imidazol-1-yl)methyl)is oxazol-5-yl)phenyl)ethynyl) benzamide | 484.3 [M+1] |
| 72 | 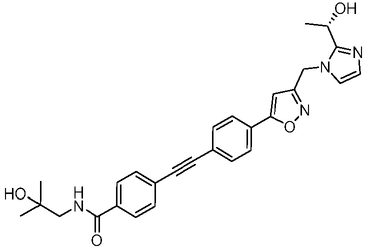 | (S)-N-(2-hydroxy-2-methylpr opyl)-4-((4-(3-((2-(1-hydroxy ethyl)-1H-imidazol-1-yl)meth yl)isoxazol-5-yl)phenyl)ethyn yl)benzamide | 485.0 [M+1] |
| 73 | 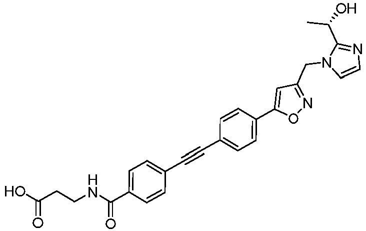 | (S)-3-(4-((4-(3-((2-(1-hydroxy ethyl)-1H-imidazol-1-yl)meth yl)isoxazol-5-yl)phenyl)ethyn yl)benzamido)propanoic acid | 484.9 [M+1] |
| 74 | 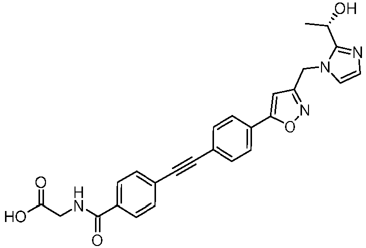 | (S)-(4-((4-(3-((2-(1-hydroxyet hyl)-1H-imidazol-1-yl)methyl )isoxazol-5-yl)phenyl)ethynyl) benzoyl)glycinate | 471.1 [M+1] |
| 75 | 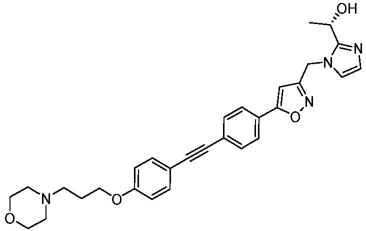 | (S)-1-(1-((5-(4-((4-(3-morphol inopropoxy)phenyl)ethynyl)ph enyl)isoxazol-3-yl)methyl)-1H -imidazol-2-yl)ethan-1-ol | 513.0 [M+1] |
| 76 | 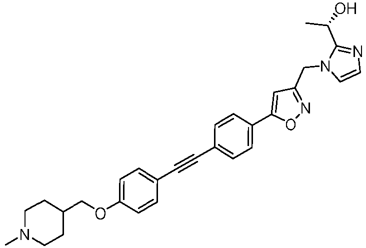 | (S)-1-(1-((5-(4-((4-((1-methyl piperidin-4-yl)methoxy)pheny l)ethynyl)phenyl)isoxazol-3-yl )methyl)-1H-imidazol-2-yl) ethan-1-ol | 497.0 [M+1] |
| 77 | 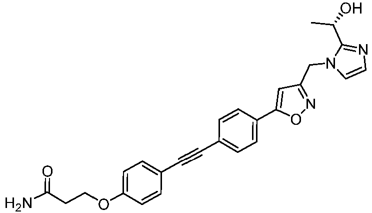 | (S)-3-(4-((4-(3-((2-(1-hydroxy ethyl)-1H-imidazol-1-yl)meth yl)isoxazol-5-yl)phenyl)ethyn yl)phenoxy)propanamide | 456.9 [M+1] |
| 78 | 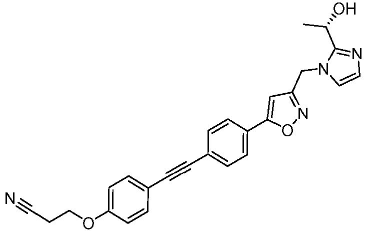 | (S)-3-(4-((4-(3-((2-(1-hydroxy ethyl)-1H-imidazol-1-yl)meth yl)isoxazol-5-yl)phenyl)ethyn yl)phenoxy)propanenitrile | 439.0 [M+1] |
| 79 | 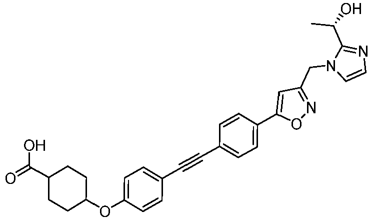 | (S)-4-(4-((4-(3-((2-(1-hydroxy ethyl)-1H-imidazol-1-yl)meth yl)isoxazol-5-yl)phenyl)ethyn yl)phenoxy)cyclohexane-1-carboxylic acid | 511.9 [M+1] |
| 80 | 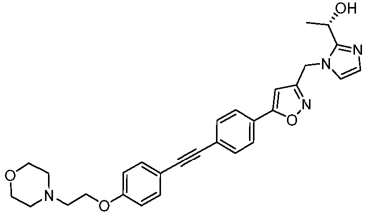 | (S)-1-(1-((5-(4-((4-(2-morphol inoethoxy)phenyl)ethynyl)phe nyl)isoxazol-3-yl)methyl)-1H-imidazol-2-yl)ethan-1-ol | 499.2 [M+1] |
| 81 | 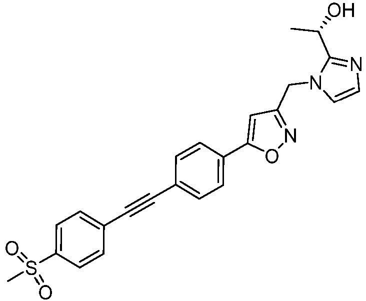 | (S)-1-(1-((5-(4-((4-(methylsulf onyl)phenyl)ethynyl)phenyl)is oxazol-3-yl)methyl)-1H-imida zol-2-yl)ethan-1-ol | 447.8 [M+1] |
| 82 | 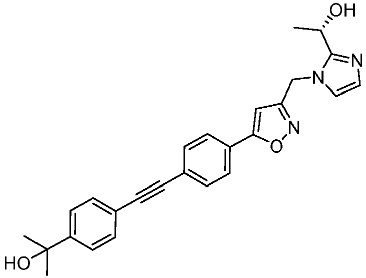 | (S)-2-(4-((4-(3-((2-(1-hydroxy ethyl)-1H-imidazol-1-yl)meth yl)isoxazol-5-yl)phenyl)ethyn yl)phenyl)propan-2-ol | 428.1 [M+1] |
| 83 | 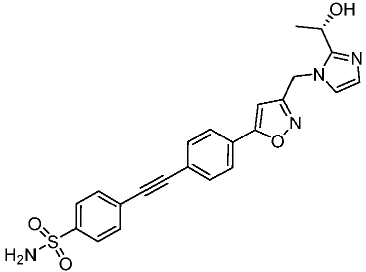 | (S)-4-((4-(3-((2-(1-hydroxyeth yl)-1H-imidazol-1-yl)methyl) isoxazol-5-yl)phenyl)ethynyl) benzenesulfonamide | 448.9 [M+1] |
| 84 | 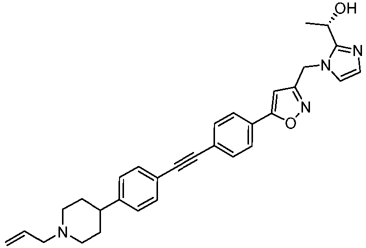 | (S)-1-(1-((5-(4-((4-(1-allylpip eridin-4-yl)phenyl)ethynyl)ph enyl)isoxazol-3-yl)methyl)-1H -imidazol-2-yl)ethan-1-ol | 493.0 [M+1] |
| 85 | 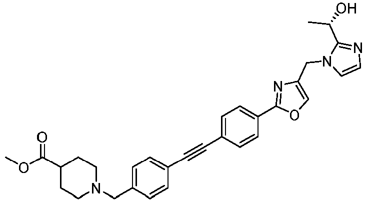 | Methyl (S)-1-(4-((4-(4-((2-(1-hydroxy ethyl)-1H-imidazol-1-yl)meth yl)oxazol-2-yl)phenyl)ethynyl )benzyl)piperidin-4-carboxylate | 525.0 [M+1] |
| 86 | 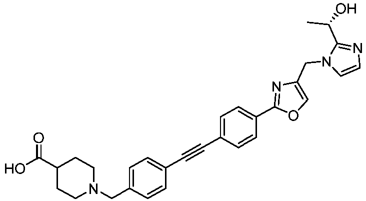 | (S)-1-(4-((4-(4-((2-(1-hydroxy ethyl)-1H-imidazol-1-yl)meth yl)oxazol-2-yl)phenyl)ethynyl )benzyl)piperidin-4-carboxylic acid | 510.9 [M+1] |
| 87 | 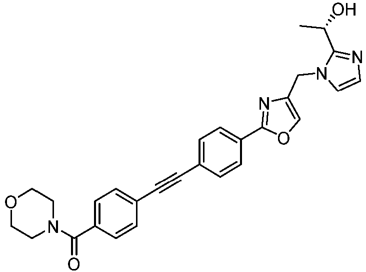 | (S)-(4-((4-(4-((2-(1-hydroxyet hyl)-1H-imidazol-1-yl)methyl )oxazol-2-yl)phenyl)ethynyl)p henyl)(morpholino)methanone | 482.9 [M+1] |
| 88 | 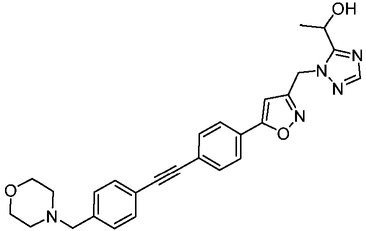 | 1-(1-((5-(4-((4-(morpholinom ethyl)phenyl)ethynyl)phenyl) isoxazol-3-yl)methyl)-1H-1,2,4 -triazol-5-yl)ethan-1-ol | 470.2 [M+1] |
| 89 | 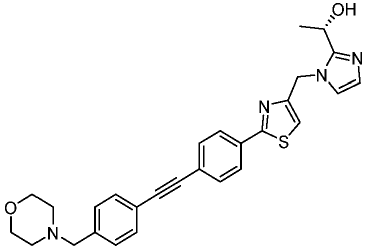 | (S)-1-(1-((2-(4-((4-(morpholin omethyl)phenyl)ethynyl)phen yl)thiazol-4-yl)methyl)-1H-im idazol-2-yl)ethan-1-ol | 484.9 [M+1] |
| 90 | 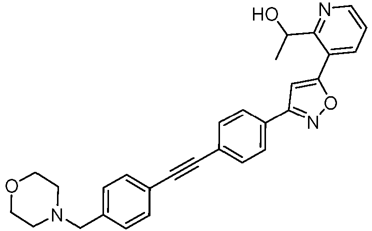 | 1-(3-(3-(4-((4-(morpholinomet hyl)phenyl)ethynyl)phenyl)iso xazol-5-yl)pyridin-2-yl)ethan-1-ol | 467.0 [M+1] |
| 91 | 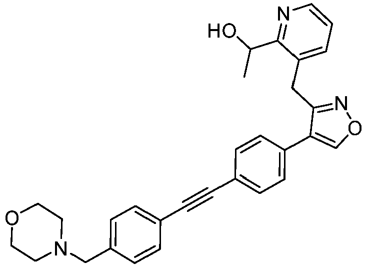 | 1-(3-((4-(4-((4-(morpholinom ethyl)phenyl)ethynyl)phenyl)i soxazol-3-yl)methyl)pyridin-2 -yl)ethan-1-ol | 480.3 [M+1] |
| 92 | 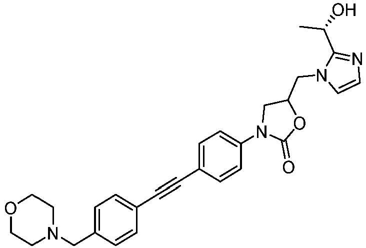 | 5-((2-((S)-1-hydroxyethyl)-1H -imidazol-1-yl)methyl)-3-(4-(( 4-(morpholinomethyl)phenyl) ethynyl)phenyl)oxazolidin-2-one | 487.1 [M+1] |
| 93 | 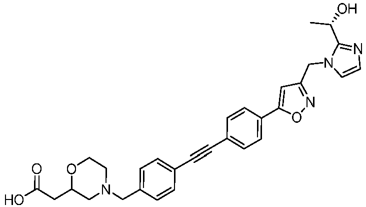 | 2-(4-(4-((4-(3-((2-((S)-1-hydr oxyethyl)-1H-imidazol-1-yl)m ethyl)isoxazol-5-yl)phenyl)eth ynyl)benzyl)morpholin-2-yl) acetic acid | 527.3 [M+1] |
| 94 | 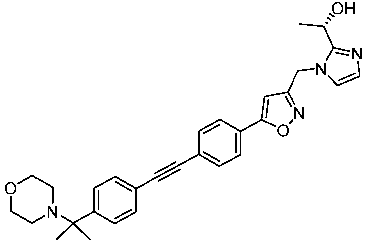 | (S)-1-(1-((5-(4-((4-(2-morphol inopropan-2-yl)phenyl)ethynyl )phenyl)isoxazol-3-yl)methyl )-1H-imidazol-2-yl)ethan-1-ol | 497.2 [M+1] |
| 95 | 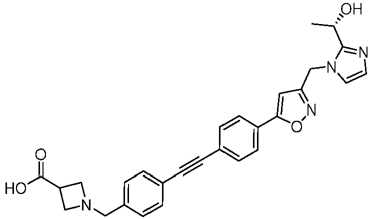 | (S)-1-(4-((4-(3-((2-(1-hydroxy ethyl)-1H-imidazol-1-yl)meth yl)isoxazol-5-yl)phenyl)ethyn yl)benzyl)azetidin-3-carboxylic acid | 483.2 [M+1] |
| 96 | 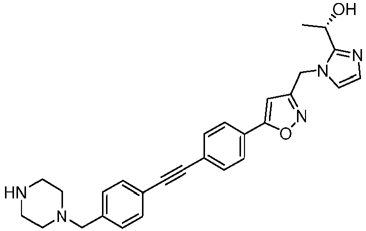 | (S)-1-(1-((5-(4-((4-(piperazin-1-ylmethyl)phenyl)ethynyl)ph enyl)isoxazol-3-yl)methyl)-1H -imidazol-2-yl)ethan-1-ol | 468.2 [M+1] |
| 97 | 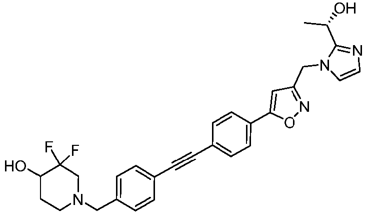 | 3,3-difluoro-1-(4-((4-(3-((2-(( S)-1-hydroxyethyl)-1H-imidaz ol-1-yl)methyl)isoxazol-5-yl) phenyl)ethynyl)benzyl)piperidin -4-ol | 519.2 [M+1] |
| 98 | 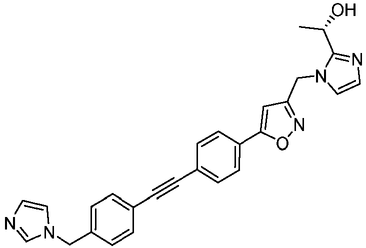 | (S)-1-(1-((5-(4-((4-((1H-imida zol-1-yl)methyl)phenyl)ethyn yl)phenyl)isoxazol-3-yl)methy l)-1H-imidazol-2-yl)ethan-1-ol | 450.0 [M+1] |
| 99 | 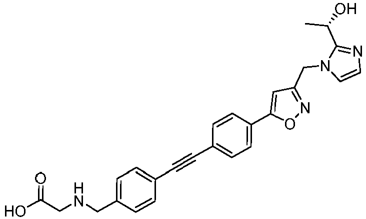 | (S)-(4-((4-(3-((2-(1-hydroxyet hyl)-1H-imidazol-1-yl)methyl )isoxazol-5-yl)phenyl)ethynyl) benzyl)glycinate | 457.2 [M+1] |
| 100 | 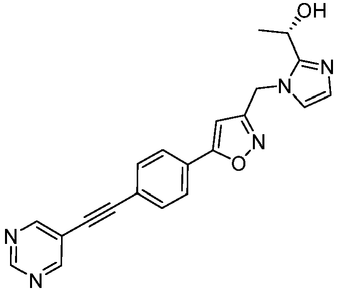 | (S)-1-(1-((5-(4-(pyrimidin-5-y lethynyl)phenyl)isoxazol-3-yl) methyl)-1H-imidazol-2-yl)eth an-1-ol | 372.0 [M+1] |
| 101 | 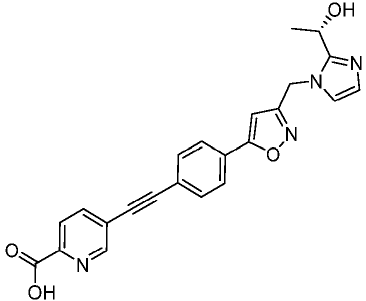 | (S)-5-((4-(3-((2-(1-hydroxyeth yl)-1H-imidazol-1-yl)methyl) isoxazol-5-yl)phenyl)ethynyl) picolinic acid | 415.1 [M+1] |
| 102 | 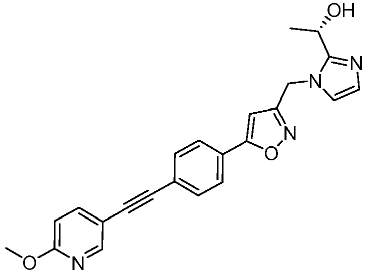 | (S)-1-(1-((5-(4-((6-methoxypy ridin-3-yl)ethynyl)phenyl) isoxazol-3-yl)methyl)-1H-imidazol -2-yl)ethan-1-ol | 401.0 [M+1] |
| 103 | 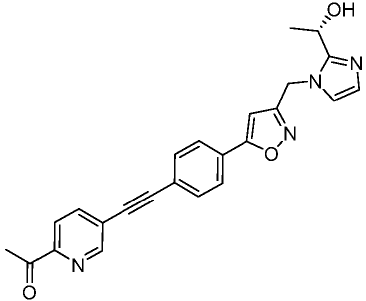 | (S)-1-(5-((4-(3-((2-(1-hydroxy ethyl)-1H-imidazol-1-yl)meth yl)isoxazol-5-yl)phenyl)ethyn yl)pyridin-2-yl)ethan-1-one | 413.1 [M+1] |
| 104 | 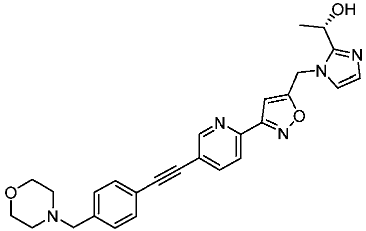 | (S)-1-(1-((3-(5-((4-(morpholin omethyl)phenyl)ethynyl)pyrid in-2-yl)isoxazol-5-yl)methyl)-1H-imidazol-2-yl)ethan-1-ol | 470.2 [M+1] |
| 105 | 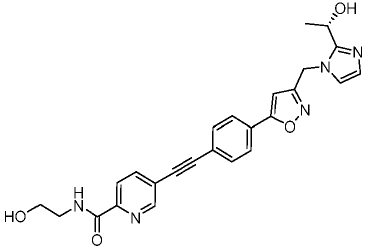 | (S)-N-(2-hydroxyethyl)-5-((4-(3-((2-(1-hydroxyethyl)-1H-imidazol-1-yl)methyl)isoxazol-5-yl)phenyl)ethynyl)picolinamide | 458.0 [M+1] |
| 106 | 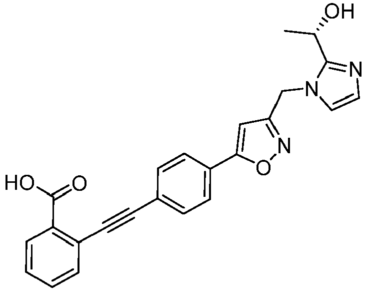 | (S)-2-((4-(3-((2-(1-hydroxyeth yl)-1H-imidazol-1-yl)methyl) isoxazol-5-yl)phenyl)ethynyl) benzoic acid | 414.0 [M+1] |
| 107 | 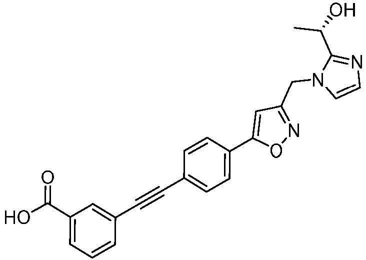 | (S)-3-((4-(3-((2-(1-hydroxyeth yl)-1H-imidazol-1-yl)methyl) isoxazol-5-yl)phenyl)ethynyl) benzoic acid | 414.0 [M+1] |
| 108 | 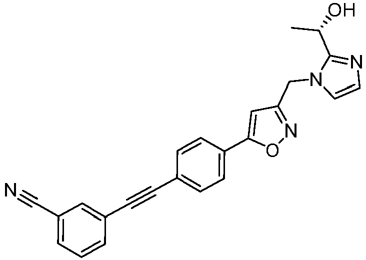 | (S)-3-((4-(3-((2-(1-hydroxyeth yl)-1H-imidazol-1-yl)methyl) isoxazol-5-yl)phenyl)ethynyl) benzonitrile | 395.0 [M+1] |
| 109 | 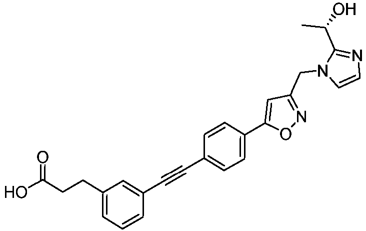 | (S)-3-(3-((4-(3-((2-(1-hydroxy ethyl)-1H-imidazol-1-yl)meth yl)isoxazol-5-yl)phenyl)ethyn yl)phenyl)propanoic acid | 442.0 [M+1] |
| 110 | 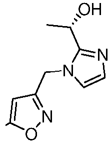 | (S)-2-(3-((4-(3-((2-(1-hydroxy ethyl)-1H-imidazol-1-yl)meth yl)isoxazol-5-yl)phenyl)ethyn yl)phenoxy)acetic acid | 443.9 [M+1] |

### Example 111

### Ethyl (S)-2-(1-(4-((4-(3-((2-(1-hydroxyethyl)-1H-imidazol-1-yl)methyl)isoxazol-5-yl)phenyl)ethynyl)b enzyl)azetidin-3-yl)acetate

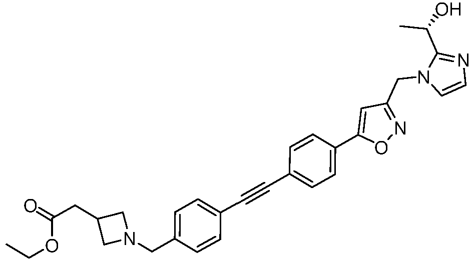

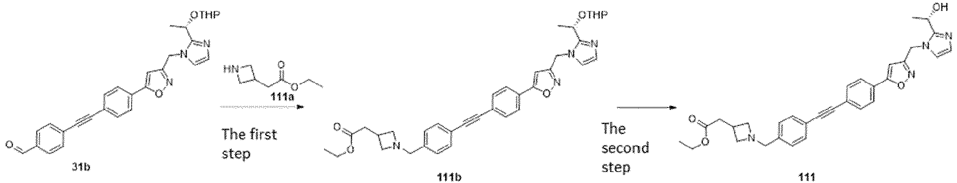

### The first step

### Ethyl 2-(1-(4-((4-(3-((2-((1S)-1-((tetrahydro-2H-pyran-2-yl)oxy)ethyl)-1H-imidazol-1-yl)methyl)isoxa zol-5-yl)phenyl)ethynyl)benzyl)azetidin-3-yl)acetate

Ethyl 4-((4-(3-((2-((1S)-1-((tetrahydro-2H-pyran-2-yl)oxy)ethyl)-1H-imidazol-1-yl)methyl)isoxazol-5-yl)phenyl)ethynyl)benzaldehyde **31b** (100 mg, 207.67 µmol) and ethyl 2-(azetidin-3-yl)acetate **111a** (133.53 mg, 519.17 µmol, commercially available) were dissolved in dichloromethane (2 mL), then added with acetic acid (12.47 mg, 207.67 µmol, 11.88 µL), stirred for 30 minutes, added with sodium acetate borohydride (70.42 mg, 332.27 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 ml×2), dried over anhydrous sodium sulfate, filtered, and concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain ethyl 2-(1-(4-((4-(3-((2-((1S)-1-((tetrahydro-2H-pyran-2-yl)oxy)ethyl)-1H-imidazol-1-yl)methyl)isoxa zol-5-yl)phenyl)ethynyl)benzyl)azetidin-3-yl)acetate **111b** (60 mg) with a yield of 47.46%.
MS m/z (ESI): 609.4 [M+1]

### The second step

### Ethyl (S)-2-(1-(4-((4-(3-((2-(1-hydroxyethyl)-1H-imidazol-1-yl)methyl)isoxazol-5-yl)phenyl)ethynyl)b enzyl)azetidin-3-yl)acetate

Ethyl 2-(1-(4-((4-(3-((2-((1S)-1-((tetrahydro-2H-pyran-2-yl)oxy)ethyl)-1H-imidazol-1-yl)methyl)isoxa zol-5-yl)phenyl)ethynyl)benzyl)azetidin-3-yl)acetate **111b** (120 mg, 197.13 µmol) and trifluoroacetic acid (0.2 mL) were sequentially added to dichloromethane (6 mL), and stirred continuously at room temperature for 12 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain ethyl (S)-2-(1-(4-((4-(3-((2-(1-hydroxyethyl)-1H-imidazol-1-yl)methyl)isoxazol-5-yl)phenyl)ethynyl)b enzyl)azetidin-3-yl)acetate 111 (90 mg) with a yield of 78.44%.
MS m/z (ESI): 525.3 [M+1]

### Example 112

### (S)-1-(1-((5-(4-((6-(((2-aminoethyl)amino)methyl)pyridin-3-yl)ethynyl)phenyl)isoxazol -3-yl)methyl)-1H-imidazol-2-yl)ethan-1-ol

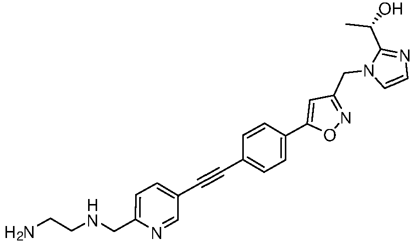

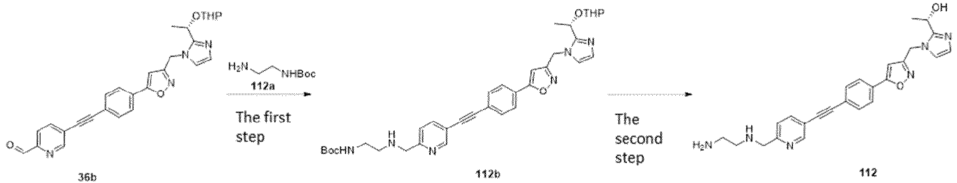

### The first step

### Tert-butyl (2-(((5-((4-(3-((2-((1S)-1-((tetrahydro-2H-pyran-2-yl)oxy)ethyl)-1H-imidazol-1-yl)methyl)isoxaz ol-5-yl)phenyl)ethynyl)pyridin-2-yl)methyl)amino)ethyl)carbamate

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde 36b (50 mg, 103.62 µmol) and tert-butyl (2-aminoethyl)carbamate **112a** (24.90 mg, 155.43 µmol, commercially available) were dissolved in dichloromethane (2 mL), then added with acetic acid (0.5 mL), stirred for 30 minutes, added with sodium acetate borohydride (35.14 mg, 165.79 µmol), and stirred continuously at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain tert-butyl (2-(((5-((4-(3-((2-((1S)-1-((tetrahydro-2H-pyran-2-yl)oxy)ethyl)-1H-imidazol-1-yl)methyl)isoxaz ol-5-yl)phenyl)ethynyl)pyridin-2-yl)methyl)amino)ethyl)carbamate **112b** (50 mg) with a yield of 76.99%, which was directly used in the next reaction.
MS m/z (ESI): 627.4 [M+1]

### The second step

### (S)-1-(1-((5-(4-((6-(((2-aminoethyl)amino)methyl)pyridin-3-yl)ethynyl)phenyl)isoxazol -3-yl)methyl)-1H-imidazol-2-yl)ethan-1-ol

Tert-butyl (2-(((5-((4-(3-((2-((1S)-1-((tetrahydro-2H-pyran-2-yl)oxy)ethyl)-1H-imidazol-1-yl)methyl)isoxaz ol-5-yl)phenyl)ethynyl)pyridin-2-yl)methyl)amino)ethyl)carbamate **112b** (50 mg, 79.78 µmol) and trifluoroacetic acid (0.2 mL) were sequentially added to dichloromethane (2 mL), and stirred continuously at room temperature for 12 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((6-(((2-aminoethyl)amino)methyl)pyridin-3-yl)ethynyl)phenyl)isoxazol-3-yl)met hyl)-1H-imidazol-2-yl)ethan-1-ol 112 (3.30 mg) with a yield of 6.81%.
MS m/z (ESI): 443.2 [M+1]

### Example 113

### (S)-1-(1-((5-(4-((6-((3-(hydroxymethyl)azetidin-1-yl)methyl)pyridin-3-yl)ethynyl)phen yl)isoxazol-3-yl)methyl)-1H-imidazol-2-yl)ethan-1-ol

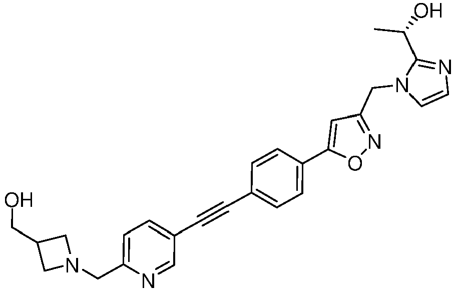

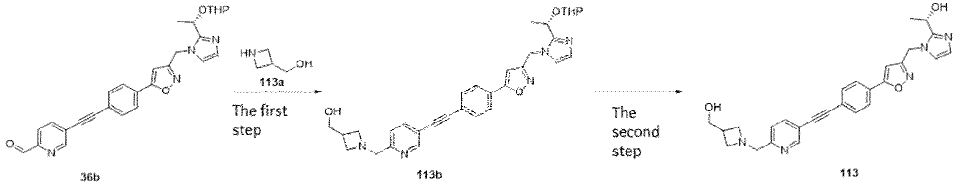

### The first step

### (1-((5-((4-(3-((2-((1S)-1-((tetrahydro-2H-pyran-2-yl)oxy)ethyl)-1H-imidazol-1-yl)meth yl)isoxazol-5-yl)phenyl)ethynyl)pyridin-2-yl)methyl)azetidin-3-yl)methanol

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (100 mg, 207.24 µmol), and azetidin-3-ylmethanol **113a** (51.22 mg, 414.48 µmol, commercially available) were dissolved in dichloromethane (1 mL), stirred for 30 minutes, added with sodium acetate borohydride (65.88 mg, 310.86 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain (1-((5-((4-(3-((2-((1S)-1-((tetrahydro-2H-pyran-2-yl)oxy)ethyl)-1H-imidazol-1-yl)methyl)isoxaz ol-5-yl)phenyl)ethynyl)pyridin-2-yl)methyl)azetidin-3-yl)methanol **113b** (80 mg) with a yield of 69.72%, which was used directly in the next reaction.
MS m/z (ESI): 554.3 [M+1]

### The second step

### (S)-1-(1-((5-(4-((6-((3-(hydroxymethyl)azetidin-1-yl)methyl)pyridin-3-yl)ethynyl)phen yl)isoxazol-3-yl)methyl)-1H-imidazol-2-yl)ethan-1-ol

(1-((5-((4-(3-((2-((1S))-1-((tetrahydro-2H-pyran-2-yl)oxy)ethyl)-1H-imidazol-1-yl)met hyl)isoxazol-5-yl)phenyl)ethynyl)pyridin-2-yl)methyl)azetidin-3-yl)methanol **113b** (80 mg, 144.50 µmol) and trifluoroacetic acid (0.5 mL) were sequentially added to dichloromethane (2 mL), and stirred continuously at room temperature for 12 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-(1-((5-(4-((6-((3-(hydroxymethyl)azetidin-1-yl)methyl)pyridin-3-yl)ethynyl)phenyl)isoxazo l-3-yl)methyl)-1H-imidazol-2-yl)ethan-1-ol **113** (6.50 mg) with a yield of 7.40%.
MS m/z (ESI): 470.2 [M+1]

### Example 114

### (S)-4-((5-((4-(3-((2-(1-hydroxyethyl)-1H-imidazol-1-yl)methyl)isoxazol-5-yl)phenyl)et hynyl)pyridin-2-yl)methyl)piperazin-2-one

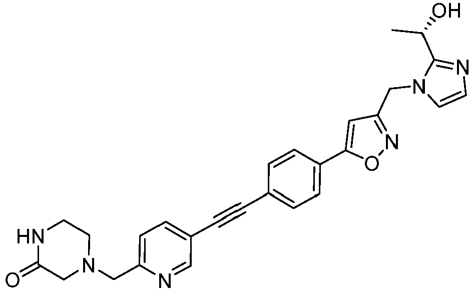

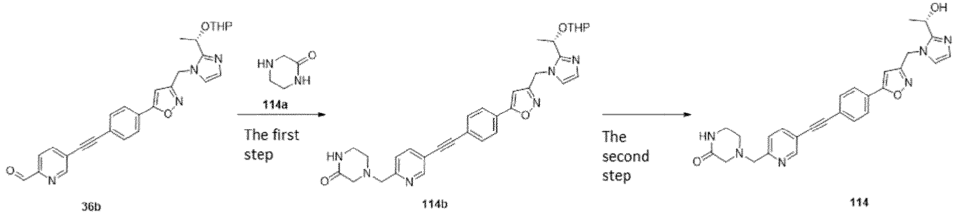

### The first step

### 4-((5-((4-(3-((2-((1S)-1-((tetrahydro-2H-pyran-2-yl)oxy)ethyl)-1H-imidazol-1-yl)methy l)isoxazol-5-yl)phenyl)ethynyl)pyridin-2-yl)methyl)piperazin-2-one

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) and piperazin-2-one **114a** (15.56 mg, 155.43 µmol, commercially available) were dissolved in dichloromethane (1 mL), added with acetic acid (6.22 mg, 103.62 µmol), stirred for 30 minutes, added with sodium acetate borohydride (32.94 mg, 155.43 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain 4-((5-((4-(3-((2-((1S)-1-((tetrahydro-2H-pyran-2-yl)oxy)ethyl)-1H-imidazol-1-yl)methyl)isoxazo l-5-yl)phenyl)ethynyl)pyridin-2-yl)methyl)piperazin-2-one **114b** (40 mg) with a yield of 68.12%, which was directly used in the next reaction.
MS m/z (ESI): 567.3 [M+1]

### The second step

### (S)-4-((5-((4-(3-((2-(1-hydroxyethyl)-1H-imidazol-1-yl)methyl)isoxazol-5-yl)phenyl)et hynyl)pyridin-2-yl)methyl)piperazin-2-one

4-((5-((4-(3-((2-((1S)-1-((tetrahydro-2H-pyran-2-yl)oxy)ethyl)-1H-imidazol-1-yl)methy l)isoxazol-5-yl)phenyl)ethynyl)pyridin-2-yl)methyl)piperazin-2-one **114b** (50 mg, 88.24 µmol) and trifluoroacetic acid (0.5 mL) were sequentially added to dichloromethane (1 mL), and stirred continuously at room temperature for 12 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-4-((5-((4-(3-((2-(1-hydroxyethyl)-1H-imidazol-1-yl)methyl)isoxazol-5-yl)phenyl)ethynyl)pyr idin-2-yl)methyl)piperazin-2-one **114** (30 mg) with a yield of 55.34%.
MS m/z (ESI): 483.2 [M+1]

### Example 115

### (S)-2-(1-((5-((4-(3-((2-(1-hydroxyethyl)-1H-imidazol-1-yl)methyl)isoxazol-5-yl)phenyl )ethynyl)pyridin-2-yl)methyl)azetidin-3-yl)acetic acid

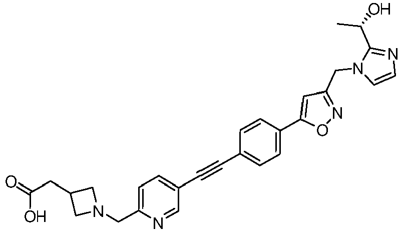

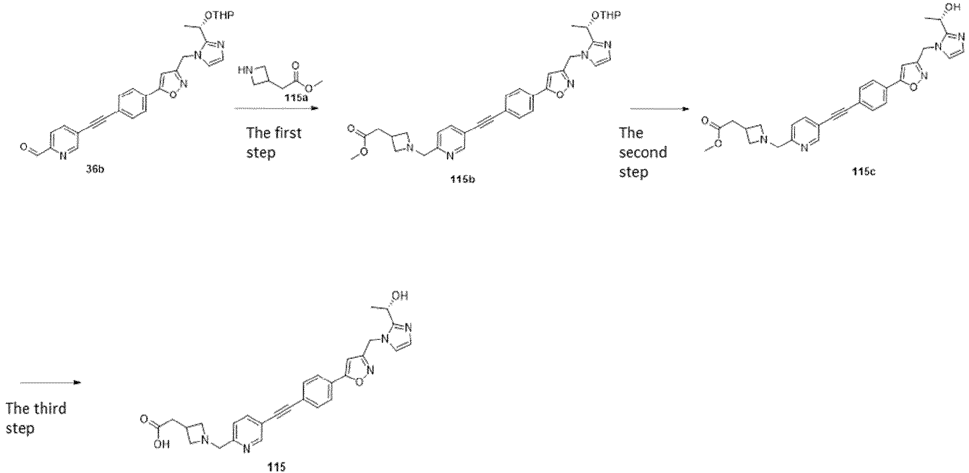

### The first step

### Methyl 2-(1-((5-((4-(3-((2-((1S)-1-((tetrahydro-2H-pyran-2-yl)oxy)ethyl)-1H-imidazol-1-yl)methyl)isoxa zol-5-yl)phenyl)ethynyl)pyridin-2-yl)methyl)azetidin-3-yl)acetate

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) and methyl 2-(azetidin-3-yl)acetate **115a** (63.00 mg, 259.05 µmol, commercially available) were dissolved in dichloromethane (1 mL), then added with acetic acid (6.22 mg, 103.62 µmol), stirred for 30 minutes, added with sodium acetate borohydride (32.94 mg, 155.43 µmol), and stirred continuously at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain methyl 2-(1-((5-((4-(3-((2-((1S)-1-((tetrahydro-2H-pyran-2-yl)oxy)ethyl)-1H-imidazol-1-yl)methyl)isoxa zol-5-yl)phenyl)ethynyl)pyridin-2-yl)methyl)azetidin-3-yl)acetate **115b** (50 mg) with a yield of 81.00%, which was directly used in the next reaction.
MS m/z (ESI): 627.4 [M+1]

### The second step

### Methyl (S)-2-(1-((5-((4-(3-((2-(1-hydroxyethyl))-1H-imidazol-1-yl)methyl)isoxazol-5-yl)phenyl)ethynyl) pyridin-2-yl)methyl)azetidin-3-yl)acetate

Methyl 2-(1-((5-((4-(3-((2-((1S)-1-((tetrahydro-2H-pyran-2-yl)oxy)ethyl)-1H-imidazol-1-yl)methyl)isoxa zol-5-yl)phenyl)ethynyl)pyridin-2-yl)methyl)azetidin-3-yl)acetate **115b** (50 mg, 83.94 µmol) and trifluoroacetic acid (0.5 mL) were sequentially added to dichloromethane (1 mL), and stirred continuously at room temperature for 12 hours. The reaction solution was concentrated under reduced pressure to obtain methyl (S)-2-(1-((5-((4-(3-((2-(1-hydroxyethyl)))-1H-imidazol-1-yl)methyl)isoxazol-5-yl)phenyl)ethynyl pyridin-2-yl)methyl)azetidin-3-yl)acetate **115c** (40 mg) with a yield of 93.15%, which was used directly in the next reaction.
MS m/z (ESI): 512.0 [M+1]

### The third step

### (S)-2-(1-((5-((4-(3-((2-(1-hydroxyethyl)-1H-imidazol-1-yl)methyl)isoxazol-5-yl)phenyl )ethynyl)pyridin-2-yl)methyl)azetidin-3-yl)acetic acid

Methyl (S)-2-(1-((5-((4-(3-((2-(1-hydroxyethyl))-1H-imidazol-1-yl)methyl)isoxazol-5-yl)phenyl)ethynyl) pyridin-2-yl)methyl)azetidin-3-yl)acetate **115c** (40 mg, 78.19 µmol) and 2.5 N sodium hydroxide aqueous solution (1 mL) were sequentially added to methanol (3 mL), and continuously stirred at room temperature for 12 hours. The system was adjusted to pH 3 with 2 N dilute hydrochloric acid, and concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA +H₂O, mobile phase B: CH₃CN) to obtain (S)-2-(1-((5-((4-(3-((2-(1-hydroxyethyl)-1H-imidazol-1-yl)methyl)isoxazol-5-yl)phenyl)ethynyl) pyridin-2-yl)methyl)azetidin-3-yl)acetic acid **115** (10 mg) with a yield of 17.98%.
MS m/z (ESI): 498.2 [M+1]

### Example 116

### (S)-((5-((4-(3-((2-(1-hydroxyethyl))-1H-imidazol-1-yl)methyl)isoxazol-5-yl)phenyl)eth ynyl)pyridin-2-yl)methyl)glycinate

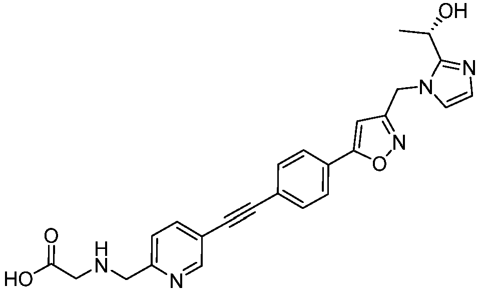

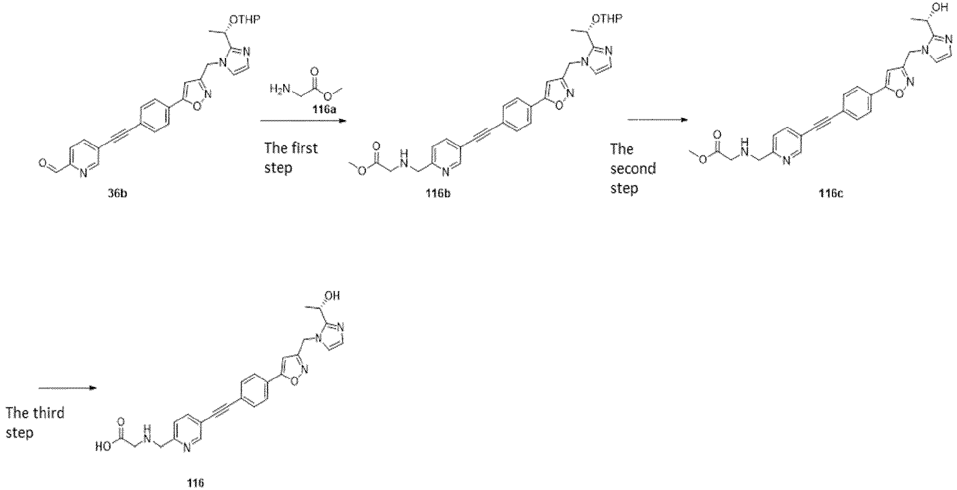

### The first step

### Methyl ((5-((4-(3-((2-((1S)-1-((tetrahydro-2H-pyran-2-yl)oxy)ethyl)-1H-imidazol-1-yl)methyl)isoxazol-5-yl)phenyl)ethynyl)pyridin-2-yl)methyl)glycinate

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) and methyl glycinate **116a** (15.61 mg, 124.34 µmol) were dissolved in dichloromethane (1 mL), then added with acetic acid (6.22 mg, 103.62 µmol), stirred for 30 minutes, added with sodium acetate borohydride (24.16 mg, 113.98 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain methyl ((5-((4-(3-((2-((1S)-1-((tetrahydro-2H-pyran-2-yl)oxy)ethyl)-1H-imidazol- 1 -yl)methyl)isoxazol-5-yl)phenyl)ethynyl)pyridin-2-yl)methyl)glycinate **116b** (50 mg) with a yield of 86.84%, which was directly used in the next reaction.
MS m/z (ESI): 556.3 [M+1]

### The second step

### Methyl (S)-((5-((4-(3-((2-(1-hydroxyethyl)-1H-imidazol-1-yl)methyl)isoxazol-5-yl)phenyl)ethynyl)pyrid in-2-yl)methyl)glycinate

Methyl ((5-((4-(3-((2-((1S)-1-((tetrahydro-2H-pyran-2-yl)oxy)ethyl)-1H-imidazol-1-yl)methyl)isoxazol-5-yl)phenyl)ethynyl)pyridin-2-yl)methyl)glycinate **116b** (50 mg, 89.99 µmol) and trifluoroacetic acid (0.5 mL) were sequentially added to dichloromethane (1.5 mL), and stirred continuously at room temperature for 12 hours. The reaction solution was concentrated under reduced pressure to obtain methyl (S)-((5-((4-(3-((2-(1-hydroxyethyl)-1H-imidazol-1-yl)methyl)isoxazol-5-yl)phenyl)ethynyl)pyrid in-2-yl)methyl)glycinate **116c** (40 mg) with a yield of 94.27%, which was directly used in the next reaction.
MS m/z (ESI): 472.2 [M+1]

### The third step

### (S)-((5-((4-(3-((2-(1-hydroxyethyl))-1H-imidazol-1-yl)methyl)isoxazol-5-yl)phenyl)eth ynyl)pyridin-2-yl)methyl)glycinate

Methyl (S)-((5-((4-(3-((2-(1-hydroxyethyl)-1H-imidazol-1-yl)methyl)isoxazol-5-yl)phenyl)ethynyl)pyrid in-2-yl)methyl)glycinate **116c** (40 mg, 84.83 µmol) and 2.5 N sodium hydroxide aqueous solution (0.5 mL) were sequentially added to methanol (1 mL), and stirred continuously at room temperature for 12 hours. The system was adjusted to pH 3 with 2 N dilute hydrochloric acid, and concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA +H₂O, mobile phase B: CH₃CN) to obtain (S)-((5-((4-(3-((2-(1-hydroxyethyl))-1H-imidazol-1-yl)methyl)isoxazol-5-yl)phenyl)ethynyl)pyri din-2-yl)methyl)glycinate 116 (15 mg) with a yield of 28.46%.
MS m/z (ESI): 458.1 [M+1]

### Example 117

### (S)-1-((5-((4-(3-((2-(1-hydroxyethyl)-1H-imidazol-1-yl)methyl)isoxazol-5-yl)phenyl)et hynyl)pyridin-2-yl)methyl)azetidin-3-carboxylic acid

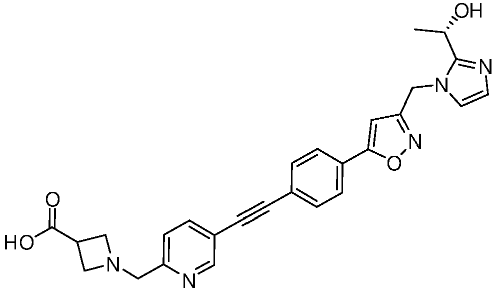

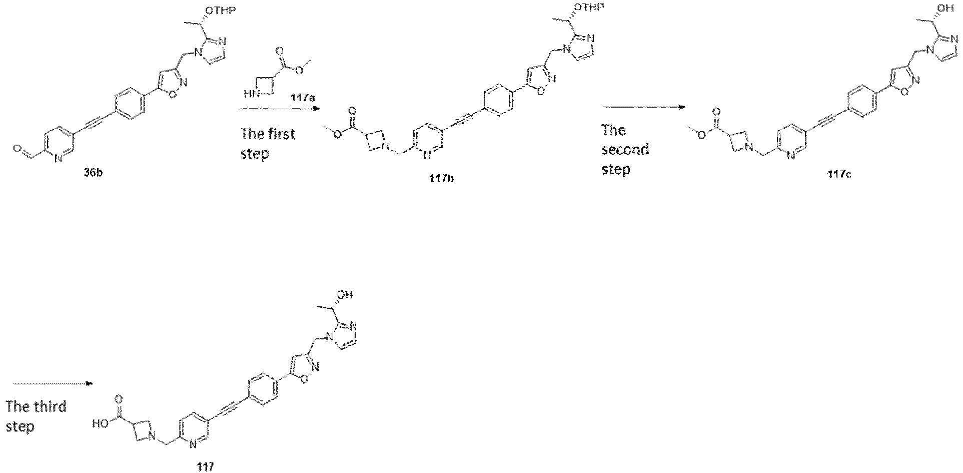

### The first step

### Methyl 1-((5-((4-(3-((2-((1S)-1-((tetrahydro-2H-pyran-2-yl)oxy)ethyl)-1H-imidazol-1-yl)methyl)isoxazo l-5-yl)phenyl)ethynyl)pyridin-2-yl)methyl)azetidin-3-carboxylate

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (100 mg, 207.24 µmol) and methyl azetidin-3-carboxylate **117a** (37.70 mg, 248.69 µmol, commercially available) were dissolved in dichloromethane (1.5 mL), added with acetic acid (12.44 mg, 207.24 µmol), stirred for 30 minutes, added with sodium acetate borohydride (43.92 mg, 207.24 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain methyl 1-((5-((4-(3-((2-((1S)-1-((tetrahydro-2H-pyran-2-yl)oxy)ethyl)-1H-imidazol-1-yl)methyl)isoxazo l-5-yl)phenyl)ethynyl)pyridin-2-yl)methyl)azetidin-3-carboxylate **117b** (100 mg) with a yield of 82.96%, which was directly used in the next reaction.
MS m/z (ESI): 582.3 [M+1]

### The second step

### Methyl (S)-1-((5-((4-(3-((2-(1-hydroxyethyl)-1H-imidazol-1-yl)methyl)isoxazol-5-yl)phenyl)ethynyl)pyr idin-2-yl)methyl)azetidin-3-carboxylate

Methyl 1-((5-((4-(3-((2-((1S)-1-((tetrahydro-2H-pyran-2-yl)oxy)ethyl)-1H-imidazol-1-yl)methyl)isoxazo l-5-yl)phenyl)ethynyl)pyridin-2-yl)methyl)azetidin-3-carboxylate **117b** (100 mg, 171.92 µmol) and trifluoroacetic acid (0.5 mL) were sequentially added to dichloromethane (2 mL), and stirred at room temperature for 12 hours. The reaction solution was concentrated under reduced pressure to obtain methyl (S)-1-((5-((4-(3-((2-(1-hydroxyethyl)-1H-imidazol-1-yl)methyl)isoxazol-5-yl)phenyl)ethynyl)pyr idin-2-yl)methyl)azetidin-3-carboxylate **117c** (60 mg) with a yield of 70.14%, which was directly used in the next reaction.
MS m/z (ESI): 498.3 [M+1]

### The third step

### (S)-1-((5-((4-(3-((2-(1-hydroxyethyl)-1H-imidazol-1-yl)methyl)isoxazol-5-yl)phenyl)et hynyl)pyridin-2-yl)methyl)azetidin-3-carboxylic acid

Methyl (S)-1-((5-((4-(3-((2-(1-hydroxyethyl)-1H-imidazol-1-yl)methyl)isoxazol-5-yl)phenyl)ethynyl)pyr idin-2-yl)methyl)azetidin-3-carboxylate **117c** (60 mg, 120.59 µmol) and 2.5 N sodium hydroxide aqueous solution (1 mL) were sequentially added to methanol (2 mL), and continuously stirred at room temperature for 12 hours. The system was adjusted to pH 3 with 2 N dilute hydrochloric acid, and concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-((5-((4-(3-((2-(1-hydroxyethyl)-1H-imidazol-1-yl)methyl)isoxazol-5-yl)phenyl)ethynyl)pyr idin-2-yl)methyl)azetidin-3-carboxylic acid **117** (20 mg) with a yield of 24.98%.
MS m/z (ESI): 484.0 [M+1]

### Example 118

### (S)-2-(1-((5-((4-(3-((2-(1-hydroxyethyl)-1H-imidazol-1-yl)methyl)isoxazol-5-yl)phenyl )ethynyl)pyridin-2-yl)methyl)azetidin-3-yl)acetonitrile

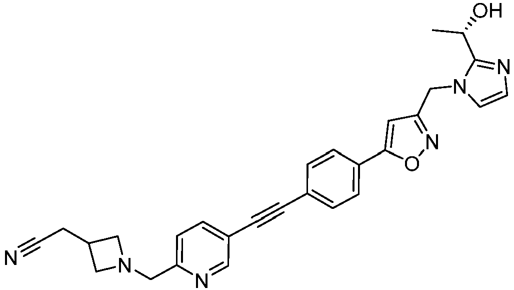

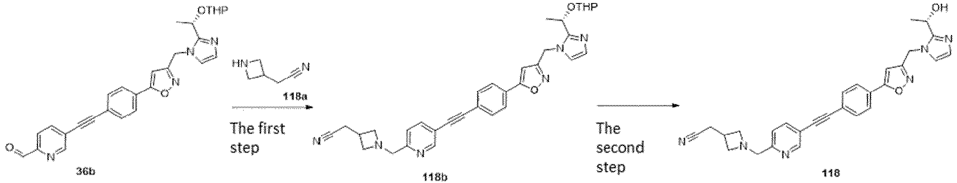

### The first step

### 2-(1-((5-((4-(3-((2-((1S))-1-((tetrahydro-2H-pyran-2-yl)oxy)ethyl)-1H-imidazol-1-yl)m ethyl)isoxazol-5-yl)phenyl)ethynyl)pyridin-2-yl)methyl)azetidin-3-yl)acetonitrile

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) and 2-(azetidin-3-yl)acetonitrile **118a** (9.96 mg, 103.62 µmol, commercially available) were dissolved in dichloromethane (1 mL), added with acetic acid (6.22 mg, 103.62 µmol), stirred for 30 minutes, added with sodium acetate borohydride (24.16 mg, 113.98 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain 2-(1-((5-((4-(3-((2-((1S)-1-((tetrahydro-2H-pyran-2-yl)oxy)ethyl)-1H-imidazol-1-yl)methyl)isoxa zol-5-yl)phenyl)ethynyl)pyridin-2-yl)methyl)azetidin-3-yl)acetonitrile **118b** (40 mg) with a yield of 68.61%, which was directly used in the next reaction.
MS m/z (ESI): 562.8 [M+1]

### The second step

### (S)-2-(1-((5-((4-(3-((2-(1-hydroxyethyl)-1H-imidazol-1-yl)methyl)isoxazol-5-yl)phenyl )ethynyl)pyridin-2-yl)methyl)azetidin-3-yl)acetonitrile

2-(1-((5-((4-(3-((2-((1S)-1-((tetrahydro-2H-pyran-2-yl)oxy)ethyl)-1H-imidazol-1-yl)me thyl)isoxazol-5-yl)phenyl)ethynyl)pyridin-2-yl)methyl)azetidin-3-yl)acetonitrile **118b** (40 mg, 71.09 µmol) and trifluoroacetic acid (0.5 mL) were sequentially added to dichloromethane (2 mL), and stirred at room temperature for 12 hours. The reaction solution was concentrated under reduced pressure. The obtained residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-2-(1-((5-((4-(3-((2-(1-hydroxyethyl)-1H-imidazol-1-yl)methyl)isoxazol-5-yl)phenyl)ethynyl) pyridin-2-yl)methyl)azetidin-3-yl)acetonitrile **118** (8.4 mg) with a yield of 19.40%.
MS m/z (ESI): 479.2 [M+1]

### Example 119

### (S)-1-((5-((4-(3-((2-(1-hydroxyethyl)-1H-imidazol-1-yl)methyl)isoxazol-5-yl)phenyl)et hynyl)pyridin-2-yl)methyl)azetidin-3-carbonitrile

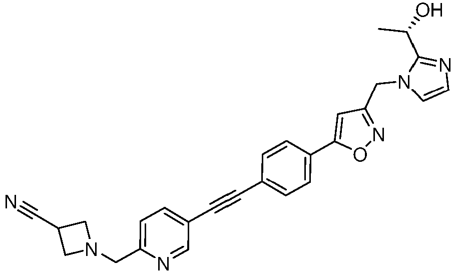

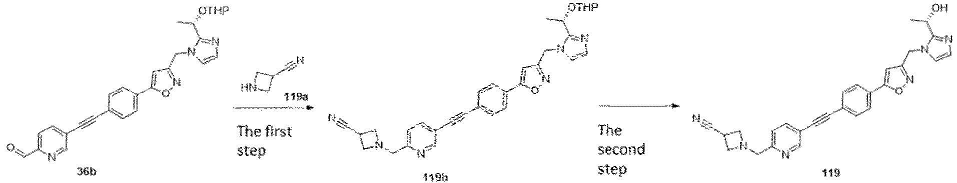

### The first step

### 1-((5-((4-(3-((2-((1S))-1-((tetrahydro-2H-pyran-2-yl)oxy)ethyl)-1H-imidazol-1-yl)meth yl)isoxazol-5-yl)phenyl)ethynyl)pyridin-2-yl)methyl)azetidin-3-carbonitrile

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) and azetidin-3-carbonitrile **119a** (8.51 mg, 103.62 µmol, commercially available) were dissolved to dichloromethane (1 mL), added with acetic acid (6.22 mg, 103.62 µmol), stirred for 30 minutes, added with sodium acetate borohydride (24.16 mg, 113.98 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain 1-((5-((4-(3-((2-((1S)-1-((tetrahydro-2H-pyran-2-yl)oxy)ethyl)-1H-imidazol-1-yl)methyl)isoxazo l-5-yl)phenyl)ethynyl)pyridin-2-yl)methyl)azetidin-3-carbonitrile **119b** (40 mg) with a yield of 70.36%, which was directly used in the next reaction.
MS m/z (ESI): 549.1 [M+1]

### The second step

### (S)-1-((5-((4-(3-((2-(1-hydroxyethyl)-1H-imidazol-1-yl)methyl)isoxazol-5-yl)phenyl)et hynyl)pyridin-2-yl)methyl)azetidin-3-carbonitrile

1-((5-((4-(3-((2-((1S)-1-((tetrahydro-2H-pyran-2-yl)oxy)ethyl)-1H-imidazol-1-yl)methy l)isoxazol-5-yl)phenyl)ethynyl)pyridin-2-yl)methyl)azetidin-3-carbonitrile **119b** (40 mg, 72.91 µmol) and trifluoroacetic acid (0.5 mL) were sequentially added to dichloromethane (2 mL), and stirred at room temperature for 12 hours. The reaction solution was concentrated under reduced pressure. The obtained residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-((5-((4-(3-((2-(1-hydroxyethyl)-1H-imidazol-1-yl)methyl)isoxazol-5-yl)phenyl)ethynyl)pyr idin-2-yl)methyl)azetidin-3-carbonitrile **119** (20 mg) with a yield of 45.90%.
MS m/z (ESI): 464.9 [M+1]

### Example 120

### (S)-5-((4-(3-((2-(1-hydroxyethyl)-1H-imidazol-1-yl)methyl)isoxazol-5-yl)phenyl)ethyn yl)picolinonitrile

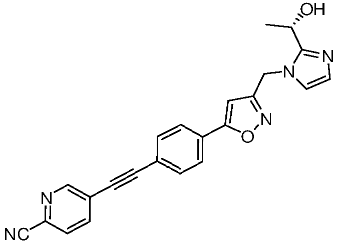

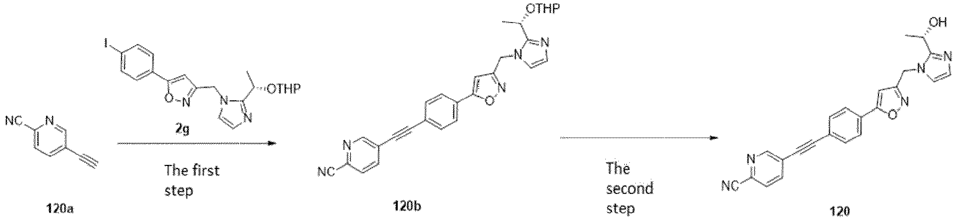

### The first step

### 5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinonitrile

5-(4-Iodophenyl)-3-((2-((1S)-1-((tetrahydro-2H-pyran-2-yl)oxy)ethyl)-1H-imidazol-1-y l)methyl)isoxazole **2g** (187.04 mg, 390.23 µmol), 5-ethynylpicolinonitrile **120a** (100 mg, 780.45 µmol), prepared according to the method in the published literature "European Journal of Chemistry, 9(4), 317-321; 2018"), allylpalladium(II) chloride dimer (56.97 mg, 156.09 µmol), triethylenediamine (262.63 mg, 2.34 mmol) and tri-tert-butylphosphine (10% toluene solution) (31.58 mg, 156.09 µmol) were sequentially added to acetonitrile (7 mL). The system was replaced with argon gas three times, and stirred continuously at room temperature overnight. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over aqueous sodium sulfate, filtered, and concentrated under reduced pressure. The obtained residue was purified by silica gel column chromatography (eluent: System A) to obtain 5-((4-(3-((2-((1S)-1-((tetrahydro-2H-pyran-2-yl)oxy)ethyl)-1H-imidazol-1-yl)methyl)isoxazol-5-yl)phenyl)ethynyl)picolinonitrile **120b** (200 mg) with a yield of 53.44%.

### The second step

### (S)-5-((4-(3-((2-(1-hydroxyethyl)-1H-imidazol-1-yl)methyl)isoxazol-5-yl)phenyl)ethyn yl)picolinonitrile

Trifluoroacetic acid (0.5 mL) was added to a solution of 5-((4-(3-((2-((1S)-1-((tetrahydro-2H-pyran-2-yl)oxy)ethyl)-1H-imidazol-1-yl)methyl)isoxazol-5-yl)phenyl)ethynyl)picolinonitrile **120b** (200 mg, 417.08 µmol) in dichloromethane (5 mL), and stirred at room temperature for 2 hours. The reaction solution was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-5-((4-(3-((2-(1-hydroxyethyl)-1H-imidazol-1-yl)methyl)isoxazol-5-yl)phenyl)ethynyl)picolin onitrile **120** (18.5 mg) with a yield of 8.54%.
MS m/z (ESI): 396.1 [M+1]

¹H NMR (400 MHz, DMSO-*d₆*) *δ* 8.96 (d, *J =* 1.2 Hz, 1H), 8.28 (dd, *J =* 8.0, 2.0 Hz, 1H), 8.15 (d, *J =* 8.0 Hz, 1H), 7.98 (d, *J =* 8.4 Hz, 2H), 7.81 (d, *J =* 8.4 Hz, 2H), 7.76 (s, 1H), 7.67 (s, 1H), 7.22 (s, 1H), 6.37 (s, 1H), 5.72 (s, 2H), 5.24 (q, *J =* 6.6 Hz, 1H), 1.49 (d, *J =* 6.8 Hz, 3H).

### Example 121

### (S)-1-((5-((4-(3-((2-(1-hydroxyethyl)-1H-imidazol-1 -yl)methyl)isoxazol-5-yl)phenyl)et hynyl)pyridin-2-yl)methyl)azetidin-3-ol

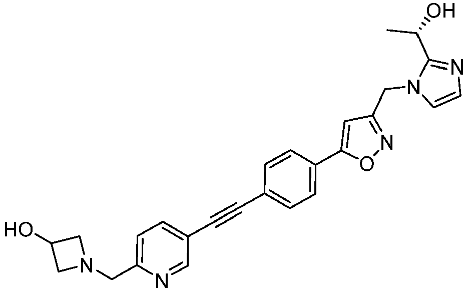

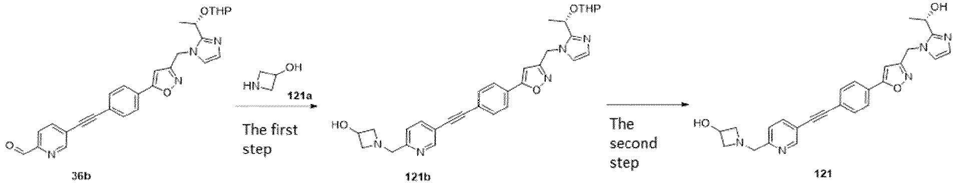

### The first step

### 1-((5-((4-(3-((2-((1S)-1-((tetrahydro-2H-pyran-2-yl)oxy)ethyl)-1H-imidazol-1-yl)methy l)isoxazol-5-yl)phenyl)ethynyl)pyridin-2-yl)methyl)azetidin-3-ol

5-((4-(3-((2-((1S)-1-((tetrahydro-2H-pyran-2-yl)oxy)ethyl)-1H-imidazol-1-yl)methyl)is oxazol-5-yl)phenyl)ethynyl)picolinaldehyde **36b** (50 mg, 103.62 µmol) and azetidin-3-ol **121a** (13.62 mg, 124.34 µmol, commercially available) were dissolved in dichloromethane (1 mL), added with acetic acid (6.22 mg, 103.62 µmol), stirred for 30 minutes, added with sodium acetate borohydride (21.96 mg, 103.62 µmol), and continuously stirred at room temperature for 12 hours. The reaction mixture was added with saturated sodium bicarbonate aqueous solution (15 mL) to quench the reaction, and then added with dichloromethane (30 mL) and water (15 mL) for layer separation. The aqueous phase was extracted with dichloromethane (30 mL×2). The combined organic phase was washed with saturated sodium chloride solution (30 mL×2), dried over anhydrous sodium sulfate, filtered, and concentrated under reduced pressure to obtain 1-((5-((4-(3-((2-((1S)-1-((tetrahydro-2H-pyran-2-yl)oxy)ethyl)-1H-imidazol-1-yl)methyl)isoxazo l-5-yl)phenyl)ethynyl)pyridin-2-yl)methyl)azetidin-3-ol **121b** (25 mg) with a yield of 44.71%, which was used directly in the next reaction.
MS m/z (ESI): 540.3 [M+1]

### The second step

### (S)-1-((5-((4-(3-((2-(1-hydroxyethyl)-1H-imidazol-1-yl)methyl)isoxazol-5-yl)phenyl)et hynyl)pyridin-2-yl)methyl)azetidin-3-ol

1-((5-((4-(3-((2-((1S))-1-((tetrahydro-2H-pyran-2-yl)oxy)ethyl)-1H-imidazol-1-yl)meth yl)isoxazol-5-yl)phenyl)ethynyl)pyridin-2-yl)methyl)azetidin-3-ol **121b** (25 mg, 46.33 µmol) and trifluoroacetic acid (0.3 mL) were sequentially added to dichloromethane (2 mL), and stirred at room temperature for 12 hours. The reaction solution was concentrated under reduced pressure. The obtained residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-((5-((4-(3-((2-(1-hydroxyethyl)-1H-imidazol-1-yl)methyl)isoxazol-5-yl)phenyl)ethynyl)pyr idin-2-yl)methyl)azetidin-3-ol **121** (10 mg) with a yield of 34.11%.
MS m/z (ESI): 456.0 [M+1]

### Example 122

### (S)-2-(1-((5-((4-(3-((2-(1-hydroxyethyl)-1H-imidazol-1-yl)methyl)isoxazol-5-yl)phenyl )ethynyl)pyridin-2-yl)methyl)azetidin-3-yl)acetamide

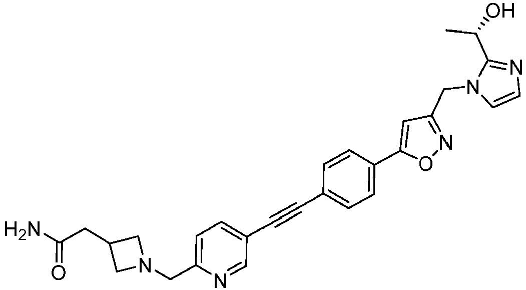

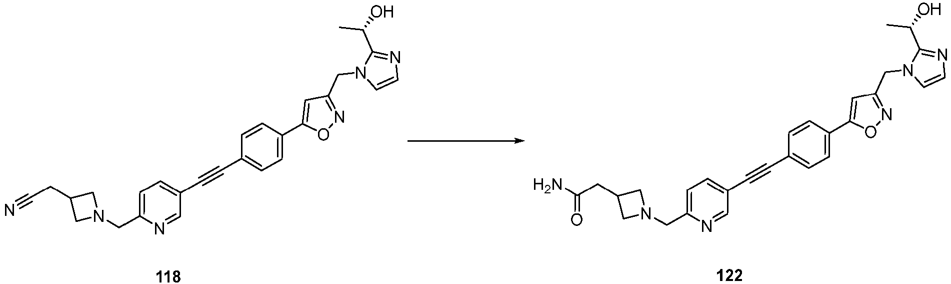

(S)-2-(1-((5-((4-(3-((2-(1-hydroxyethyl)-1H-imidazol-1-yl)methyl)isoxazol-5-yl)phenyl )ethynyl)pyridin-2-yl)methyl)azetidin-3-yl)acetonitrile **118** (20 mg, 41.79 µmol) and 2.5 M sodium hydroxide aqueous solution (0.5 mL) were sequentially added into dimethyl sulfoxide (1 mL), added with hydrogen peroxide (0.3 mL) dropwise in an ice bath, and continuously stirred at room temperature for 0.5 hours. The reaction mixture was added with ethyl acetate (30 mL) and water (15 mL) for layer separation. The aqueous phase was extracted with ethyl acetate (30 mL×2). The combined organic phase was washed with saturated sodium chloride solution (30 mL×2), dried over anhydrous sodium sulfate, filtered, and concentrated under reduced pressure. The residue was purified by preparative liquid chromatography (separation column AKZONOBEL Kromasil; 250×21.2 mm I.D.; 5µm, 20 mL/min; mobile phase A: 0.05% TFA+H₂O, mobile phase B: CH₃CN) to obtain (S)-2-(1-((5-((4-(3-((2-(1-hydroxyethyl)-1H-imidazol-1-yl)methyl)isoxazol-5-yl)phenyl)ethynyl) pyridin-2-yl)methyl)azetidin-3-yl)acetamide **122** (5.0 mg) with a yield of 17.05%.
MS m/z (ESI): 496.9 [M+1]

### Example 123

### (S)-1-((5-((4-(3-((2-(1-hydroxyethyl)-1H-imidazol-1 -yl)methyl)isoxazol-5-yl)phenyl)et hynyl)pyridin-2-yl)methyl)azetidin-3-carboxamide

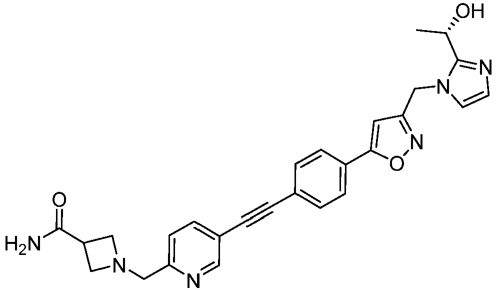

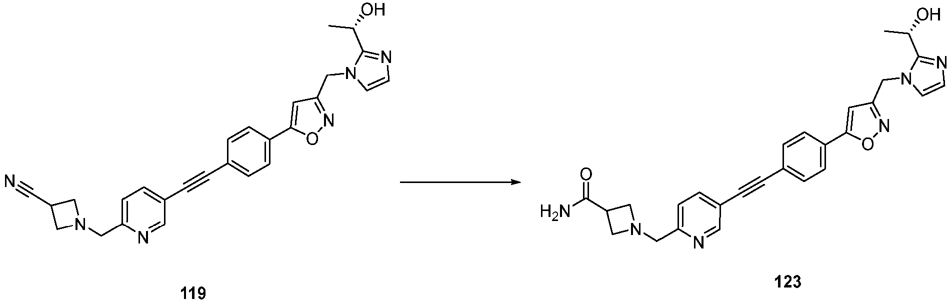

(S)-1-((5-((4-(3-((2-(1-hydroxyethyl)-1H-imidazol-1-yl)methyl)isoxazol-5-yl)phenyl)et hynyl)pyridin-2-yl)methyl)azetidin-3-carbonitrile **119** (20 mg, 43.06 µmol) and 2.5 M sodium hydroxide aqueous solution (0.5 mL) were sequentially added to dimethyl sulfoxide (1 mL), added with hydrogen peroxide (0.3 mL) dropwise in an ice bath, and stirred continuously at room temperature for 0.5 hours. The reaction mixture was added with ethyl acetate (30 mL) and water (15 mL) for layer separation. The aqueous phase was extracted with ethyl acetate (30 mL×2). The combined organic phase was added with saturated sodium chloride solution (30 mL×2), dried over anhydrous sodium sulfate, filtered, and concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (S)-1-((5-((4-(3-((2-(1-hydroxyethyl)-1H-imidazol-1-yl)methyl)isoxazol-5-yl)phenyl)ethynyl)pyr idin-2-yl)methyl)azetidin-3-carboxamide 123 (10.0 mg) with a yield of 34.96%.
MS m/z (ESI): 483.0 [M+1]

### Biological evaluation

### Test Example 1. Determination of inhibition of LpxC enzymatic activity by the compounds of the present disclosure

The degree of inhibition of the enzymatic activity of recombinant Pseudomonas aeruginosa LpxC by the compounds of the present disclosure under in vitro conditions was determined using the following method.

The experimental procedure is briefly described as follows: The test compound was first dissolved in DMSO to prepare a 10 mM stock solution. The reaction was carried out in a 96-well microplate. First, 20 µL of recombinant Pseudomonas aeruginosa LpxC (purchased from Signalway Antibody, product number AP74647-2) was added to each well, with a final concentration of 5 nM. 5 µL of the compound to be tested was then added, and the compound was diluted 4 times to 8 concentration points with a concentration range of 0.61-10,000 nM. 5 µL of LpxC substrate UDP-3-O-(R-3-hydroxydecanoyl)-GlcNAc (purchased from Biosynth Carbosynth, product number: mu75071) was added, with a final concentration of the substrate of 10 µM. The plate was incubated at 25°C for 120 minutes. Then 20 µL of 2.0 mg/mL fluorescamine (purchased from sigmaaldrich, product number: F9015, solvent: 1:1 dimethylformamide/acetonitrile) was added to the reaction system, and mixed well for 10 minutes of reaction. Finally, 50 µL of 200 mM sodium phosphate buffer (pH 8.0) was added to terminate the reaction. Reading was carried out using a microplate reader (BMG), and the excitation and emission wavelengths were 390 and 495 nm respectively. By comparing with the fluorescence intensity ratio of the control group (0.1% DMSO), the percentage inhibition rate of the compound at each concentration was calculated. Nonlinear regression analysis was performed using the logarithmic value of compound concentration - inhibition rate by GraphPad Prism 5 software to obtain IC₅₀ value of the compound, as shown in Table 1 below.

**Table 1 Results of inhibition of LpxC enzymatic activity by compounds of the present disclosure**

| Compound No. | IC₅₀/nM |
|---|---|
| Example 2 | 3.07 |
| Example 3 | 24.91 |
| Example 5 | 72.26 |
| Example 6 | 12.9 |
| Example 7 | 73.07 |
| Example 9 | 27.07 |
| Example 10 | 30.38 |
| Example 17 | 6.7 |
| Example 18 | 9.84 |
| Example 20 | 9.86 |
| Example 22 | 98.94 |
| Example 23 | 7.75 |
| Example 24 | 22.16 |
| Example 25 | 20.3 |
| Example 26 | 71.69 |
| Example 27 | 4.25 |
| Example 28 | 19.9 |
| Example 29 | 13.99 |
| Example 30 | 12.65 |
| Example 31 | 6.06 |
| Example 32 | 19.77 |
| Example 33 | 28.06 |
| Example 34 | 4.86 |
| Example 35 | 14.92 |
| Example 36 | 8.33 |
| Example 37 | 3.55 |
| Example 38 | 50.8 |
| Example 39 | 19.92 |
| Example 40 | 16.69 |
| Example 41 | 26.34 |
| Example 42 | 5.78 |
| Example 43 | 36.67 |
| Example 44 | 18.64 |
| Example 46 | 22.96 |
| Example 47 | 11.15 |
| Example 48 | 9.77 |
| Example 49 | 11.08 |
| Example 50 | 22.21 |
| Example 51 | 6.7 |
| Example 52 | 11.25 |
| Example 53 | 13.69 |
| Example 54 | 16.18 |
| Example 55 | 24.39 |
| Example 56 | 14.65 |
| Example 57 | 14.1 |
| Example 58 | 21.96 |
| Example 59 | 21.8 |
| Example 60 | 26.41 |
| Example 61 | 74.16 |
| Example 62 | 21.34 |
| Example 63 | 65.08 |
| Example 64 | 21.59 |
| Example 65 | 64.59 |
| Example 66 | 67.92 |
| Example 67 | 10.45 |
| Example 68 | 68.35 |
| Example 69 | 10.44 |
| Example 70 | 12.9 |
| Example 71 | 24.2 |
| Example 72 | 26.57 |
| Example 73 | 33.06 |
| Example 74 | 21.24 |
| Example 75 | 61.17 |
| Example 76 | 12.88 |
| Example 79 | 42.51 |
| Example 80 | 64.91 |
| Example 83 | 54.69 |
| Example 93 | 16.23 |
| Example 94 | 76.45 |
| Example 95 | 19.91 |
| Example 96 | 15.9 |
| Example 97 | 31.85 |
| Example 98 | 78.51 |
| Example 99 | 19.43 |
| Example 103 | 46.84 |
| Example 104 | 58.64 |
| Example 105 | 22.65 |
| Example 106 | 68.87 |
| Example 107 | 16.34 |
| Example 109 | 23.36 |
| Example 110 | 11.16 |
| Example 111 | 8.59 |
| Example 113 | 24.62 |
| Example 114 | 10.48 |
| Example 115 | 24.49 |
| Example 116 | 47.92 |
| Example 117 | 18.5 |
| Example 118 | 16.48 |
| Example 119 | 7.42 |
| Example 120 | 77.12 |
| Example 121 | 22.28 |
| Example 122 | 6.71 |
| Example 123 | 8.22 |

Conclusion: The compounds of the present disclosure had an IC₅₀ for inhibiting the enzymatic activity of recombinant Pseudomonas aeruginosa LpxC of less than 100 nM, showing a significant inhibitory effect on the LpxC enzymatic activity.

### Test Example 2. Evaluation of antibacterial activity of the compounds of the present disclosure

The in vitro minimum inhibitory concentration (MIC) was determined in accordance with the CLSI guidelines, and was measured using the broth microdilution method.

The experimental process is briefly described as follows: The test compound was dissolved in DMSO to prepare a 12.8 mg/mL stock solution, which was then prepared into 11 twice-diluted 100× high-concentration working solutions (the final concentration of the system was 64 µg/mL-0.06 µg/mL) using DMSO. The strains (*K. Pneumoniae* ATCC13883 and *E. coli* ATCC 25922) frozen in -80°C glycerol were inoculated into solid agar medium, placed in an incubator at 35°C for 18-24 hours of incubation. After the preparation of strains was completed, an appropriate amount of solid plate culture was collected, resuspended in physiological saline, and mixed well. The bacterial suspension was adjusted to a suitable turbidity using a turbidity meter, containing approximately 1×10⁸ cfu/mL bacteria. Then the bacterial suspension with the adjusted turbidity was diluted to a bacterial concentration of 5×10⁵ cfu/ml using the culture medium for measurement, to complete the preparation of the inoculum solution. 198 µL of the inoculum solution was inoculated into a 96-well plate, and then added with 2 µL of a 100× high-concentration working solution of the compound. Then the 96-well plate was placed at 35°C for 18 to 24 hours of incubation. After the incubation was completed, the test plate was obserced with the naked eye. The lowest drug concentration that completely inhibited bacterial growth is the minimum inhibitory concentration (MIC) of the compound, as shown in Table 2.

**Table 2 Measurement results of antibacterial activity of compounds of the present disclosure**

| **Compound No.** | **MIC**(K.Pneumoniae)/µg·mL⁻¹ | **MIC**(E. coli)/ µg·mL⁻¹ |
|---|---|---|
| Example 2 | 1 | 0.5 |
| Example 3 | 2 | 1 |
| Example 5 | N/A | 1 |
| Example 6 | 4 | 2 |
| Example 7 | 0.5 | 0.25 |
| Example 9 | 2 | 1 |
| Example 10 | 1 | 0.5 |
| Example 17 | 8 | 4 |
| Example 18 | 4 | 2 |
| Example 20 | N/A | 4 |
| Example 22 | N/A | 4 |
| Example 23 | 1 | 0.25 |
| Example 24 | 8 | 4 |
| Example 25 | 4 | 1 |
| Example 26 | 0.5 | 0.125 |
| Example 27 | 2 | 1 |
| Example 28 | 2 | 1 |
| Example 29 | 0.25 | 0.25 |
| Example 30 | 1 | 0.5 |
| Example 31 | 4 | 2 |
| Example 32 | 4 | 2 |
| Example 33 | 0.25 | 0.125 |
| Example 34 | 2 | 1 |
| Example 35 | 0.5 | 0.25 |
| Example 36 | 4 | 2 |
| Example 37 | 2 | 1 |
| Example 38 | 1 | 0.5 |
| Example 39 | 1 | 0.5 |
| Example 40 | 4 | 1 |
| Example 42 | 4 | 2 |
| Example 43 | 8 | 1 |
| Example 44 | 8 | 2 |
| Example 46 | 0.5 | 0.25 |
| Example 47 | N/A | 8 |
| Example 48 | 8 | 2 |
| Example 49 | 4 | 1 |
| Example 50 | 4 | 2 |
| Example 51 | 8 | 2 |
| Example 52 | 1 | 0.25 |
| Example 53 | 4 | 1 |
| Example 54 | 4 | 2 |
| Example 55 | 8 | 8 |
| Example 56 | 4 | 2 |
| Example 57 | 4 | 2 |
| Example 58 | 8 | 4 |
| Example 59 | 8 | 2 |
| Example 60 | 4 | 2 |
| Example 61 | 4 | 1 |
| Example 62 | 4 | 1 |
| Example 63 | 4 | 2 |
| Example 64 | 2 | 4 |
| Example 67 | N/A | 2 |
| Example 69 | N/A | 8 |
| Example 70 | N/A | 8 |
| Example 71 | N/A | 8 |
| Example 72 | N/A | 8 |
| Example 73 | N/A | 8 |
| Example 75 | 2 | 1 |
| Example 76 | N/A | 8 |
| Example 93 | N/A | 8 |
| Example 95 | N/A | 8 |
| Example 96 | N/A | 4 |
| Example 99 | N/A | 4 |
| Example 107 | N/A | 8 |
| Example 109 | N/A | 4 |
| Example 110 | N/A | 8 |
| Example 111 | 1 | 1 |
| Example 114 | 8 | 2 |
| Example 115 | 8 | 2 |

| | | |
|---|---|---|
| Note: N/A means not determined | | |

Conclusion: The compounds of the present disclosure had good inhibitory effect on both *Klebsiella Pneumoniae* (*K. Pneumoniae* ATCC13883) and *Escherichia coli* (*E. coli* ATCC 25922).

### Test Example 3. Pharmacokinetic assay of the compounds of the present disclosure in mice

### 1. Experimental purpose

ICR mice were used as test animals. The injected compounds 17 and 32 of the present disclosure were measured using the LC/MS/MS method to determine the drug concentrations in plasma at different times, in order to study the pharmacokinetic characteristics of the compounds of the present disclosure in mice.

### 2. Experimental scheme

### 2.1 Experimental drugs and animals;

### Compounds 17 and 32;

**ICR** mice, male, weighing 27.8-38 g, were purchased from VitalRiver Laboratory Animal Technology Co., Ltd.

### 2.2 Preparation of drugs

An appropriate amount of the pharmaceutical compound was weighed, added with an appropriate amount of 10% HP-β-CD, vortexed, sonicated for 20 minutes to dissolve the particles, and filtered (PTFE, 0.45 µm) to obtain 3 mg/kg of a colorless solution.

### 2.3 Administration

ICR mice were divided into groups of injection of each test compound (nine mice in each group), fasted overnight, then administered by injection (IV, at an amount of 15 mg/kg and a volume of 5 mL/kg), and fed 4 hours after administration.

### 3. Operation

About 0.1 mL of blood was collected from the orbit before administration and 0.083 hours, 0.25 hours, 0.5 hours, 1 hour, 2 hours, 4 hours, 8 hours, 12 hours and 24 hours after administration. The whole blood samples were placed in EDTA-K2-containing anticoagulant tubes. After collected, the blood samples were placed on ice, and centrifuged to separate plasma (centrifugation conditions: 1500 g, 10 minutes). The collected plasma was stored at -40 to -20°C before analysis.

The content of the compound to be tested in the plasma of mice after injection was determined using LC-MS/MS.

### 4. Pharmacokinetic parameter results

The pharmacokinetic parameters of the compounds of the present disclosure are shown in Table 3 below.

**Table 3 Pharmacokinetic parameters of compounds of the present disclosure in mice**

| Compound No. | Pharmacokinetic assays (15 mg/kg, IV) | | | |
|---|---|---|---|---|
| | Area under curve AUC_{0-∞} (ng-h/mL) | Half life T1/2 (h) | Clearance CL (mL·kg⁻¹·min⁻¹) | Volume of distribution Vdss (L·kg⁻¹) |
| Example 17 | 22500 | 1.3 | 11.1 | 0.48 |
| Example 32 | 20300 | 1.21 | 12.3 | 0.612 |

Conclusion: Both the compound of Example 17 and the compound of Example 32 of the present disclosure had large areas under the curve and had good pharmacokinetic properties.

### Test Example 4. Evaluation of the efficacy of the compounds of the present disclosure against pulmonary infection caused by Klebsiella pneumoniae

The efficacy of the compounds of the present disclosure against pulmonary infection caused by *Klebsiella pneumoniae* ATCC 51504 was determined by the following method.

The experimental process is briefly described as follows: The experimental day of inoculating *Klebsiella pneumoniae* ATCC 51504 was defined as Day 0. On Day -4 and Day -1, CD-1 female mice (6-8 weeks old, purchased from VitalRiver Experimental Animal Technology Co., Ltd., divided into 3 groups, 5 animals in each group) were intraperitoneally injected with cyclophosphamide to induce an immunosuppressive state in mice. *Klebsiella pneumoniae* ATCC 51504 cells were cultured in advance on a Muaeller-Hinton agar culture plate in a 37°C incubator overnight. The single colonies were collected, suspended in physiological saline, and adjusted to the required concentration using a turbidity meter. CD-1 female mice were inoculated via intranasal instillation. Each mouse was inoculated with 50 µL of bacterial solution, with an inoculation volume of 1×10⁷ CFU/mouse. Two hours after infection, CD-1 female mice were intravenously administered with compound 17 and compound 32 at 150 mg/kg (solvent: 10% HP-β-CD in Sterile Water for Injection). 24 hours after infection, CD-1 female mice were euthanized. The lung tissue was taken, homogenized with physiological saline, and then diluted 10 times in a gradient for a total of 5 times. Then, 10 µL of lung tissue homogenate of each dilution ratio was taken, and inoculated into bacterial culture plates, which were incubated in a 37°C incubator overnight, then photographed and saved. A culture plate with an appropriate dilution ratio was selected to count the number of colonies and calculate the bacterial load in the lung tissue to evaluate the efficacy of the drug in inhibiting bacteria. The determination results of the efficacy of the compounds of the present disclosure on pulmonary infection caused by *Klebsiella pneumoniae* are shown in Tables 4 and 5 below.

**Table 4 Determination results of efficacy of the compound of Example 17 of the present disclosure on pulmonary infection caused by Klebsiella pneumoniae**

| Group | LOG (CFU/mouse) | |
|---|---|---|
| | Mean | Standard deviation |
| Control group 2 hours | 6.96 | 0.08 |
| Control group 24 hours | 8.95 | 0.32 |
| Example 17 | 4.17 | 0.307 |

Conclusion: Compared with the control group, 150 mg/kg of the compound of Example 17 can reduce the total bacterial load of *Klebsiella pneumoniae* ATCC 51504 in the lungs by 4.78 log value, showing a good antibacterial activity. There was no significant change in the body weight of the mice.

**Table 5 Determination results of efficacy of the compound of Example 32 of the present disclosure on pulmonary infection caused by Klebsiella pneumoniae**

| Group | LOG (CFU/mouse) | |
|---|---|---|
| | Mean | Standard deviation |
| Control group 2 hours | 6.94 | 0.08 |
| Control group 24 hours | 8.66 | 0.3 |
| Example 32 | 4.42 | 0.32 |

Conclusion: Compared with the control group, 150 mg/kg of the compound of Example 32 can reduce the total bacterial load of *Klebsiella pneumoniae* ATCC 51504 in the lungs by 4.24 log value, showing a good antibacterial activity. There was no significant change in the body weight of the mice.

## Claims

1. A compound represented by general formula (A-I) or a stereoisomer, tautomer or pharmaceutically acceptable salt thereof: 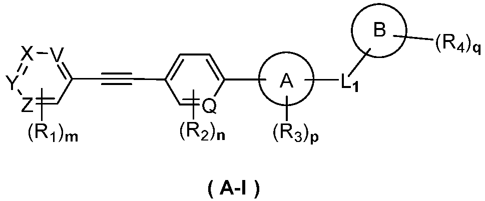 wherein:
ring A is 5- to 6-membered heteroaryl or 5- to 6-membered heterocyclyl, preferably 5-membered heteroaryl or 5-membered heterocyclyl;
ring B is 5- to 10-membered heteroaryl;
Q is C or N;
X, Y, Z, and V are each independently C or N, wherein X and Y are not N simultaneously, and Z and Y are not N simultaneously;
R₁ is the same or different, each independently being -G₁-R₅;
G₁ is selected from the group consisting of a single bond, -O-, -CH₂- and -C(=O)-;
L₁ is -(CH₂)ₛ-, preferably -CH₂-;
R₂ is the same or different, each independently selected from the group consisting of hydroxyl, cyano, halogen, C₁-C₆ alkyl, C₁-C₆ alkoxy, C₃-C₇ cycloalkyl, 5- to 7-membered monocyclic or a 7-to 10-membered bicyclic or tricyclic heterocyclyl, C₆-C₁₀ aryl and C₆-C₁₀ heteroaryl, wherein the C₁-C₆ alkyl, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with one or more substituents selected from the group consisting of halogen, hydroxyl, cyano and C₁-C₆ alkoxy;
R₃ is the same or different, each independently selected from the group consisting of hydroxyl, cyano, halogen, C₁-C₆ alkyl and C₁-C₆ alkoxy;
alternatively, two R₃ form -C(=O)- together with the C atom to which they are connected;
R₄ is the same or different, each independently selected from the group consisting of hydroxyl, cyano, halogen, C₁-C₆ alkyl, C₁-C₆ alkoxy, C₃-C₇ cycloalkyl, 5- to 7-membered monocyclic or a 7-to 10-membered bicyclic or tricyclic heterocyclyl, C₆-C₁₀ heteroaryl, -C(O)R₆, -C(O)OR₆, - OC(O)R₆, -NR₇R₈, -C(O)NR₇R₈, -SO₂NR₇R₈ and -NR₇C(O)R₈, wherein the C₁-C₆ alkyl, C₃-C₇ cycloalkyl, 5- to 7-membered monocyclic or a 7- to 10-membered bicyclic or tricyclic heterocyclyl or C₆-C₁₀ heteroaryl is optionally further substituted with one or more substituents selected from the group consisting of halogen, hydroxyl, cyano, C₁-C₆ alkoxy, and amino;
R₅ is selected from the group consisting of cyano, halogen, C₁-C₆ alkyl, hydroxyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, -C(O)R₆, -C(O)OR₆, -OC(O)R₆, -NR₁₂R₁₃, - C(O)NR₁₂R₁₃, -SO₂R₆, -SO₂NR₁₂R₁₃ and -NR₁₂C(O)R₁₃, wherein the C₁-C₆ alkoxy, C₁-C₆ alkyl, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with one or more R_{A};
R_{A} is selected from the group consisting of halogen, hydroxyl, cyano, hydroxy C₁-C₆ alkyl, C₁-C₆ alkoxy, C₁-C₆ alkyl, C₂-C₄ alkenyl, C₃-C₇ cycloalkyl, 5- to 7-membered monocyclic or a 7-to 10-membered bicyclic or tricyclic heterocyclyl, C₆-C₁₀ aryl, C₆-C₁₀ heteroaryl, -C(O)R₆, - C(O)OR₆, -OC(O)R₆, -NR₇R₈, -C(O)NR₇R₈, -SO₂NR₇R₈ and -NR₇C(O)R₈, wherein the C₁-C₆ alkyl, C₃-C₇ cycloalkyl, 5- to 7-membered monocyclic or a 7- to 10-membered bicyclic or tricyclic heterocyclyl, C₆-C₁₀ aryl, or C₆-C₁₀ heteroaryl is optionally further substituted with one or more substituents selected from the group consisting of halogen, hydroxyl, cyano, amino, halo C₁-C₆ alkyl, hydroxy C₁-C₆ alkyl, C₁-C₆ alkoxy, C₁-C₆ alkyl, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, -C(O)R₆, -C(O)OR₆, -OC(O)R₆, -NR₇R₈, -C(O)NR₇R₈, -SO₂NR₇R₈ and -NR₇C(O)R₈;
alternatively, two R_{A} form -C(O)- together with the same carbon atom to which they are connected;
R₆ is selected from the group consisting of a hydrogen atom, C₁-C₆ alkyl, C₃-C₇ cycloalkyl, 5- to 7-membered monocyclic or a 7- to 10-membered bicyclic or tricyclic heterocyclyl, C₆-C₁₀ aryl and C₆-C₁₀ heteroaryl, wherein the C₁-C₆ alkyl, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with one or more substituents selected from the group consisting of hydroxyl, halogen, nitro, cyano, C₁-C₆ alkyl, C₁-C₆ alkoxy, halo C₁-C₆ alkyl, halo C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, =O, -C(O)R₉, -C(O)OR₉, -OC(O)R₉, - NR₁₀R₁₁, -C(O)NR₁₀R₁₁, -SO₂NR₁₀R₁₁ and -NR₁₀C(O)R₁₁;
R₇, R₈, R₁₂ and R₁₃ are each independently selected from the group consisting of a hydrogen atom, hydroxyl, halogen, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl and C₆-C₁₀ heteroaryl, wherein the C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with one or more substituents selected from the group consisting of hydroxyl, halogen, nitro, cyano, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, =O, -C(O)R₉, -C(O)OR₉, -OC(O)R₉, -NR₁₀R₁₁, -C(O)NR₁₀R₁₁, -SO₂R₉, -SO₂NR₁₀R₁₁ and -NR₁₀C(O)R₁₁;
alternatively, R₇ and R₈ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, =O, -C(O)R₉, -C(O)OR₉, -OC(O)R₉, - NR₁₀R₁₁, -C(O)NR₁₀R₁₁, -SO₂NR₁₀R₁₁ and -NR₁₀C(O)R₁₁;
alternatively, R₁₂ and R₁₃ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, =O, -C(O)R₉, -C(O)OR₉, -OC(O)R₉, - NR₁₀R₁₁, -C(O)NR₁₀R₁₁, -SO₂NR₁₀R₁₁ and -NR₁₀C(O)R₁₁;
R₉, R₁₀ and R₁₁ are each independently selected from the group consisting of a hydrogen atom, C₁-C₆ alkyl, amino, C₃-C₇ cycloalkyl, 5- to 7-membered monocyclic or a 7- to 10-membered bicyclic or tricyclic heterocyclyl, C₆-C₁₀ aryl and C₆-C₁₀ heteroaryl, wherein the C₁-C₆ alkyl, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with one or more substituents selected from the group consisting of hydroxyl, halogen, nitro, amino, cyano, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, carboxyl, and a carboxylate group;
m is 0, 1, 2 or 3;
n is 0, 1 or 2, preferably 0;
p is 0, 1 or 2;
s is 1 or 2; and
q is 1, 2 or 3.

2. The compound or the stereoisomer, tautomer, or pharmaceutically acceptable salt thereof according to claim 1, which is a compound represented by general formula (I) or a stereoisomer, tautomer or pharmaceutically acceptable salt thereof: 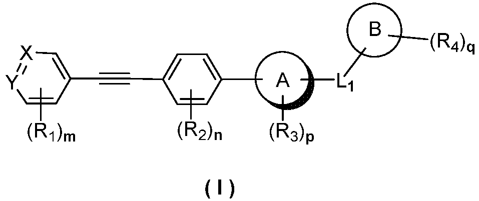 wherein:
X and Y are each independently C or N, wherein X and Y are not N simultaneously;
R₁ is the same or different, each independently being -G₁-R₅;
G₁ is selected from the group consisting of a single bond, -CH₂- and -C(=O)-;
R₅ is selected from the group consisting of cyano, halogen, C₁-C₆ alkyl, hydroxyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, -C(O)R₆, -C(O)OR₆, -OC(O)R₆, -NR₁₂R₁₃, - C(O)NR₁₂R₁₃, -SO₂NR₁₂R₁₃ and -NR₁₂C(O)R₁₃, wherein the C₁-C₆ alkoxy, C₁-C₆ alkyl, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with one or more R_{A};
R_{A} is selected from the group consisting of halogen, hydroxyl, cyano, hydroxy C₁-C₆ alkyl, C₁-C₆ alkoxy, C₁-C₆ alkyl, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, -C(O)R₆, -C(O)OR₆, -OC(O)R₆, - NR₇R₈, -C(O)NR₇R₈, -SO₂NR₇R₈ and -NR₇C(O)R₈, wherein the C₁-C₆ alkyl, C₃-C₇ cycloalkyl, 5-to 7-membered monocyclic or a 7- to 10-membered bicyclic or tricyclic heterocyclyl, C₆-C₁₀ aryl or C₆-C₁₀ heteroaryl is optionally further substituted with one or more substituents selected from the group consisting of halogen, hydroxyl, amino, halo C₁-C₆ alkyl, hydroxy C₁-C₆ alkyl, C₁-C₆ alkoxy, C₁-C₆ alkyl, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, -C(O)R₆, -C(O)OR₆, -OC(O)R₆, - NR₇R₈, -C(O)NR₇R₈, -SO₂NR₇R₈ and -NR₇C(O)R₈;
R₇, R₈, R₁₂ and R₁₃ are each independently selected from the group consisting of a hydrogen atom, hydroxyl, halogen, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl and C₆-C₁₀ heteroaryl, wherein the C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with one or more substituents selected from the group consisting of hydroxyl, halogen, nitro, cyano, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, =O, -C(O)R₉, -C(O)OR₉, -OC(O)R₉, -NR₁₀R₁₁, -C(O)NR₁₀R₁₁, -SO₂NR₁₀R₁₁ and - NR₁₀C(O)R₁₁;
alternatively, R₇ and R₈ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, =O, -C(O)R₉, -C(O)OR₉, -OC(O)R₉, - NR₁₀R₁₁, -C(O)NR₁₀R₁₁, -SO₂NR₁₀R₁₁ and -NR₁₀C(O)R₁₁;
alternatively, R₁₂ and R₁₃ form 4- to 8-membered heterocyclyl together with the atom to which they are connected, wherein the 4- to 8-membered heterocyclyl contains one or more N, O, S or SO₂, and the 4- to 8-membered heterocyclyl is optionally further substituted with one or more substituents selected from the group consisting of hydroxyl, halogen, nitro, cyano, C₁-C₆ alkyl, C₁-C₆ alkoxy, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl, =O, -C(O)R₉, -C(O)OR₉, -OC(O)R₉, - NR₁₀R₁₁, -C(O)NR₁₀R₁₁, -SO₂NR₁₀R₁₁ and -NR₁₀C(O)R₁₁; and
ring A, ring B, R₂-R₄, R₆, R₉-R₁₁, L₁, m, n, p and q are as defined in claim 1.

3. The compound or the stereoisomer, tautomer, or pharmaceutically acceptable salt thereof according to claim 1, which is a compound represented by general formula (II-1), (II-2), (II-3), (II-4) or (II-5) or a stereoisomer, tautomer or pharmaceutically acceptable salt thereof: 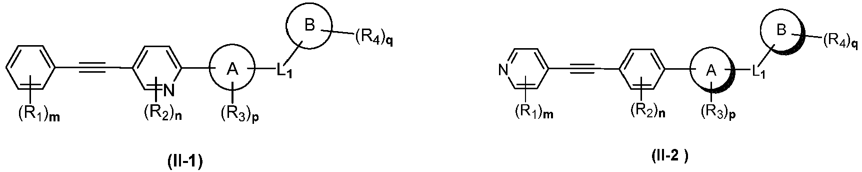 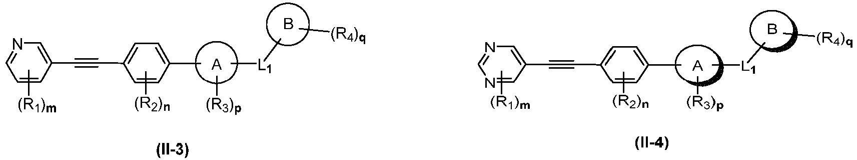 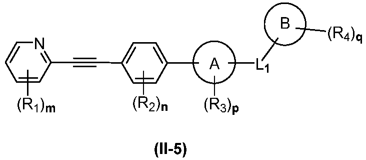 wherein, ring A, ring B, R₁-R₄, L₁, m, n, p and q are as defined in claim 1.

4. The compound or the stereoisomer, tautomer, or pharmaceutically acceptable salt thereof according to any one of claims 1 to 3, which is a compound represented by general formula (III-1) or (III-2) or a stereoisomer, tautomer or pharmaceutically acceptable salt thereof: 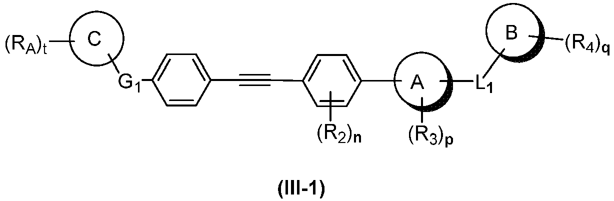 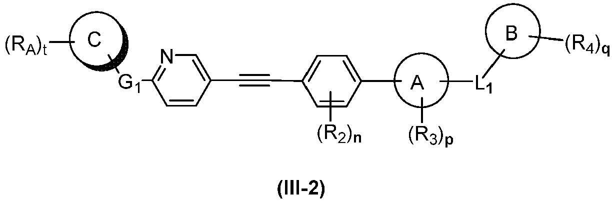 wherein:
ring C is selected from the group consisting of C₄-C₈ cycloalkyl, 5- to 6-membered heteroaryl and 4- to 8-membered heterocyclyl;
R_{A} is the same or different, each independently selected from the group consisting of halo C₁-C₆ alkyl, hydroxy C₁-C₆ alkyl, C₁-C₆ alkoxy, C₁-C₆ alkyl, C₂-C₄ alkenyl, hydroxyl, halogen, cyano, -C(O)NH₂, C₃-C₇ cycloalkyl, 5- to 7-membered monocyclic or a 7- to 10-membered bicyclic or tricyclic heterocyclyl, C₆-C₁₀ aryl, C₆-C₁₀ heteroaryl and carboxyl, wherein the C₁-C₆ alkyl, C₃-C₇ cycloalkyl, 5- to 7-membered monocyclic or a 7- to 10-membered bicyclic or tricyclic heterocyclyl, C₆-C₁₀ aryl or C₆-C₁₀ heteroaryl is optionally further substituted with a substituent selected from the group consisting of hydroxyl, cyano, carboxyl and an ester group;
alternatively, two R_{A} form -C(O)- together with the same carbon atom to which they are connected; t is 0, 1, 2 or 3; and
ring A, ring B, R₂-R₄, G₁, L₁, n, p and q are as defined in claim 1.

5. The compound or the stereoisomer, tautomer, or pharmaceutically acceptable salt thereof according to any one of claims 1 to 4, wherein ring A is selected from the group consisting of: 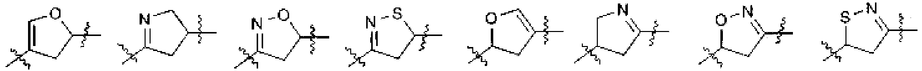 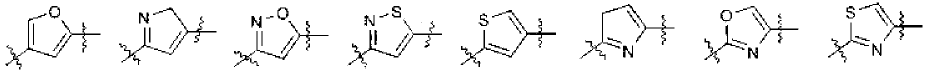 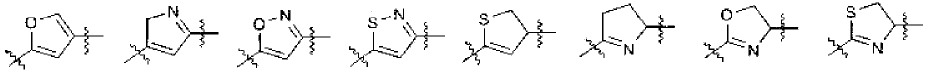 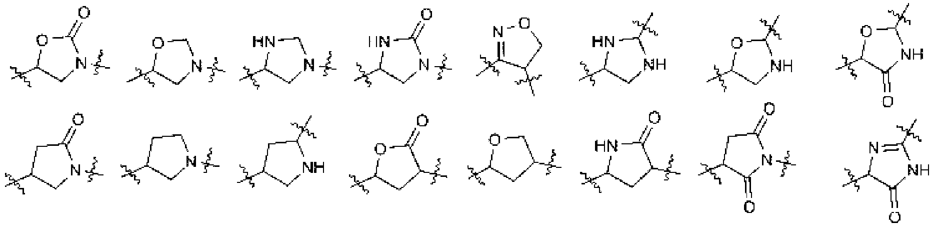 and

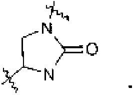

6. The compound or the stereoisomer, tautomer, or pharmaceutically acceptable salt thereof according to any one of claims 1 to 4, wherein ring B is selected from the group consisting of:

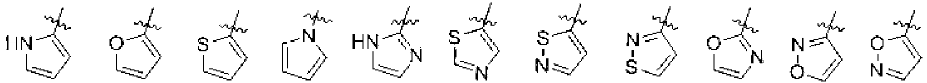

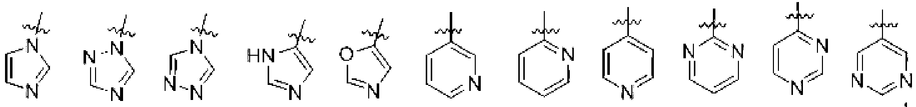

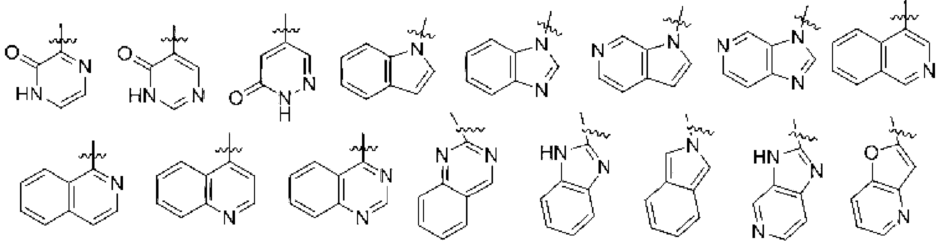

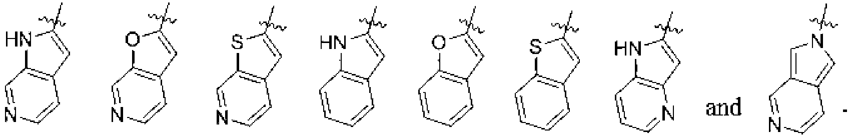

7. The compound or the stereoisomer, tautomer, or pharmaceutically acceptable salt thereof according to any one of claims 4 to 6, wherein ring C is selected from the group consisting of:

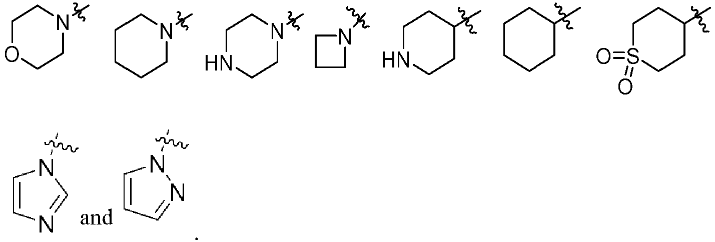

8. The compound or the stereoisomer, tautomer, or pharmaceutically acceptable salt thereof according to any one of claims 1 to 7, wherein:
R₄ is selected from the group consisting of C₁-C₆ alkyl, -C(O)R₆, -C(O)OR₆ and -C(O)NR₇R₈, wherein the C₁-C₆ alkyl is optionally further substituted with one or more substituents of hydroxyl or halogen;
R₆ is selected from the group consisting of a hydrogen atom, hydroxyl, C₁-C₆ alkyl and C₁-C₆ alkoxy; and
R₇ and R₈ are each independently a hydrogen atom or C₁-C₆ alkyl.

9. The compound or the stereoisomer, tautomer, or pharmaceutically acceptable salt thereof according to any one of claims 1 to 8, wherein R₄ is selected from the group consisting of:

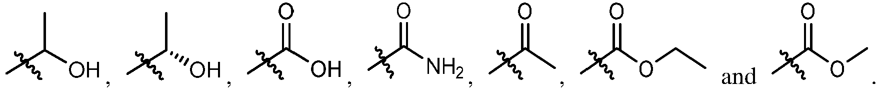

10. The compound or the stereoisomer, tautomer, or pharmaceutically acceptable salt thereof according to any one of claims 1 to 9, wherein the compound is selected from the group consisting of: 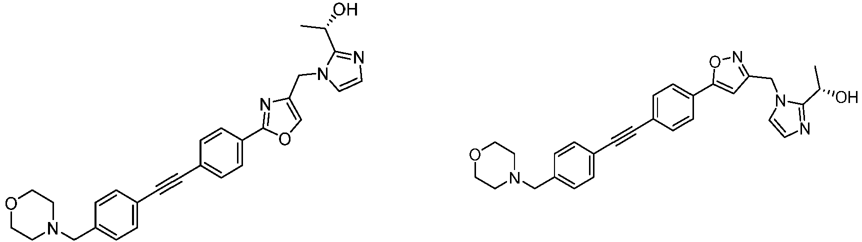 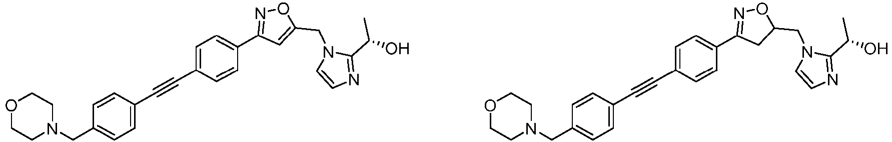 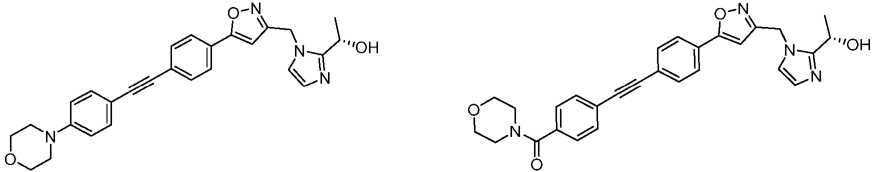 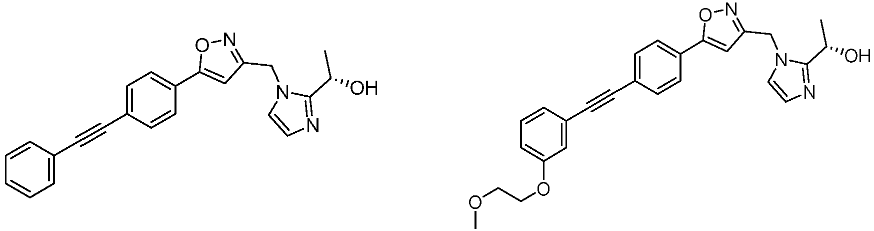 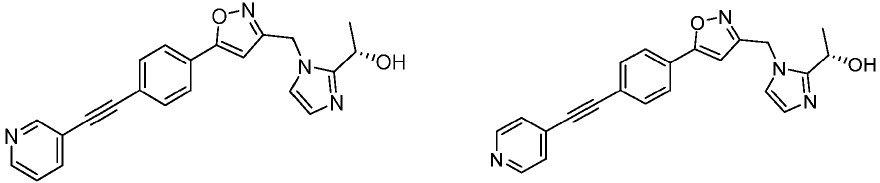 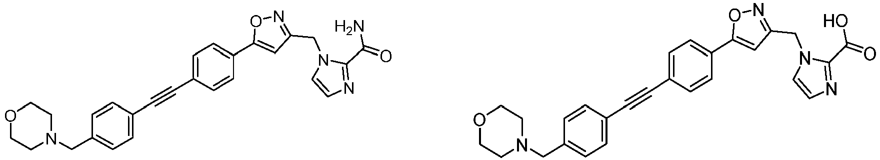 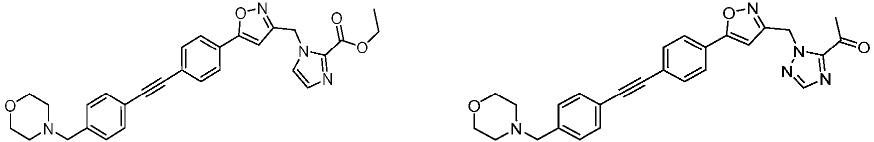 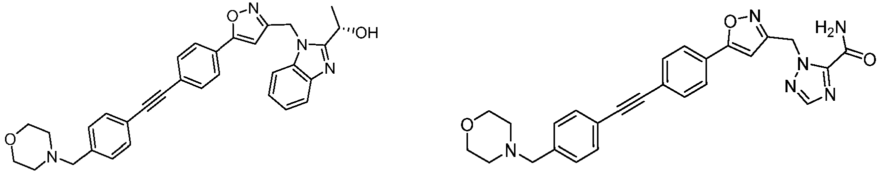 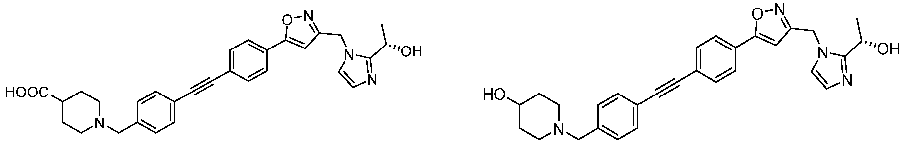 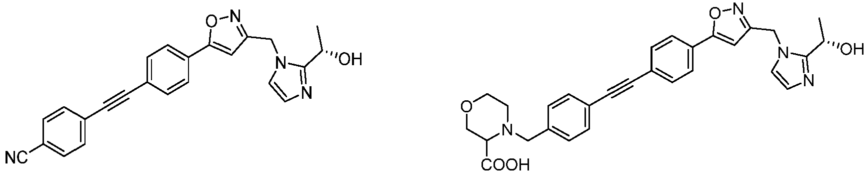 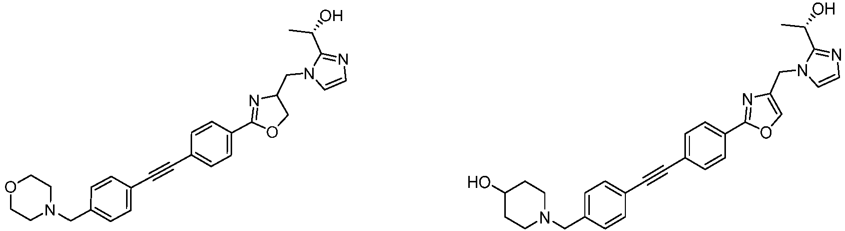 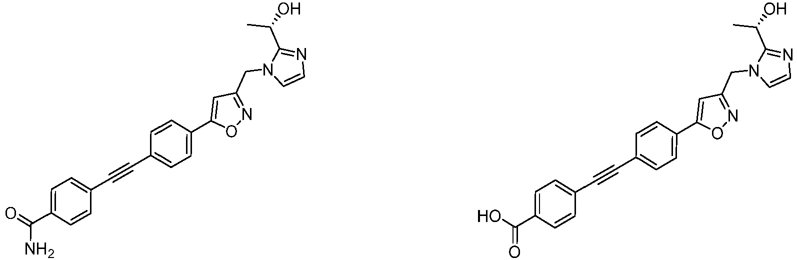 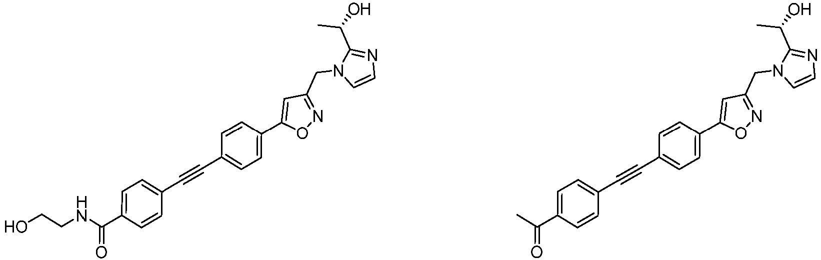 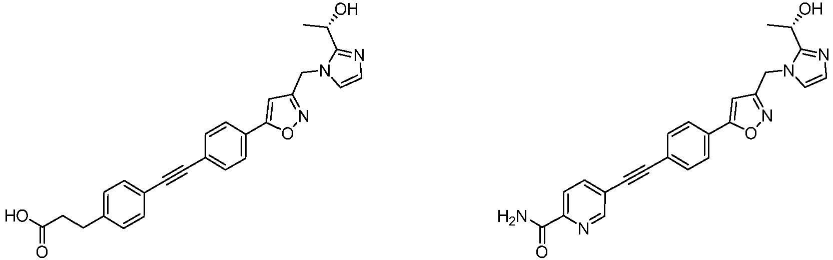 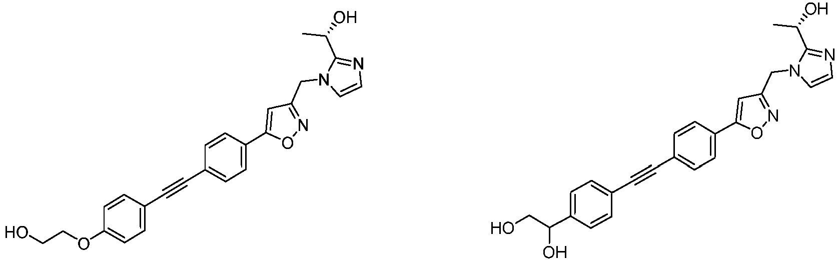 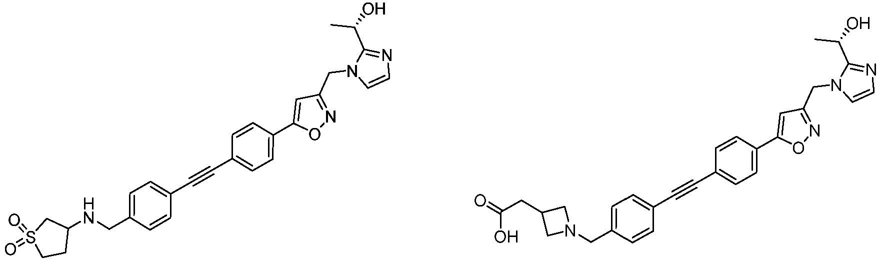 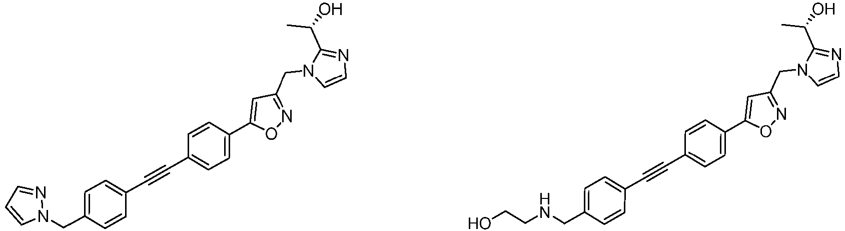 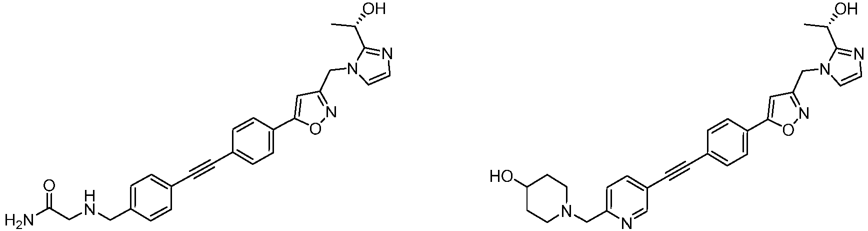 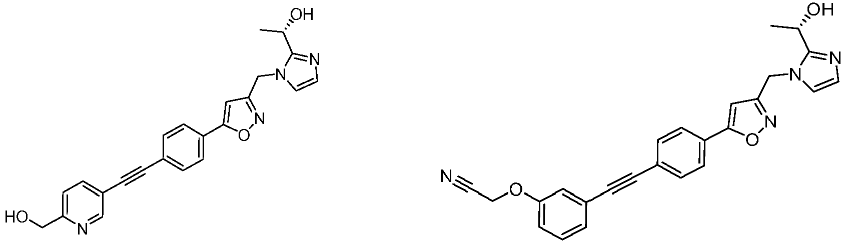 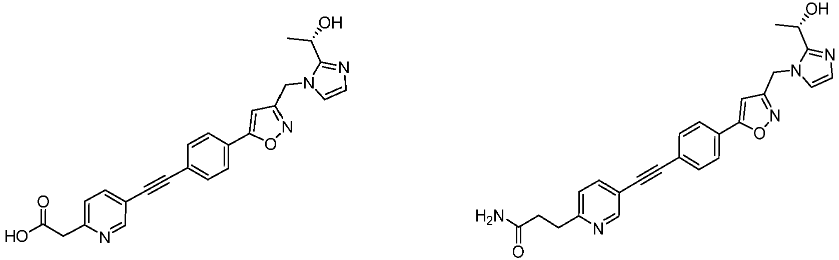 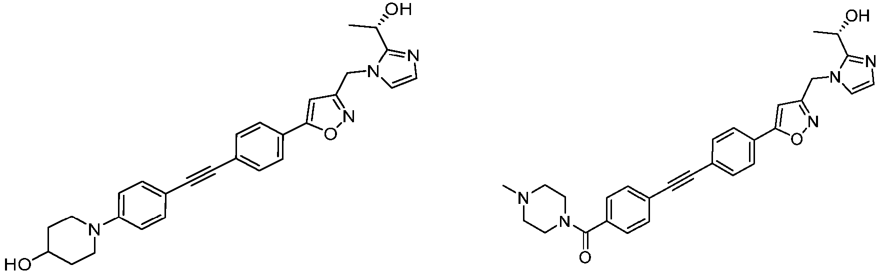 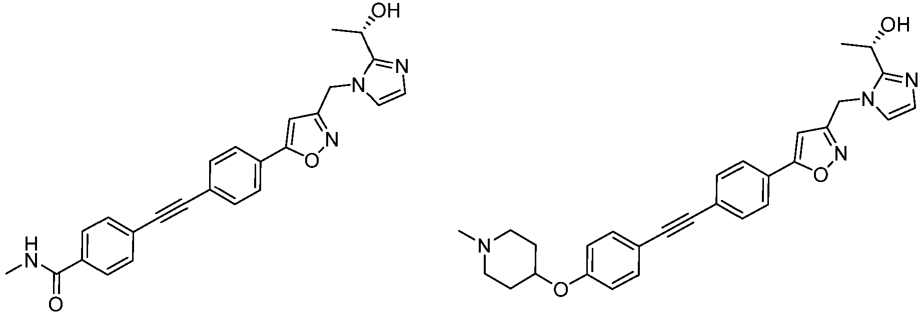 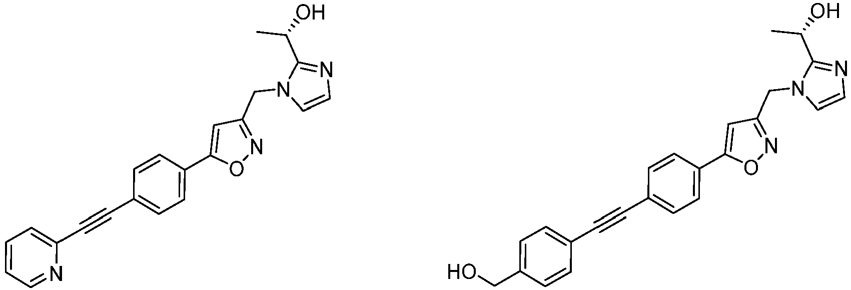 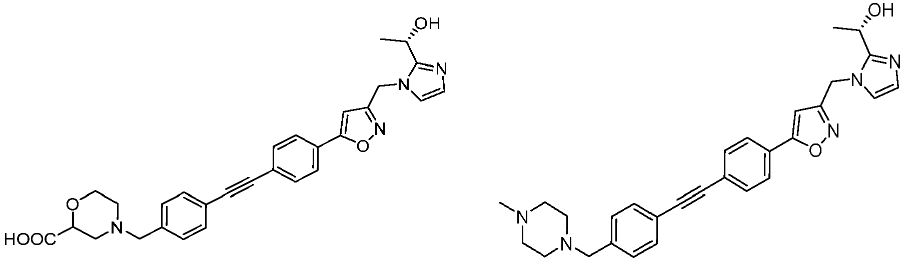 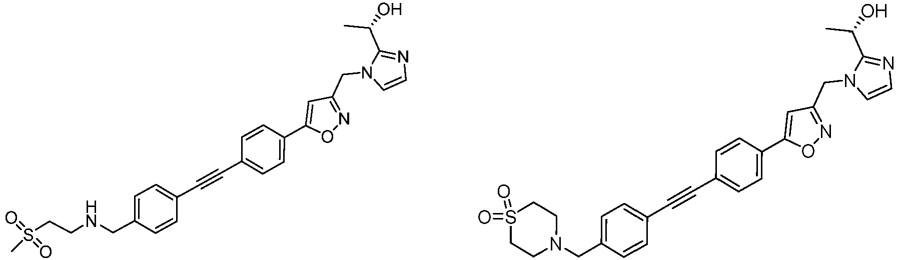 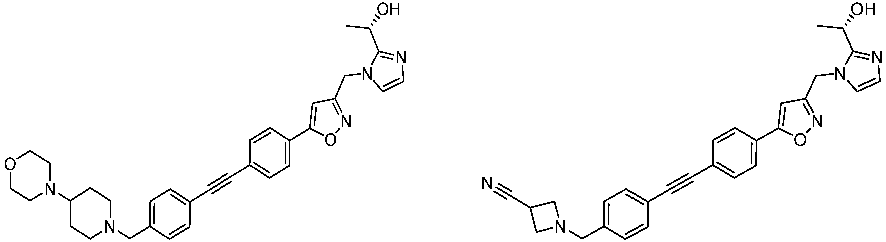 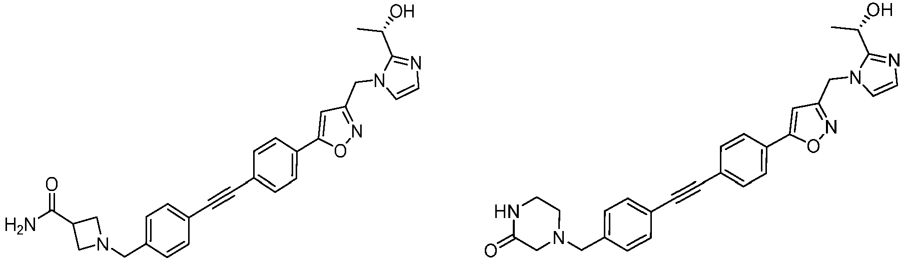 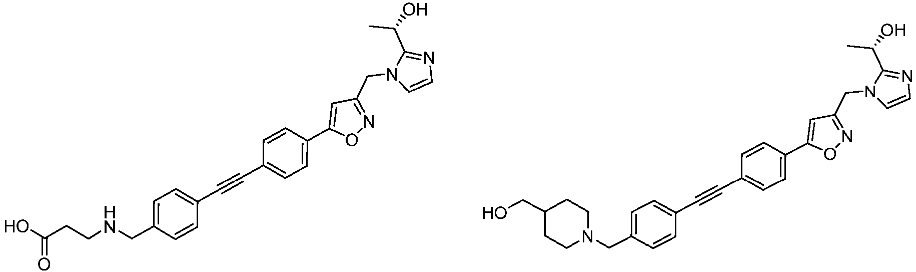 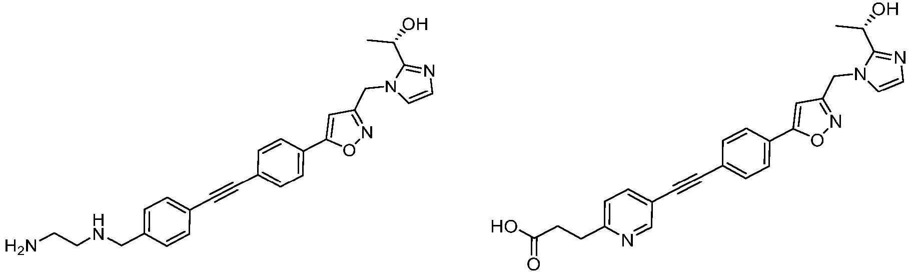 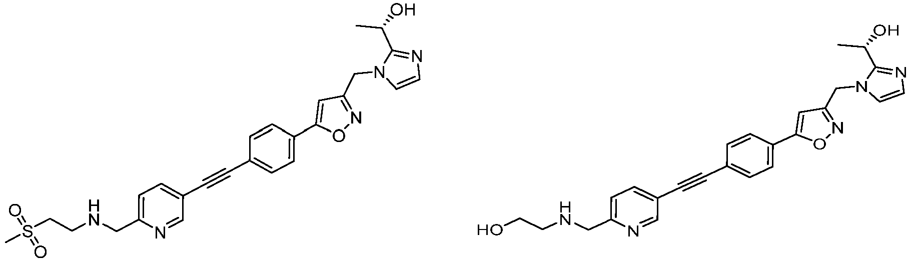 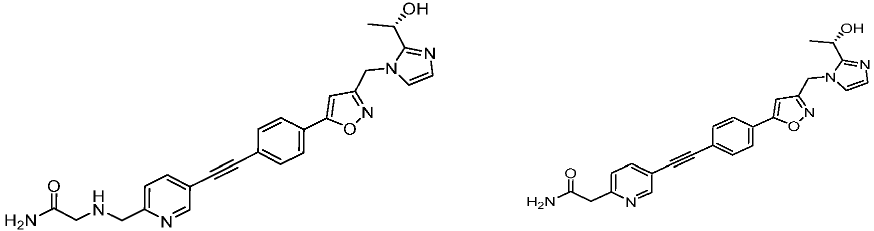 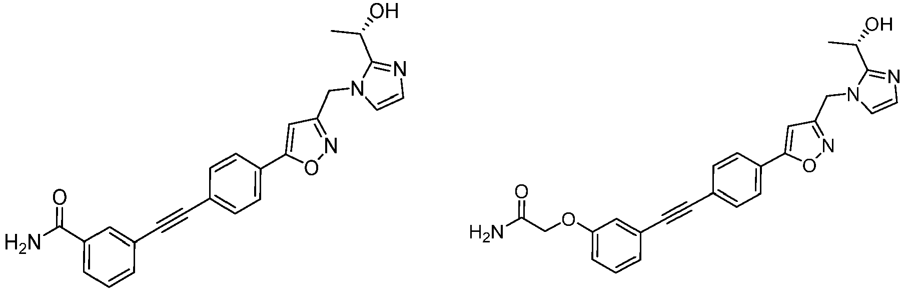 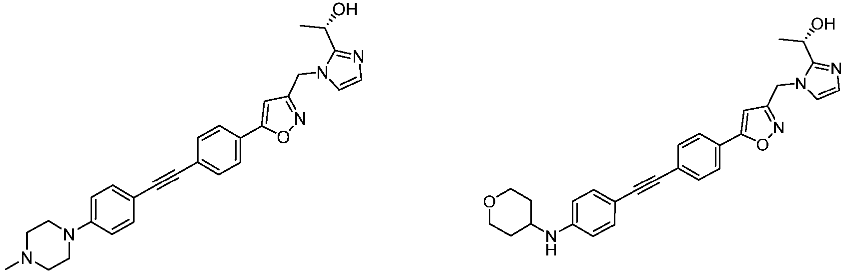 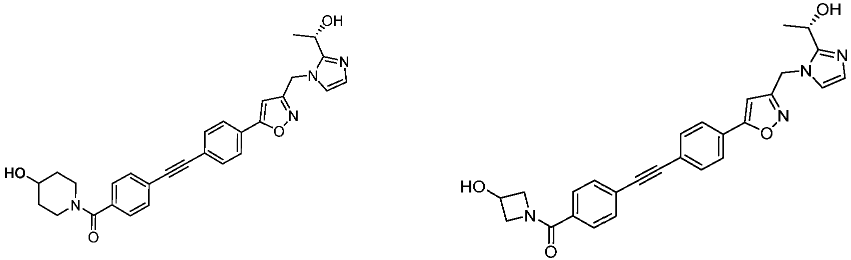 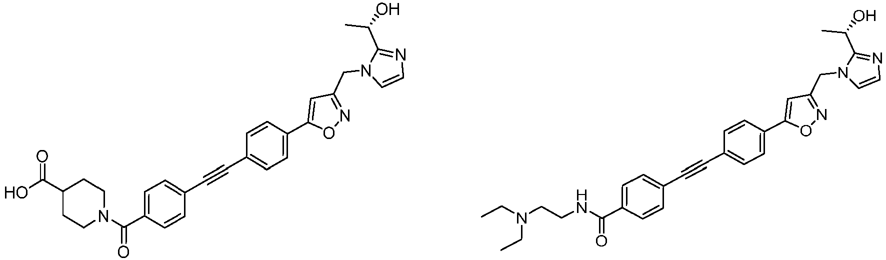 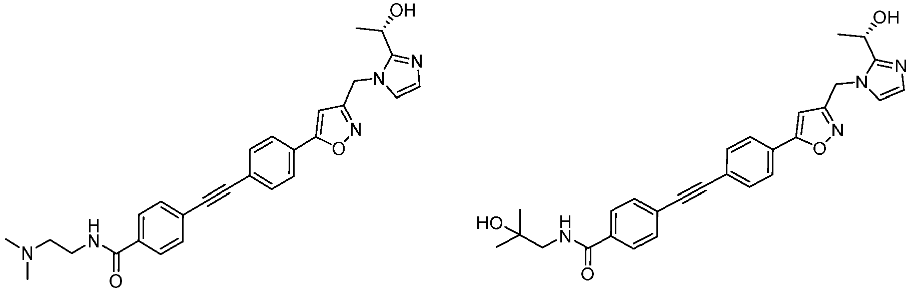 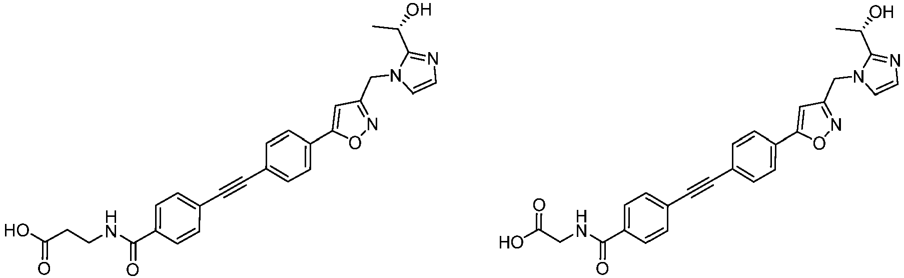 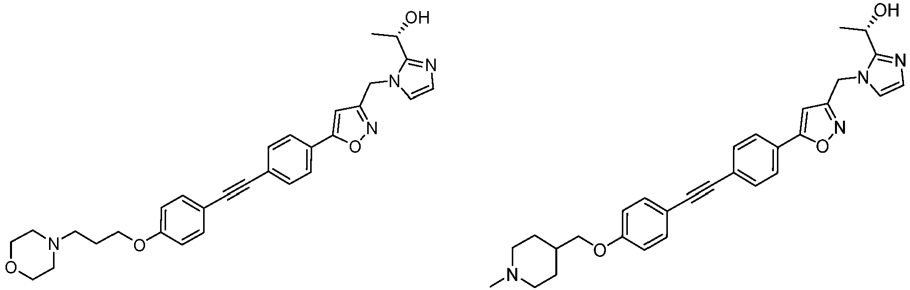 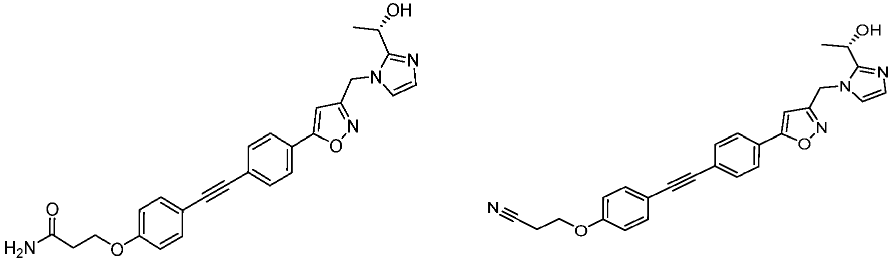 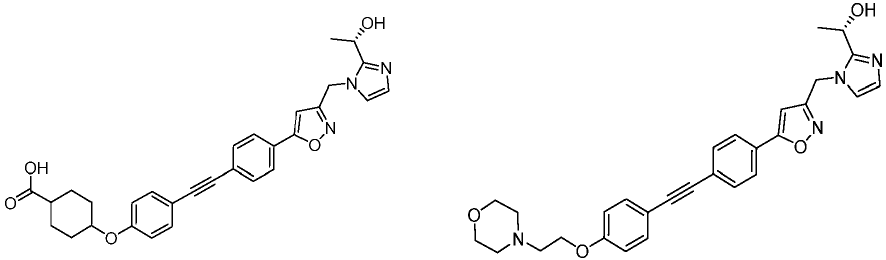 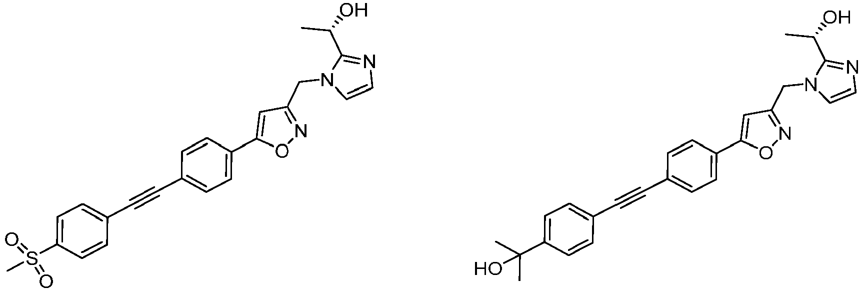 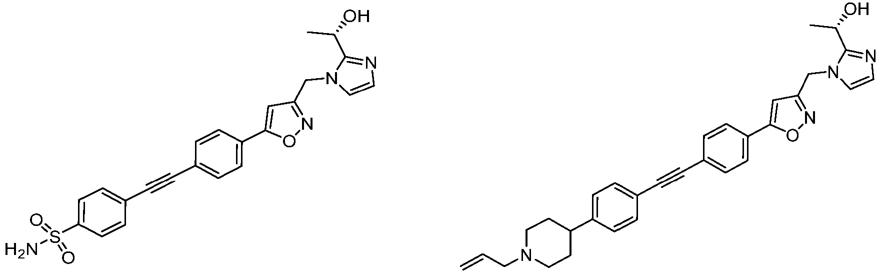 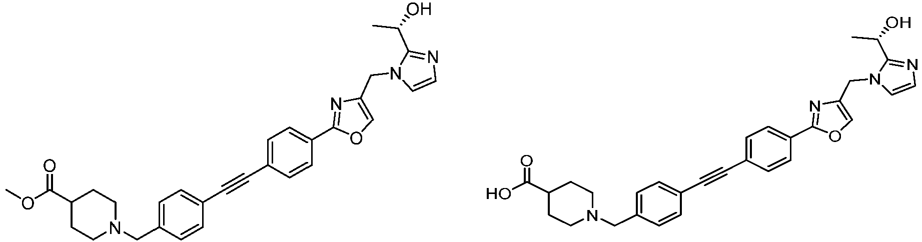 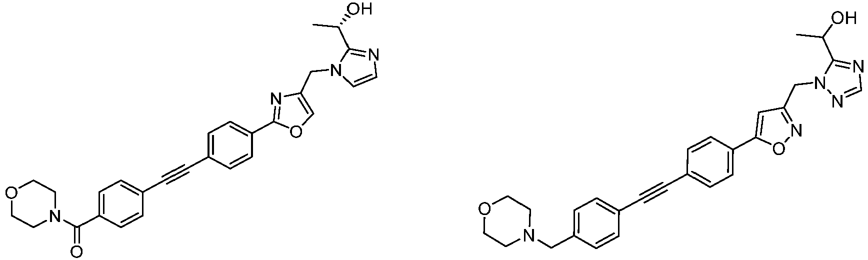 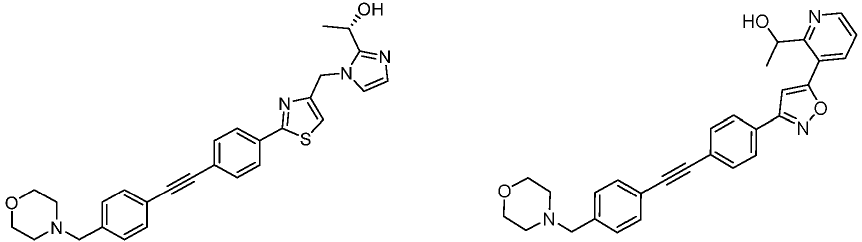 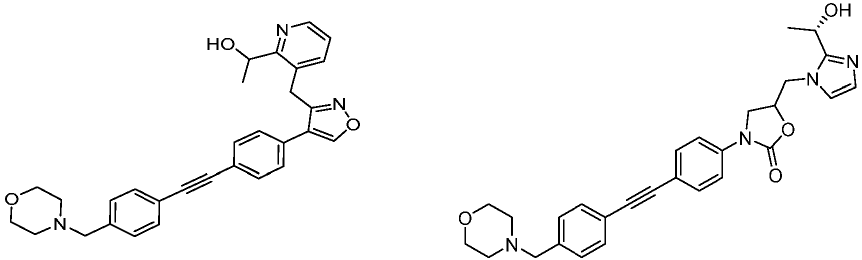 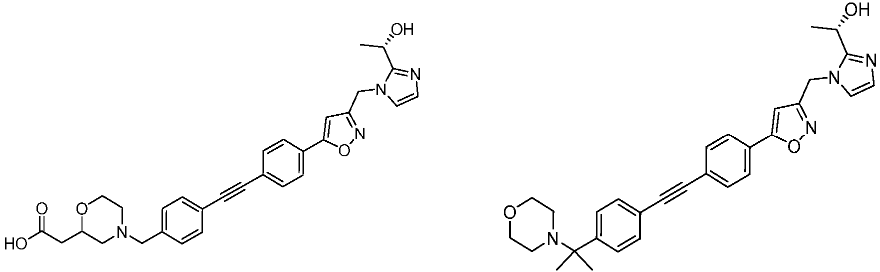 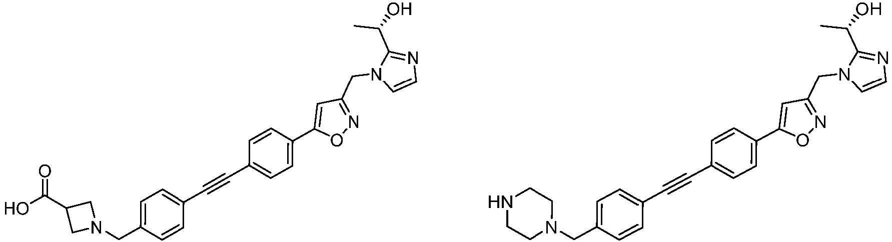 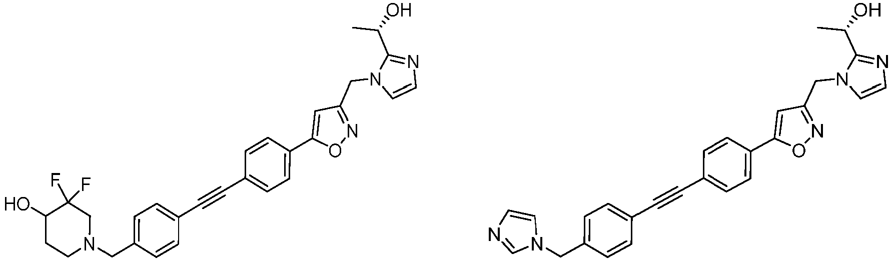 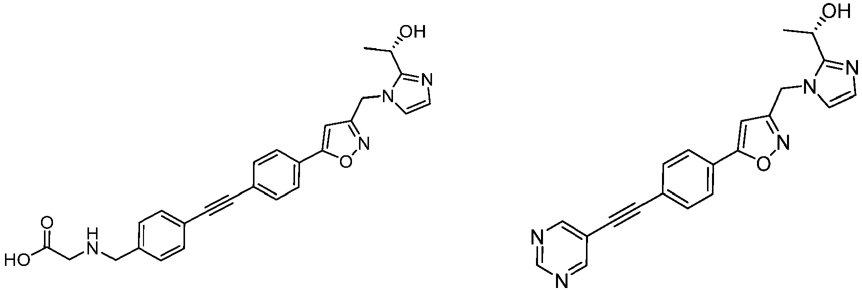 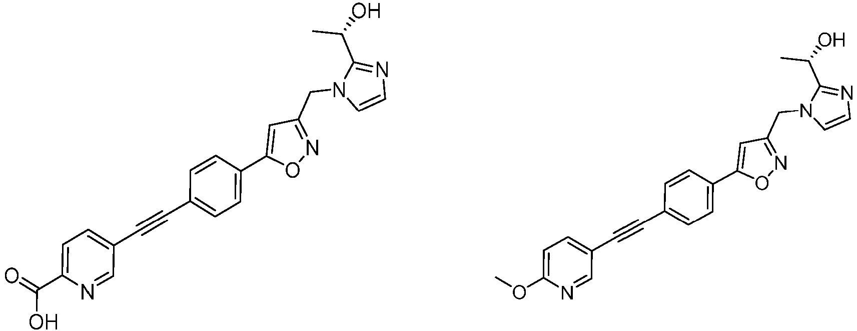 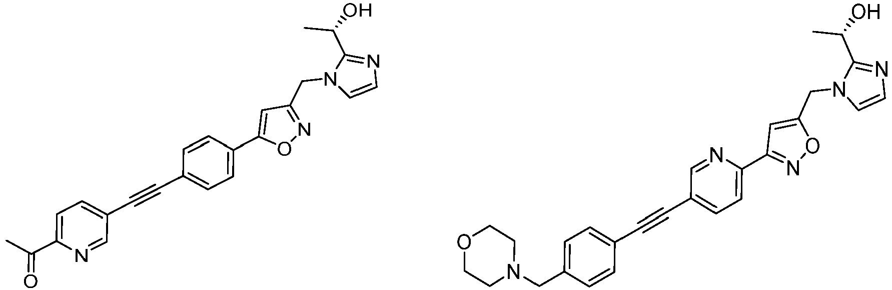 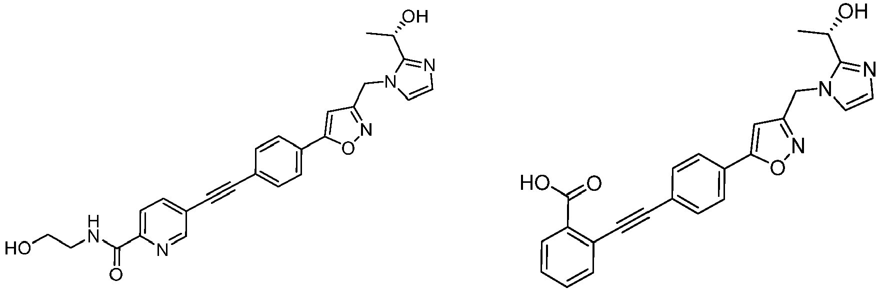 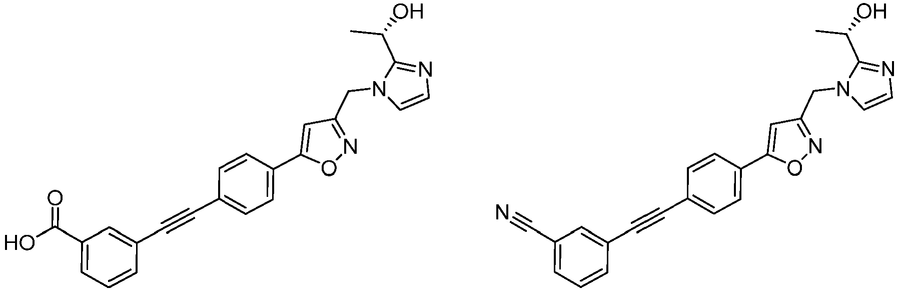 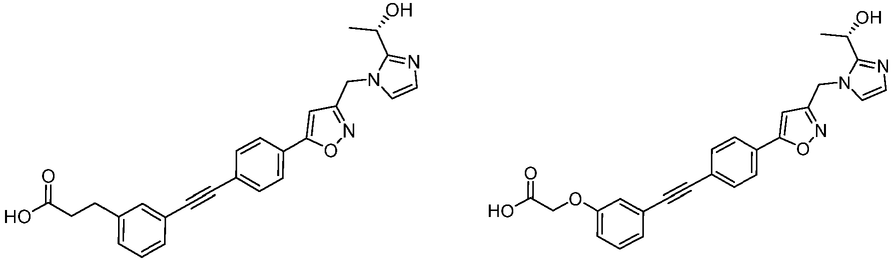 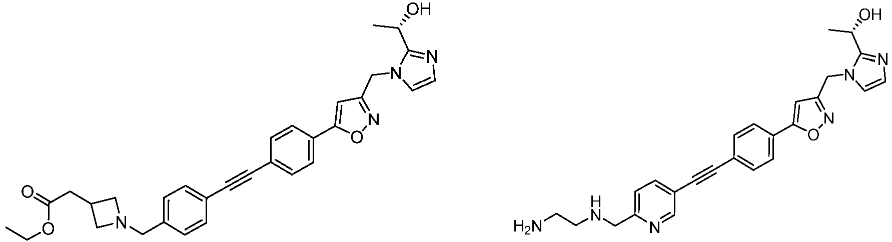 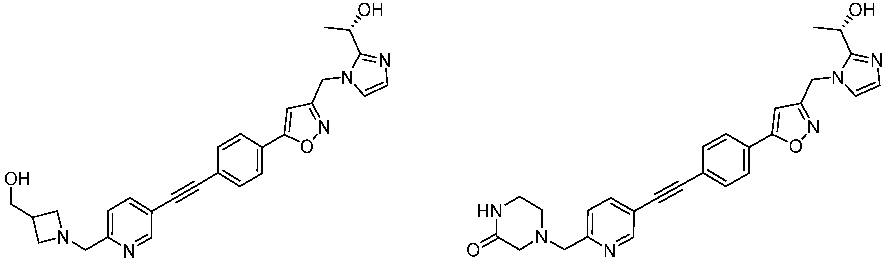 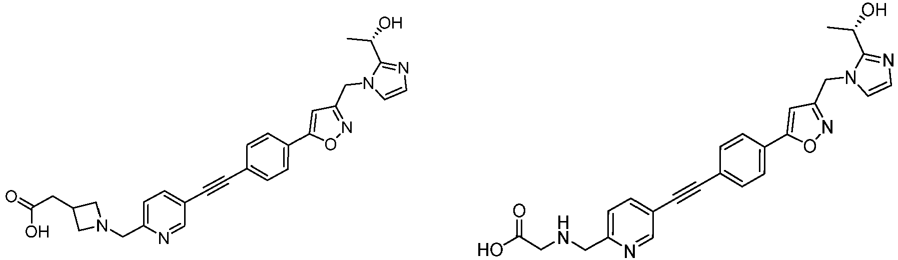 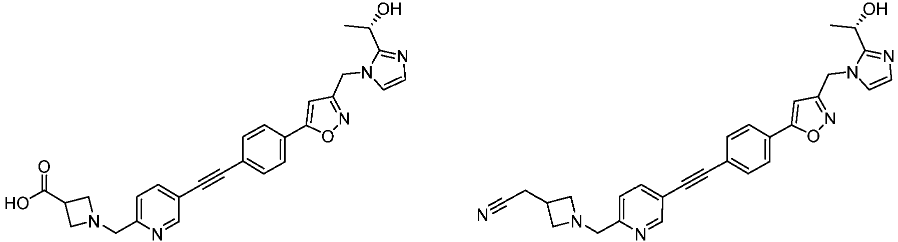 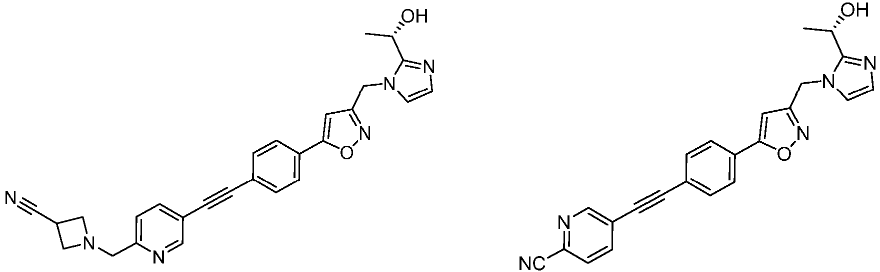 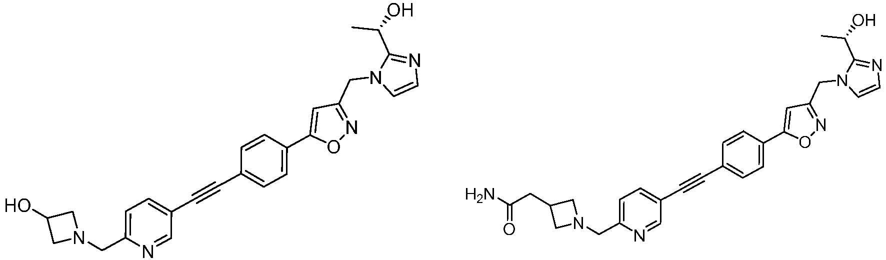 and

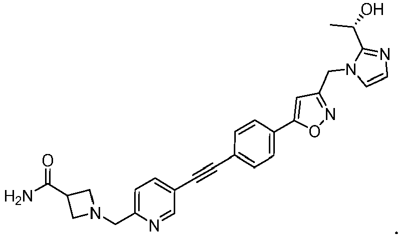

11. A method for producing the compound represented by general formula (A-I) or the stereoisomer, tautomer, or pharmaceutically acceptable salt thereof according to claim 1, comprising: 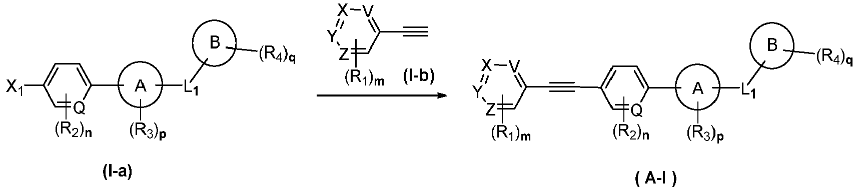
subjecting a compound represented by general formula (I-a) and a compound represented by general formula (I-b) to a coupling reaction under the action of a catalyst, and optionally further performing one or more steps of deprotection, hydrolysis, reduction, reductive amination or acid-amine condensation reaction to obtain the compound represented by general formula (A-I);
wherein:
X₁ is halogen; and
ring A, ring B, X, Y, Z, V, Q, R₁-R₄, L₁, n, m, p, and q are as defined in claim 1;
or 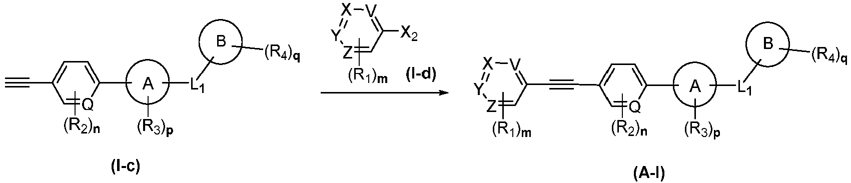
subjecting a compound represented by general formula (I-c) and a compound represented by general formula (I-d) to a coupling reaction under the action of a catalyst, and optionally further performing one or more steps of deprotection, hydrolysis, reduction, reductive amination or acid-amine condensation reaction to obtain the compound represented by general formula (A-I);
wherein:
X₂ is halogen; and
ring A, ring B, X, Y, Z, V, Q, R₁-R₄, L₁, n, m, p, and q are as defined in claim 1;
or 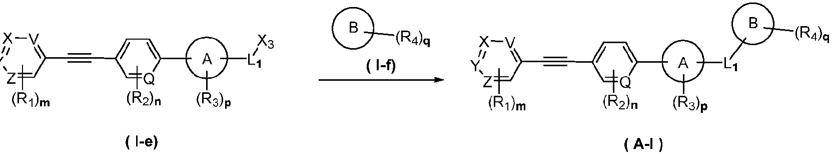
subjecting a compound represented by general formula (I-e) and a compound represented by (I-f) to a substitution reaction under the action of an alkaline reagent, and optionally further performing a deprotection reaction to obtain the compound represented by general formula (A-I);
wherein:
X₃ is halogen; and
ring A, ring B, X, Y, Z, V, Q, R₁-R₄, L₁, n, m, p, and q are as defined in claim 1.

12. A pharmaceutical composition, comprising the compound or the stereoisomer, tautomer, or pharmaceutically acceptable salt thereof according to any one of claims 1 to 10, and a pharmaceutically acceptable carrier.

13. A compound or the stereoisomer, tautomer, or pharmaceutically acceptable salt thereof according to any one of claims 1 to 10 for use in inhibiting LPXC.

14. A compound or the stereoisomer, tautomer, or pharmaceutically acceptable salt thereof according to any one of claims 1 to 10 for use in treating a disease mediated by LPXC, wherein the disease mediated by LPXC is a bacterial infection caused by a Gram-negative bacterium.

15. The compound or the stereoisomer, tautomer, or pharmaceutically acceptable salt thereof for use according to claim 14, wherein the Gram-negative bacterium is selected from the group consisting of *Escherichia coli, Pseudomonas aeruginosa, Proteus, Shigella dysenteriae, Klebsiella Pneumoniae, Brucella, Salmonella typhi, Acinetobacter, Yersinia, Legionella pneumophila, Bordetella pertussis, Shigella, Pasteurella, Vibrio cholerae,* and *Neisseria meningitidis.*

## Patentansprüche

1. Verbindung, die durch die allgemeine Formel (A-I) dargestellt ist, oder ein Stereoisomer, Tautomer oder pharmazeutisch akzeptables Salz davon: 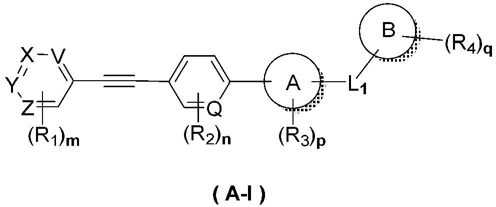 wobei:
Ring A ein 5- bis 6-gliedriges Heteroaryl oder 5- bis 6-gliedriges Heterocyclyl, vorzugsweise ein 5-gliedriges Heteroaryl oder 5-gliedriges Heterocyclyl ist;
Ring B ein 5- bis 10-gliedriges Heteroaryl ist;
Q C oder N ist;
X, Y, Z und V jeweils unabhängig voneinander C oder N sind, wobei X und Y nicht gleichzeitig N sind und Z und Y nicht gleichzeitig N sind;
R₁ gleich oder verschieden ist, wobei jedes unabhängig -G₁-R₅ ist;
G₁ aus der Gruppe ausgewählt ist, bestehend aus einer Einfachbindung, -O-, -CH₂- und -C(=O)-;
L₁ -(CH₂)ₛ-, vorzugsweise -CH₂- ist;
R₂ gleich oder verschieden ist, wobei jedes unabhängig aus der Gruppe ausgewählt ist, bestehend aus Hydroxyl, Cyano, Halogen, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl und C₆-C₁₀-Heteroaryl, wobei das C₁-C₆-Alkyl, C₃-C₇-Cycloalkyl, 5- bis 7-gliedrige monocyclische oder ein 7- bis 10-gliedriges bicyclisches oder tricyclisches Heterocyclyl, das C₆-C₁₀-Aryl oder C₆-C₁₀-Heteroaryl optional ferner mit einem oder mehreren Substituenten substituiert ist, ausgewählt aus der Gruppe bestehend aus Halogen, Hydroxyl, Cyano und C₁-C₆-Alkoxy;
R₃ gleich oder verschieden ist, wobei jedes unabhängig aus der Gruppe ausgewählt ist, bestehend aus Hydroxyl, Cyano, Halogen, C₁-C₆-Alkyl und C₁-C₆-Alkoxy;
alternativ zwei R₃ zusammen mit dem C-Atom, an das sie gebunden sind, - C(=O)- bilden;
R₄ gleich oder verschieden ist, wobei jedes unabhängig aus der Gruppe ausgewählt ist, bestehend aus Hydroxyl, Cyano, Halogen, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Heteroaryl, -C(O)R₆, -C(O)OR₆, - OC(O)R₆, -NR₇R₈, -C(O)NR₇R₈, -SO₂NR₇R₈ und -NR₇C(O)R₈, wobei das C₁-C₆-Alkyl, C₃-C₇-Cycloalkyl, 5- bis 7-gliedrige monocyclische oder ein 7- bis 10-gliedriges bicyclisches oder tricyclisches Heterocyclyl oder C₆-C₁₀-Heteroaryl optional ferner mit einem oder mehreren Substituenten substituiert ist, ausgewählt aus der Gruppe bestehend aus Halogen, Hydroxyl, Cyano, C₁-C₆-Alkoxy und Amino;
R₅ aus der Gruppe ausgewählt ist, bestehend aus Cyano, Halogen, C₁-C₆-Alkyl, Hydroxyl, C₁-C₆-Alkoxy, C₃-C₇ Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, -C(O)R₆, -C(O)OR₆, -OC(O)R₆, -NR₁₂R₁₃, -C(O)NR₁₂R₁₃, -SO₂R₆, -SO₂NR₁₂R₁₃ und - NR₁₂C(O)R₁₃, wobei das C₁-C₆-Alkoxy, C₁-C₆-Alkyl, C₃-C₇ Cycloalkyl, 5- bis 7-gliedrige monocyclische oder ein 7- bis 10-gliedriges bicyclisches oder tricyclisches Heterocyclyl, C₆-C₁₀-Aryl- oder C₆-C₁₀-Heteroaryl optional ferner mit einem oder mehreren R_{A} substituiert ist;
R_{A} aus der Gruppe ausgewählt ist, bestehend aus Halogen, Hydroxyl, Cyano, Hydroxy-C₁-C₆ -Alkyl, C₁-C₆-Alkoxy, C₁-C₆-Alkyl, C₂-C₄-Alkenyl, C₃-C₇ -Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, -C(O)R₆, -C(O)OR₆, -OC(O)R₆, NR₇R₈, - C(O)NR₇R₈, -SO₂NR₇R₈und -NR₇C(O)R₈, wobei das C₁-C₆-Alkyl, C₃-C₇-Cycloalkyl, 5- bis 7-gliedrige monocyclische oder ein 7- bis 10-gliedriges bicyclisches oder tricyclisches Heterocyclyl, C₆-C₁₀-Aryl- oder C₆-C₁₀-Heteroaryl optional mit einem oder mehreren Substituenten substituiert ist, ausgewählt aus der Gruppe bestehend aus Halogen, Hydroxyl, Cyano, Amino, Halogen C₁-C₆-Alkyl, Hydroxy-C₁-C₆-Alkyl, C₁-C₆-Alkoxy, C₁-C₆-Alkyl, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, -C(O)R₆, - C(O)OR₆, -OC(O)R6, -NR₇R8, -C(O)NR₇R₈, -SO₂NR7R₈ und -NR₇C(O)R₈;
alternativ zwei R_{A} zusammen mit demselben Kohlenstoffatom, an das sie gebunden sind, -C(O)- bilden;
R₆ aus der Gruppe ausgewählt ist, bestehend aus einem Wasserstoffatom, C₁-C₆-Alkyl, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl und C₆-C₁₀-Heteroaryl, wobei das C₁-C₆-Alkyl, C₃-C₇-Cycloalkyl, 5- bis 7-gliedrige monocyclische oder 7- bis 10-gliedrige bicyclische oder tricyclische Heterocyclyl, C₆-C₁₀-Aryl oder C₆-C₁₀-Heteroaryl optional ferner mit einem oder mehreren Substituenten substituiert ist, ausgewählt aus der Gruppe bestehend aus Hydroxyl, Halogen, Nitro, Cyano, C₁-C₆-Alkyl, C₁-C₆-Alkoxy, Halogen-C₁-C₆-Alkyl, Halogen-C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, =O, -C(O)R₉, -C(O)OR₉, -OC(O)R₉, -NR₁₀R₁₁, - C(O)NR₁₀R₁₁, -SO₂NR₁₀R₁₁ und -NR₁₀C(O)R₁₁;
R₇, R₈, R₁₂ und R₁₃ jeweils unabhängig voneinander aus der Gruppe ausgewählt sind, bestehend aus Wasserstoffatom, Hydroxyl, Halogen, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl und C₆-C₁₀-Heteroaryl, wobei das C₁-C₆-Alkyl, C₁-C₆-Alkoxy, C₃-C₇-Cycloalkyl, 5- bis 7-gliedrige monocyclische oder ein 7- bis 10-gliedriges bicyclisches oder tricyclisches Heterocyclyl, C₆-C₁₀-Aryl oder C₆-C₁₀-Heteroaryl optional ferner mit einem oder mehreren Substituenten substituiert ist, ausgewählt aus der Gruppe bestehend aus Hydroxyl, Halogen, Nitro, Cyano, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, =O, -C(O)R₉, -C(O)OR₉, -OC(O)R₉, -NR₁₀R₁₁, -C(O)NR₁₀R₁₁, -SO₂R₉, -SO₂NR₁₀R₁₁ und -NR₁₀C(O)R₁₁;
alternativ R₇ und R₈ zusammen mit dem Atom, an das sie gebunden sind, ein 4- bis 8-gliedriges Heterocyclyl bilden, wobei das 4- bis 8-gliedrige Heterocyclyl ein oder mehrere N, O, S oder SO₂ enthält und optional mit einem oder mehreren Substituenten substituiert ist, ausgewählt aus der Gruppe bestehend aus Hydroxyl, Halogen, Nitro, Cyano, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, =O, -C(O)R₉, -C(O)OR₉, -OC(O)R₉, -NR₁₀R₁₁, -C(O)NR₁₀R₁₁, -SO₂NR₁₀R₁₁ und - NR₁₀C(O)R₁₁;
alternativ R₁₂ und R₁₃ zusammen mit dem Atom, an das sie gebunden sind, ein 4- bis 8-gliedriges Heterocyclyl bilden, wobei das 4- bis 8-gliedrige Heterocyclyl ein oder mehrere N-, O-, S- oder SO₂ enthält und das 4- bis 8-gliedrige Heterocyclyl optional ferner mit einem oder mehreren Substituenten substituiert ist, ausgewählt aus der Gruppe bestehend aus Hydroxyl, Halogen, Nitro, Cyano, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, =O, -C(O)R₉, - C(O)OR₉, -OC(O)R₉, -NR₁₀R₁₁, -C(O)NR₁₀R₁₁, -SO₂NR₁₀R₁₁ und -NR₁₀C(O)R₁₁;
R₉, R₁₀ und R₁₁ jeweils unabhängig voneinander aus der Gruppe ausgewählt sind, bestehend aus einem Wasserstoffatom, C₁-C₆-Alkyl, Amino, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl und C₆-C₁₀-Heteroaryl, wobei das C₁-C₆-Alkyl, C₃-C₇-Cycloalkyl, 5- bis 7-gliedrige monocyclische oder ein 7- bis 10-gliedriges bicyclisches oder tricyclisches Heterocyclyl, C₆-C₁₀-Aryl oder C₆-C₁₀-Heteroaryl optional ferner mit einem oder mehreren Substituenten substituiert ist, ausgewählt aus der Gruppe bestehend aus Hydroxyl, Halogen, Nitro, Amino, Cyano, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, Carboxyl und einer Carboxylatgruppe;
m 0, 1, 2 oder 3 ist;
n 0, 1 oder 2, vorzugsweise 0 ist;
p 0, 1 oder 2 ist;
s 1 oder 2 ist; und
q 1, 2 oder 3 ist.

2. Verbindung oder das Stereoisomer, Tautomer oder pharmazeutisch akzeptable Salz davon nach Anspruch 1, die eine Verbindung, dargestellt durch die allgemeine Formel (I) oder ein Stereoisomer, Tautomer oder pharmazeutisch akzeptables Salz davon ist: 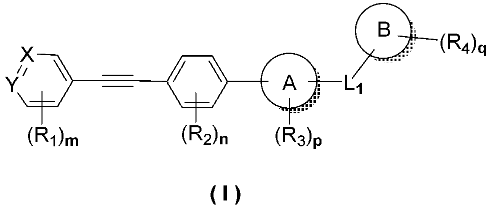 wobei:
X und Y jeweils unabhängig voneinander C oder N sind, wobei X und Y nicht gleichzeitig N sind;
R₁ gleich oder verschieden ist, wobei jedes unabhängig -G₁-R₅ ist;
G₁ aus der Gruppe ausgewählt ist, bestehend aus einer Einfachbindung, -CH₂- und -C(=O)-;
R₅ aus der Gruppe ausgewählt ist, bestehend aus Cyano, Halogen, C₁-C₆-Alkyl, Hydroxyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, -C(O)R₆, -C(O)OR₆, -OC(O)R₆, -NR₁₂R₁₃, -C(O)NR₁₂R₁₃, -SO₂NR₁₂R₁₃ und - NR₁₂C(O)R₁₃, wobei das C₁-C₆-Alkoxy, C₁-C₆-Alkyl, C₃-C₇-Cycloalkyl, 5- bis 7-gliedrige monocyclische oder ein 7- bis 10-gliedriges bicyclisches oder tricyclisches Heterocyclyl,C₆-C₁₀-Aryl oder C₆-C₁₀-Heteroaryl ferner mit einem oder mehreren R_{A}substituiert ist;
R_{A} aus der Gruppe ausgewählt ist, bestehend aus Halogen, Hydroxyl, Cyano, Hydroxy-C₁-C₆-Alkyl, C₁-C₆-Alkoxy, C₁-C₆-Alkyl, C₃-C₇-Cycloalkyl , 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, -C(O)R₆, -C(O)OR₆, -OC(O)R₆, -NR₇R₈, - C(O)NR₇R₈, -SO₂NR₇R₈ und -NR₇C(O)R₈, wobei das C₁-C₆ -Alkyl, C₃-C₇-Cycloalkyl, 5- bis 7-gliedrige monocyclische oder ein 7- bis 10-gliedriges bicyclisches oder tricyclisches Heterocyclyl, C₆-C₁₀-Aryl- oder C₆-C₁₀-Heteroaryl optional mit einem oder mehreren Substituenten substituiert ist, ausgewählt aus der Gruppe bestehend aus Halogen, Hydroxyl, Amino, Halogen-C₁-C₆-Alkyl, Hydroxy-C₁-C₆ -Alkyl, C₁-C₆-Alkoxy, C₁-C₆-Alkyl, C₃-C₇-Cycloalkyl, 5- bis 7-gliedrigem monocyclischen oder einem 7- bis 10-gliedrigem bicyclischen oder tricyclischen Heterocyclyl, C₆-C₁₀-Aryl-, C₆-C₁₀-Heteroaryl, -C(O)R₆, - C(O)OR₆, -OC(O)R₆, -NR₇R8, -C(O)NR₇R₈, -SO₂NR₇R₈ und -NR₇C(O)R₈;
R₇, R₈, R₁₂ und R₁₃ jeweils unabhängig voneinander aus der Gruppe, ausgewählt sind bestehend aus Wasserstoffatom, Hydroxyl, Halogen, C₁-C₆-Alkyl, C₁-C₆-Alkoxy, C₃-C₇-Cycloalkyl, 5- bis 7-gliedrigem monocyclischen oder einem 7- bis 10-gliedrigem bicyclischen oder tricyclischen Heterocyclyl, C₆-C₁₀-Aryl und C₆-C₁₀-Heteroaryl, wobei das C₁-C₆-Alkyl, C₁-C₆-Alkoxy, C₃-C₇-Cycloalkyl, 5- bis 7-gliedrige monocyclische oder ein 7- bis 10-gliedriges bicyclisches oder tricyclisches Heterocyclyl, C₆-C₁₀-Aryl oder C₆-C₁₀-Heteroaryl optional ferner mit einem oder mehreren Substituenten substituiert ist, ausgewählt aus der Gruppe bestehend aus Hydroxyl, Halogen, Nitro, Cyano, C₁-C₆-Alkyl, C₁-C₆-Alkoxy, C₃-C₇ Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, =O, -C(O)R₉, -C(O)OR₉, -OC(O)R₉, -NR₁₀R₁₁, -C(O)NR₁₀R₁₁, -SO₂NR₁₀R₁₁ und -NR₁₀C(O)R₁₁;
alternativ R₇ und R₈ zusammen mit dem Atom, an das sie gebunden sind, ein 4- bis 8-gliedriges Heterocyclyl bilden, wobei das 4- bis 8-gliedrige Heterocyclyl ein oder mehrere N, O, S oder SO₂ enthält und optional mit einem oder mehreren Substituenten substituiert ist, ausgewählt aus der Gruppe bestehend aus Hydroxyl, Halogen, Nitro, Cyano, C₁-C₆-Alkyl, C₁-C₆-Alkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, =O, -C(O)R₉, -C(O)OR₉, -OC(O)R₉, -NR₁₀R₁₁, -C(O)NR₁₀R₁₁, -SO₂NR₁₀R₁₁ und - NR₁₀C(O)R₁₁;
alternativ R₁₂ und R₁₃ zusammen mit dem Atom, an das sie gebunden sind, ein 4- bis 8-gliedriges Heterocyclyl bilden, wobei das 4- bis 8-gliedrige Heterocyclyl ein oder mehrere N-, O-, S- oder SO₂ enthält und optional mit einem oder mehreren Substituenten substituiert ist, ausgewählt aus der Gruppe bestehend aus Hydroxyl-, Halogen-, Nitro-, Cyano-, C₁-C₆-Alkyl, C₁-C₆-Allkoxy, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, C₆-C₁₀-Heteroaryl, =O, -C(O)R₉, -C(O)OR₉, -OC(O)R₉, -NR₁₀R₁₁, - C(O)NR₁₀R₁₁, -SO₂NR₁₀R₁₁ und -NR₁₀C(O)R₁₁; und
Ring A, Ring B, R₂-R₄, R₆, R₉-R₁₁, L₁, m, n, p und q nach Anspruch 1 definiert sind.

3. Verbindung oder das Stereoisomer, Tautomer oder pharmazeutisch akzeptable Salz davon nach Anspruch 1, die eine Verbindung, dargestellt durch allgemeine Formel (II-1), (II-2), (II-3), (II-4) oder (II-5), oder ein Stereoisomer, Tautomer oder pharmazeutisch akzeptables Salz davon ist: 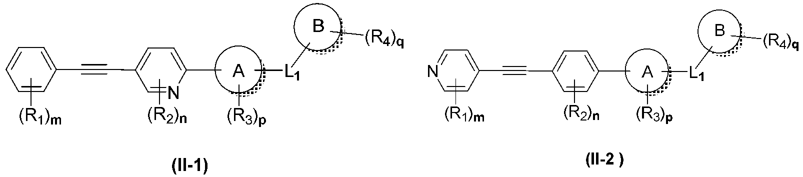 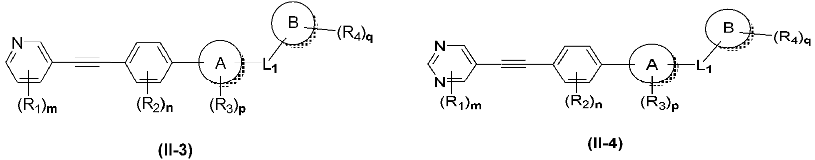 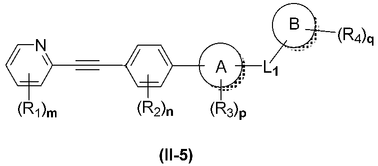 wobei Ring A, Ring B, R₁-R₄, L₁, m, n, p und q nach Anspruch 1 definiert sind.

4. Verbindung oder das Stereoisomer, Tautomer oder pharmazeutisch akzeptable Salz davon nach einem der Ansprüche 1 bis 3, die eine Verbindung, dargestellt durch allgemeine Formel (III-1) oder (III-2), oder ein Stereoisomer, Tautomer oder pharmazeutisch akzeptables Salz davon ist: 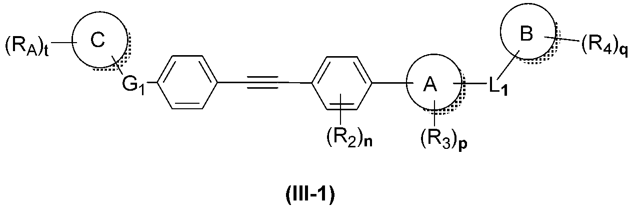 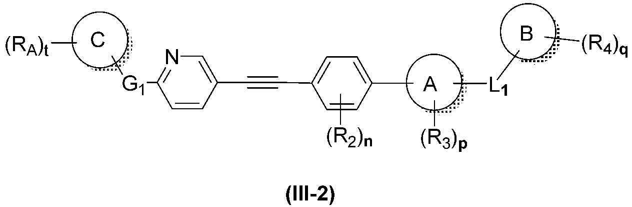 wobei:
Ring C aus der Gruppe ausgewählt ist, bestehend aus C₄-C₈-Cycloalkyl, 5- bis 6-gliedrigem Heteroaryl und 4- bis 8-gliedrigem Heterocyclyl;
R_{A} gleich oder verschieden ist, wobei jedes unabhängig aus der Gruppe ausgewählt ist, bestehend aus Halogen-C₁-C₆-Alkyl, Hydroxy-C₁-C₆-Alkyl, C₁-C₆-Alkoxy, C₁-C₆-Alkyl, C₂-C₄-Alkenyl, Hydroxyl, Halogen, Cyano, -C(O)NH₂, C₃-C₇-Cycloalkyl, 5- bis 7-gliedrigem monocyclischen oder einem 7- bis 10-gliedrigen bicyclischen oder tricyclischen Heterocyclyl, C₆-C₁₀-Aryl, V₆-C₁₀₋-Heteroaryl und Carboxyl, wobei das C₁-C₆-Alkyl, C₃-C₇₋-Cycloalkyl, 5- bis 7-gliedrige monocyclische oder ein 7- bis 10-gliedriges bicyclisches oder tricyclisches Heterocyclyl, C₆-C₁₀-Aryl oder C₆-C₁₀-Heteroaryl optional ferner mit einem Substituenten substituiert ist, ausgewählt aus der Gruppe bestehend aus Hydroxyl, Cyano, Carboxyl und Ester;
alternativ zwei RA_{A} gemeinsam mit demselben Kohlenstoffatom, an das sie gebunden sind, -C(O)- bilden; wobei t 0, 1, 2 oder 3 ist; und
Ring A, Ring B, R₂-R₄, G₁, L₁, n, p und q nach Anspruch 1 definiert sind.

5. Verbindung oder das Stereoisomer, Tautomer oder pharmazeutisch akzeptable Salz davon nach einem der Ansprüche 1 bis 4, wobei Ring A aus der Gruppe ausgewählt ist, bestehend aus: 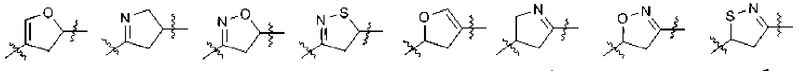 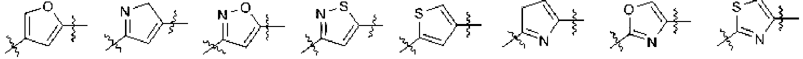 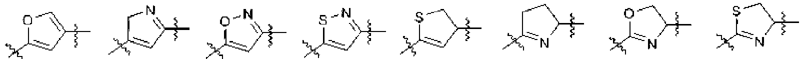 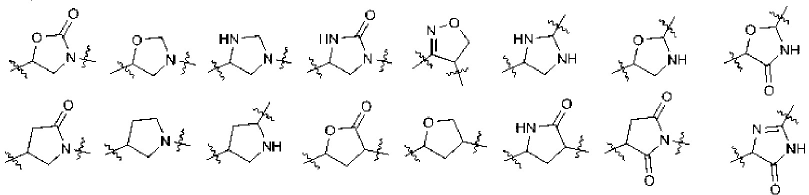 un

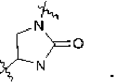

6. Verbindung oder das Stereoisomer, Tautomer oder pharmazeutisch akzeptable Salz davon nach einem der Ansprüche 1 bis 4, wobei Ring B aus der Gruppe ausgewählt ist, bestehend aus:

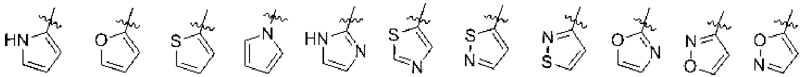

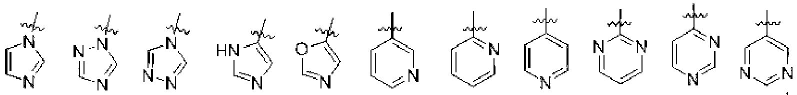

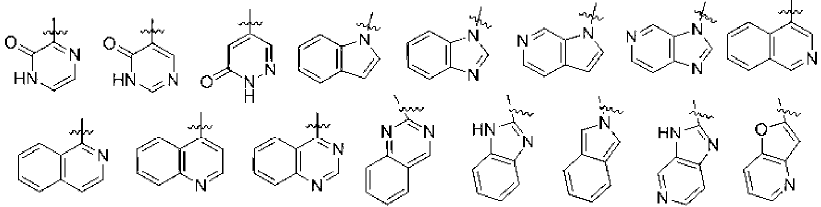

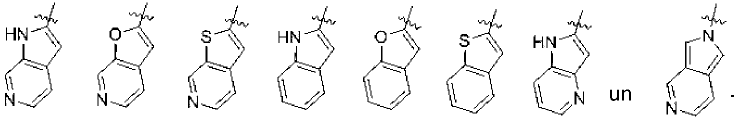

7. Verbindung oder das Stereoisomer, Tautomer oder pharmazeutisch akzeptable Salz davon nach einem der Ansprüche 4 bis 6, wobei Ring C aus der Gruppe ausgewählt ist, bestehend aus:

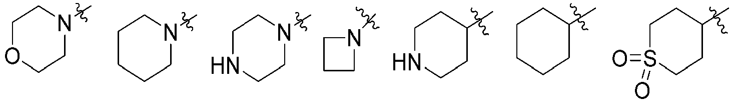

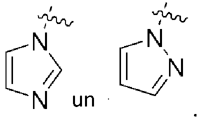

8. Verbindung oder das Stereoisomer, Tautomer oder ein pharmazeutisch akzeptables Salz davon nach einem der Ansprüche 1 bis 7, wobei:
R₄ aus der Gruppe ausgewählt ist, bestehend aus C₁-C₆-Alkyl, -C(O)R₆, -C(O)OR₆ und -C(O)NR₇R₈, wobei das C₁-C₆-Alkyl optional ferner mit einem oder mehreren Hydroxyl- oder Halogensubstituenten substituiert ist;
R₆ aus der Gruppe ausgewählt ist, bestehend aus einem Wasserstoffatom, Hydroxyl, C₁-C₆-Alkyl und C₁-C₆-Alkoxy; und
R₇ und R_{8'} jeweils unabhängig voneinander Wasserstoff oder C₁-C₆-Alkyl sind;

9. Verbindung oder das Stereoisomer, Tautomer oder pharmazeutisch akzeptable Salz davon nach einem der Ansprüche 1 bis 8, wobei R₄ aus der Gruppe ausgewählt ist, bestehend aus:

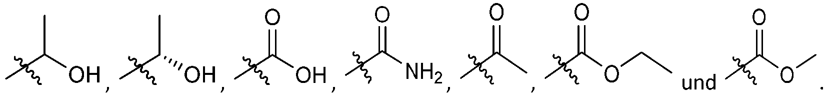

10. Verbindung oder das Stereoisomer, Tautomer oder pharmazeutisch akzeptable Salz davon nach einem der Ansprüche 1 bis 9, wobei die Verbindung aus der Gruppe ausgewählt ist, bestehend aus:

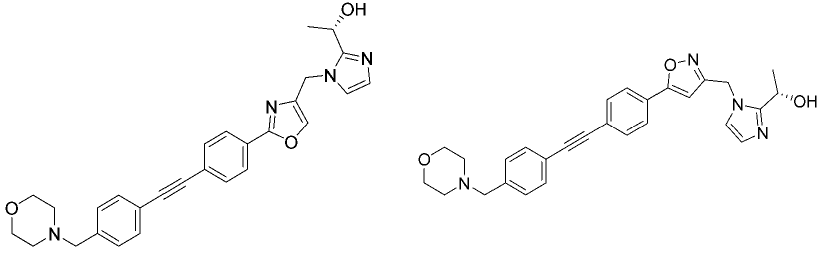

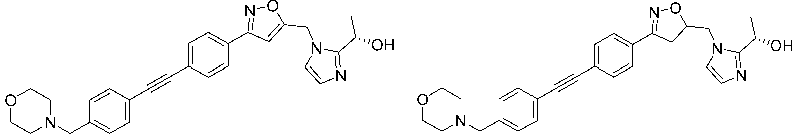

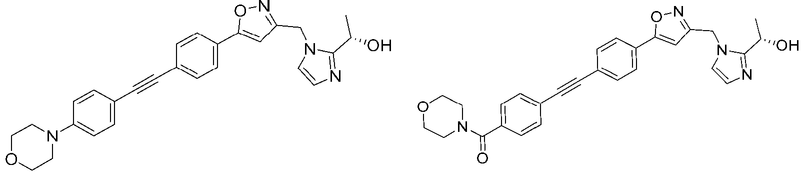

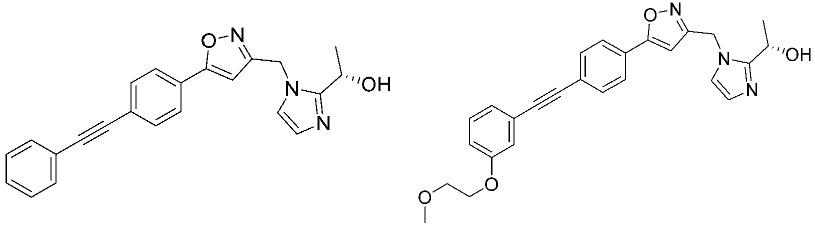

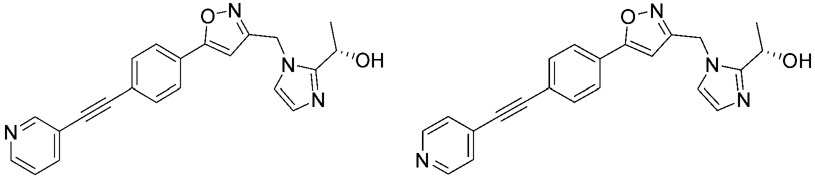

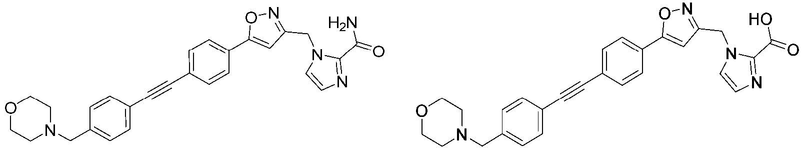

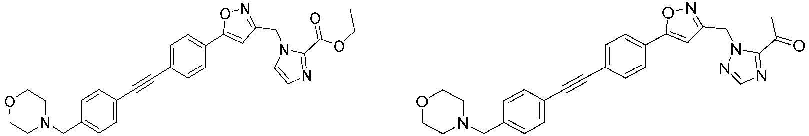

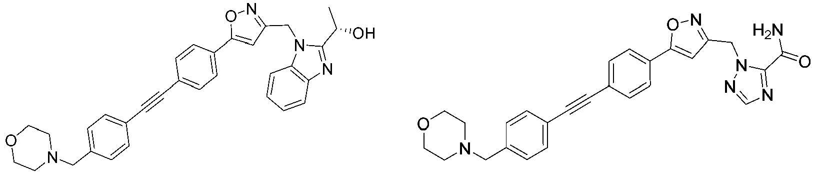

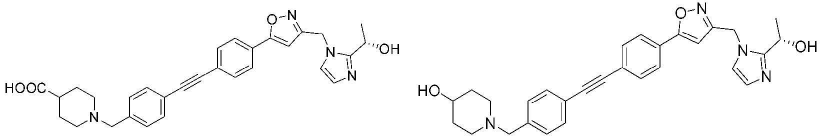

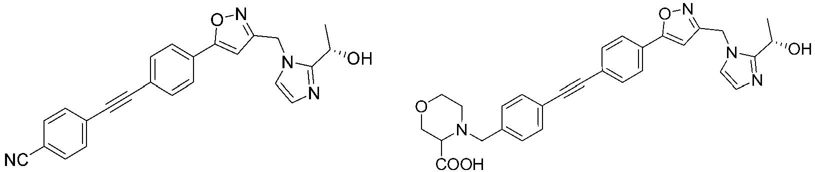

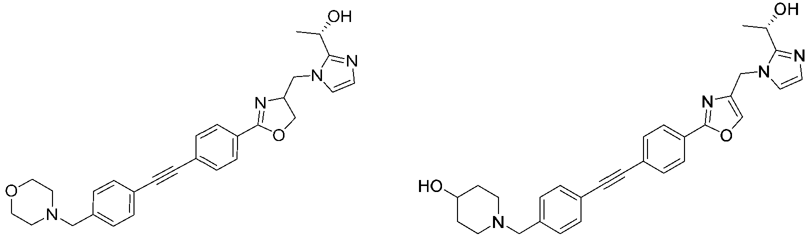

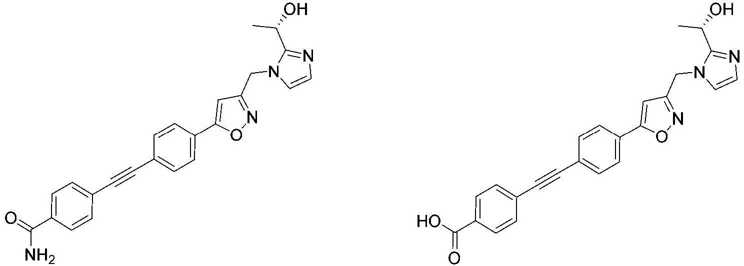

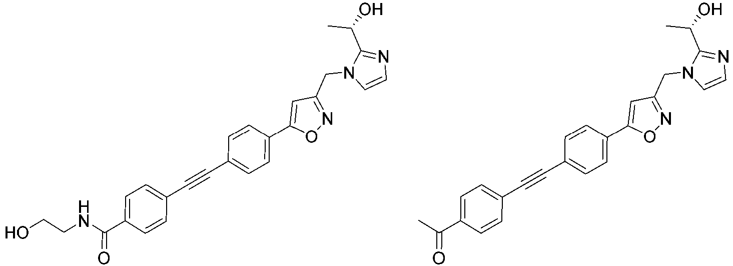

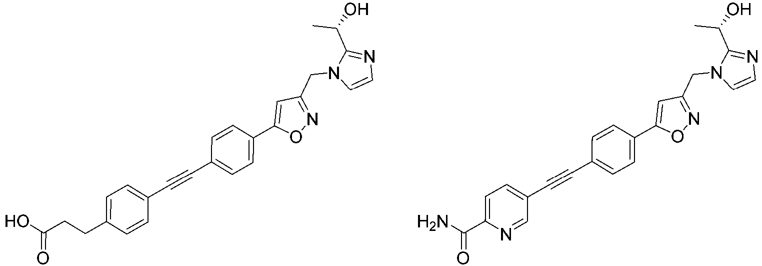

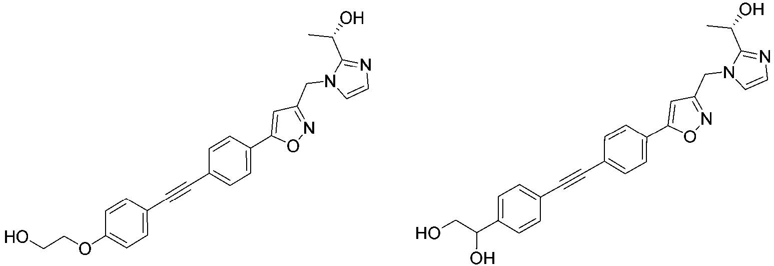

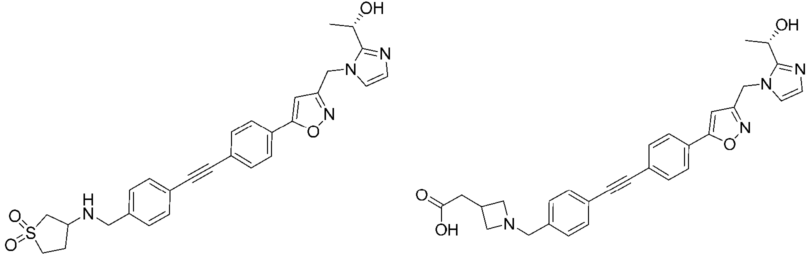

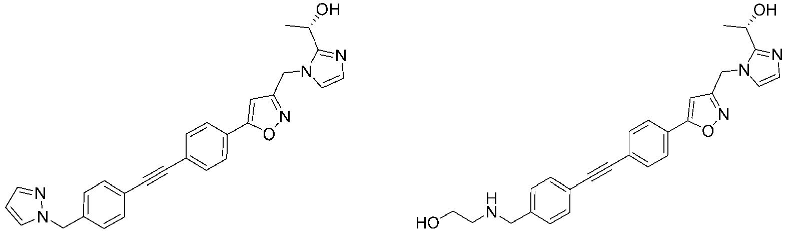

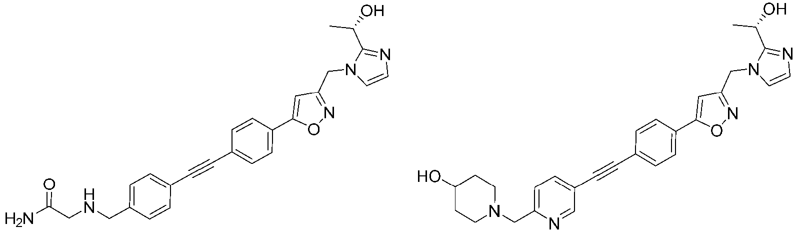

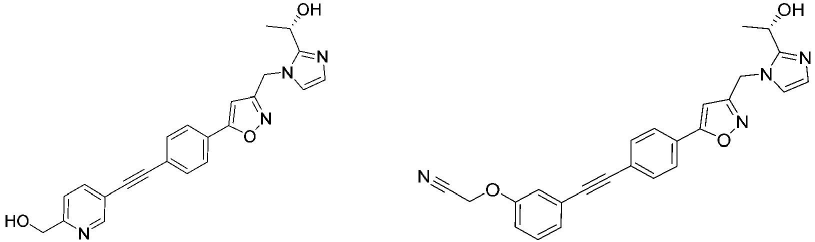

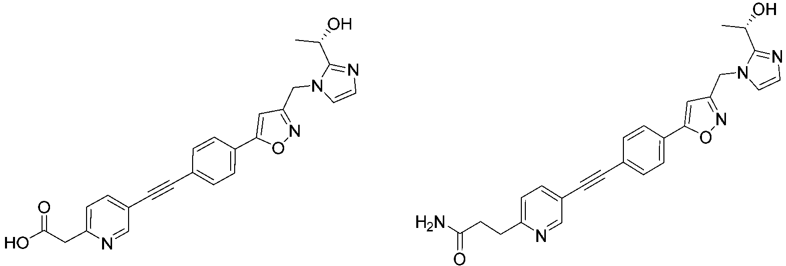

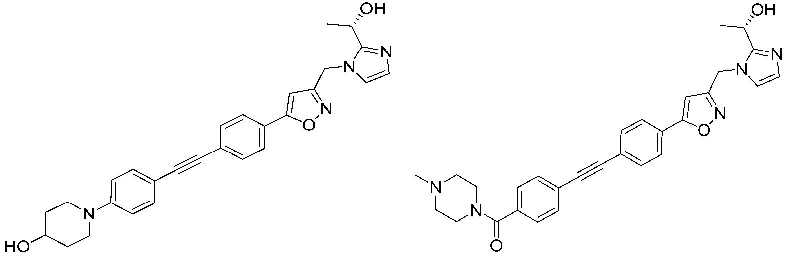

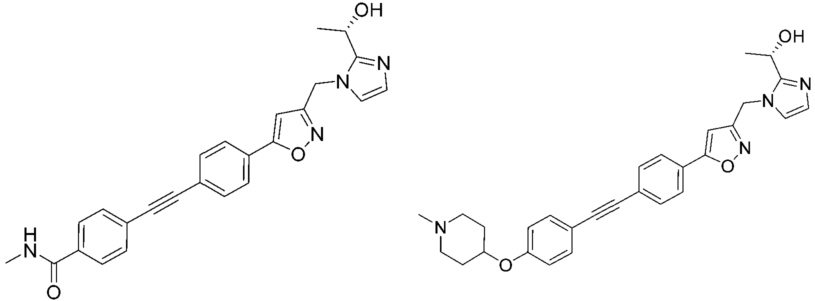

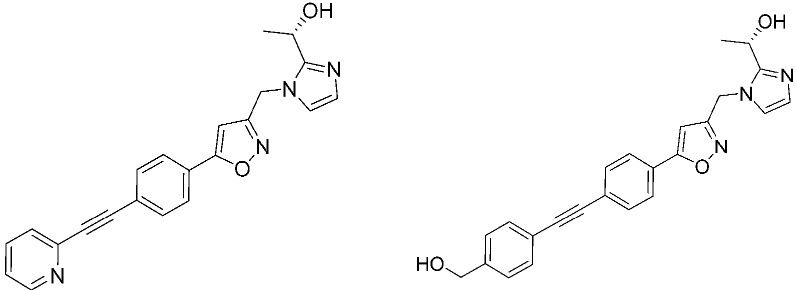

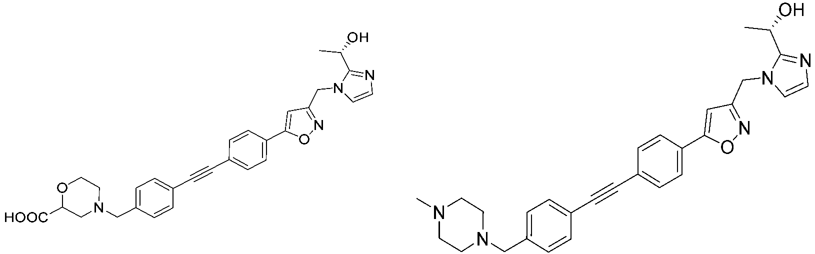

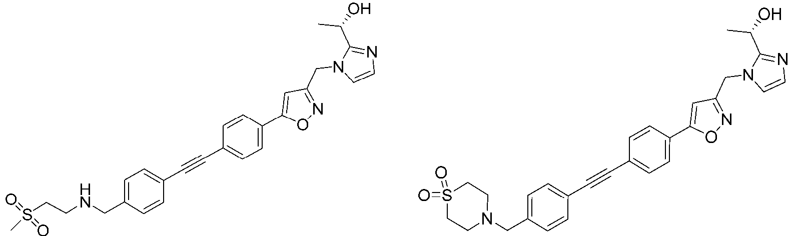

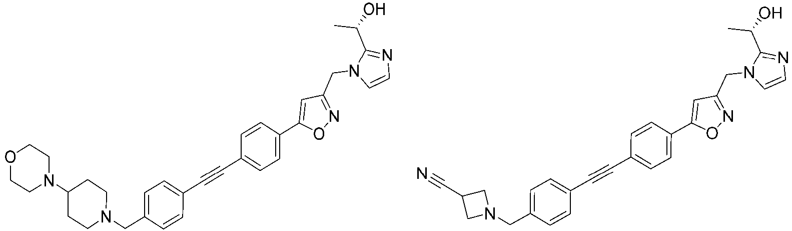

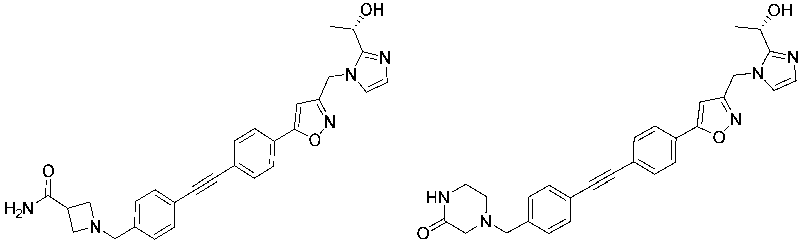

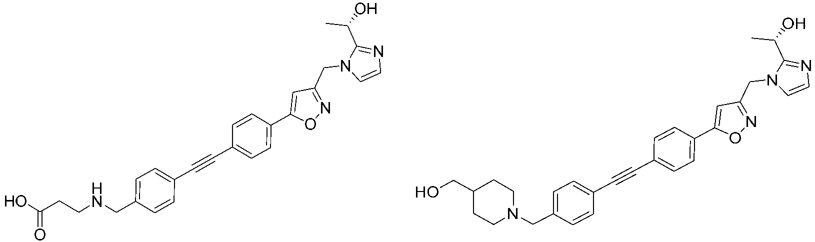

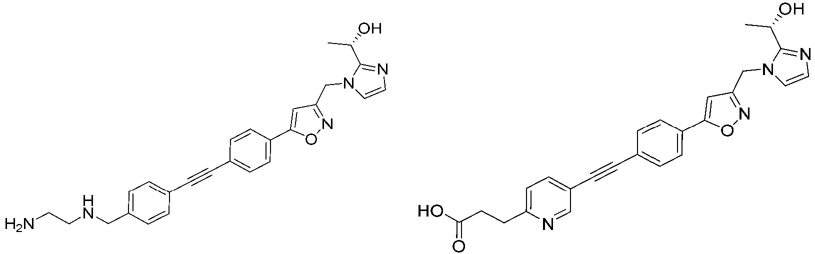

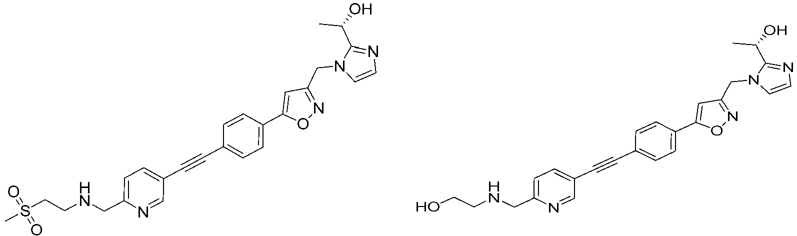

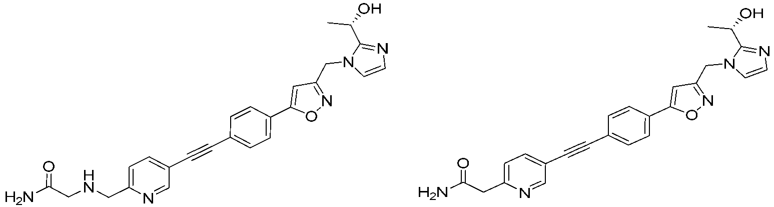

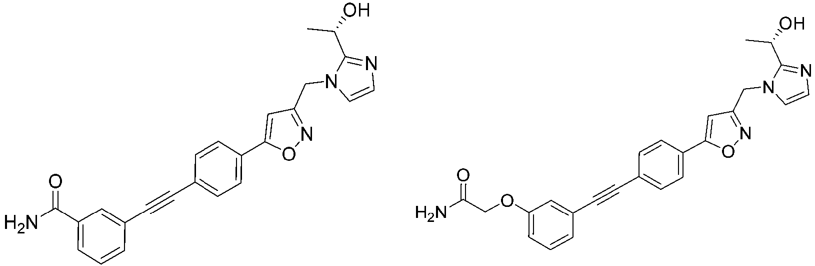

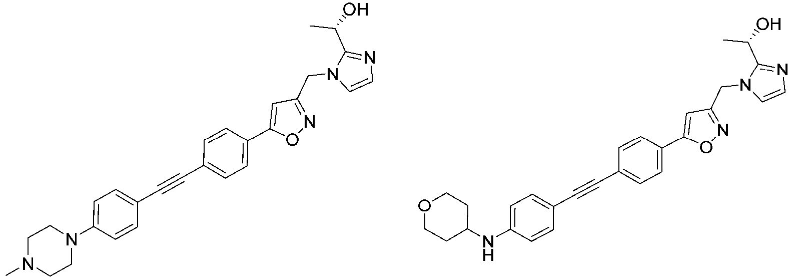

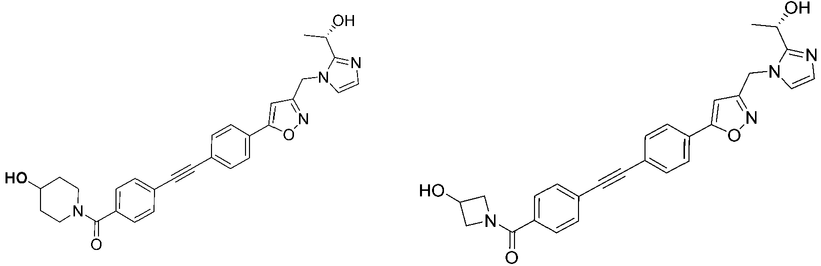

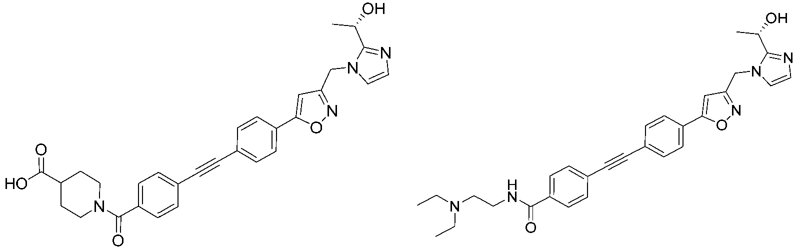

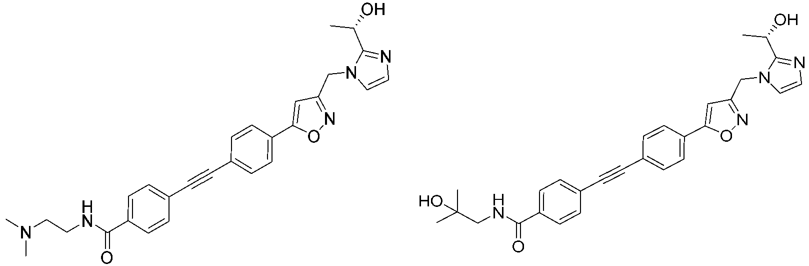

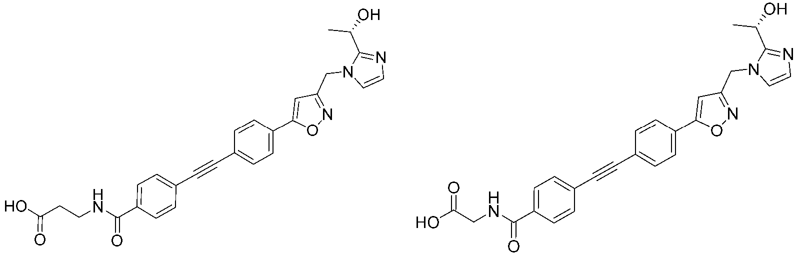

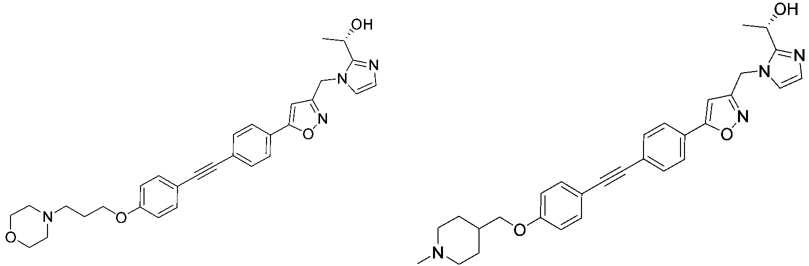

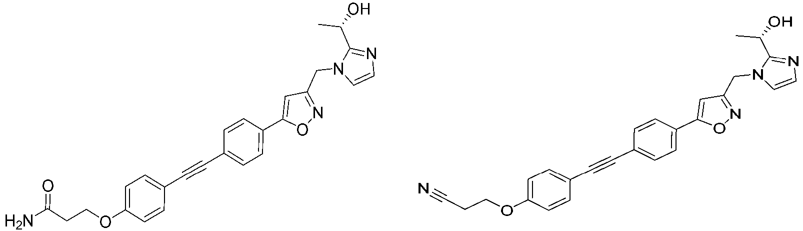

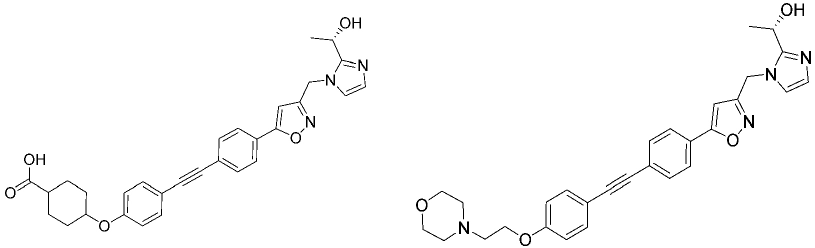

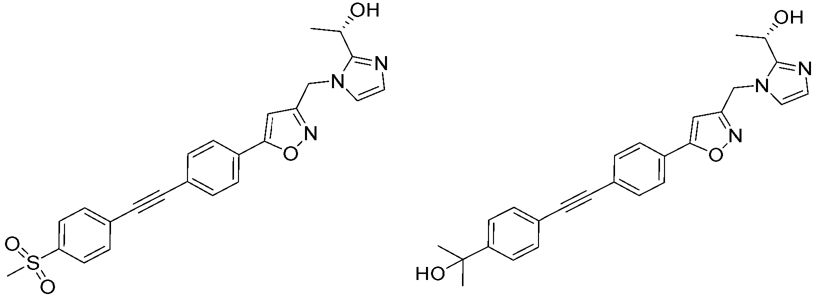

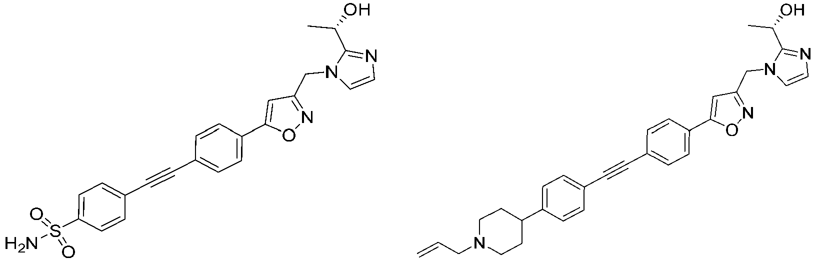

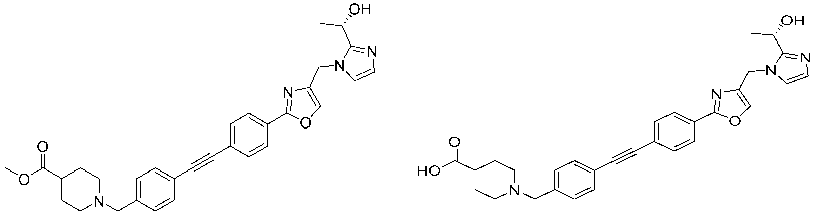

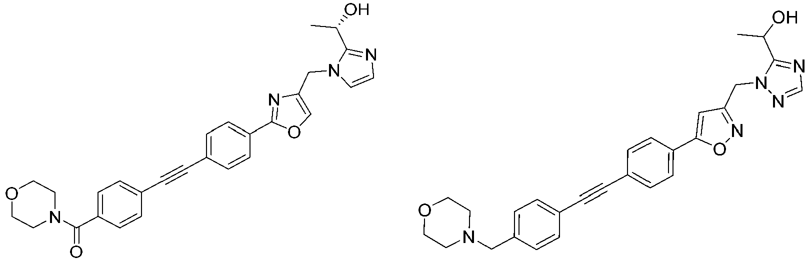

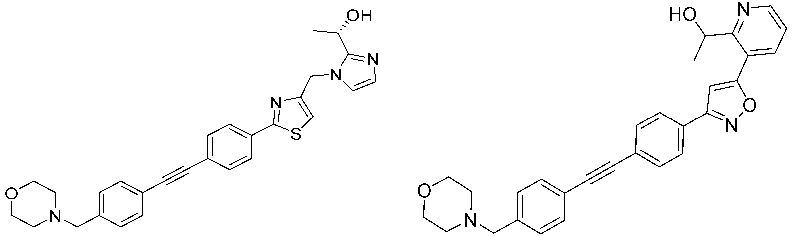

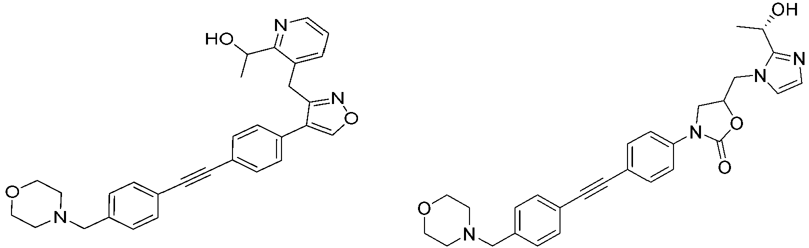

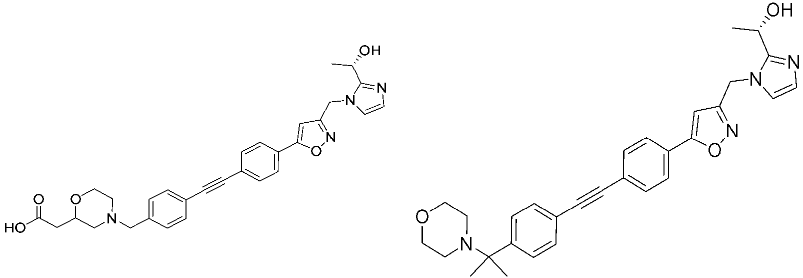

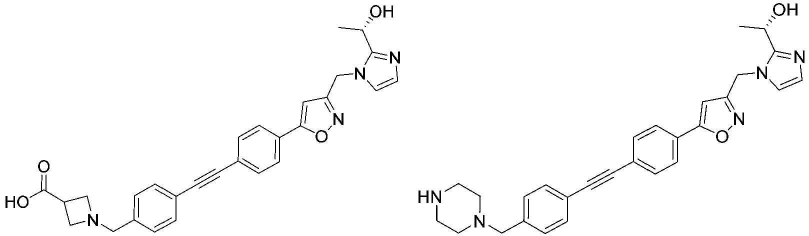

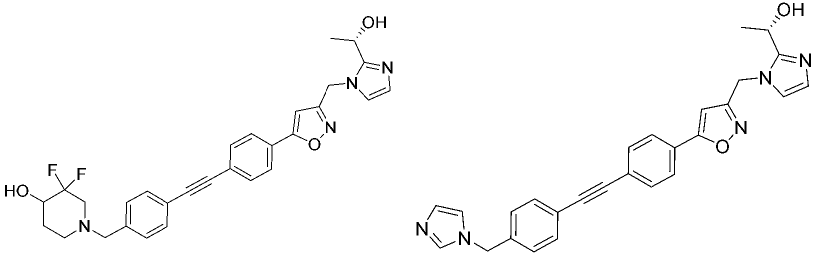

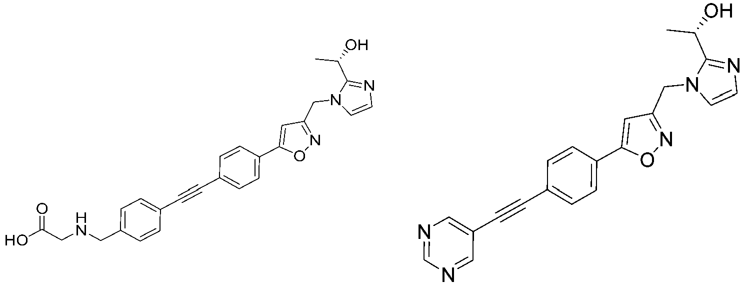

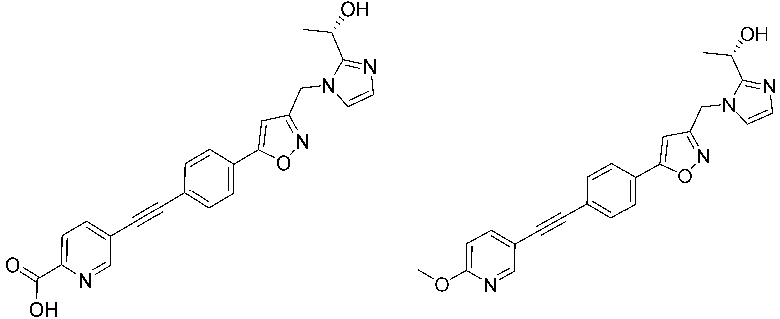

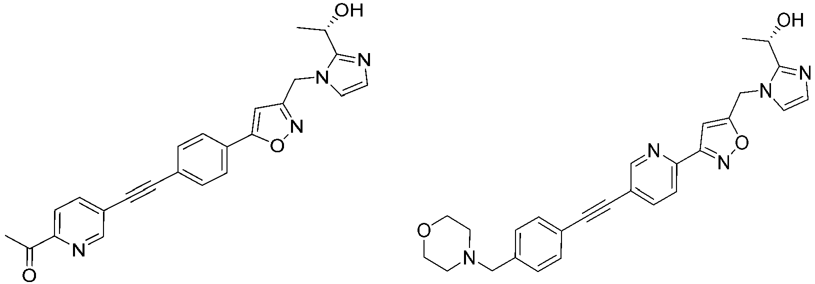

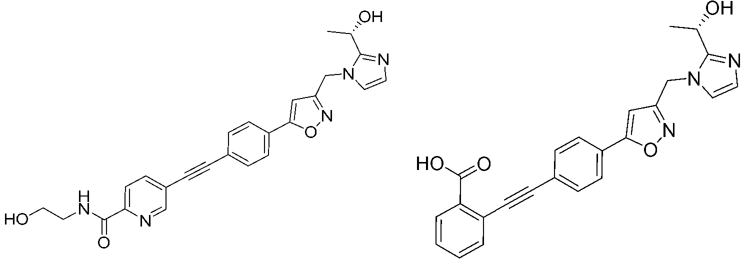

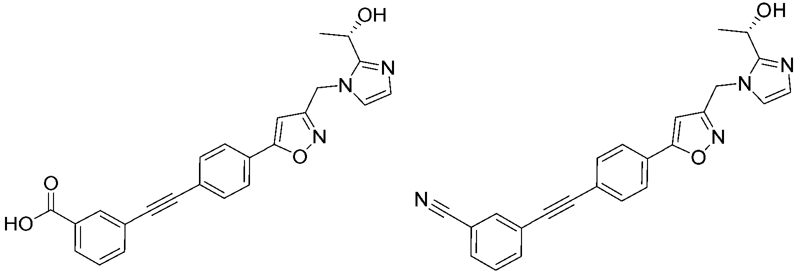

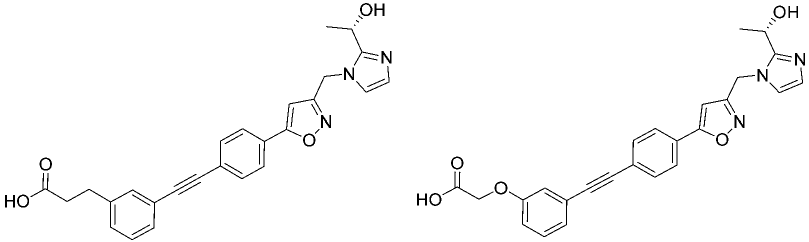

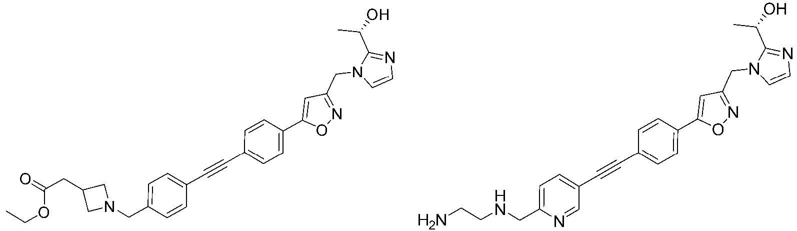

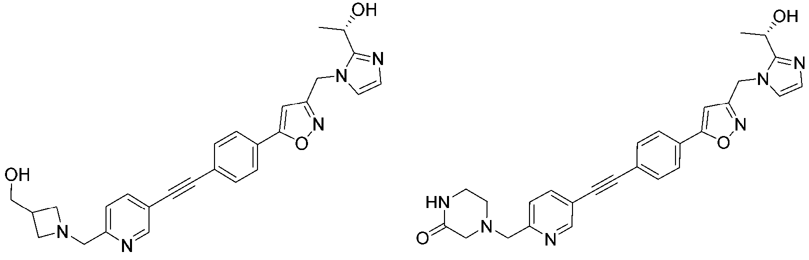

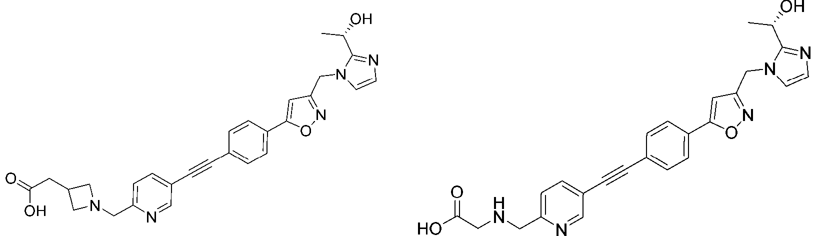

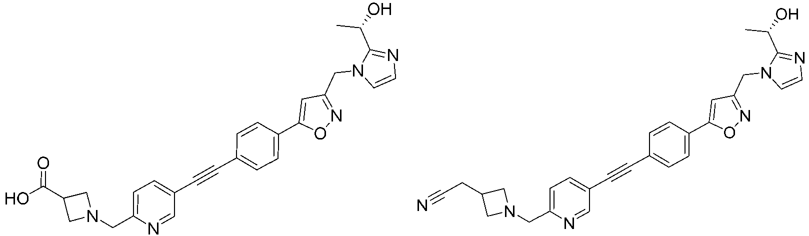

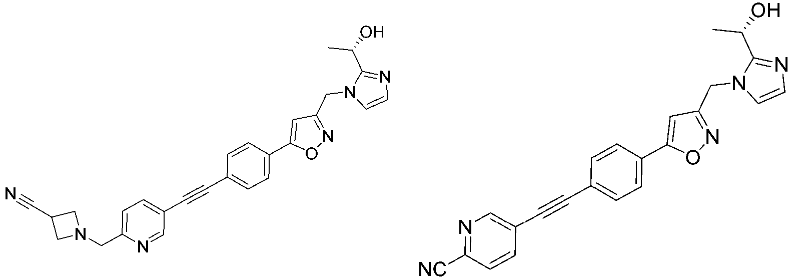

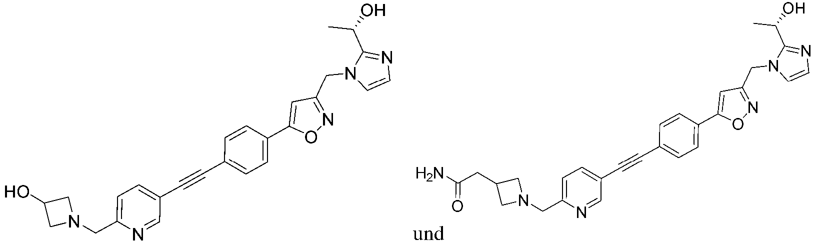

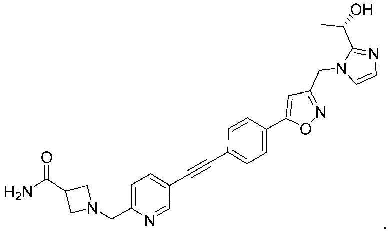

11. Verfahren zur Herstellung der durch die allgemeine Formel (A-I) dargestellten Verbindung oder des Stereoisomers, Tautomers oder pharmazeutisch akzeptablen Salzes davon nach Anspruch 1, umfassend: 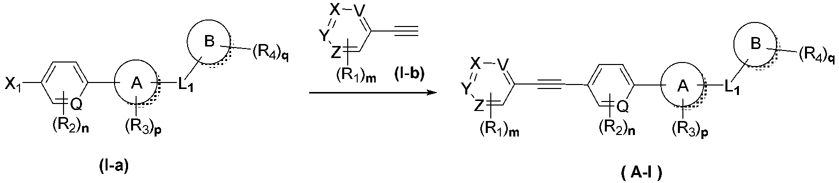
Unterziehen einer Verbindung, die durch allgemeine Formel (I-a) dargestellt ist, und einer Verbindung, die durch allgemeine Formel (I-b) dargestellt ist, einer Kupplungsreaktion unter Einwirkung eines Katalysators und optional ferner Durchführen eines oder mehrerer Schritte der Entschützung, Hydrolyse, Reduktion, reduktiven Aminierung oder Säure-Amin-Kondensationsreaktion, um die Verbindung zu erhalten, die durch die allgemeine Formel (A-I) dargestellt ist;
wobei:
X₁ Halogen ist; und
Ring A, Ring B, X, Y, Z, V, Q, R₁-R₄, L₁, n, m, p und q nach Anspruch 1 definiert sind;
oder 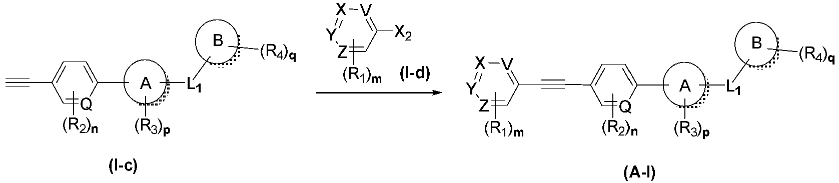
Unterziehen einer Verbindung, die durch allgemeine Formel (I-c) dargestellt ist, und einer Verbindung, die durch allgemeine Formel (I-d) dargestellt ist, einer Kupplungsreaktion unter Einwirkung eines Katalysators und optional ferner Durchführen eines oder mehrerer Schritte der Entschützung, Hydrolyse, Reduktion, reduktiven Aminierung oder Säure-Amin-Kondensationsreaktion, um die Verbindung zu erhalten, die durch allgemeine Formel (A-I) dargestellt ist;
wobei:
X₂ Halogen ist; und
Ring A, Ring B, X, Y, Z, V, Q, R₁-R₄, L₁, n, m, p und q nach Anspruch 1 definiert sind;
oder 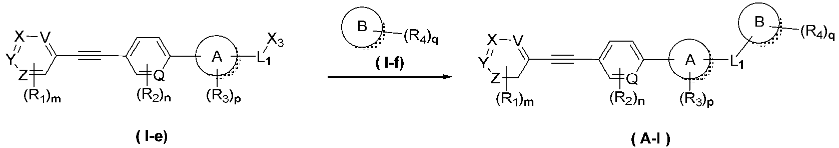
Unterziehen einer Verbindung, die durch allgemeine Formel (I-e) dargestellt ist, und einer Verbindung, die durch allgemeine Formel (I-f) dargestellt ist, einer Substitutionsreaktion unter Einwirkung eines alkalischen Reagenz und optional ferner Durchführen einer Entschützungsreaktion, um die Verbindung zu erhalten, die durch allgemeine Formel (A-I) dargestellt ist;
wobei:
X₃ Halogen ist; und
Ring A, Ring B, X, Y, Z, V, Q, R₁-R₄, L₁, n, m, p und q nach Anspruch 1 definiert sind.

12. Pharmazeutische Zusammensetzung, umfassend die Verbindung oder das Stereoisomer, Tautomer oder pharmazeutisch akzeptable Salz davon nach einem der Ansprüche 1 bis 10 und einen pharmazeutisch akzeptablen Träger.

13. Verbindung oder das Stereoisomer, Tautomer oder pharmazeutisch akzeptable Salz davon nach einem der Ansprüche 1 bis 10 zur Verwendung bei der Hemmung von LPXC.

14. Verbindung oder das Stereoisomer, Tautomer oder pharmazeutisch akzeptable Salz davon nach einem der Ansprüche 1 bis 10 zur Verwendung bei der Behandlung einer durch LPXC vermittelten Krankheit, wobei die durch LPXC vermittelte Krankheit eine bakterielle Infektion ist, die durch ein gramnegatives Bakterium verursacht wird.

15. Verbindung oder das Stereoisomer, Tautomer oder pharmazeutisch akzeptable Salz davon zur Verwendung nach Anspruch 14, wobei das gramnegative Bakterium aus der Gruppe ausgewählt ist, bestehend aus *Escherichia coli, Pseudomonas aeruginosa, Proteus, Shigella dysenteriae, Klebsiella Pneumoniae, Brucella, Salmonella typhi, Acinetobacter, Yersinia, Legionella pneumophila, Bordetella pertussis, Shigella, Pasteurella, Vibrio choleraeund Neisseria meningitidis.*

## Revendications

1. Composé représenté par la formule générale (A-I) ou stéréoisomère, tautomère ou sel pharmaceutiquement acceptable de celui-ci : 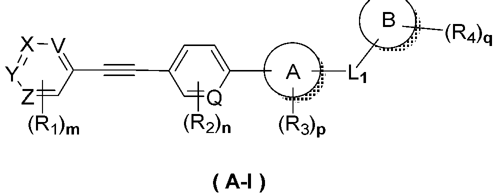 dans lequel :
le cycle A est hétéroaryle de 5 ou 6 chaînons ou hétérocyclyle de 5 ou 6 chaînons, de préférence hétéroaryle de 5 chaînons ou hétérocyclyle de 5 chaînons ;
le cycle B est hétéroaryle de 5 à 10 chaînons ;
Q est C ou N ;
X, Y, Z et V sont chacun indépendamment C ou N, dans lequel X et Y ne sont pas simultanément N, et Z et Y ne sont pas simultanément N ;
R₁ est identique ou différent, chacun étant indépendamment -G₁-R₅ ;
G₁ est sélectionné dans le groupe consistant en une liaison simple, -O-, -CH₂- et -C(=O)- ;
L₁ est -(CH₂)ₛ-, de préférence -CH₂- ;
R₂ est identique ou différent, chacun étant sélectionné indépendamment dans le groupe consistant en hydroxyle, cyano, halogène, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ et hétéroaryle en C₆-C₁₀, dans lequel alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs substituants sélectionnés dans le groupe consistant en halogène, hydroxyle, cyano et alcoxy en C₁-C₆ ;
R₃ est identique ou différent, chacun étant sélectionné indépendamment dans le groupe consistant en hydroxyle, cyano, halogène, alkyle en C₁-C₆ et alcoxy en C₁-C₆ ;
alternativement, deux R₃ forment -C(=O)- conjointement avec l'atome C auquel ils sont liés ;
R₄ est identique ou différent, chacun étant sélectionné indépendamment dans le groupe consistant en hydroxyle, cyano, halogène, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, hétéroaryle en C₆-C₁₀, -C(O)R₆, -C(O)OR₆, -OC(O)R₆, -NR₇R₈, -C(O)NR₇R₈, -SO₂NR₇R₈ et -NR₇C(O)R₈, dans lequel alkyle en C₁-C₆, cycloalkyle en C₃-C₇, hétérocyclyle monocyclique de 5 à 7 chaînons ou bicyclique ou tricyclique de 7 à 10 chaînons ou hétéroaryle en C₆-C₁₀ est en outre éventuellement substitué par un ou plusieurs substituants sélectionnés dans le groupe consistant en halogène, hydroxyle, cyano, alcoxy en C₁-C₆ et amino ;
R₅ est sélectionné dans le groupe consistant en cyano, halogène, alkyle en C₁-C₆, hydroxyle, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, -C(O)R₆, -C(O)OR₆, -OC(O)R₆, -NR₁₂R₁₃, -C(O)NR₁₂R₁₃, -SO₂R₆, -SO₂NR₁₂R₁₃ et - NR₁₂C(O)R₁₃, dans lequel alcoxy en C₁-C₆, alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs R_{A};
R_{A} est sélectionné dans le groupe consistant en halogène, hydroxyle, cyano, hydroxyalkyle en C₁-C₆, alcoxy en C₁-C₆, alkyle en C₁-C₆, alcényle en C₂-C₄, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, -C(O)R₆, -C(O)OR₆, -OC(O)R₆, -NR₇R₈, - C(O)NR₇R₈, -SO₂NR₇R₈ et -NR₇C(O)R₈, dans lequel alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, ou hétéroaryle en C₆-C₁₀ est en outre éventuellement substitué par un ou plusieurs substituants sélectionnés dans le groupe consistant en halogène, hydroxyle, cyano, amino, haloalkyle en C₁-C₆, hydroxyalkyle en C₁-C₆, alcoxy en C₁-C₆, alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, -C(O)R₆, -C(O)OR₆, -OC(O)R₆, -NR₇R₈, -C(O)NR₇R₈, -SO₂NR₇R₈ et -NR₇C(O)R₈ ;
alternativement, deux R_{A} forment -C(O)- conjointement avec le même atome Carbone auquel ils sont liés ;
R₆ est sélectionné dans le groupe consistant en un atome d'hydrogène, alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ et hétéroaryle en C₆-C₁₀, dans lequel alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs substituants sélectionnés dans le groupe consistant en hydroxyle, halogène, nitro, cyano, alkyle en C₁-C₆, alcoxy en C₁-C₆, haloalkyle en C₁-C₆, haloalcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, =O, -C(O)R₉, -C(O)OR₉, -OC(O)R₉, -NR₁₀R₁₁, -C(O)NR₁₀R₁₁, -SO₂NR₁₀R₁₁ et -NR₁₀C(O)R₁₁;
R₇, R₈, R₁₂ et R₁₃ sont chacun indépendamment sélectionnés dans le groupe consistant en un atome d'hydrogène, hydroxyle, halogène, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ et hétéroaryle en C₆-C₁₀, dans lequel alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs substituants sélectionnés dans le groupe consistant en hydroxyle, halogène, nitro, cyano, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, =O, -C(O)R₉, -C(O)OR₉, -OC(O)R₉, -NR₁₀R₁₁, -C(O)NR₁₀R₁₁, -SO₂R₉, -SO₂NR₁₀R₁₁ et -NR₁₀C(O)R₁₁;
alternativement, R₇ et R₈ forment hétérocyclyle de 4 à 8 chaînons conjointement avec l'atome auquel ils sont liés, dans lequel hétérocyclyle de 4 à 8 chaînons contient un ou plusieurs N, O, S ou SO₂, et hétérocyclyle de 4 à 8 chaînons est en outre éventuellement substitué par un ou plusieurs substituants sélectionnés dans le groupe consistant en hydroxyle, halogène, nitro, cyano, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, =O, -C(O)R₉, -C(O)OR₉, -OC(O)R₉, -NR₁₀R₁₁, -C(O)NR₁₀R₁₁, -SO₂NR₁₀R₁₁ et -NR₁₀C(O)R₁₁;
alternativement, R₁₂ et R₁₃ forment hétérocyclyle de 4 à 8 chaînons conjointement avec l'atome auquel ils sont liés, dans lequel hétérocyclyle de 4 à 8 chaînons contient un ou plusieurs N, O, S ou SO₂, et hétérocyclyle de 4 à 8 chaînons est en outre éventuellement substitué par un ou plusieurs substituants sélectionnés dans le groupe consistant en hydroxyle, halogène, nitro, cyano, alkyle C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, =O, -C(O)R₉, -C(O)OR₉, -OC(O)R₉, -NR₁₀R₁₁, -C(O)NR₁₀R₁₁, -SO₂NR₁₀R₁₁ et -NR₁₀C(O)R₁₁;
R₉, R₁₀ et R₁₁ sont chacun indépendamment sélectionnés dans le groupe consistant en un atome d'hydrogène, alkyle en C₁-C₆, amino, cycloalkyle en C₃-C₇, hétérocyclyle monocyclique de 5 à 7 chaînons ou bicyclique ou tricyclique de 7 à 10 chaînons, aryle en C₆-C₁₀ et hétéroaryle en C₆-C₁₀, dans lequel alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs substituants sélectionnés dans le groupe consistant en hydroxyle, halogène, nitro, amino, cyano, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, carboxyle, et un groupe carboxylate ;
m vaut 0, 1, 2 ou 3 ;
n vaut 0, 1 ou 2, de préférence 0 ;
p vaut 0,1 ou 2 ;
s vaut 1 ou 2 ; et
q vaut 1, 2 ou 3.

2. Composé ou stéréoisomère, tautomère ou sel pharmaceutiquement acceptable de celui-ci selon la revendication 1, qui est un composé représenté par la formule générale (I) ou un stéréoisomère, un tautomère ou un sel pharmaceutiquement acceptable de celui-ci : 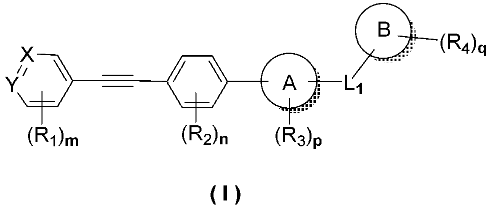 dans lequel :
X et Y sont chacun indépendamment C ou N, dans lequel X et Y ne sont pas simultanément N ;
R₁ est identique ou différent, chacun étant indépendamment -G₁-R₅;
G₁ est sélectionné dans le groupe consistant en une liaison simple, -CH₂- et - C(=O)- ;
R₅ est sélectionné dans le groupe consistant en cyano, halogène, alkyle en C₁-C₆, hydroxyle, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, -C(O)R₆, -C(O)OR₆, -OC(O)R₆, -NR₁₂R₁₃, -C(O)NR₁₂R₁₃, -SO₂NR₁₂R₁₃ et -NR₁₂C(O)R₁₃, dans lequel alcoxy en C₁-C₆, alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs R_{A}
R_{A} est sélectionné dans le groupe consistant en halogène, hydroxyle, cyano, hydroxyalkyle en C₁-C₆, alcoxy en C₁-C₆, alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, -C(O)R₆, -C(O)OR₆, -OC(O)R₆, -NR₇R₈, -C(O)NR₇R₈, -SO₂NR₇R₈ et -NR₇C(O)R₈, dans lequel alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs substituants sélectionnés dans le groupe consistant en halogène, hydroxyle, amino, haloalkyle en C₁-C₆, hydroxyalkyle en C₁-C₆, alcoxy en C₁-C₆, alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, -C(O)R₆, -C(O)OR₆, -OC(O)R₆, -NR₇R₈, - C(O)NR₇R₈, -SO₂NR₇R₈ et -NR₇C(O)R₈;
R₇, R₈, R₁₂ et R₁₃ sont chacun indépendamment sélectionnés dans le groupe consistant en un atome d'hydrogène, hydroxyle, halogène, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ et hétéroaryle en C₆-C₁₀, dans lequel alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs substituants sélectionnés dans le groupe consistant en hydroxyle, halogène, nitro, cyano, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, =O, -C(O)R₉, -C(O)OR₉, -OC(O)R₉, -NR₁₀R₁₁, -C(O)NR₁₀R₁₁, -SO₂NR₁₀R₁₁ et -NR₁₀C(O)R₁₁;
alternativement, R₇ et R₈ forment hétérocyclyle de 4 à 8 chaînons conjointement avec l'atome auquel ils sont liés, dans lequel hétérocyclyle de 4 à 8 chaînons contient un ou plusieurs N, O, S ou SO₂, et hétérocyclyle de 4 à 8 chaînons est en outre éventuellement substitué par un ou plusieurs substituants sélectionnés dans le groupe consistant en hydroxyle, halogène, nitro, cyano, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, =O, -C(O)R₉, -C(O)OR₉, -OC(O)R₉, -NR₁₀R₁₁, -C(O)NR₁₀R₁₁, -SO₂NR₁₀R₁₁ et -NR₁₀C(O)R₁₁;
alternativement, R₁₂ et R₁₃ forment hétérocyclyle de 4 à 8 chaînons conjointement avec l'atome auquel ils sont liés, dans lequel hétérocyclyle de 4 à 8 chaînons contient un ou plusieurs N, O, S ou SO₂, et hétérocyclyle de 4 à 8 chaînons est en outre éventuellement substitué par un ou plusieurs substituants sélectionnés dans le groupe consistant en hydroxyle, halogène, nitro, cyano, alkyle en C₁-C₆, alcoxy en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀, =O, -C(O)R₉, -C(O)OR₉, -OC(O)R₉, -NR₁₀R₁₁, -C(O)NR₁₀R₁₁, -SO₂NR₁₀R₁₁ et -NR₁₀C(O)R₁₁; et
le cycle A, le cycle B, R₂-R₄, R₆, R₉-R₁₁, L₁, m, n, p et q sont tels que définis en revendication 1.

3. Composé ou stéréoisomère, tautomère ou sel pharmaceutiquement acceptable de celui-ci selon la revendication 1, qui est un composé représenté par la formule générale (II-1), (II-2), (II-3), (II-4) ou (II-5) ou un stéréoisomère, un tautomère ou un sel pharmaceutiquement acceptable de celui-ci : 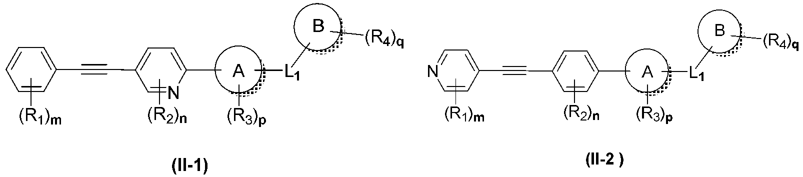 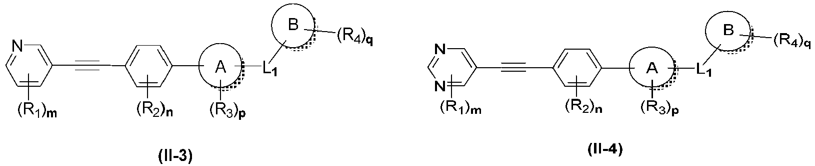 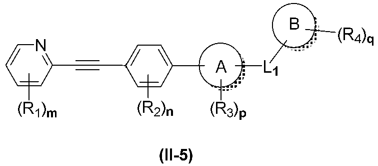 dans lequel, le cycle A, le cycle B, R₁-R₄, L₁, m, n, p et q sont tels que définis en revendication 1.

4. Composé ou stéréoisomère, tautomère ou sel pharmaceutiquement acceptable de celui-ci selon l'une quelconque des revendications 1 à 3, qui est un composé représenté par la formule générale (III-1) ou (III-2) ou un stéréoisomère, un tautomère ou un sel pharmaceutiquement acceptable de celui-ci : 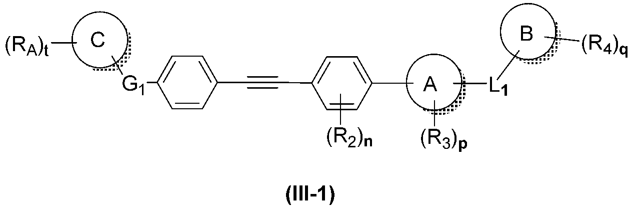 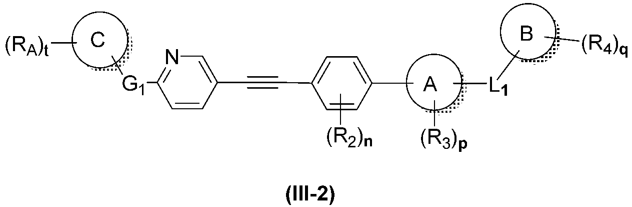 dans lequel :
le cycle C est sélectionné dans le groupe consistant en cycloalkyle en C₄-C₈, hétéroaryle de 5 à 6 chaînons et hétérocyclyle à 4 à 8 chaînons ;
R_{A} est identique ou différent, chacun étant indépendamment sélectionné dans le groupe consistant en haloalkyle en C₁-C₆, hydroxyalkyle en C₁-C₆, alcoxy en C₁-C₆, alkyle en C₁-C₆, alcényle en C₂-C₄, hydroxyle, halogène, cyano, -C(O)NH₂, cycloalkyle en C₃-C₇, hétérocyclyle monocyclique de 5 à 7 chaînons ou bicyclique ou tricyclique de 7 à 10 chaînons, aryle en C₆-C₁₀, hétéroaryle en C₆-C₁₀ et carboxyle, dans lequel alkyle en C₁-C₆, cycloalkyle en C₃-C₇, hétérocyclyle monocyclique de 5 à 7 chaînons ou bicyclique ou tricyclique de 7 à 10 chaînons, aryle en C₆-C₁₀ ou hétéroaryle en C₆-C₁₀ est en outre éventuellement substitué par un ou plusieurs substituants sélectionnés dans le groupe consistant en hydroxyle, cyano, carboxyle et un groupe ester ;
alternativement, deux R_{A} forment -C(O)- conjointement avec le même atome Carbone auquel ils sont liés ; t vaut 0, 1, 2 ou 3 ; et
le cycle A, le cycle B, R₂-R₄, G₁, L₁, n, p et q sont tels que définis en revendication 1.

5. Composé ou stéréoisomère, tautomère ou sel pharmaceutiquement acceptable de celui-ci selon l'une quelconque des revendications 1 à 4, dans lequel le cycle A est sélectionné dans le groupe consistant en : 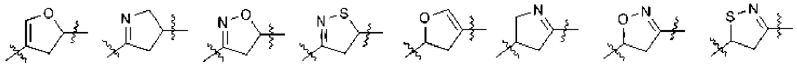 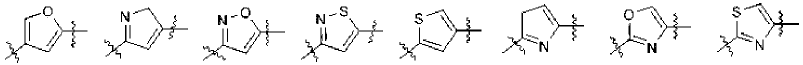 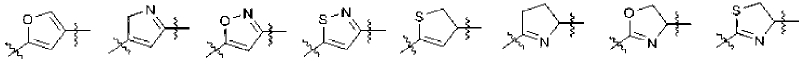 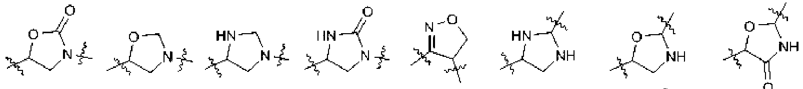 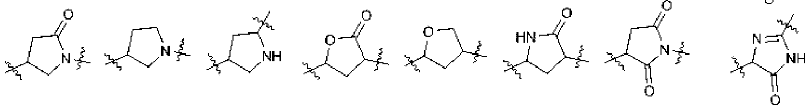 et

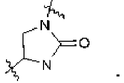

6. Composé ou stéréoisomère, tautomère ou sel pharmaceutiquement acceptable de celui-ci selon l'une quelconque des revendications 1 à 4, dans lequel le cycle B est sélectionné dans le groupe consistant en :

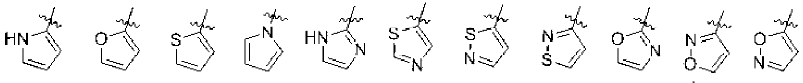

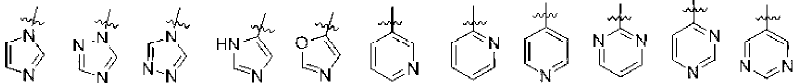

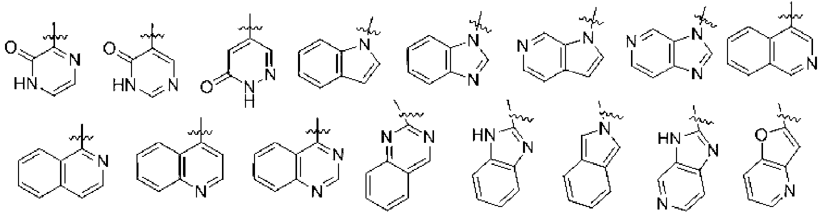

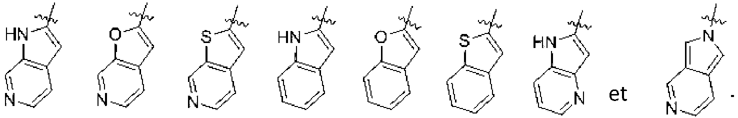

7. Composé ou stéréoisomère, tautomère ou sel pharmaceutiquement acceptable de celui-ci selon l'une quelconque des revendications 4 à 6, dans lequel le cycle C est sélectionné dans le groupe consistant en :

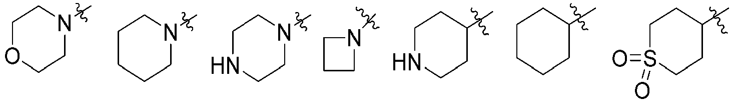

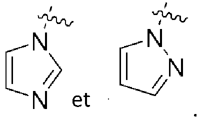

8. Composé, ou stéréoisomère, tautomère ou sel pharmaceutiquement acceptable de celui-ci selon l'une quelconque des revendications 1 à 7, dans lequel :
R₄ est sélectionné dans le groupe consistant en alkyle en C₁-C₆, -C(O)R₆, -C(O)OR₆ et -C(O)NR₇R₈, dans lequel alkyle en C₁-C₆ est en outre éventuellement substitué par un ou plusieurs substituants parmi hydroxyle ou halogène ;
R₆ est sélectionné dans le groupe consistant en un atome d'hydrogène, hydroxyle, alkyle en C₁-C₆ et alcoxy en C₁-C₆ ; et
R₇ et R₈ sont chacun indépendamment un atome d'hydrogène ou alkyle en C₁-C₆.

9. Composé ou stéréoisomère, tautomère ou sel pharmaceutiquement acceptable de celui-ci selon l'une quelconque des revendications 1 à 8, dans lequel R₄ est sélectionné dans le groupe consistant en :

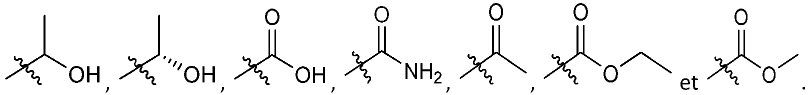

10. Composé ou stéréoisomère, tautomère ou sel pharmaceutiquement acceptable de celui-ci selon l'une quelconque des revendications 1 à 9, dans lequel le composé est sélectionné dans le groupe consistant en :

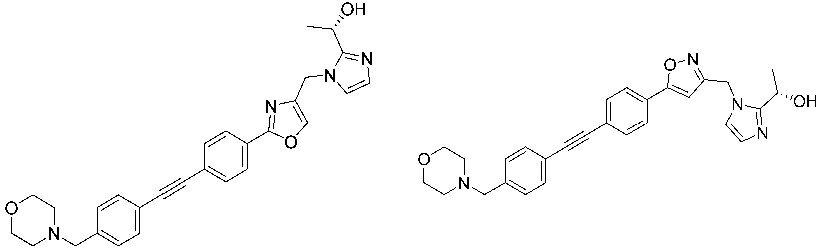

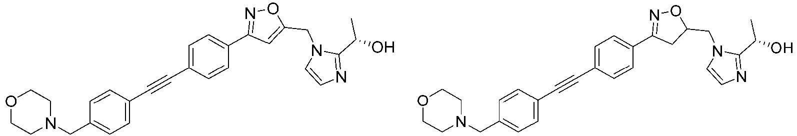

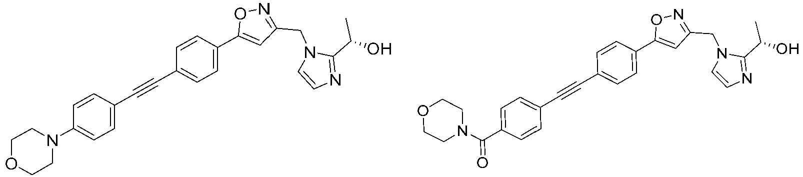

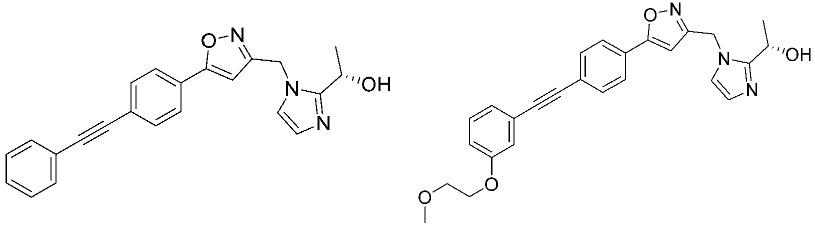

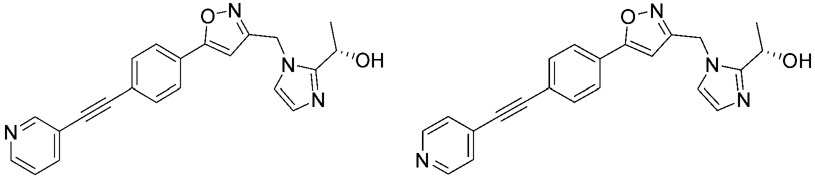

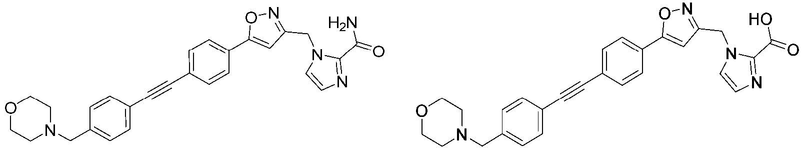

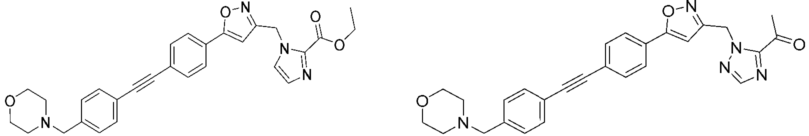

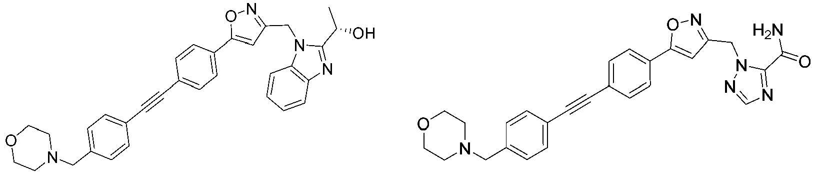

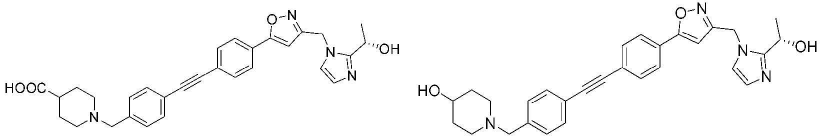

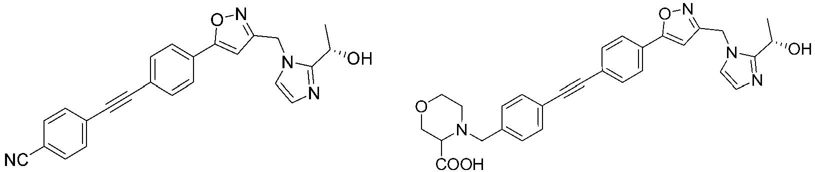

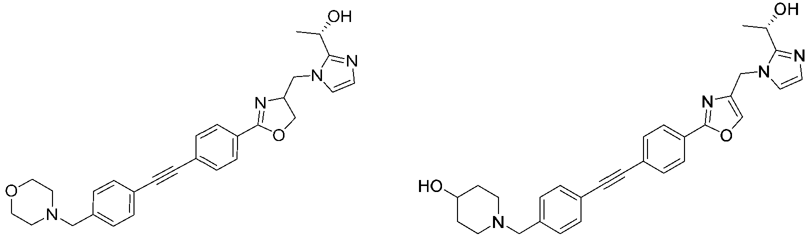

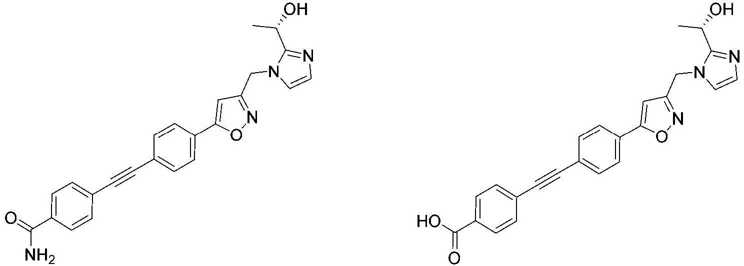

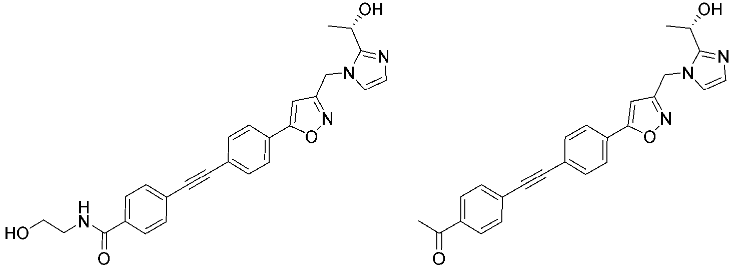

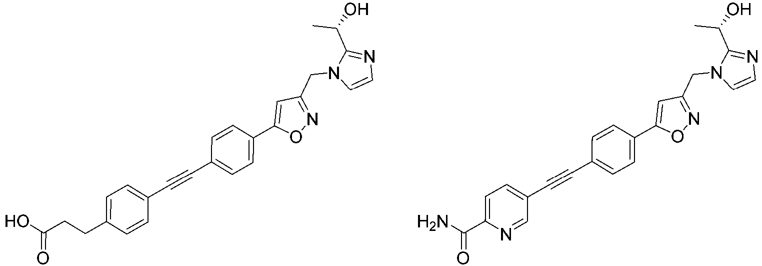

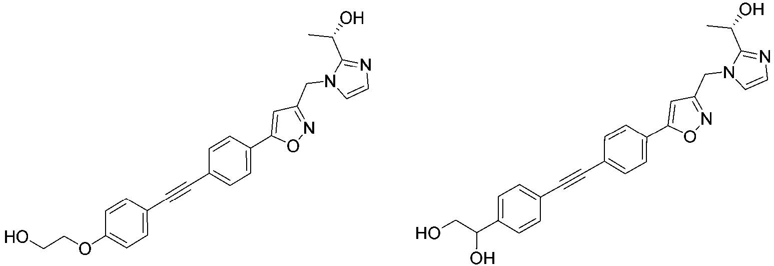

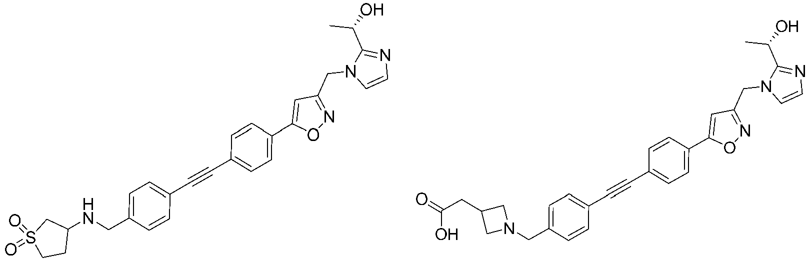

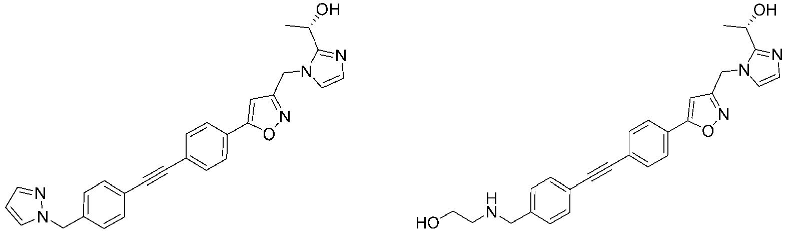

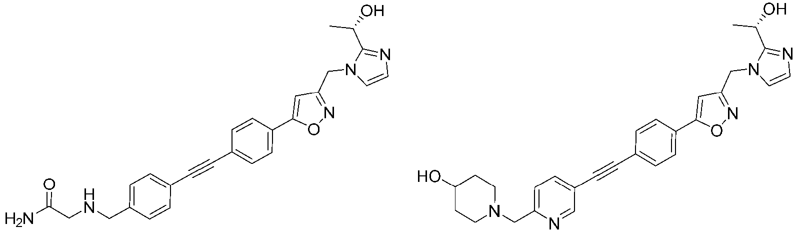

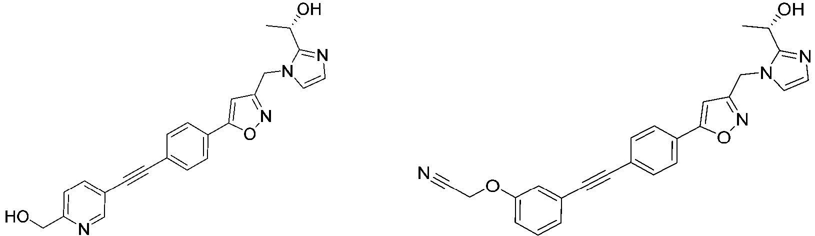

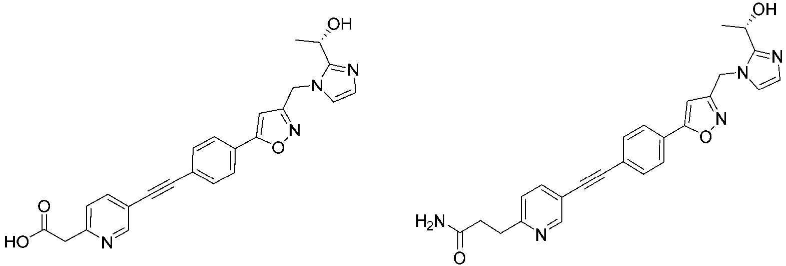

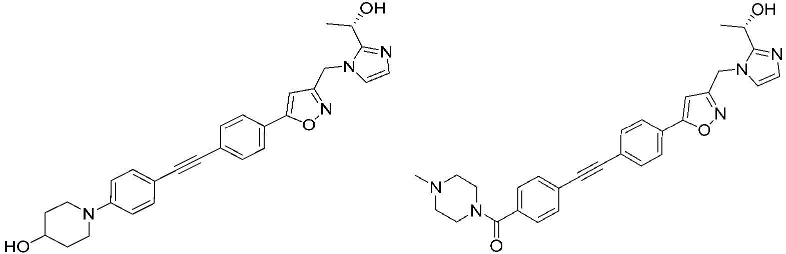

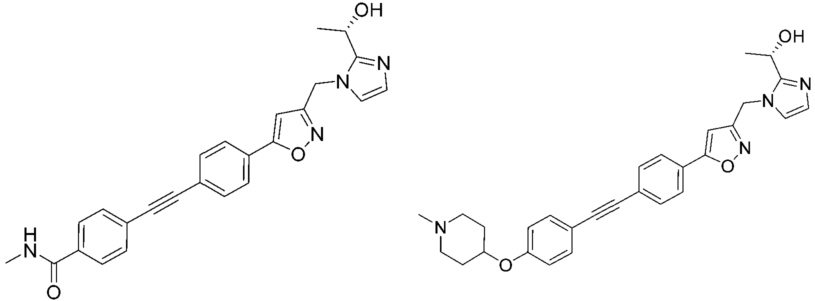

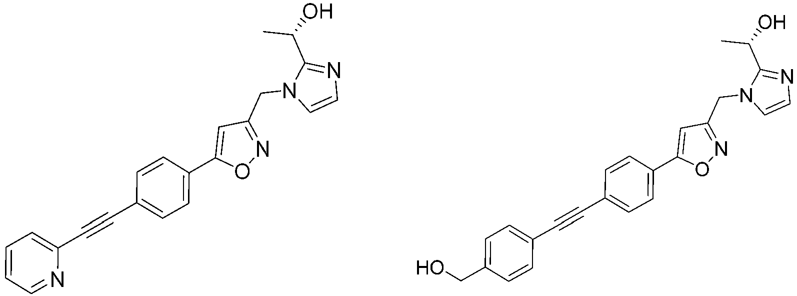

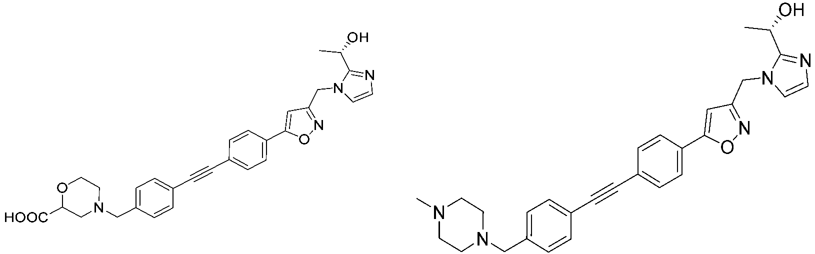

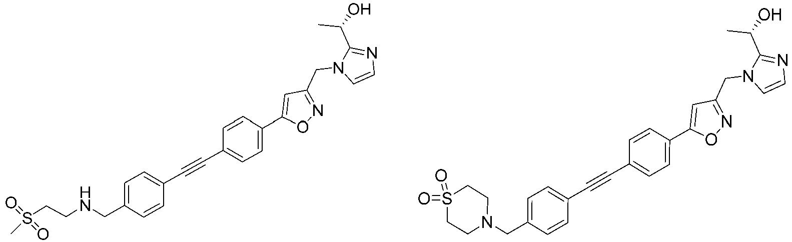

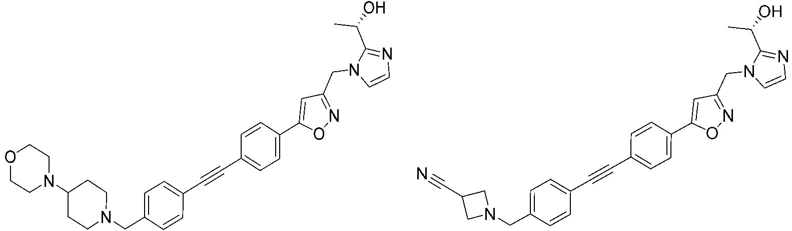

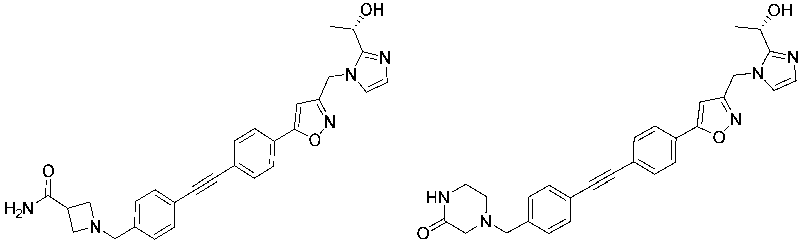

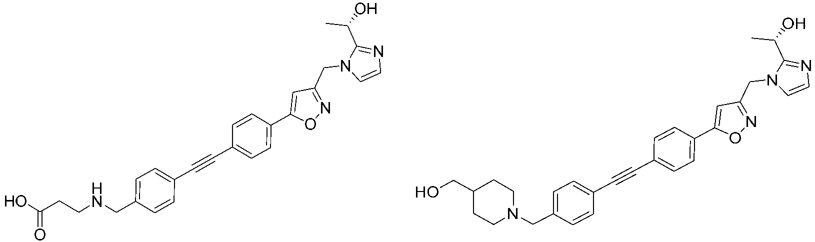

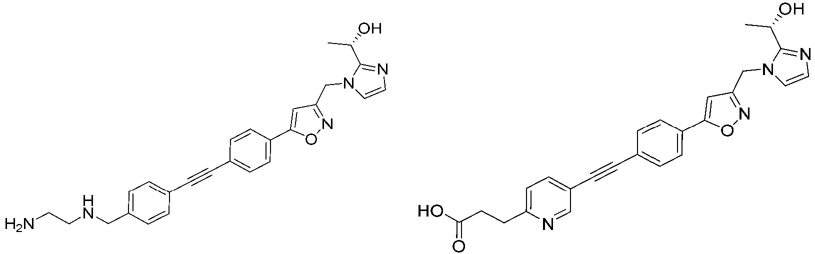

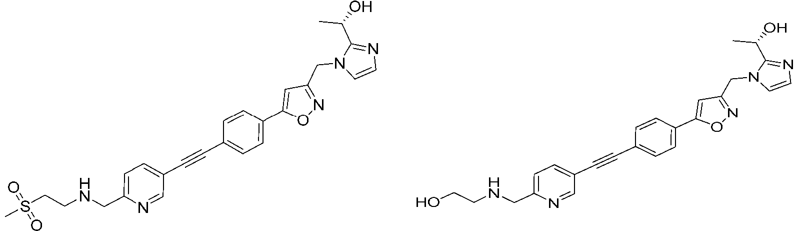

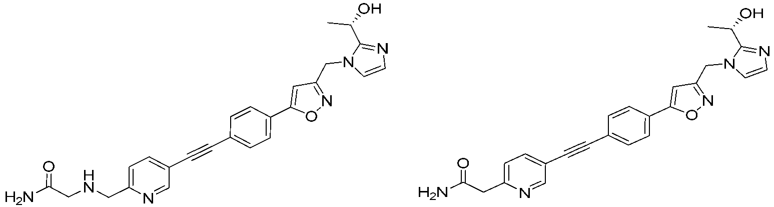

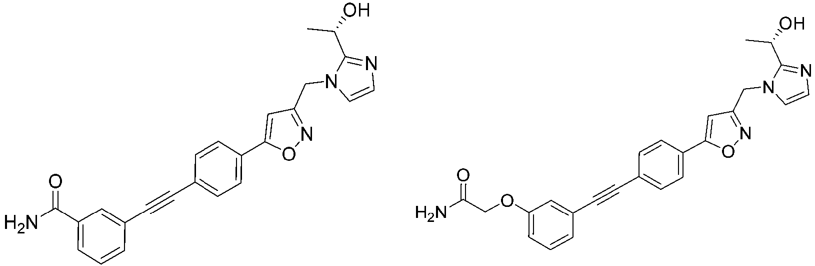

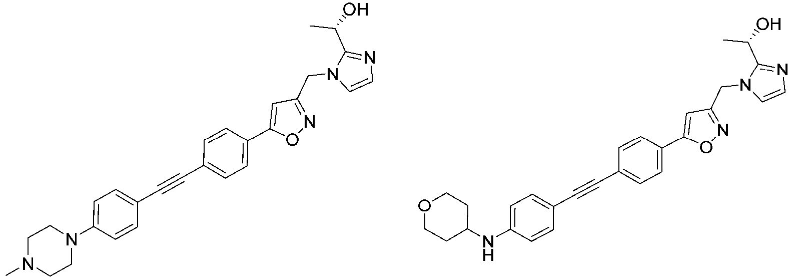

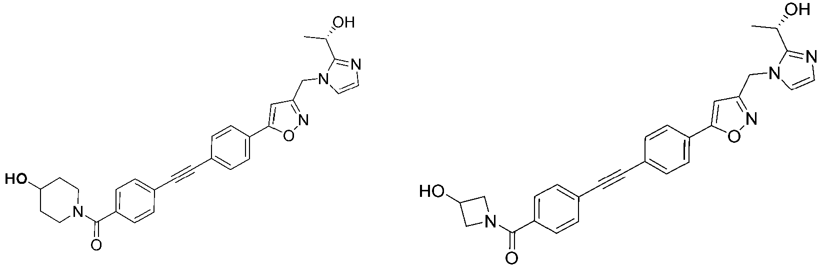

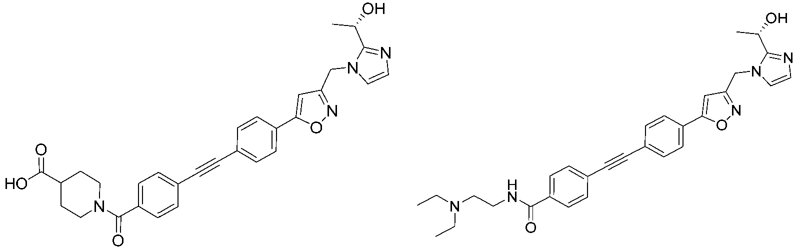

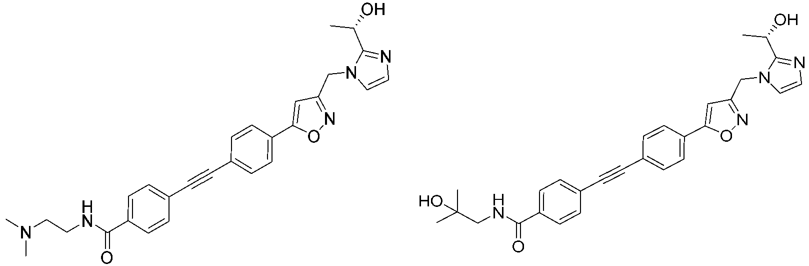

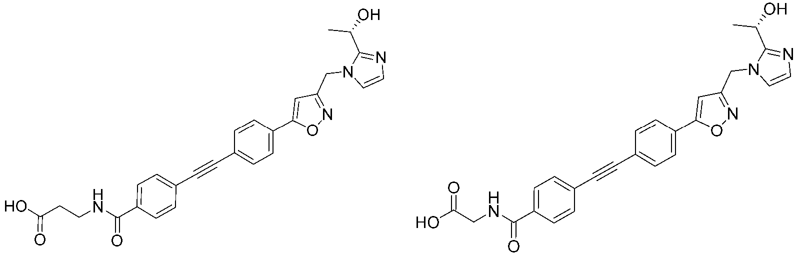

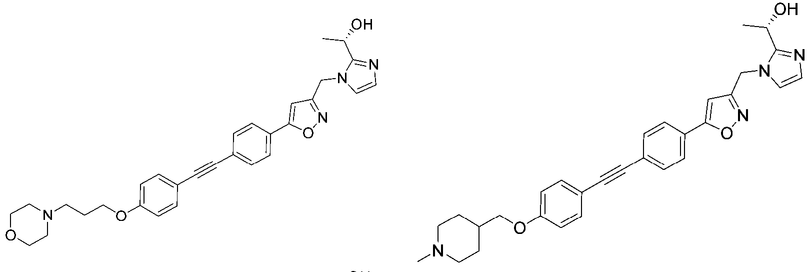

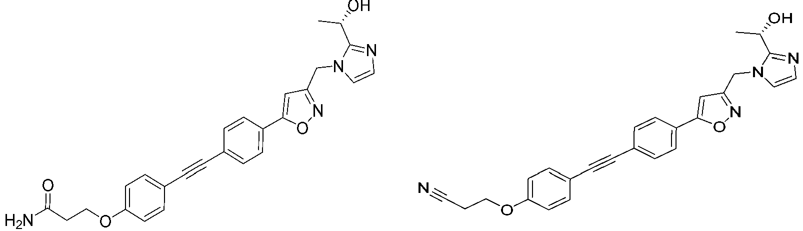

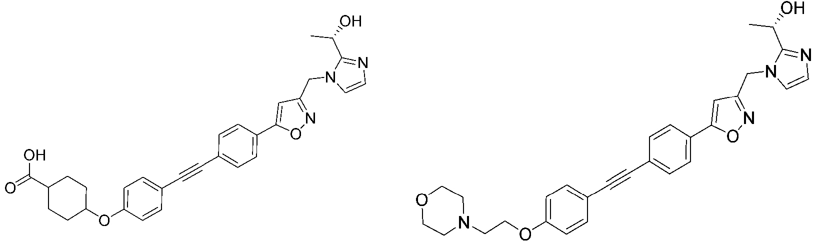

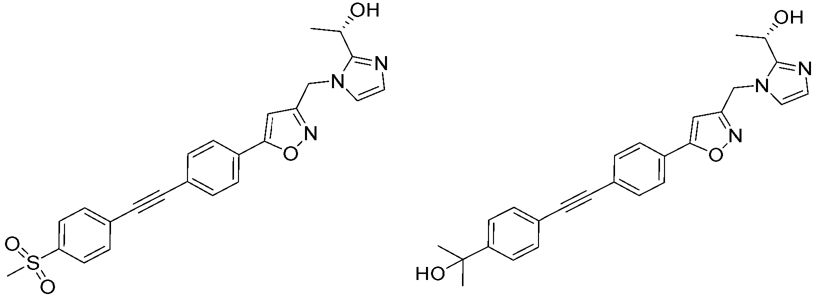

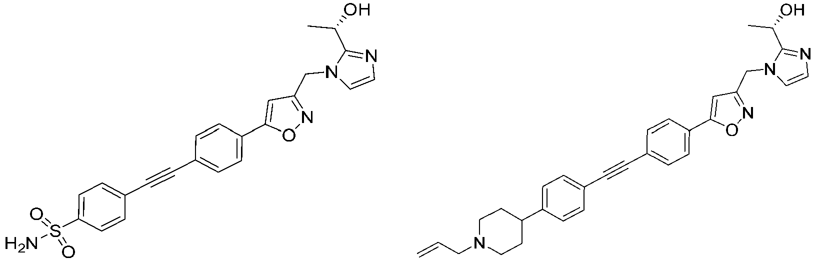

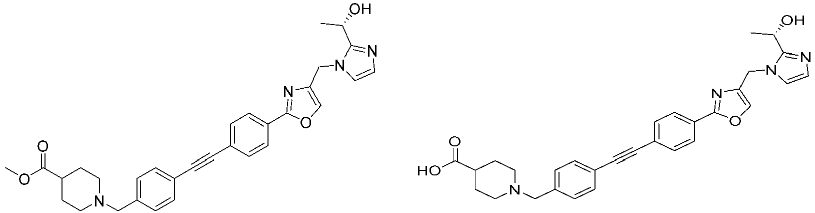

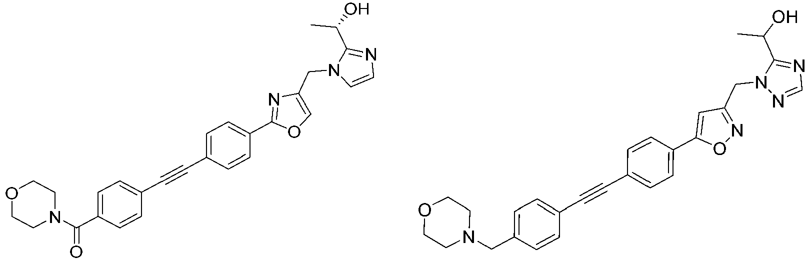

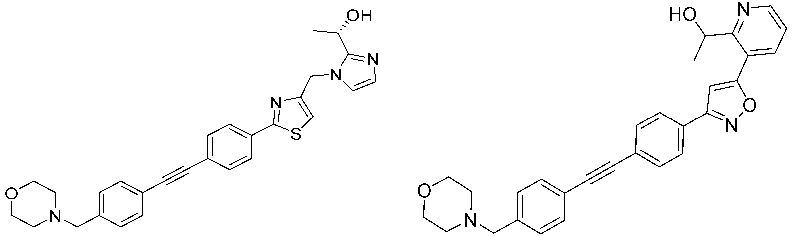

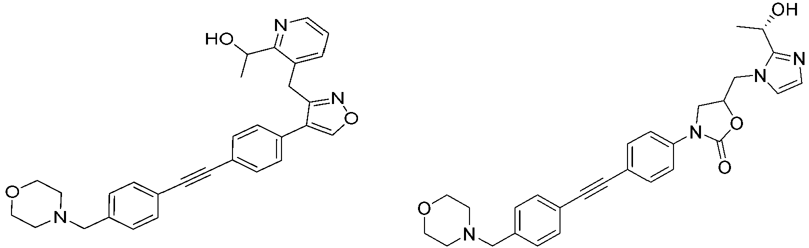

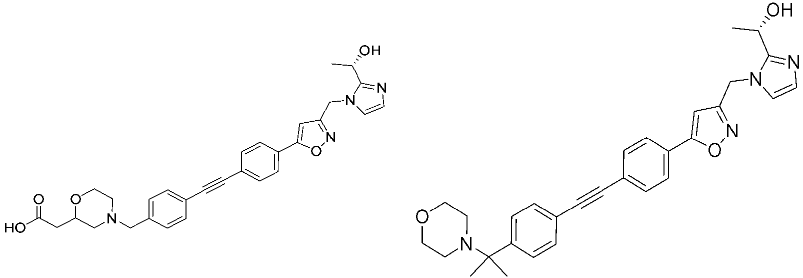

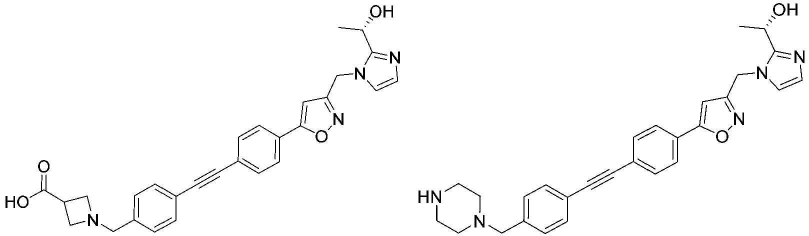

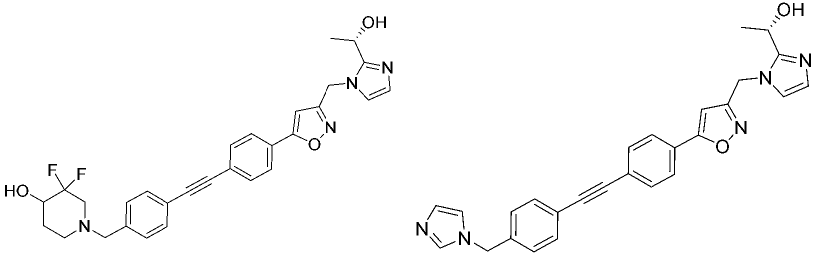

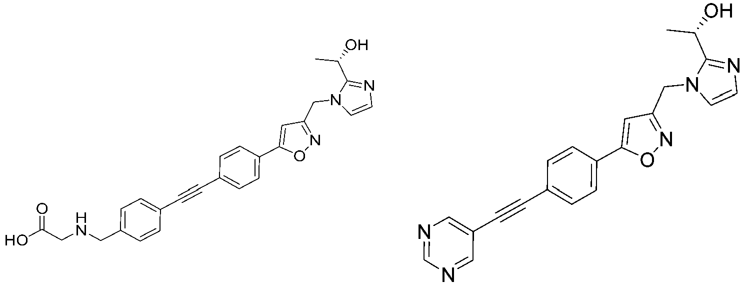

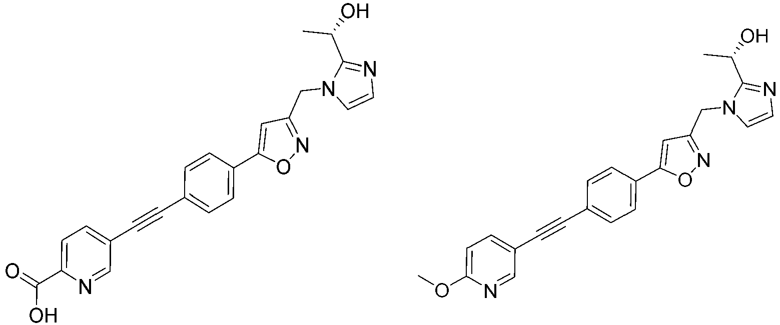

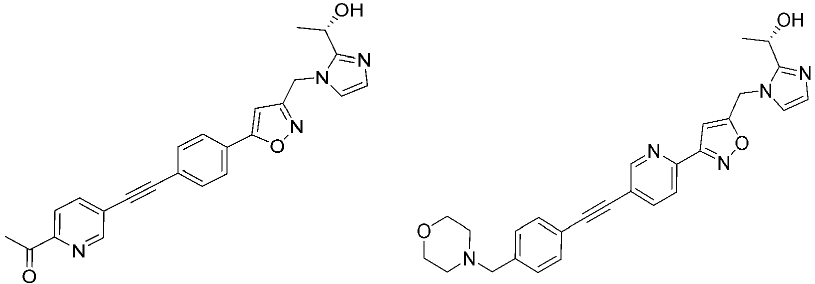

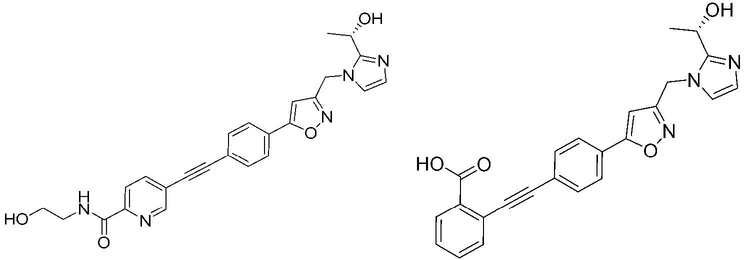

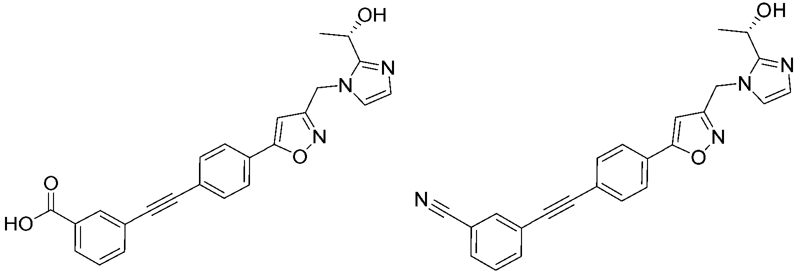

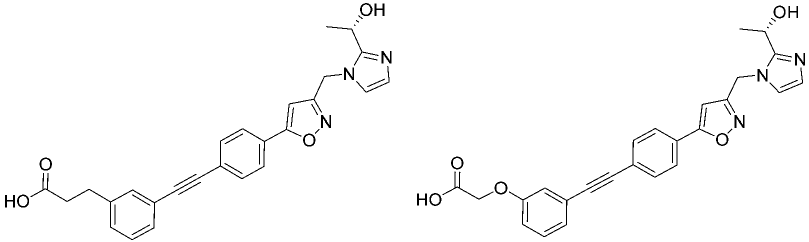

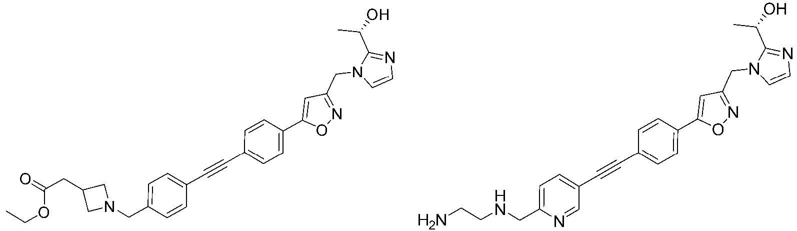

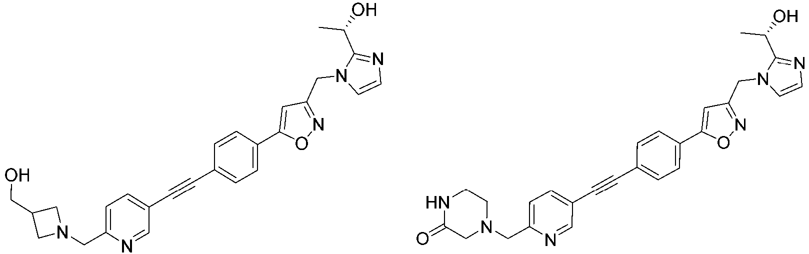

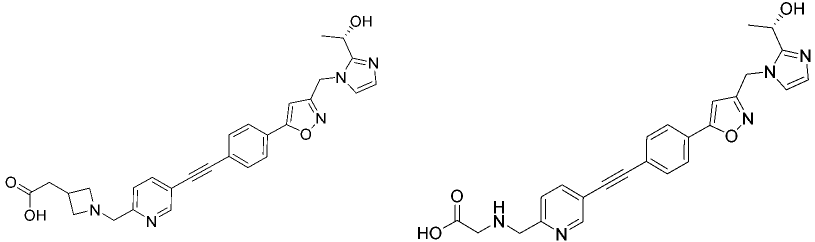

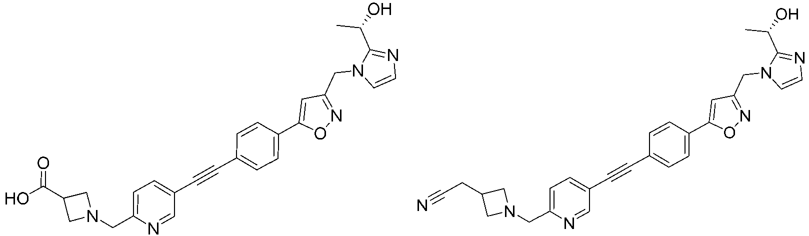

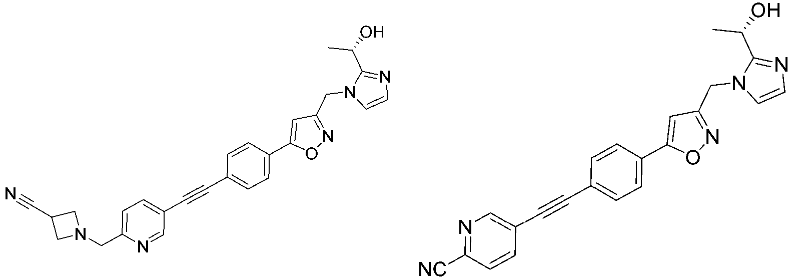

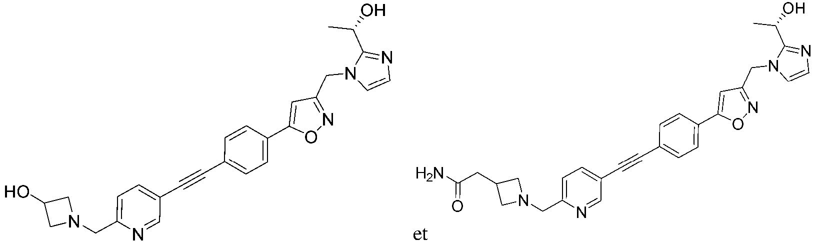

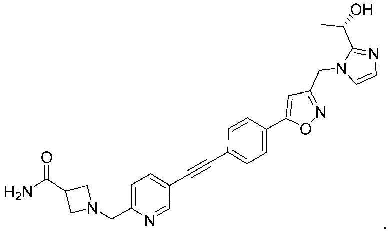

11. Procédé de production du composé représenté par la formule générale (A-I) ou du stéréoisomère, tautomère ou sel pharmaceutiquement acceptable de celui-ci selon la revendication 1, comprenant : 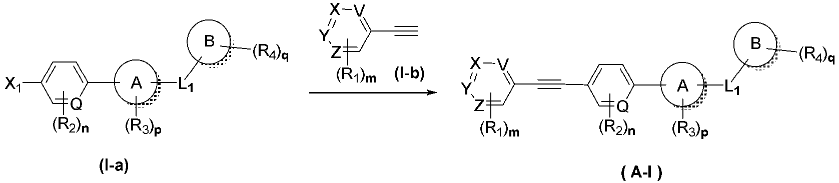
la soumission d'un composé représenté par la formule générale (I-a) et d'un composé représenté par la formule générale (I-b) à une réaction de couplage sous l'action d'un catalyseur, et éventuellement la réalisation, en outre, d'une ou de plusieurs étapes de déprotection, d'hydrolyse, de réduction, d'amination réductrice ou de réaction de condensation acide-amine pour obtenir le composé représenté par la formule générale (A-I) ;
dans lequel :
X₁ est halogène ; et
le cycle A, le cycle B, X, Y, Z, V, Q, R₁-R₄, L₁, n, m, p, et q sont tels que définis en revendication 1 ;
ou 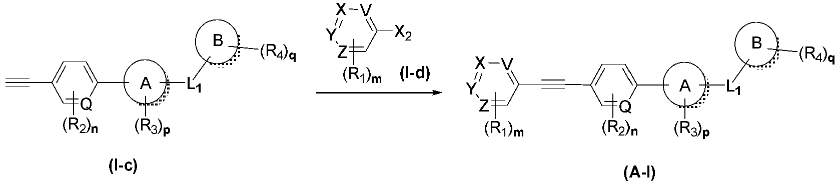
la soumission d'un composé représenté par la formule générale (I-c) et d'un composé représenté par la formule générale (I-d) à une réaction de couplage sous l'action d'un catalyseur, et éventuellement la réalisation, en outre, d'une ou de plusieurs étapes de déprotection, d'hydrolyse, de réduction, d'amination réductrice ou de réaction de condensation acide-amine pour obtenir le composé représenté par la formule générale (A-I) ;
dans lequel :
X₂ est halogène ; et
le cycle A, le cycle B, X, Y, Z, V, Q, R₁-R₄, L₁, n, m, p, et q sont tels que définis en revendication 1;
ou 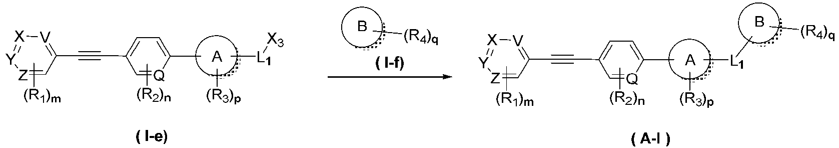
la **soumission** d'un composé représenté par la formule générale (I-e) et d'un composé représenté par (I-f) à une réaction de substitution sous l'action d'un réactif alcalin, et éventuellement la réalisation, en outre, d'une réaction de déprotection pour obtenir le composé représenté par la formule générale (A-I) ;
dans lequel :
X₃ est halogène ; et
le cycle A, le cycle B, X, Y, Z, V, Q, R₁-R₄, L₁, n, m, p, et q sont tels que définis en revendication 1.

12. Composition pharmaceutique comprenant le composé ou le stéréoisomère, le tautomère ou le sel pharmaceutiquement acceptable de celui-ci selon l'une quelconque des revendications 1 à 10, et un support pharmaceutiquement acceptable.

13. Composé, ou stéréoisomère, tautomère ou sel pharmaceutiquement acceptable de celui-ci, selon l'une quelconque des revendications 1 à 10, pour utilisation dans l'inhibition de LPXC.

14. Composé ou stéréoisomère, tautomère ou sel pharmaceutiquement acceptable de celui-ci selon l'une quelconque des revendications 1 à 10, pour utilisation dans le traitement d'une maladie médiée par LPXC, dans lequel la maladie médiée par LPXC est une infection bactérienne causée par une bactérie Gram-négative.

15. Composé ou stéréoisomère, tautomère ou sel pharmaceutiquement acceptable de celui-ci pour utilisation selon la revendication 14, dans lequel la bactérie Gram-négative est sélectionnée dans le groupe consistant en *Escherichia coli, Pseudomonas aeruginosa, Proteus, Shigella dysenteriae, Klebsiella pneumoniae, Brucella, Salmonella typhi, Acinetobacter, Yersinia, Legionella pneumophila, Bordetella pertussis, Shigella, Pasteurella, Vibrio cholerae* et *Neisseria meningitidis.*
